(19) Europäisches Patentamt
European Patent Office
Office européen des brevets

(11) EP 4 293 022 A1

(12) **EUROPEAN PATENT APPLICATION**
published in accordance with Art. 153(4) EPC

(43) Date of publication:
**20.12.2023 Bulletin 2023/51**

(21) Application number: **22752233.1**

(22) Date of filing: **08.02.2022**

(51) International Patent Classification (IPC):
***C07D 487/04*** (2006.01) ***A61K 31/4985*** (2006.01)
***A61P 31/12*** (2006.01)

(52) Cooperative Patent Classification (CPC):
**A61K 31/4985; A61K 31/506; A61P 31/12; A61P 31/20; C07D 487/04**

(86) International application number:
**PCT/CN2022/075494**

(87) International publication number:
**WO 2022/171072 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.02.2021 CN 202110182679**
**08.07.2021 CN 202110773266**

(71) Applicant: **Shanghai Visonpharma Co., Ltd.**
**Shanghai 201321 (CN)**

(72) Inventors:
- **TANG, Guozhi**
  **Shanghai 201321 (CN)**
- **MA, Dawei**
  **Shanghai 200030 (CN)**
- **CHEN, Junli**
  **Shanghai 201321 (CN)**
- **HUANG, Mengwei**
  **Shanghai 201321 (CN)**
- **ZHANG, Jinliang**
  **Shanghai 201321 (CN)**
- **ZHANG, Guoliang**
  **Shanghai 201321 (CN)**

(74) Representative: **Cabinet Laurent & Charras**
**Le Contemporain**
**50 Chemin de la Bruyère**
**69574 Dardilly Cedex (FR)**

(54) **DIHYDROPYRIMIDINE COMPOUND, PREPARATION METHOD THEREFOR AND APPLICATION THEREOF**

(57) Disclosed are a dihydropyrimidine compound, a preparation method therefor and an application thereof. Specifically, disclosed are a dihydropyrimidine compound represented by formula 1' or a pharmaceutically acceptable salt thereof, a composition comprising the compound, and an application of the compound in preparing a drug for treating and preventing hepatitis B virus (HBV) infection related diseases. The disclosed dihydropyrimidine compound can effectively suppress the activity of HBV in HepG2.2.15 cells, and is less toxic to liver cancer cells.

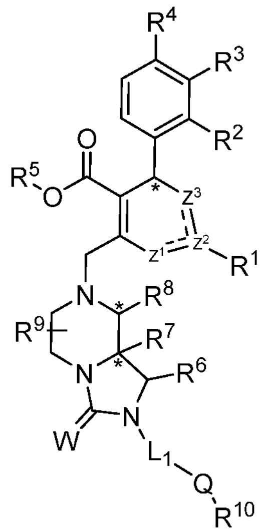

EP 4 293 022 A1

## Description

This application claims priority of Chinese patent application 2021101826790, filed on February 9, 2021, and Chinese patent application 202110773266X, filed on July 8, 2021., the contents of both are incorporated herein by reference in their entirety .

## Technical field

The present invention relates to dihydropyrimidine compounds, a preparation method therefor, and application thereof.

## Background

Hepatitis B virus (HBV) is a small, enveloped, predominantly hepatotropic DNA virus, classified in the Hepadnaviridae and has a circular, partially double-stranded DNA genome of approximately 3.2 kb in size. HBV infection has been a major public health problem. Despite the availability of safe and effective preventive HBV vaccines, it is estimated that approximately 240 million people worldwide are chronically infected with HBV. Chronic HBV infection puts patients at high risk for cirrhosis and liver cancer, and more than 686,000 death per year are estimated from hepatitis B complications. (WHO. Global hepatitis report 2017)

Current therapies are limited to two classes of drugs, which are nucleoside (nucleotide) analogues (lamivudine, adefovir, tenofovir, telbivudine and entecavir) and interferon-$\alpha$ (INF-$\alpha$, both unpegylated and pegylated). Although both therapies reduce HBV DNA and normalize liver enzymes, neither therapy provides a high level of clinical functional cure, as defined by HBV surface antigen (HBsAg) loss (with or without seroconversion). Interferon-based therapies are poorly tolerated and effective only against certain viral genotypes. Nucleoside (nucleotide) analogues often require prolonged or possibly lifelong treatment, and some drugs have the issues of drug resistance, low efficacy, and/or tolerability. Therefore, there is still a great medical need for the discovery and development of effective and safe anti-HBV drugs with novel mechanisms of action to improve disease cure rates. (Marcellin, Patrick, et al. The Lancet 381.9865 (2013): 468-475; Tang, Lydia SY, et al. Jama 319.17 (2018): 1802-1813.)

The HBV capsid, assembled from the HBV core protein, protects the viral genome and creates an environment for reverse transcription of pre-genomic RNA (pgRNA) into DNA. Capsid proteins play a crucial role in the life cycle of the virus. Modulators of HBV capsid assembly have been demonstrated to interfere with capsid assembly and function, and block multiple steps in the HBV life cycle. These compounds interfere with both new virus production and nucleocapsid transport to the nucleus, thus prevent the replenishment of new cccDNA. If administered in combination with approved anti-HBV nucleotide analogues and other anti-HBV agents, these functions can provide potent clinical antiviral activity with synergistic effects. (Diab, Ahmed, et al. Antiviral research 149 (2018): 211-220.)

Different chemical series of HBV capsid assembly modulators have been developed. However, there is still a need for further development of highly potent, safer and therapeutically more effective modulators of capsid assembly.

## Summary

The technical problem to be solved by the present invention is the problem of a unitary drug for the treatment and prevention of HBV infection in the prior art, and provides a dihydropyrimidine compound, preparation method therefor and application thereof, the dihydropyrimidine compound can be used to prepare drugs for the treatment and prevention of HBV infection.

The present invention solves the above-mentioned technical problems by means of the following technical solutions.

The present invention provides a dihydropyrimidine compound of formula I' or a pharmaceutically acceptable salt thereof,

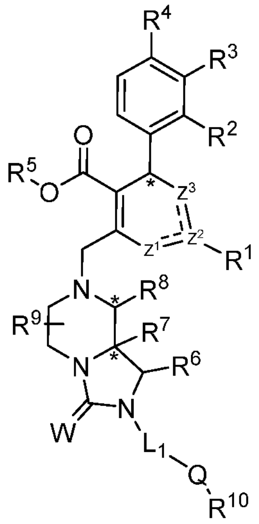

I'

wherein,

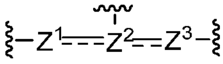 is 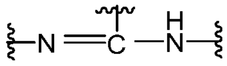 or 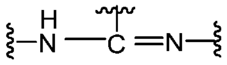;

W is O or S;

$R^1$ is 2-thiazolyl or 2-thiazolyl substituted by one or more $R^{11}$;

$R^{11}$ is halogen or methyl;

$R^2$, $R^3$ and $R^4$ are independently selected from H, halogen and $C_1$-$C_3$ alkyl;

$R^5$ is methyl or ethyl;

$R^6$ is H or $C_1$-$C_3$ alkyl;

$R^7$ is H, halogen, $C_1$-$C_3$alkyl, $C_1$-$C_3$alkoxy or

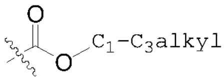

;

$R^8$ is H, COOH, $C_1$-$C_3$alkyl substituted with $C_1$-$C_3$alkoxy,

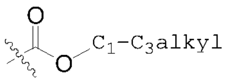

;

$R^9$ is H, halogen, $C_1$-$C_3$alkyl substituted with $C_1$-$C_3$alkoxy, or

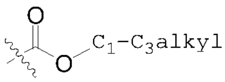

;

$L_1$ is single bond or $C_1$-$C_3$alkylene;

Q is a 5-8-membered bridged cycloalkyl or a 5-8-membered heterocyclyl; the 5-8-membered heterocyclyl is bridged-linked, the heteroatoms in the 5-8-membered heterocyclyl are selected from N, O, and S, and the number of heteroatoms is one, two, or three;

$R^{10}$ is COOH, CN, $C_1$-$C_6$ alkyl substituted by one or more $R^A$, $C_1$-$C_6$ alkyl,

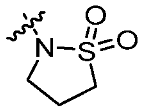,

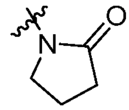,

$NR^BR^C$, or

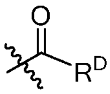;

$R^A$ is a hydroxyl, halogen, $C_1$-$C_6$ alkoxy, COOH,

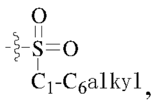, 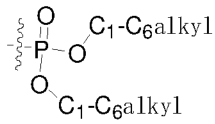,

CN,

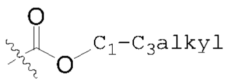,

$C_4$-$C_6$ heterocyclyl substituted by a carbonyl,

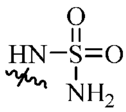,

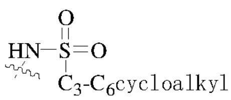, 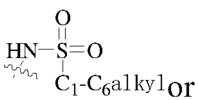 or 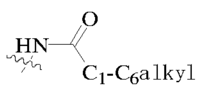;

wherein the number of heteroatoms in the $C_4$-$C_6$ heterocyclyl is one, two, or three, and the heteroatoms are selected from N, O, and S;
$R^B$ and $R^C$ are independently H,

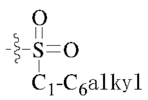, 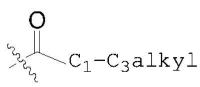,

$C_1$-$C_6$ alkyl,

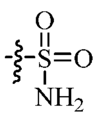,

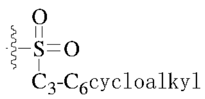

or -$C_1$-$C_6$ alkylene-COOH;

$R^D$ is $C_4$-$C_6$ heterocyclyl, $NH_2$-, -$NHC_1$-$C_6$ alkyl,

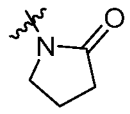 or 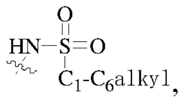

wherein the number of heteroatoms in the $C_4$-$C_6$ heterocyclyl is one, two, or three, and the heteroatoms are selected from N, O, and S;

a carbon atom with "*" indicates that when the carbon atom is a chiral carbon atom, the carbon atom with "*" is in the S configuration, R configuration, or a mixture thereof; and

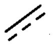 represents a single or double bond.

In certain preferred embodiments of the present invention, certain groups in the dihydropyrimidine compounds of formula I' or pharmaceutically acceptable salts thereof are as defined below, and the groups not mentioned are as described in any of the embodiments(for this expression, hereinafter referred to as "in an embodiment of the present invention"):

In an embodiment of the present invention, the dihydropyrimidine compound of formula I' or a pharmaceutically acceptable salt thereof are shown in formula I,

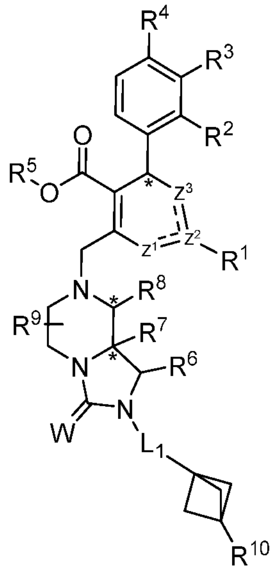

I

wherein,

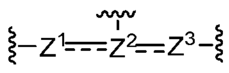 is 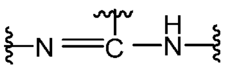 or 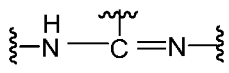;

W is O or S;

$R^1$ is 2-thiazolyl or 2-thiazolyl substituted by one or more $R^{11}$;

$R^{11}$ is halogen or methyl;

$R^2$, $R^3$ and $R^4$ are independently selected from H, halogen and $C_1$-$C_3$ alkyl;

$R^5$ is methyl or ethyl;
$R^6$ is H or $C_1$-$C_3$ alkyl;
$R^7$ is H, halogen, $C_1$-$C_3$alkl, $C_1$-$C_3$alkoxy or

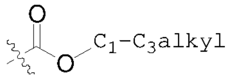

;

$R^8$ is H, COOH, $C_1$-$C_3$alkyl substituted with $C_1$-$C_3$alkoxy,

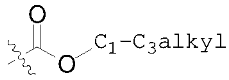

;

$R^9$ is H, halogen, $C_1$-$C_3$alkyl substituted with $C_1$-$C_3$alkoxy, or

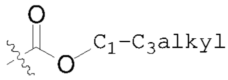

;

$L_1$ is single bond or $C_1$-$C_3$alkylene;
$R^{10}$ is COOH, CN, $C_1$-$C_6$ alkyl substituted by one or more $R^A$, $C_1$-$C_6$ alkyl,

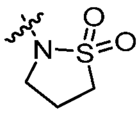

,

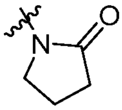

,

$NR^BR^C$, or

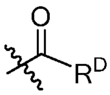

;

$R^A$ is hydroxyl, halogen, $C_1$-$C_6$ alkoxy, COOH,

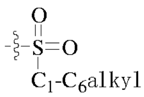

,

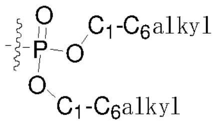

,

CN,

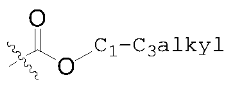

,

$C_4$-$C_6$ heterocyclyl substituted by a carbonyl,

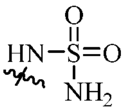,

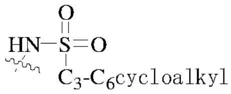, 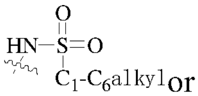 or 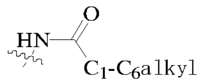;

wherein the number of heteroatoms in the $C_4$-$C_6$ heterocyclyl is one, two, or three, and the heteroatoms are selected from N, O, and S;
$R^B$ and $R^C$ are independently H,

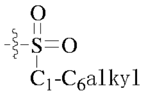, 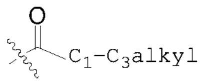,

$C_1$-$C_6$ alkyl,

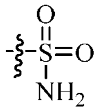,

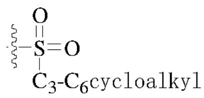

or -$C_1$-$C_6$ alkylene-COOH;
$R^D$ is $C_4$-$C_6$ heterocyclyl, $NH_2$-, -$NHC_1$-$C_6$ alkyl,

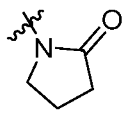 or 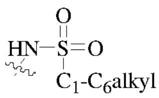,

wherein the number of heteroatoms in the $C_4$-$C_6$ heterocyclyl is one, two, or three, and the heteroatoms are selected from N, O, and S;

A carbon atom with "*" indicates that when the carbon atom is a chiral carbon atom, the carbon atom with "*" is in the S configuration, R configuration, or a mixture thereof;

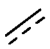 represents a single or double bond.

In an embodiment of the present invention: when $R^{11}$ is halogen, the halogen is fluorine, chlorine, bromine, or iodine.

In an embodiment of the present invention: when $R^2$, $R^3$, and $R^4$ are independently halogen, the halogen is fluorine, chlorine, or bromine, preferably fluorine, chlorine or bromine.

In an embodiment of the present invention: when $R^2$, $R^3$, and $R^4$ are independently $C_1$-$C_3$ alkyl, the $C_1$-$C_3$ alkyl is methyl, ethyl, n-propyl, or isopropyl, preferably methyl.

In an embodiment of the present invention: when $R^6$ is $C_1$-$C_3$ alkyl, the $C_1$-$C_3$ alkyl is methyl, ethyl, n-propyl or isopropyl.

In an embodiment of the present invention: when $R^7$ is halogen, the halogen is fluorine, chlorine, bromine, or iodine.

In an embodiment of the present invention: when $R^7$ is $C_1$-$C_3$ alkyl, the $C_1$-$C_3$ alkyl is methyl, ethyl, n-propyl or isopropyl.

In an embodiment of the present invention: when $R^7$ is $C_1$-$C_3$ alkoxy, the $C_1$-$C_3$ alkyl is methoxy, ethoxy, n-propoxy or isopropoxy.

In an embodiment of the present invention: when $R^7$ is

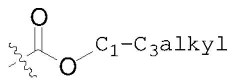,

the $C_1$-$C_3$ alkyl is methyl, ethyl, n-propyl or isopropyl.

In an embodiment of the present invention: when $R^8$ is $C_1$-$C_3$ alkyl substituted by $C_1$-$C_3$ alkoxy, the $C_1$-$C_3$ alkyl is methyl, ethyl, n-propyl or isopropyl, preferably methyl.

In an embodiment of the present invention: when $R^8$ is $C_1$-$C_3$ alkyl substituted by $C_1$-$C_3$ alkoxy, the $C_1$-$C_3$ alkoxy is methoxy, ethoxy, n-propoxy or isopropoxy, preferably methoxy.

In an embodiment of the present invention: when $R^8$ is $C_1$-$C_3$ alkyl substituted by $C_1$-$C_3$ alkoxy, the $C_1$-$C_3$ alkyl substituted by $C_1$-$C_3$ alkoxy is

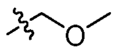.

In an embodiment of the present invention: when $R^8$ is

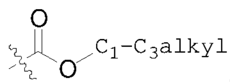,

the $C_1$-$C_3$ alkyl is methyl, ethyl, n-propyl or isopropyl, preferably ethyl.

In an embodiment of the present invention: when $R^9$ is halogen, the halogen is fluorine, chlorine, bromine, or iodine.

In an embodiment of the present invention: when $R^9$ is $C_1$-$C_3$ alkyl substituted by $C_1$-$C_3$ alkoxy, the $C_1$-$C_3$ alkyl is methyl, ethyl, n-propyl or isopropyl.

In an embodiment of the present invention: when $R^9$ is $C_1$-$C_3$ alkyl substituted by $C_1$-$C_3$ alkoxy, the $C_1$-$C_3$ alkoxy is methoxy, ethoxy, n-propoxy or isopropoxy.

In an embodiment of the present invention: when $R^9$ is

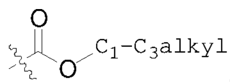,

the $C_1$-$C_3$ alkyl is methyl, ethyl, n-propyl or isopropyl.

In an embodiment of the present invention: when $L_1$ is $C_1$-$C_3$alkylene, the $C_1$-$C_3$alkylene is methylene,

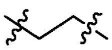, 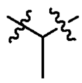, 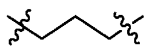, 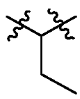, 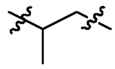 or 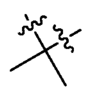,

preferably methylene.

In an embodiment of the present invention: when $R^{10}$ is $C_1$-$C_6$ alkyl substituted by one or more $R^A$, the $C_1$-$C_6$alkyl is methyl, ethyl, n-propyl, isopropyl, n-butyl, isobutyl, tert butyl, n-pentyl, isopentyl or neopentyl, preferably methyl, ethyl, isopropyl, or isobutyl.

In an embodiment of the present invention: when $R^A$ is halogen, the halogen is fluorine, chlorine, bromine, or iodine, preferably fluorine.

In an embodiment of the present invention: when $R^A$ is $C_1$-$C_6$ alkoxy, the $C_1$-$C_6$ alkoxy is methoxy, ethoxy, n-propoxy, isopropoxy, n-butoxy, isobutyloxy, tert butoxy, n-pentoxy, isopentoxy or neopentoxy, preferably methoxy.

In an embodiment of the present invention: when $R^A$ is

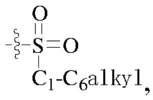

the $C_1$-$C_6$ alkyl is methyl, ethyl, n-propyl, isopropyl, n-butyl, isobutyl, tert butyl, n-pentyl, isopentyl or neopentyl, preferably methyl.

In an embodiment of the present invention: when $R^A$ is

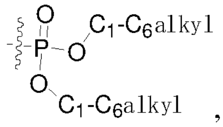

the $C_1$-$C_6$ alkyl is independently methyl, ethyl, n-propyl, isopropyl, n-butyl, isobutyl, tert butyl, n-pentyl, isopentyl or neopentyl, preferably ethyl.

In an embodiment of the present invention: when $R^A$ is

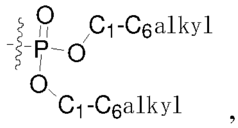

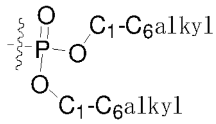

is

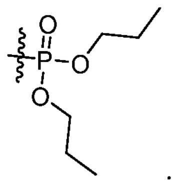

In an embodiment of the present invention: when $R^A$ is

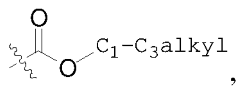

the $C_1$-$C_3$ alkyl is methyl, ethyl, n-propyl or isopropyl, preferably methyl.

In an embodiment of the present invention: when $R^A$ is $C_4$-$C_6$ heterocyclyl substituted by one carbonyl, the $C_4$-$C_6$ heterocyclyl is tetrahydropyrrolyl, tetrahydrofuryl, tetrahydrothienyl, piperidinyl, tetrahydropyranyl or morpholinyl, preferably tetrahydropyrrolyl.

In an embodiment of the present invention: when $R^A$ is $C_4$-$C_6$ heterocyclyl substituted by one carbonyl, the $C_4$-$C_6$ heterocyclyl substituted by one carbonyl is

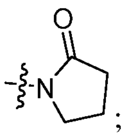

In an embodiment of the present invention: when $R^A$ is

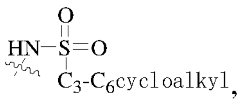

the $C_3$-$C_6$ cycloalkyl is cyclopropyl, cyclobutyl, or cyclopentyl, preferably cyclopropyl.

In an embodiment of the present invention: when $R^A$ is

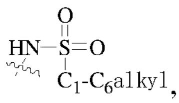

the $C_1$-$C_6$ alkyl is methyl, ethyl, n-propyl, isopropyl, n-butyl, isobutyl, tert butyl, n-pentyl, isopentyl or neopentyl, preferably methyl.

In an embodiment of the present invention: when $R^A$ is

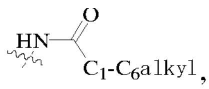

the $C_1$-$C_6$ alkyl is methyl, ethyl, n-propyl, isopropyl, n-butyl, isobutyl, tert butyl, n-pentyl, isopentyl or neopentyl, preferably methyl.

In an embodiment of the present invention: when $R^{10}$ is $C_1$-$C_6$ alkyl substituted by one $R^A$, the $C_1$-$C_6$ alkyl substituted by one $R^A$ is

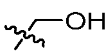, 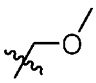, 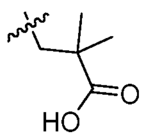, 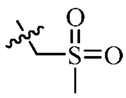,

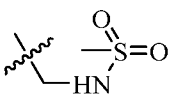, 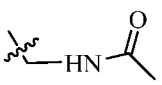, 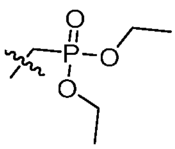, 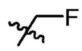, 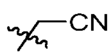, 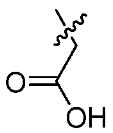, 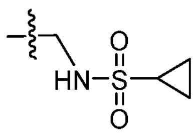,

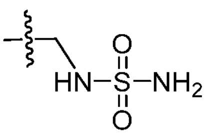, 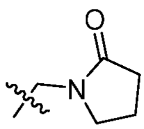, 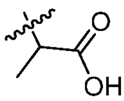, 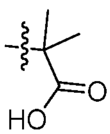 or 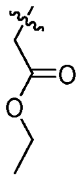.

In an embodiment of the present invention: when $R^{10}$ is $C_1$-$C_6$ alkyl substituted by more $R^A$, the $C_1$-$C_6$ alkyl substituted by more $R^A$ is

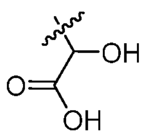 or 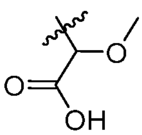.

In an embodiment of the present invention: when $R^{10}$ is $C_1$-$C_6$ alkyl, the $C_1$-$C_6$ alkyl is methyl, ethyl, n-propyl, isopropyl, n-butyl, isobutyl, tert butyl, n-pentyl, isopentyl or neopentyl.

In an embodiment of the present invention: when $R^B$ and $R^C$ are independently

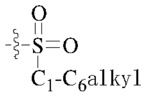,

the $C_1$-$C_6$ alkyl is methyl, ethyl, n-propyl, isopropyl, n-butyl, isobutyl, tert butyl, n-pentyl, isopentyl or neopentyl, preferably methyl.

In an embodiment of the present invention: when $R^B$ and $R^C$ are independently

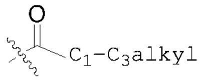,

the $C_1$-$C_3$ alkyl is methyl, ethyl, n-propyl or isopropyl, preferably methyl.

In an embodiment of the present invention: when $R^B$ and $R^C$ are independently $C_1$-$C_6$ alkyl, the $C_1$-$C_6$ alkyl is methyl, ethyl, n-propyl, isopropyl, n-butyl, isobutyl, tert butyl, n-pentyl, isopentyl or neopentyl, preferably methyl.

In an embodiment of the present invention: when $R^B$ and $R^C$ are independently

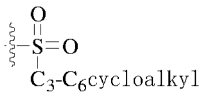,

the $C_3$-$C_6$ cycloalkyl is cyclopropyl, cyclobutyl, cyclopentyl or cyclohexyl, preferably cyclopropyl.

In an embodiment of the present invention: when $R^B$ and $R^C$ are independently

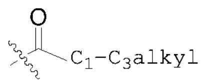,

the $C_1$-$C_3$ alkyl is methyl, ethyl, n-propyl or isopropyl, preferably methyl.

In an embodiment of the present invention: when $R^B$ and $R^C$ are independently $-C_1$-$C_6$ alkylene-COOH, the $C_1$-$C_6$ alkylene is methylene, ethylene, propylene, butylene, pentylene, or hexylidene, preferably methylene.

In an embodiment of the present invention: when $R^{10}$ is $NR^BR^C$, $NR^BR^C$ is

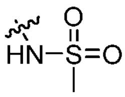,

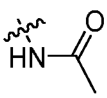, 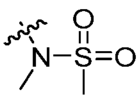, 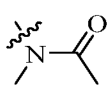, 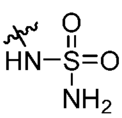, 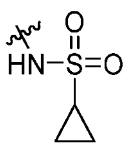 or 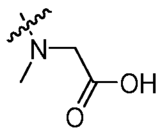.

In an embodiment of the present invention: when $R^D$ is $C_4$-$C_6$ heterocyclyl, the $C_4$-$C_6$ heterocyclyl is tetrahydropyrrolyl, tetrahydrofuryl, tetrahydrothienyl, piperidinyl, tetrahydropyranyl or morpholinyl, preferably

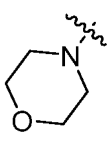 or 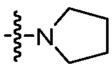.

In an embodiment of the present invention: when $R^D$ is $-NHC_1$-$C_6$ alkyl, the $C_1$-$C_6$ alkyl is methyl, ethyl, n-propyl, isopropyl, n-butyl, isobutyl, tert butyl, n-pentyl, isopentyl or neopentyl, preferably methyl.

In an embodiment of the present invention: when $R^D$ is

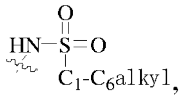

the $C_1$-$C_6$ alkyl is methyl, ethyl, n-propyl, isopropyl, n-butyl, isobutyl, tert butyl, n-pentyl, isopentyl or neopentyl, preferably methyl.

In an embodiment of the present invention: when $R^{10}$ is

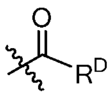,
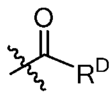

is

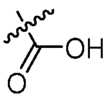, 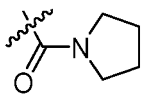, 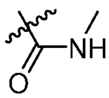, 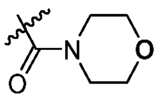 or 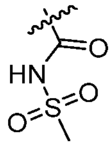.

In an embodiment of the present invention: when Q is 5-8-membered heterocyclyl; the 5-8-membered heterocyclyl is bridged-linked, the heteroatom in the 5-8-membered heterocyclyl is O, and the number of heteroatoms is one.

In an embodiment of the present invention: when Q is 5-8-membered bridged cycloalkyl; the 5-8 membered bridged cycloalkyl is 5-membered bridged cycloalkyl, for example

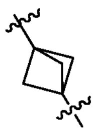.

In an embodiment of the present invention: when Q is 5-8-membered bridged cycloalkyl; the 5-8 membered bridged cycloalkyl is 6-8 membered bridged cycloalkyl, for exmaple,

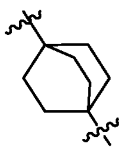, 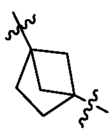, 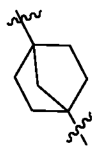 or 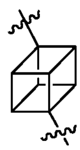.

In an embodiment of the present invention, the compound of formula I is any of the following compounds:

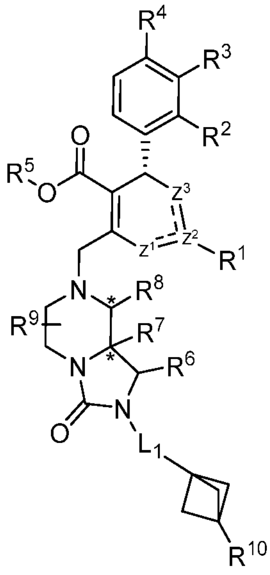,
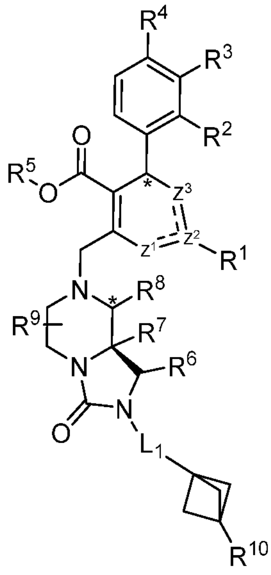 or
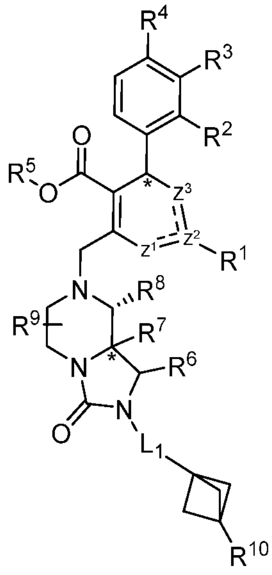.

In an embodiment of the present invention, the compound of formula I' is any of the following compounds:

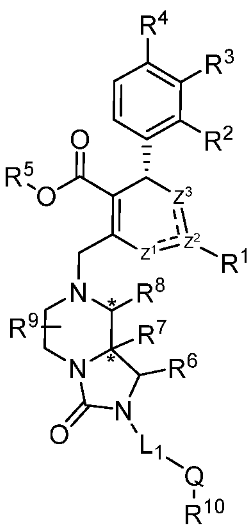,
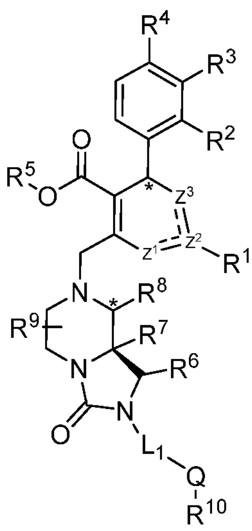 or
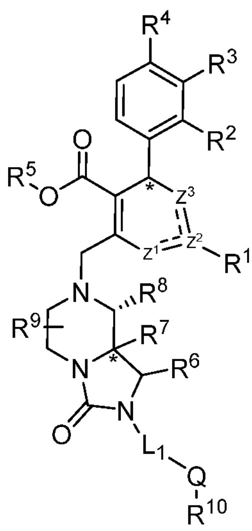.

In an embodiment of the present invention, the compound of formula I is any of the following compounds:

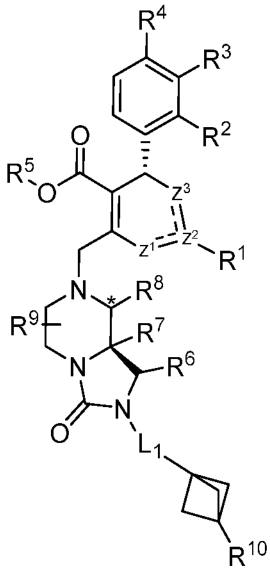
,
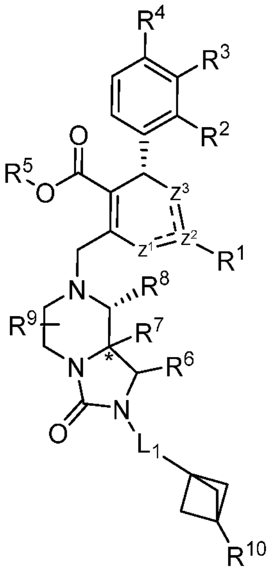
or
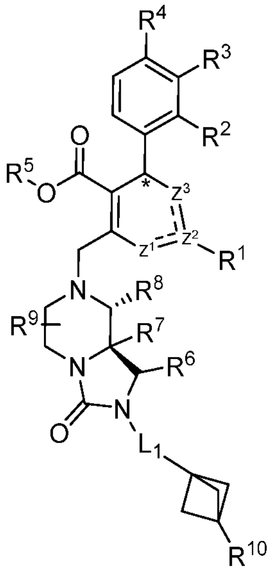
.

In an embodiment of the present invention, the compound of formula I' is any of the following compounds:

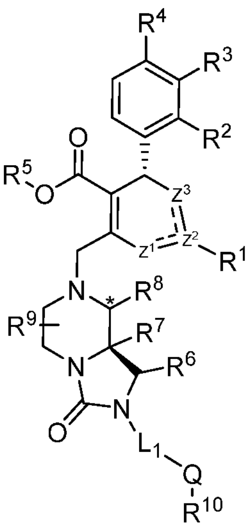
,
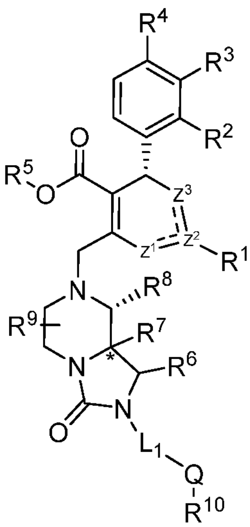
or
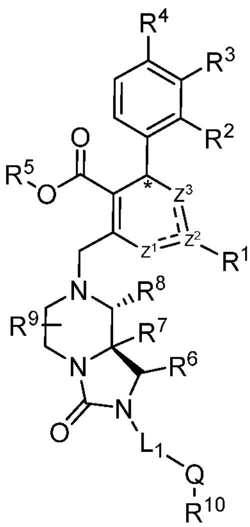
.

In an embodiment of the present invention, the compound of formula I is

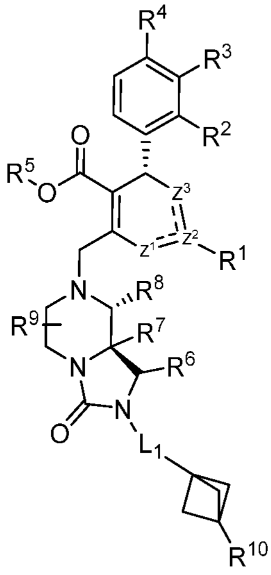

.

In an embodiment of the present invention, the compound of formula I' is

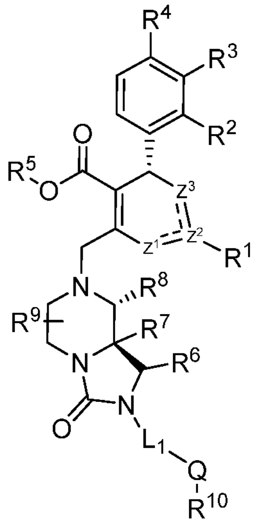

.

In an embodiment of the present invention, $R^1$ is 2-thiazolyl.

In an embodiment of the present invention, $R^2$, $R^3$, and $R^4$ are independently H, fluorine, chlorine, bromine, or methyl, preferably H, fluorine, or methyl, and $R^3$ and $R^4$ are preferably not fluorine at the same time.

In an embodiment of the present invention, Rand $R^4$ are preferably not halogen at the same time.

In an embodiment of the present invention, $R^6$ is H.

In an embodiment of the present invention, $R^7$ is H.

In an embodiment of the present invention, $R^8$ is H,

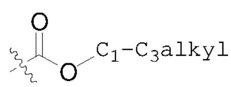

,

preferably H.

In an embodiment of the present invention, $R^9$ is H.

In an embodiment of the present invention, $L_1$ is single bond or methylene, preferably single bond.

In an embodiment of the present invention, $R^{10}$ is CN, $C_1$-$C_6$ alkyl substituted by one $R^A$, $C_1$-$C_6$ alkyl,

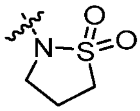, 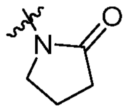,

$NR^BR^C$, or

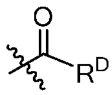.

In an embodiment of the present invention, $R^{10}$ is COOH, CN, $C_1$-$C_6$ alkyl substituted by one $R^A$, $C_1$-$C_6$ alkyl,

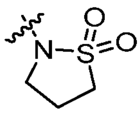, 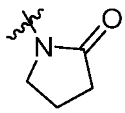,

$NR^BR^C$, or

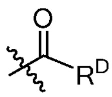.

In an embodiment of the present invention, Q is

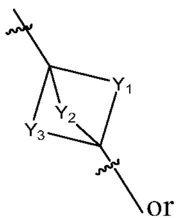 or 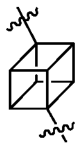;

wherein $Y_1$ is - $CH_2$- or -$CH_2CH_2$-; $Y_2$ is -$CH_2$- or -$CH_2CH_2$-; $Y_3$ is - $CH_2$-, -$CH_2CH_2$-, -O-$CH_2$-, or -$CH_2$-O-.

In an embodiment of the present invention, $R^A$ are independently hydroxyl, halogen, $C_1$-$C_6$ alkoxy,

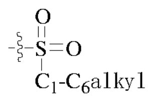, 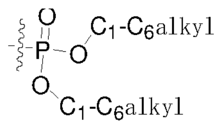,

CN,

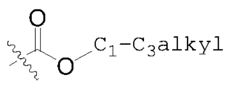,

$C_4$-$C_6$ heterocyclyl substituted by a carbonyl,

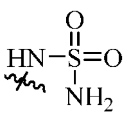, 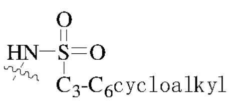, 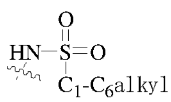 or 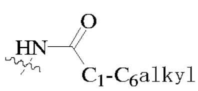.

In an embodiment of the present invention, $R^B$ and $R^C$ are independently H,

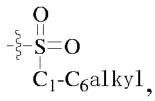

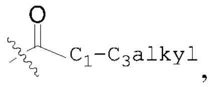

$C_1$-$C_6$ alkyl or

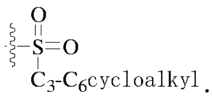

In an embodiment of the present invention, $R^D$ is $C_4$-$C_6$ heterocyclyl, $NH_2$- or -$NHC_1$-$C_6$alkyl.

In an embodiment of the present invention,
wherein,

$R^1$ is 2-thiazolyl;
$R^6$ is H;
$R^7$ is H;
$R^9$ is H; and
$L_1$ is single bond or methylene.

In an embodiment of the present invention,
wherein,

$R^1$ is 2-thiazolyl;
$R^6$ is H;
$R^7$ is H;
$R^8$ is H,

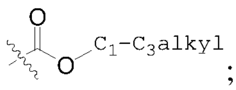

$R^9$ is H;
$L_1$ is single bond; and
$R^{10}$ is COOH, CN, $C_1$-$C_6$ alkyl substituted by one $R^A$, $C_1$-$C_6$ alkyl,

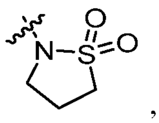

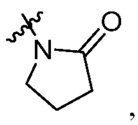

$NR^BR^C$, or

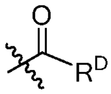.

In an embodiment of the present invention, wherein,

$R^1$ is 2-thiazolyl;
$R^6$ is H;
$R^7$ is H;
$R^8$ is H,

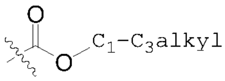;

$R^9$ is H;
$L_1$ is single bond;
$R^{10}$ is COOH, CN, $C_1$-$C_6$ alkyl substituted by one $R^A$, $C_1$-$C_6$ alkyl,

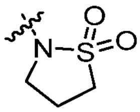,

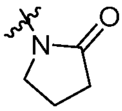,

$NR^BR^C$, or

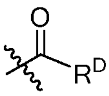;

and Q is

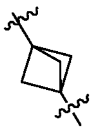.

In an embodiment of the present invention, wherein,

$R^1$ is 2-thiazolyl;
$R^6$ is H;
$R^7$ is H;
$R^8$ is H;
$R^9$ is H;
$L_1$ is single bond;
$R^{10}$ is COOH, CN, $C_1$-$C_6$ alkyl substituted by one $R^A$, $C_1$-$C_6$ alkyl,

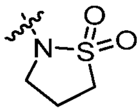,

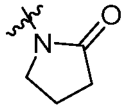,

$NR^BR^C$, or

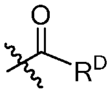;

and Q is

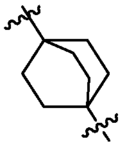, 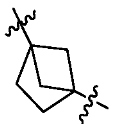, 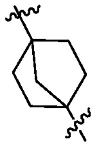 or 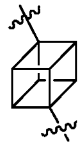.

In an embodiment of the present invention, wherein,

W is O;
$R^1$ is 2-thiazolyl;
$R^2$, $R^3$, and $R^4$ are independently H, fluorine, or methyl; and $R^3$ and $R^4$ are preferably not fluorine at the same time;
$R^6$ is H;
$R^7$ is H;
$R^8$ is H;
$R^9$ is H;
$L_1$ is single bond;
$R^{10}$ is CN, $C_1$-$C_6$ alkyl substituted by one $R^A$, $C_1$-$C_6$ alkyl,

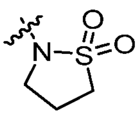, 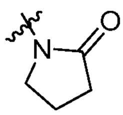,

$NR^BR^C$, or

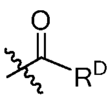;

$R^D$ is $C_4$-$C_6$ heterocyclyl, $NH_2$- or -$NHC_1$-$C_6$alkyl; and

In an embodiment of the present invention, wherein,

W is O;
$R^1$ is 2-thiazolyl;
$R^2$, $R^3$, and $R^4$ are independently H, fluorine, or methyl; and $R^3$ and $R^4$ are preferably not fluorine at the same time;

$R^6$ is H;
$R^7$ is H;
$R^8$ is H;
$R^9$ is H;
$L_1$ is single bond;
$R^{10}$ is CN, $C_1$-$C_6$ alkyl substituted by one $R^A$, $C_1$-$C_6$ alkyl,

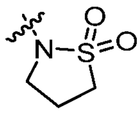 , 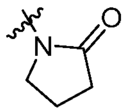 ,

$NR^BR^C$, or

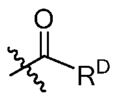 ;

$R^D$ is $C_4$-$C_6$ heterocyclyl, $NH_2$- or -$NHC_1$-$C_6$alkyl; and Q is

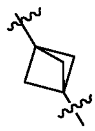 .

In an embodiment of the present invention,
wherein,

W is O;
$R^1$ is 2-thiazolyl;
$R^2$, $R^3$, and $R^4$ are independently H, fluorine, or methyl; and $R^3$ and $R^4$ are preferably not fluorine at the same time;
$R^6$ is H;
$R^7$ is H;
$R^8$ is H;
$R^9$ is H;
$L_1$ is single bond;
$R^{10}$ is COOH or $C_1$-$C_6$ alkyl substituted by one $R^A$, wherein, $R^A$ is COOH; and
Q is

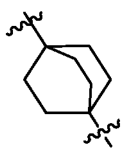 , 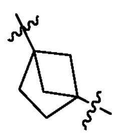 , 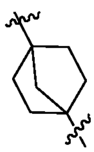 or 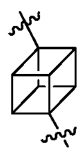 .

In an embodiment of the present invention,
wherein,

W is O;
$R^1$ is 2-thiazolyl;
$R^2$, $R^3$, and $R^4$ are independently H, fluorine, or methyl;
$R^6$ is H;
$R^7$ is H;
$R^8$ is H;
$R^9$ is H;
$L_1$ is single bond;

$R^{10}$ is CN, $C_1$-$C_6$ alkyl substituted by one $R^A$, $C_1$-$C_6$ alkyl,

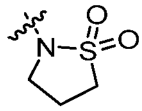, 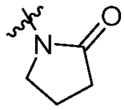,

$NR^BR^C$, or

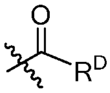;

$R^A$ is hydroxyl, halogen, $C_1$-$C_6$ alkoxy,

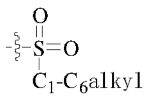, 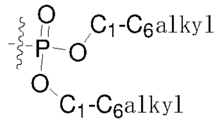,

CN,

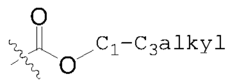,

$C_4$-$C_6$ heterocyclyl substituted by a carbonyl,

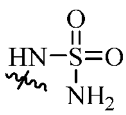,

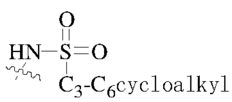, 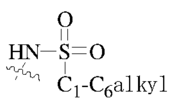 or 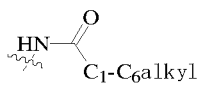;

$R^B$ and $R^C$ are independently H,

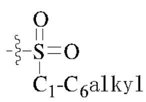, 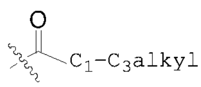,

$C_1$-$C_6$ alkyl or

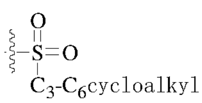;

and
$R^D$ is $C_4$-$C_6$ heterocyclyl, $NH_2$- or -$NHC_1$-$C_6$alkyl.

In an embodiment of the present invention,

wherein,

$W$ is O;
$R^1$ is 2-thiazolyl;
$R^2$, $R^3$, and $R^4$ are independently H, fluorine, or methyl;
$R^6$ is H;
$R^7$ is H;
$R^8$ is H;
$R^9$ is H;
$L_1$ is single bond;

$R^{10}$ is CN, $C_1$-$C_6$ alkyl substituted by one $R^A$, $C_1$-$C_6$ alkyl, 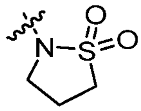, 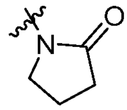, $NR^BR^C$, or

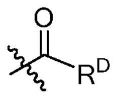;

$R^A$ is hydroxyl, halogen, $C_1$-$C_6$ alkoxy,

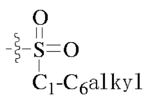, 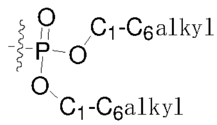,

CN,

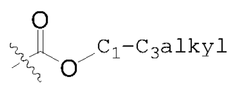,

$C_4$-$C_6$ heterocyclyl substituted by a carbonyl,

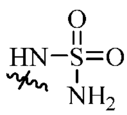,

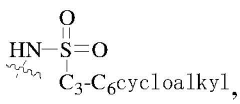, 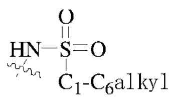 or 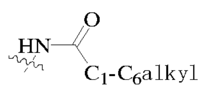;

$R^B$ and $R^C$ are independently H,

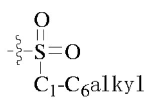, 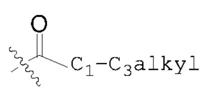,

$C_1$-$C_6$ alkyl or

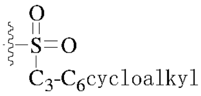;

$R^D$ is $C_4$-$C_6$ heterocyclyl, $NH_2$- or -$NHC_1$-$C_6$alkyl; and Q is

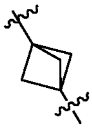.

In an embodiment of the present invention, wherein,

W is O;
$R^1$ is 2-thiazolyl;
$R^2$, $R^3$, and $R^4$ are independently H, fluorine, or methyl;
$R^6$ is H;
$R^7$ is H;
$R^8$ is H;
$R^9$ is H;
$L_1$ is single bond;
$R^{10}$ is COOH; and Q is

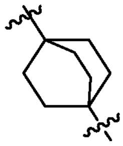.

In an embodiment of the present invention,

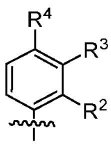 is 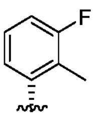, 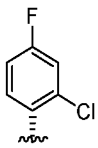, 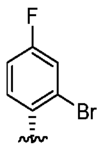, 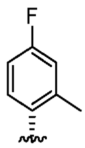, 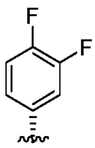, 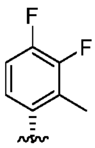 or 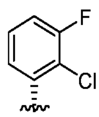.

In an embodiment of the present invention,

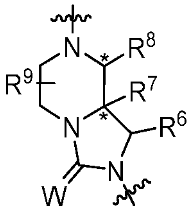

is

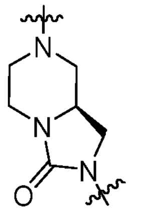,

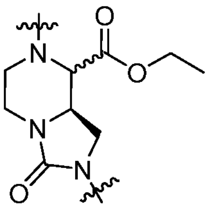, 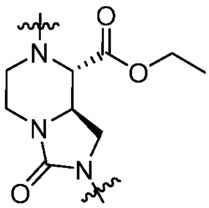, 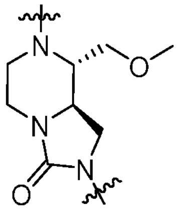 or 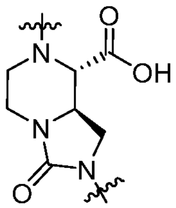.

In an embodiment of the present invention, Q is

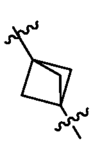, 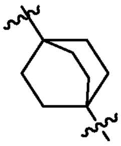, 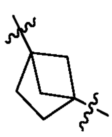, 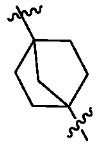

or

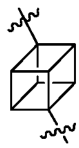.

In an embodiment of the present invention, $R^{10}$ is COOH,

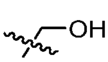, 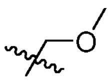,

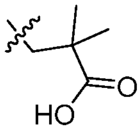, 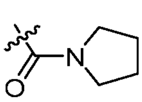, 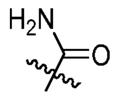, 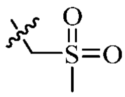, 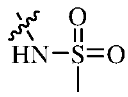, 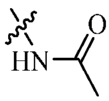, 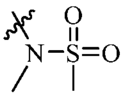, 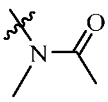,

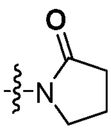, 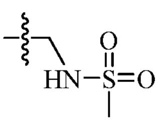, 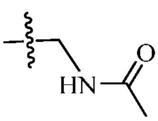, 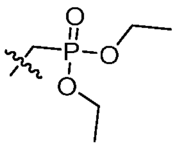, CN, 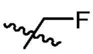, 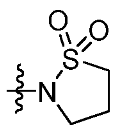, 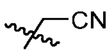,

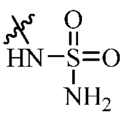, 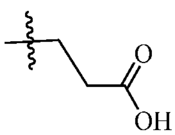, 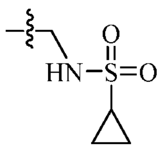, 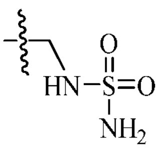, 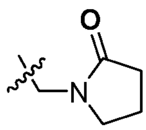, 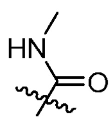, 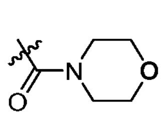,

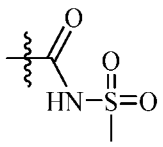, 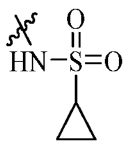, 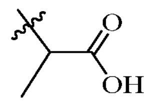, 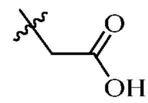, 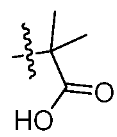, 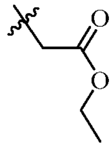, 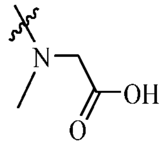,

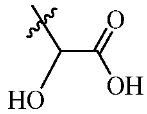 or 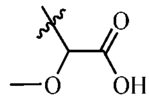.

In an embodiment of the present invention, the dihydropyrimidine compound shown in formula I' is selected from the following compounds:

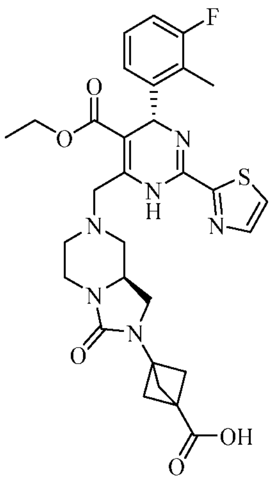, 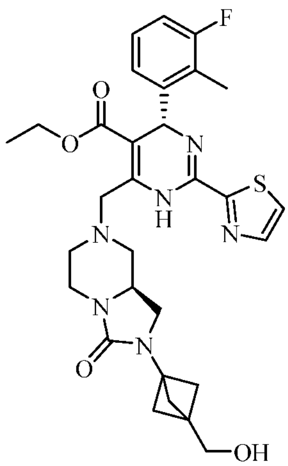、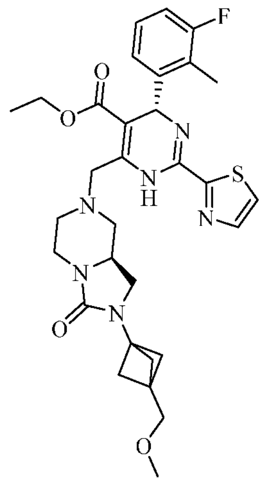, 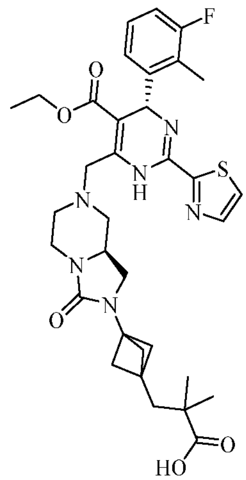,

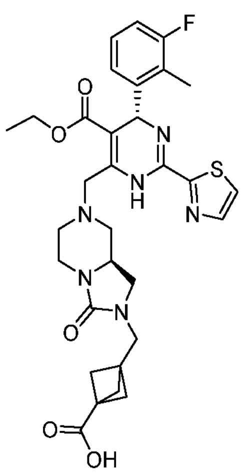, 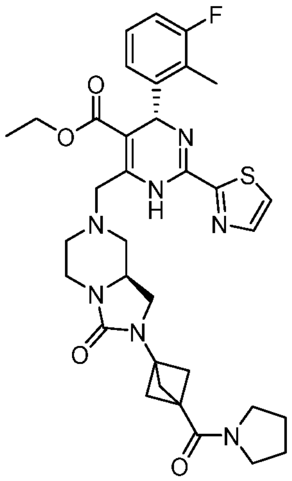, 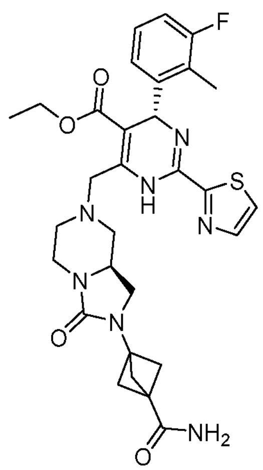, 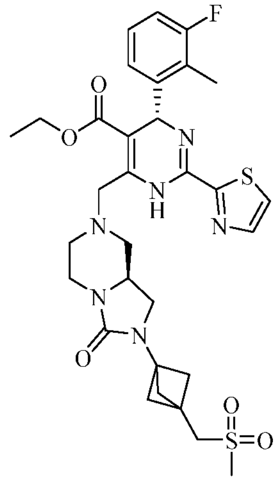,

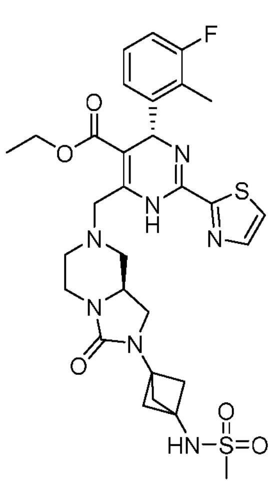 , 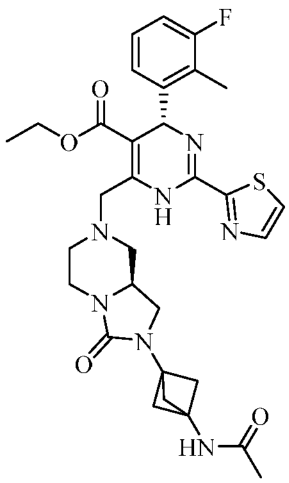 , 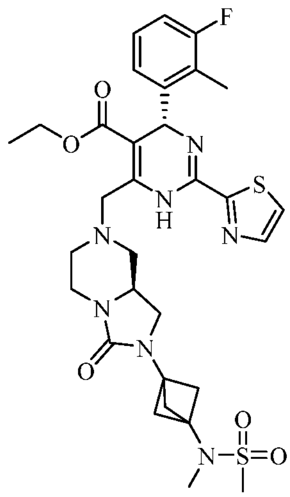 , 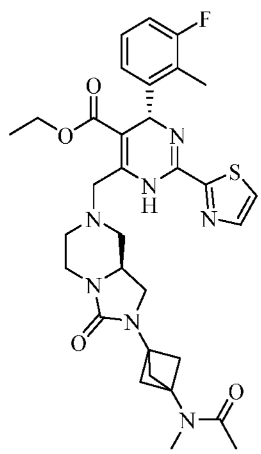 ,

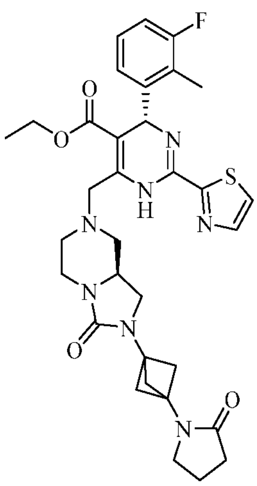 , 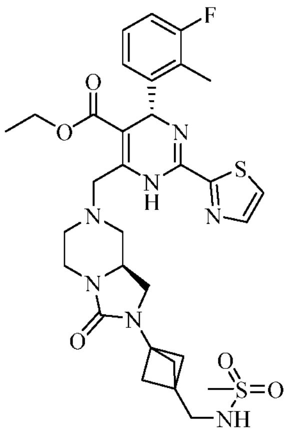 , 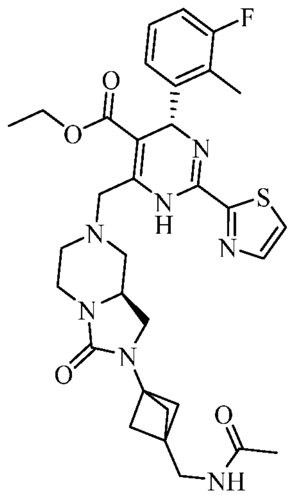 , 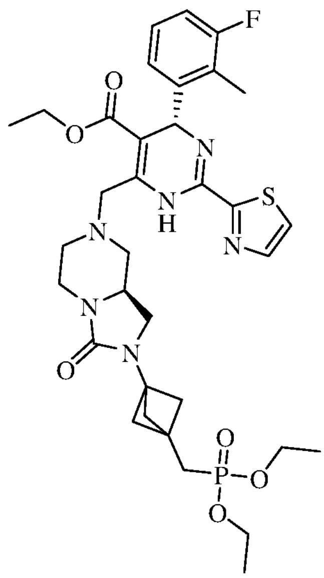 ,

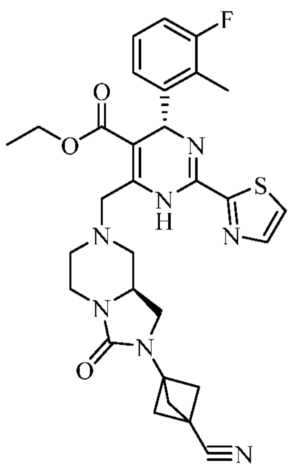 , 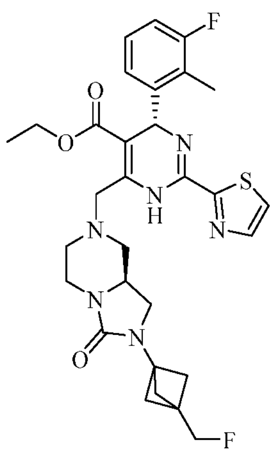 , 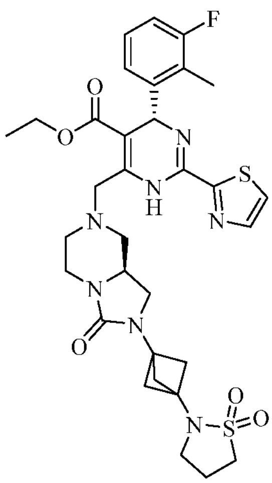 , 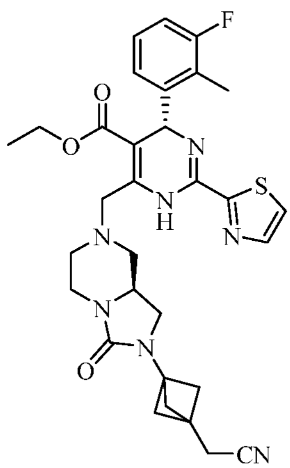 ,

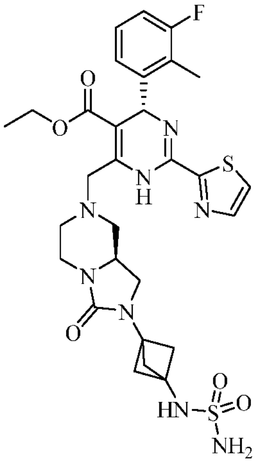 , 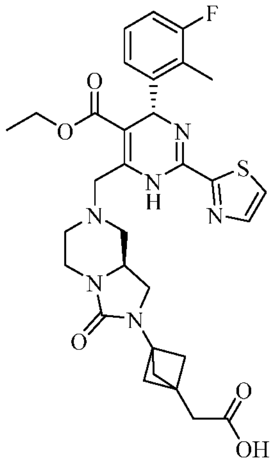 , 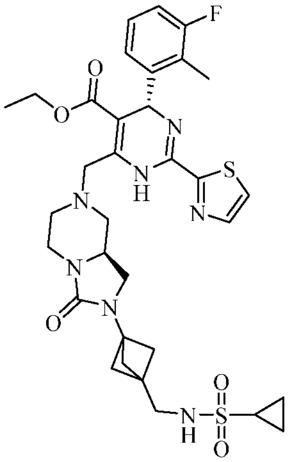 , 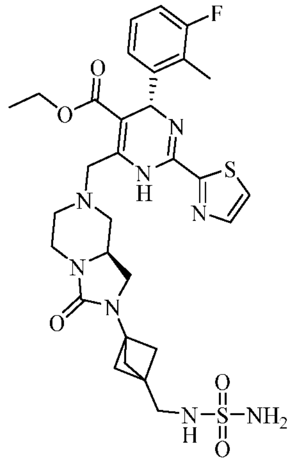 ,

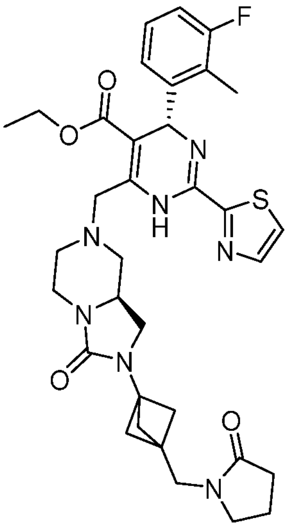 , 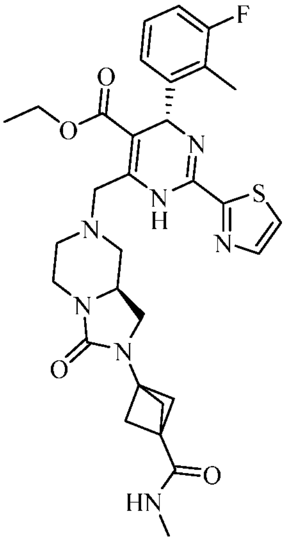 , 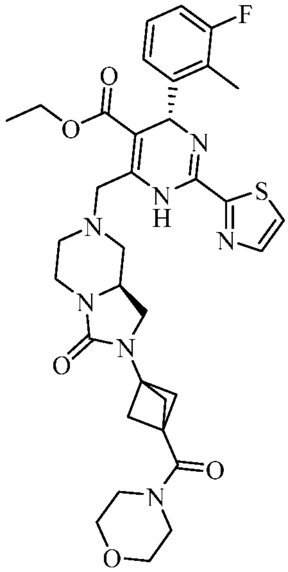 , 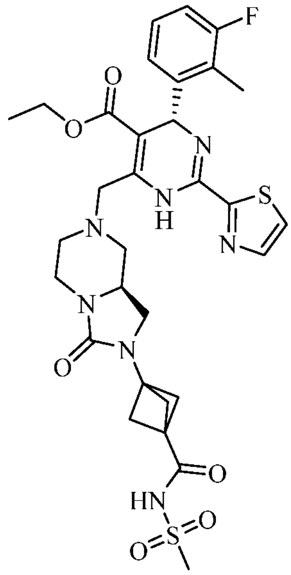 ,

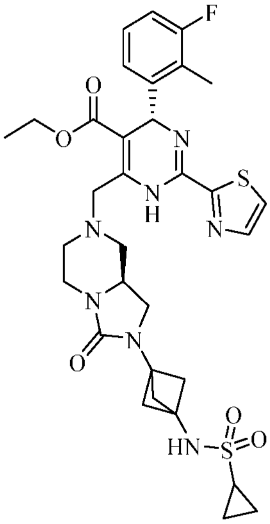 , 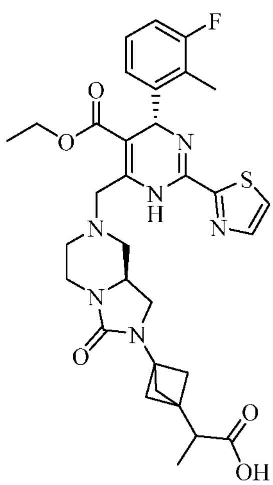 , 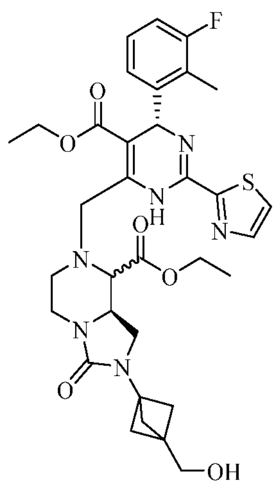 , 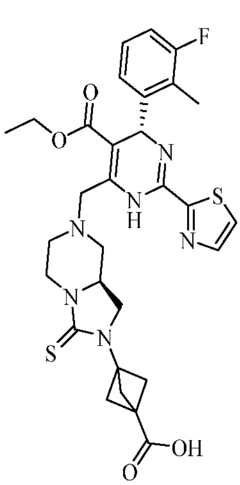 ,

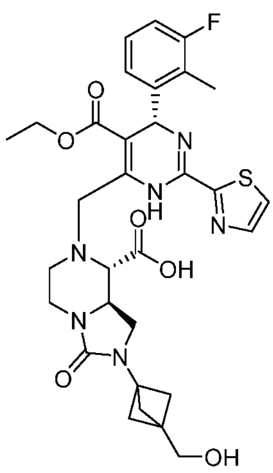,
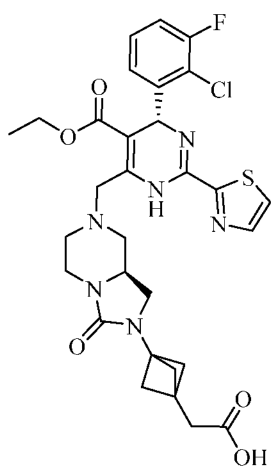,
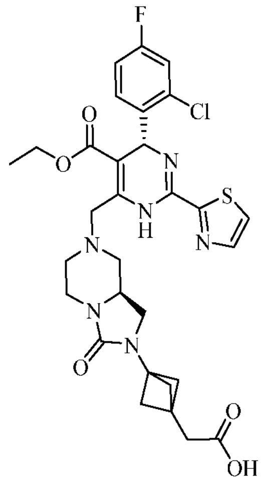,
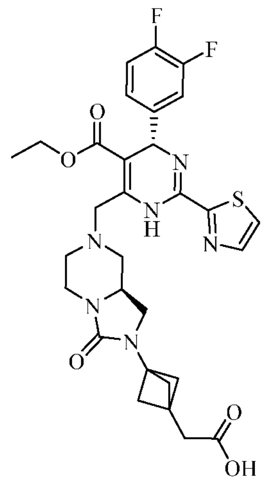,

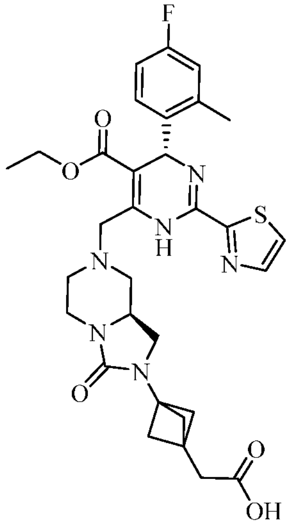,
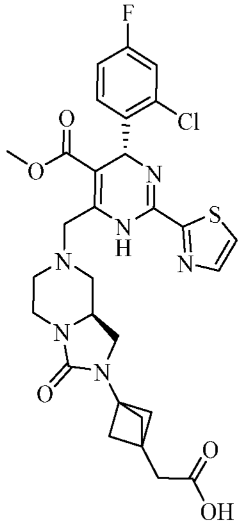,
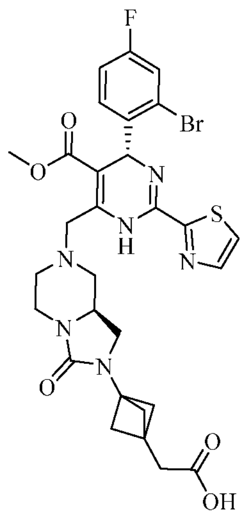,
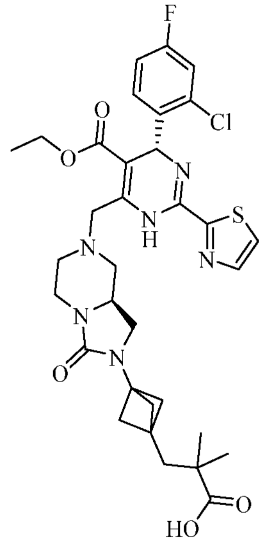,

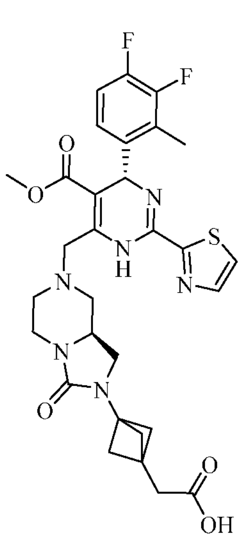,
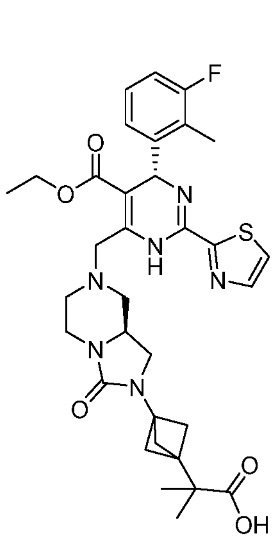,
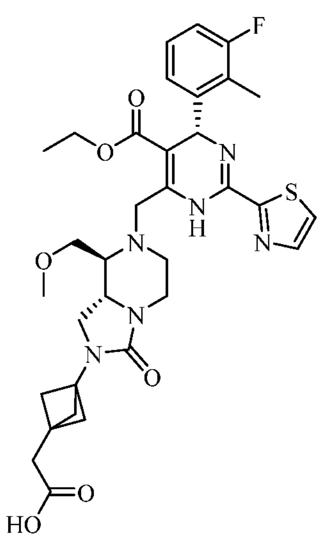,
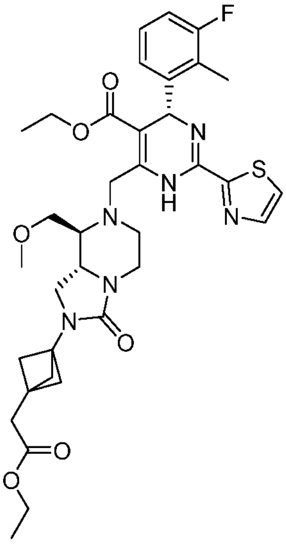,

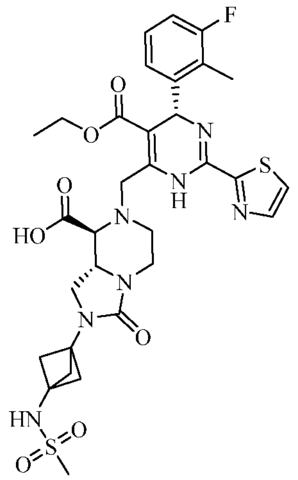 , 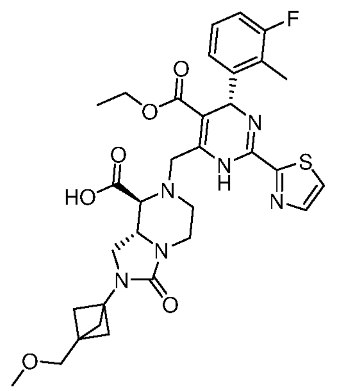 , 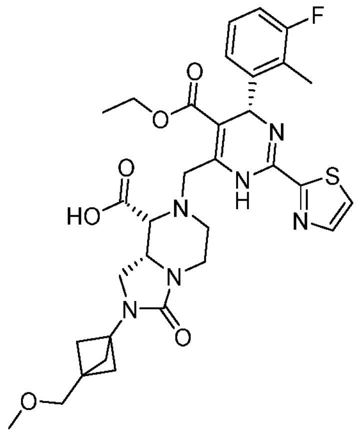 ,

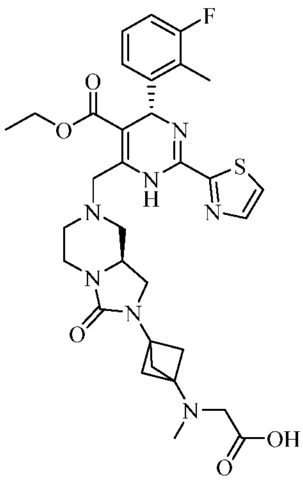 , 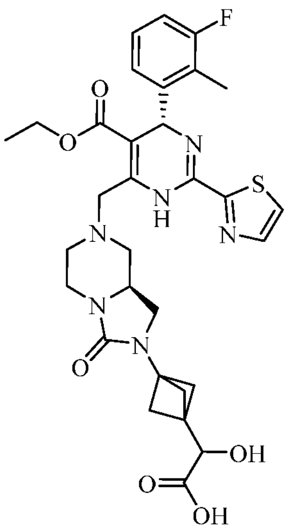 , 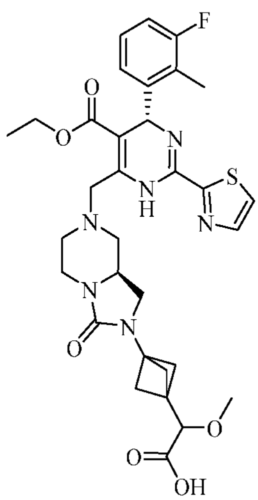 , 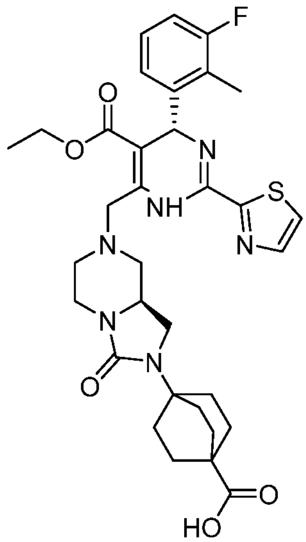 ,

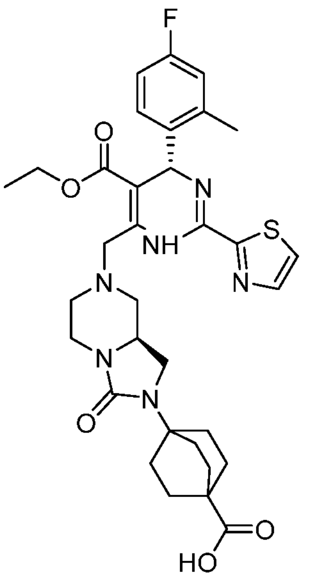 , 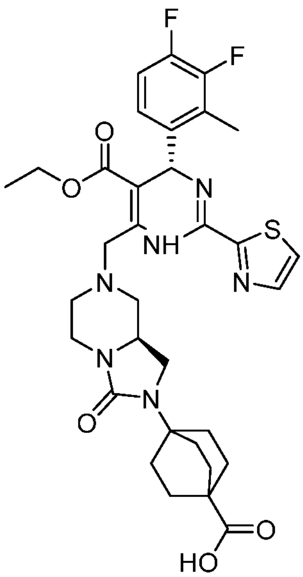 , 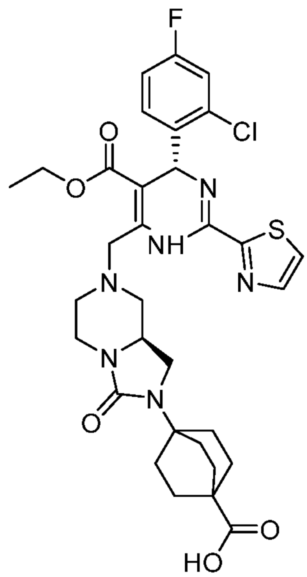 ,

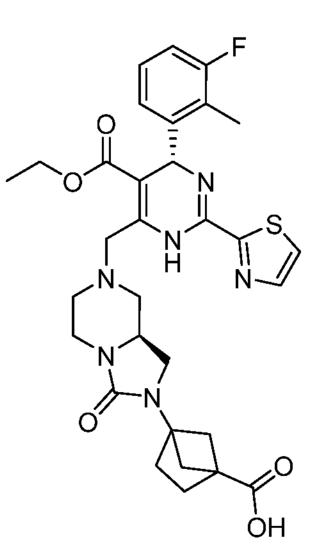
,
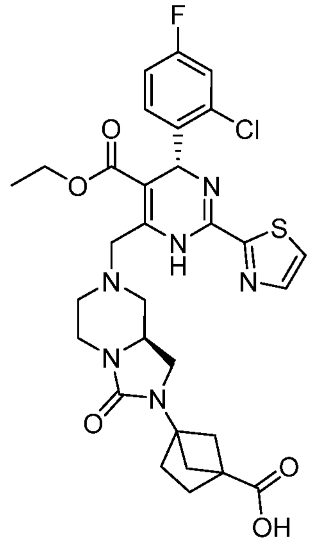
,
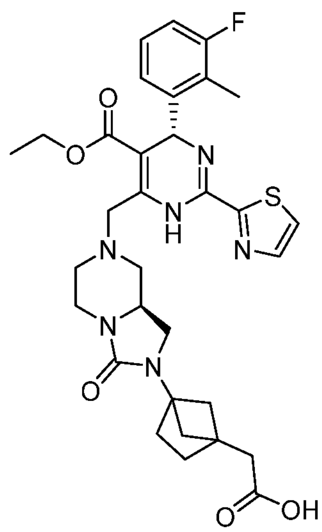
,

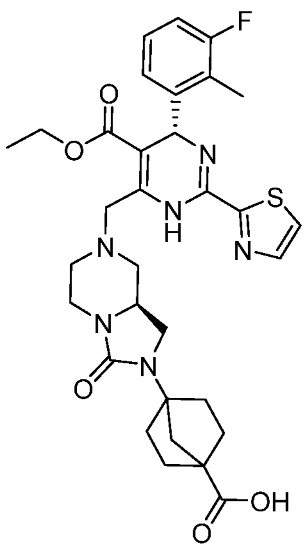
,
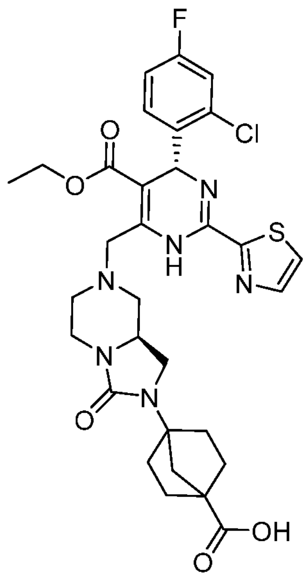
,
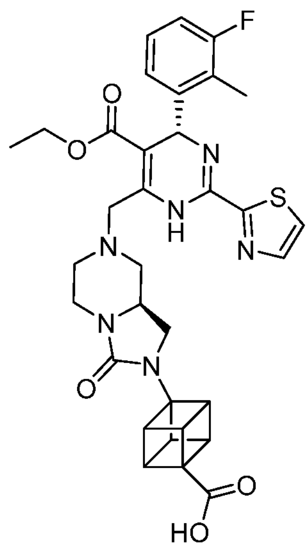
,

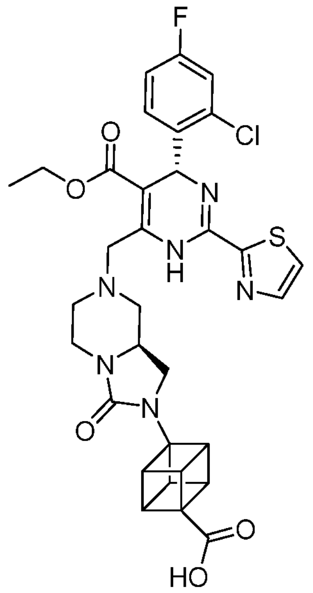
,
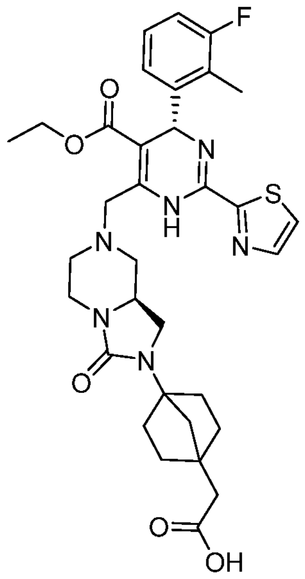
,
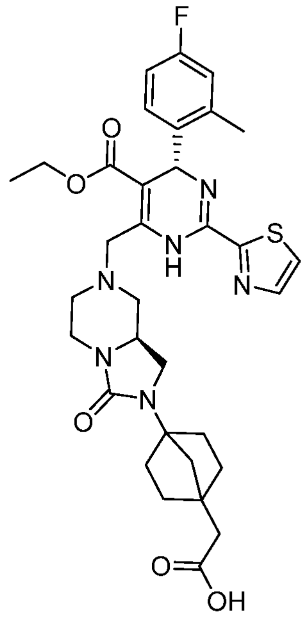

or

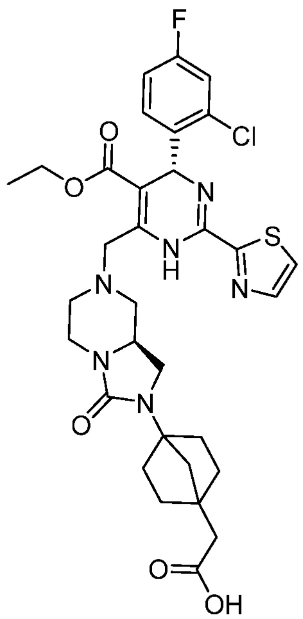
.

The invention further provides a preparation method for the above-mentioned compound of formula I, comprising the following steps:

in a solvent, in the presence of base, the compound of Formula II and the compound of Formula III undergo the following substitution reaction to obtain the compound of Formula I;

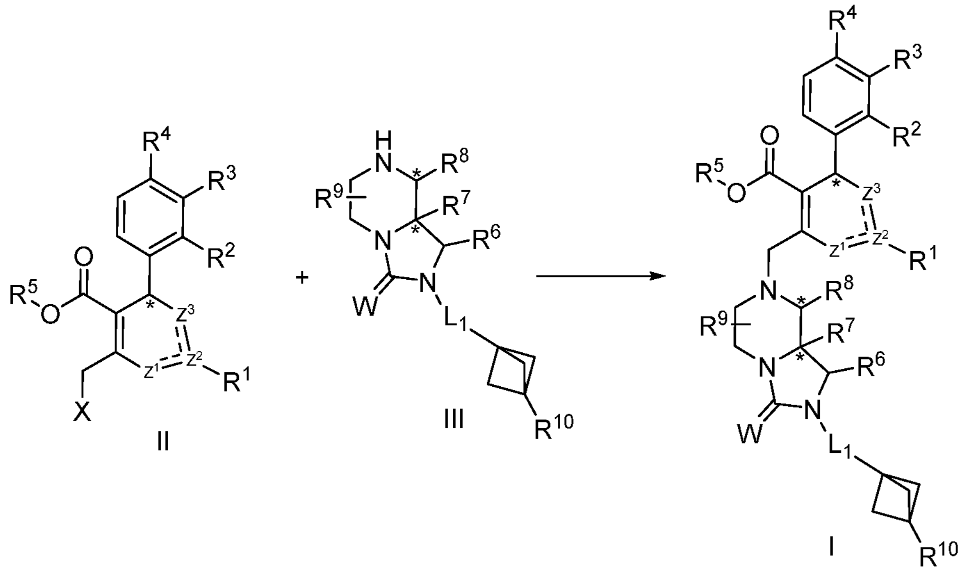

II III I

X is halogen,

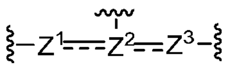,

$R^1$, $R^2$, $R^3$, $R^4$, $R^5$, $R^6$, $R^7$, $R^8$, $R^9$, $R^{10}$, $L_1$, Q, and W are as defined above.

The conditions and steps of the substitution reaction can be the conventional conditions and steps of this kind of substitution reaction in the art. The invention is particularly preferred as follows:
In the Substitution reaction, where X is halogen, the halogen is fluorine, chlorine, bromine or iodine, preferably bromine.
In the substitution reaction, the solvent can be a conventional solvent for the substitution reaction in the art, preferably halogenated alkane solvents, more preferably dichloromethane.
In the substitution reaction, the base can be a conventional base of this type of substitution reaction in the art, preferably 2,2,6,6-tetramethylpiperidine.
In the substitution reaction, the molar ratio of the compound of Formula II to the compound of Formula I can be a conventional ratio of this type of substitution reaction in the art, preferably 0.8:1 to 1.2:1, more preferably 1:1.
In the substitution reaction, the concentration of the compound of Formula II in the solvent can be a conventional concentration of this type of substitution reaction in the art, preferably 10mmol/L~100mmol/L, preferably 56mmol/L.
In the substitution reaction, the reaction temperature can be the conventional temperature of this kind of substitution reaction in the art, preferably 0°C~40°C, more preferably 20°C.
In the substitution reaction, the end point of the substitution reaction is that the compound of Formula II disappears or no longer reacts, and the reaction time is preferably 8 to 24 hours, more preferably 12 hours.
The invention further provides a preparation method for the above-mentioned compound of formula I', comprising the following steps:

in a solvent, in the presence of base, the compound of formula II and the compound of formula III' undergo the following substitution reaction to obtain the compound of formula I';

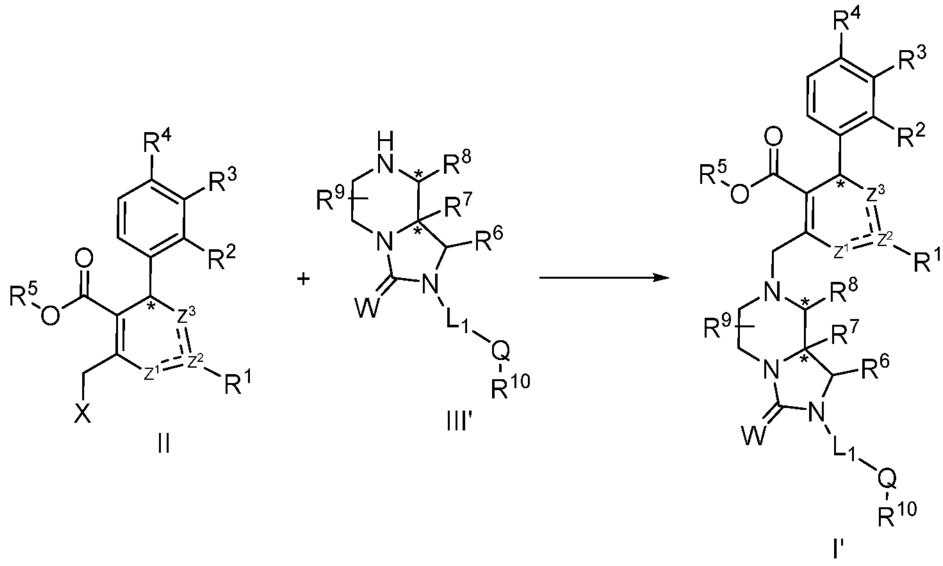

II III' I'

X is halogen,

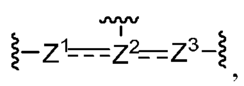

$R^1$, $R^2$, $R^3$, $R^4$, $R^5$, $R^6$, $R^7$, $R^8$, $R^9$, $R^{10}$, $L_1$, Q, and W are as defined above.

The conditions and steps of the substitution reaction can be the conventional conditions and steps of this kind of substitution reaction in the art. The invention is particularly preferred as follows:
In the substitution reaction, where X is halogen, the halogen is fluorine, chlorine, bromine or iodine, preferably bromine.

In the substitution reaction, the solvent can be a conventional solvent for the substitution reaction in the art, preferably halogenated alkane solvents, more preferably dichloromethane.

In the substitution reaction, the base can be a conventional base of this type of substitution reaction in the art, preferably 2,2,6,6-tetramethylpiperidine.

In the substitution reaction, the molar ratio of the compound of Formula II to the compound of Formula I' can be a conventional ratio of this type of substitution reaction in the art, preferably 0.8:1 to 1.2:1, more preferably 1:1.

In the substitution reaction, the concentration of the compound of Formula II in the solvent can be a conventional concentration of this type of substitution reaction in the art, preferably 10mmol/L~100mmol/L, preferably 56mmol/L.

In the substitution reaction, the reaction temperature can be the conventional temperature of this kind of substitution reaction in the art, preferably 0°C~40°C, more preferably 20°C.

In the substitution reaction, the end point of the substitution reaction is that the compound of Formula II disappears or no longer reacts, and the reaction time is preferably 8 to 24 hours, more preferably 12 hours.

Therefore, throughout this specification, those skilled in the art can select the groups and their substituents in the dihydropyrimidine compounds shown in formulas I and I' or pharmaceutically acceptable salts thereof to provide stable dihydropyrimidine compounds of formulas I and I' or pharmaceutically acceptable salts thereof, including but not limited to the compounds described in embodiments of the present invention.

The dihydropyrimidine compounds of formulas I and I' or pharmaceutically acceptable salts thereof as described in the present invention, can be synthesized by methods similar to those commonly known in the chemical field. The steps and conditions of the synthesis can refer to those of similar reactions in the present field, especially according to the instructions herein. The starting materials are usually from commercial sources, such as Aldrich or can be easily prepared using methods known to those skilled in the art (obtained through SciFinder, Reasys online databases).

In the present invention, the dihydropyrimidine compounds of formulas I and I' can also be obtained by peripheral modification of the prepared dihydropyrimidines as shown in formulae I and I' by conventional methods in the art, and thereby obtaining other heterocyclyl compounds as shown in formulae I and I'.

Generally, the compounds of the present invention can be prepared by the methods described in the present invention, unless further specified, the substituent are as defined in formulas I and I'. The following reaction scheme and embodiments are used to further exemplify the present invention.

The necessary raw materials or reagents for preparing compounds as shown in formulas I and I' can be

commercially available or prepared through known synthesis methods in the art. The compounds of the invention can be prepared as free bases or salts thereof formed by the addition of acids by the method described in the following experimental section. The term pharmaceutically acceptable salt refers to the pharmaceutically acceptable salt defined herein, which has all the pharmacological activities of the parent compound. The pharmaceutically acceptable salt can be prepared by adding the corresponding acid to the organic base in suitable organic solvent and treated according to conventional methods.

Examples of salt formation include: salt formed with inorganic acid, such as hydrochloric acid, hydrobromic acid, sulfuric acid, nitric acid, phosphoric acid; and salt formed with organic acid, such as acetic acid, benzenesulfonic acid, benzoic acid, camphorsulfonic acid, citric acid, ethanesulfonic acid, fumaric acid, glucoheptonic acid, glutamic acid, glycolic acid, naphtholcarboxylic acid, 2-hydroxyethanesulfonic acid, lactic acid, maleic acid, malic acid, malonic acid, mandelic acid, methanesulfonic acid, 2-naphthalenesulfonic acid, propionic acid, salicylic acid, succinic acid, tartaric acid, p-toluenesulfonic acid or trimethylacetic acid.

Dihydropyrimidine compounds of Formula I and I' may have one or more chiral carbon atoms, so they can be separated to obtain optically pure isomers, such as pure enantiomers, or racemates, or mixed isomers. Pure single isomers can be obtained through separation methods in the field, such as chiral crystallization into salts or chiral preparative column separation.

The chemicals used in the synthesis route described in the present patent include solvents, reagents, catalysts and protecting groups, deprotecting groups, and the protecting groups including tert butoxycarbonyl (Boc). The above methods further include steps before or after the steps specifically described herein, and suitable protecting groups can be added or removed to obtain target compound. In addition, various synthesis steps can be carried out alternately or sequentially to obtain final target product.

The present invention provides a pharmaceutical composition comprising dihydropyrimidine compounds of formula I as described above or pharmaceutically acceptable salts thereof and (one or more) pharmaceutical excipients. For example, the pharmaceutical composition may comprise one or more additional dihydropyrimidine compounds of formula I or pharmaceutically acceptable salts thereof. In the pharmaceutical composition, the dihydropyrimidine compound of formula I or pharmaceutically acceptable salts thereof can be therapeutically effective amount.

The present invention further provides a use of a dihydropyrimidine compound of formula I or a pharmaceutically acceptable salt thereof, or a pharmaceutical composition comprising thereof, in the preparation of HBV inhibitors. In the use, the HBV inhibitor can be used in mammalian organisms; it can also be used in vitro, mainly for experimental purposes, such as providing comparisons as standard samples or reference samples, or be prepared as a kit according to conventional methods in this field to provide rapid detection of HBV inhibition effect.

The present invention further provides use of the dihydropyrimidine compound of formula I, or pharmaceutically acceptable salts, or the pharmaceutical composition comprising thereof in the preparation of medication, the medication is used for the prevention and/or treatment of infections associated with HBV.

Another aspect of the present invention relates to a method of preventing and/or treating HBV infection, comprising administering to a patient therapeutically effective amount of the dihydropyrimidine compound as shown in Formula I, or pharmaceutically acceptable salts thereof, or pharmaceutical compositions comprising thereof.

Another aspect of the present invention relates to a medication used for the treatment and/or prevention of HBV infection, comprising the dihydropyrimidine compound of formula I or a pharmaceutically acceptable salt thereof, or a pharmaceutical composition comprising thereof.

The present invention provides a pharmaceutical composition, comprising the dihydropyrimidine compound of formula I' as described above or pharmaceutically acceptable salts thereof and (one or more) pharmaceutical excipients. For example, the pharmaceutical composition may comprise one or more additional dihydropyrimidine compounds of formula I' or pharmaceutically acceptable salts thereof. In the pharmaceutical composition, the dihydropyrimidine compound of formula I' or pharmaceutically acceptable salts thereof can be of therapeutically effective amount.

The present invention further provides a use of a dihydropyrimidine compound of formula I' or a pharmaceutically acceptable salt thereof, or a pharmaceutical composition comprising thereof, in the preparation of HBV inhibitors. In the use, the HBV inhibitor can be used in mammalian organisms; it can also be used in vitro, mainly for experimental purposes, such as providing comparisons as standard samples or reference samples, or be prepared as a kit according to conventional methods in this field to provide rapid detection of HBV inhibition effect.

The present invention further provides use of the dihydropyrimidine compound of formula I', or pharmaceutically acceptable salts, or the pharmaceutical composition comprising thereof in the preparation of a medication, the medication is used for the prevention and/or treatment of infections associated with HBV.

Another aspect of the present invention relates to a method of preventing and/or treating HBV infection, comprising administering to a patient therapeutically effective amount of the dihydropyrimidine compound as shown in Formula I', or pharmaceutically acceptable salts thereof, or pharmaceutical compositions comprising thereof.

Another aspect of the present invention relates to a medication used for the treatment and/or prevention of HBV infection, comprising the dihydropyrimidine compound of formula I' or a pharmaceutically acceptable salt thereof,

or a pharmaceutical composition comprising thereof.

The compounds and pharmaceutical compositions of the present invention can be administered locally or throughout the body, for example, for enteral administration, such as rectal or oral administration, or for parenteral administration to mammals (especially humans). Exemplary composition for rectal administration include suppositories, which may comprise, for example, a suitable non irritating excipient, such as cocoa butter, synthetic triglyceride, or polyethylene glycol, which is solid at room temperature, but melts and/or dissolves in the rectal cavity to release the drug. The compounds of the present invention can also be administered parenterally, for example, through inhalation, injection or infusion, such as intravenously, intraarterially, intraosseously, intramuscularly, intracerebrally, extraventricularly, synovially, intrasternally, intrathecally, intralesionally, intracranially, intratumorally, intradermally, and subcutaneously injected or administered.

The compounds and pharmaceutical compositions described in the present invention can be administered locally or throughout the body, for example, for enteral administration, such as rectal or oral administration, or for parenteral administration to mammals (especially humans), and include therapeutically effective amount of compounds or pharmaceutically acceptable salts as active ingredients according to the present invention, along with pharmaceutically acceptable excipients, such as pharmaceutically acceptable carriers. The therapeutically effective amount of active ingredients is defined by the context and depends on the type of mammal, body weight, age, individual condition, individual pharmacokinetic parameters, disease to be treated, and mode of administration. For enteral administration, such as oral medication, the compound of the present invention can be formulated into a wide variety of dosage forms.

The effective amount of the compound, pharmaceutical composition or drug of the invention can be easily determined by routine experiments, and the most effective and convenient route of administration and the most appropriate preparation can also be determined by routine experiments.

The pharmaceutical excipients mentioned can be those widely used in the field of drug production. Excipients are mainly used to provide a safe, stable, and functional pharmaceutical composition, and can also provide methods to enable the active ingredients to dissolve at a desired rate after the subject receives the medication, or to promote the effective absorption of the active ingredients after the subject receives the medication of the composition. The pharmaceutical excipients can be inert fillers or provide certain functions, such as stabilizing the overall pH value of the composition or preventing the degradation of the active ingredients of the composition. The pharmaceutical excipients described may include one or more of the following excipients: adhesives, suspension aids, emulsifiers, diluents, fillers, granulators, adhesives, disintegrants, lubricants, anti adhesion agents, flow aids, wetting agents, gelling agents, absorption retardants, dissolution inhibitors, enhancers, adsorbents, buffering agents, chelating agents, preservatives, colorants, flavorings and sweeteners.

Substances that can be used as pharmaceutically acceptable excipients include, but are not limited to, ion exchanger, aluminum, aluminum stearate, lecithin, serum protein, such as human serum proteins, buffering substances, such as phosphate, glycine, sorbic acid, potassium sorbate, partial glyceride mixture of saturated vegetable fatty acids, water, salt or electrolyte, such as protamine sulfate, disodium hydrogen phosphate, potassium hydrogen phosphate, sodium chloride, zinc salt, colloidal silica, magnesium trisilicate, polyvinylpyrrolidone, polyacrylate, wax, polyethylene-polyoxypropylene blocking polymer, lanolin, sugar, such as lactose, glucose and sucrose; starches such as corn starch and potato starch; cellulose and its derivatives such as sodium carboxymethyl cellulose, ethyl cellulose and cellulose acetate; arabic gum; malt; gelatin; talc; excipients such as cocoa butter and suppository wax; oil such as peanut oil, cottonseed oil, safflower oil, sesame oil, olive oil, corn oil and soybean oil; glycol compounds, such as propylene glycol and polyethylene glycol; esters such as ethyl oleate and ethyl laurate; agar; buffers such as magnesium hydroxide and aluminium hydroxide; alginate; pyrogen-free water; isotonic salt; Ringer's solution; ethanol, phosphate buffer solution, and other non-toxic suitable lubricants such as sodium lauryl sulfate and magnesium stearate, colorants, releasing agents, coatings, sweeteners, flavorings and spices, preservatives and antioxidants.

The pharmaceutical composition of the present invention can be prepared using any method known to those skilled in the art according to the disclosed content. For example, conventional mixing, dissolution, granulation, emulsification, grinding, encapsulation, embedding, or freeze-drying processes.

The pharmaceutical dosage forms of the compounds of the present invention can be provided in the form of rapid release, controlled release, sustained release, or target drug release systems. For example, commonly used dosage forms include solutions and suspensions, (micro) lotion, ointments, gel and patches, liposomes, tablets, sugar coated pills, soft or hard shell capsules, suppositories, ovules, implants, amorphous or crystalline powders, aerosols and freeze-dried preparations. Depending on the route of administration used, special devices may be required to administer or give drugs, such as syringes and needles, inhalers, pumps, injector pen, applicators or special flasks. Drug dosage forms often consist of drugs, excipients, and container/sealing systems. One or more excipients (also known as inactive ingredients) can be added to the compounds of the present invention to improve or promote the manufacture, stability, administration, and safety of drugs, and a method for obtaining the required drug release curve can be provided. Therefore, the type of excipient added to the drug can be determined by various factors, such as the physical and chemical properties of the drug, the route of administration and the preparation steps. There are medicinal excipients

in this field and include those listed in various pharmacopoeia. (See U.S. Pharmacopeia, USP, Japanese Pharmacopeia, JP, European Pharmacopoeia, EP and British Pharmacopeia, BP); the U.S. Food and Drug Administration (www.fda.gov) Center for Drug Evaluation and Research (CEDR) publications, such as the "Inactive Ingredient Guide" (1996); Hand book of Pharmaceutical Additives (2002) written by Ash and Ash, Synapse Information Resources, Inc., Endicott NY; etc.

The pharmaceutical dosage form of the compound of the invention can be manufactured by any method well known in the art, such as conventional mixing, screening, dissolution, melting, granulation, manufacture of sugar-coated pills, tablet pressing, suspension, extrusion, spray drying, grinding, emulsification, (nanometer/micron level) encapsulation, packaging or freeze-drying processes. As mentioned above, the composition of the present invention may include one or more physiologically acceptable non-active ingredients that promote the processing of active molecules into formulations for pharmaceutical purposes.

The pharmaceutical composition and dosage form may include one or more compounds of the present invention or one or more pharmaceutically acceptable salts thereof as active ingredients. pharmaceutically acceptable carriers can be solid or liquid. Solid formulations include powders, tablets, pills, lozenges, capsules, suppositories, and dispersible granules. Solid carriers can be one or more substances used as diluents, flavorings, solubilizers, lubricants, suspending agents, adhesives, preservatives, tablet disintegrators, or encapsulating materials. In powders, the carrier is usually a finely ground solid, which is a mixture of finely ground active ingredients. In tablets, the active component is usually mixed with a carrier with necessary adhesive ability in an appropriate ratio and compacted according to the desired shape and size. Suitable carriers include but are not limited to magnesium carbonate, magnesium stearate, talc, sugar, lactose, pectin, dextrin, starch, gelatin, methyl cellulose, sodium carboxymethyl cellulose, low melting point wax, cocoa butter, etc. The dosage form of active ingredients can include encapsulating materials as carriers, providing capsules, where the active ingredients with or without carriers are surrounded by the carriers to which it is bound.

Other forms suitable for oral administration include liquid preparations, including lotion, syrup, elixir, aqueous solution, aqueous suspension, or solid preparations intended to be converted into liquid preparations shortly before use. The lotion can be prepared in a solution, such as propylene glycol aqueous solution, or can contain emulsifiers, such as lecithin, dehydrated sorbitol monooleate or gum arabic. Aqueous solutions can be prepared by dissolving the active ingredients in water and adding appropriate colorants, spices, stabilizers, and thickeners. Aqueous suspensions can be prepared by dispersing the active ingredients of fine particles in water with binders such as natural or synthetic gum, resin, methyl cellulose, carboxymethyl cellulose and other commonly used suspending agents. Solid formulations include solutions, suspensions and lotion, in addition to active ingredients, they can also contain colorants, spices, stabilizers, buffers, artificial and natural sweeteners, dispersants, thickeners, solubilizers, etc.

For parenteral administration, the pharmaceutical composition of the present invention can be in the form of sterile injectable or infusible injectable formulations, for example, as sterile aqueous or oily suspensions. The suspension can be prepared by using suitable dispersant or wetting agent (such as Tween 80) and suspending agent according to the technology known in the art. Sterile injectable or infusible formulations can also be non-toxic injectable or infusible solutions or suspensions in parenterally acceptable diluents or solvents. For example, the pharmaceutical composition can be a solution in 1,3-butanediol. Other examples of acceptable media and solvents that can be used in the pharmaceutical compositions of the present invention include, but are not limited to, mannitol, water, Ringer's solution, and isotonic sodium chloride solution. In addition, sterile non-volatile oils are commonly used as solvents or suspension media. For this purpose, any mild non-volatile oil, including synthetic glycerol monoesters or diglycerides, can be used. Fatty acids such as oleic acid and its glycerol derivatives can be used to prepare injections, as well as natural pharmaceutically acceptable oils, such as olive oil or castor oil, especially their polyoxyethylated forms. These oil solutions or suspensions can also contain long-chain alcohol diluents or dispersants. Solutions for parenteral use may also include suitable stabilizers and, if necessary, buffering agent. Suitable stabilizers include antioxidants, such as sodium bisulfate, sodium sulfite or ascorbic acid, citric acid, and their salts and EDTA sodium salts alone or in combination. Parenteral solutions may also include preservatives such as benzalkonium chloride, p-hydroxybenzoic acid or propyl p-hydroxybenzoate and chlorobutanol.

The therapeutically effective amount may first be estimated using various methods well known in the art. The initial dose used for animal research can be based on the effective concentration established in cell culture assay. The dose range suitable for individual humans, for example, can be determined using data obtained from animal research and cell culture measurements. In certain embodiments, the compound of the present invention can be prepared as a medication for oral use.

An effective amount or therapeutically effective amount or dosage of a medication (such as a compound of the present invention) refers to the amount of medication or compound that causes improvement in individual symptoms or prolongation of survival. The toxicity and therapeutic efficacy of the molecule can be measured in cell culture or experimental animals through standard pharmaceutical procedures, such as $LD_{50}$ (the dose that causes 50% mortality in the population) and $ED_{50}$ (the dose that is effective for 50% treatment in the population). The dose ratio of toxic effects to therapeutic effects is the therapeutic index, which can be expressed as $LD_{50}/ED_{50}$. Medications showing a high therapeutic index are preferred.

An effective amount or therapeutically effective amount is the amount of a compound or pharmaceutical composition that will trigger a biological or medical response in tissue, system, animal or human being that is being explored by researchers, veterinarians, physicians or other clinicians. The dosage is preferably within the range of circulating concentrations including extremely toxic or non-toxic $ED_{50}$. The dosage can vary within this range, depending on the dosage form used and/or the route of administration used. The correct formulation, route of administration, dosage, and dosing interval should be selected according to methods known in the field, taking into account the specificity of the individual condition.

The dosage and interval time can be individually adjusted to provide plasma levels of the active fraction sufficient to achieve the desired effect; i.e., minimal effective concentration (MEC). The MEC will vary from compound to compound, but can be estimated, for example, from in vitro (in vitro) data and animal experiments. The dose necessary to obtain MEC will depend on individual characteristics and route of administration. In the case of local administration or selective uptake, the effective local concentration of the drug may be independent of the plasma concentration.

The amount of medication or composition administered can vary depending on various factors, including the gender, age, and weight of the treated individual, the severity of the pain, the mode of administration, and the judgment of the prescribing physician.

When desired, the composition of the present invention may be provided in packages or dispensing devices containing one or more unit dosage forms (containing the active ingredient). For example, the package or device may include metal or plastic foil (such as foam packaging) or glass and rubber stoppers, as in small bottles. The package or dispensing device may be accompanied by medication instructions. Compositions of compounds of the present invention formulated in compatible pharmaceutical carriers can also be prepared, placed in appropriate containers, and labeled for the treatment of specified conditions.

Unless otherwise specified, all technical and scientific terms used in this article have the standard meanings of the field that require the protection of the subject matter. If there are multiple definitions for a certain term, the definition herein shall prevail.

Definition of groups

Unless otherwise specified, the following definitions used herein should be applied. For the purpose of the present invention, chemical elements and periodic table CAS edition, and Handbook of Chemistry and Physics, 75th Edition, 1994 are the same. In addition, the general principles of organic chemistry can be referred to in "Organic Chemistry", Thomas Sorrell, University Science Books, Sausalito: 1999, and "March's Advanced Organic Chemistry" by Michael B. Smith and Jerry March, John Wiley&Sons, New York: 2007, all of which are incorporated herein by reference.

In the present specification, the groups and substituents thereof can be selected by those skilled in the art to provide stable structural moieties and compounds. When a substituent is described by a conventional formula written from left to right, the substituent likewise includes the chemically equivalent substituent obtained when the formula is written from right to left.

Certain chemical groups defined herein are preceded by a simplified symbol to indicate the total number of carbon atoms present in that group. For example, $C_1$-$C_6$ alkyl refers to an alkyl group as defined below having a total of 1, 2, 3, 4, 5 or 6 carbon atoms. The total number of carbon atoms in the simplified symbol does not include carbons that may be present in substituent of the group.

In this specification, numerical ranges defined in substituents such as 0 to 4, 1-4, 1 to 3 indicate integers within that range, such as 1-6 being 1, 2, 3, 4, 5, and 6.

In addition to the foregoing, the following terms, when used in the present specification and claims, have the meanings set forth below unless otherwise specified.

The term "include" is an open-ended expression, i.e., means including what is specified in the present invention, but does not exclude other aspects.

The term "substituted" refers to the substitution of any one or more hydrogen atoms on a particular atom by substituents, including deuterium and hydrogen variants, provided that the valence state of the particular atom is normal and the substituted compound is stable.

Generally speaking, the term "substituted" indicates that one or more hydrogen atoms in a given structure have been replaced by specific substituents. Furthermore, when the group is replaced by more than one substituent, the substituents are independent of each other, that is, the substituents of more than one substituents of a given group can be different or the same. Unless otherwise indicated, a substituents can be substituted at various substitutable positions of the substituted group. When more than one position in the given structural formula can be substituted by one or more substituents selected from specific groups, then the substituents may be substituted at various positions identically or differently.

In various parts of this specification, the substituents of compounds disclosed in the present invention are

disclosed according to the type or range of functional groups. Specifically, the present invention includes each independent secondary combination of each member of these functional group types and ranges. For example, the term "$C_1$-$C_6$ alkyl" or "$C_{1-6}$ alkyl" specifically refers to independently disclosed methyl, ethyl, $C_3$ alkyl, $C_4$ alkyl, $C_5$ alkyl, and $C_6$ alkyl; $C_{1-4}$ alkyl specifically refers to independently disclosed methyl, ethyl, $C_3$ alkyl (i.e. propyl, including n-propyl and isopropyl), and $C_4$ alkyl (i.e. butyl, including n-butyl, isobutyl, sec-butyl, and tert butyl).

The term "halogen" is selected from F, Cl, Br, or I, especially referring to F or Cl.

In this application, 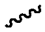 bond, when represents single bond, refers to a mixture of 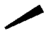 and 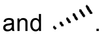.

In the present application, the term "alkyl", as a group or part of another group, means saturated aliphatic hydrocarbon group, having from 1 to 12 carbon atoms, preferably having from 1 to 6 carbon atoms. General formula is $C_nH_{2n+1}$. The term "$C_1$-$C_6$ alkyl", "$C_{1-6}$ alkyl" refers to the alkyl moiety containing 1, 2, 3, 4, 5, or 6 carbon atoms. In a certain embodiment, the "alkyl" refers to $C_1$-$C_6$ alkyl. In a certain embodiment, the "alkyl" refers to $C_1$-$C_4$ alkyl.

Lower alkyls containing 1 to 6 carbon atoms, non limiting examples include methyl, ethyl, n-propyl, isopropyl, n-butyl, isobutyl, tert butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Non limiting examples of alkyls containing 1 to 12 carbon atoms include examples of lower alkyls containing 1 to 6 carbon atoms mentioned above, as well as 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-quinyl, 3,3-diethylhexyl, 2,2-diethylhexyl, and various branched isomers.

In this application, as a group or as part of another group, unless otherwise specified, the term "cycloalkyl" refers to a saturated monocyclic ring, polycyclic ring, or bridged carboncyclic ring substituent composed solely of carbon and hydrogen atoms, which can be connected to the rest of the molecule via any suitable carbon atom by a single bond; when it is polycyclic, it can be a fused or spiral ring system (i.e., the two adjacent hydrogen atoms on the carbon atom are replaced by alkylene) that connected in paralle or spiral. Cycloalkyl substituents can be connected to the central molecule via any suitable carbon atom. In some embodiments, a ring with 3-10 carbon atoms can be represented as a $C_3$-$C_{10}$ cycloalkyl. In some embodiments, $C_3$-$C_{10}$ cycloalkyl includes cyclopropyl ($C_3$), cyclobutyl ($C_4$), cyclopentyl ($C_5$), and cyclohexyl ($C_6$). In some embodiments, examples of $C_3$-$C_{10}$ cycloalkyl include the aforementioned $C_3$-$C_6$ cycloalkyls, along with cycloheptyl ($C_7$), cyclooctyl ($C_8$), cyclononyl ($C_9$), and cyclodecyl ($C_{10}$).

In this application, the term "bridged cycloalkyl", as a group or as part of another group, refers to a carbocyclic substituent of a saturated bridged ring which may be linked to the rest of the molecule by a single bond via any suitable carbon atom; e.g., a 3-10-membered bridged cycloalkyl with 3-10 carbon atoms, preferably a 5-8-membered bridged cycloalkyl with 5-8 carbon atoms.

In the present application, as a group or as part of another group, the term "heterocyclyl" refers to a stable 3- to 10-membered saturated cyclic group consisting of 2 -6 carbon atoms and 1 -4 heteroatoms selected from nitrogen, oxygen and sulfur. In the embodiments, "heterocyclyl" refers to a stable 3-to 7-membered saturated cyclic group consisting of 2-6 carbon atoms and 1-4 heteroatoms selected from nitrogen, oxygen and sulfur. Examplary 3-membered heterocyclyl includes, but are not limited to, azirdinyl, oxirane group, and thiocyclopropyl, or stereoisomers thereof; examplary 4-membered heterocyclyl includes, but are not limited to, azetidinyl, epoxypropanyl, thietane, or isomers and stereoisomers thereof; examplary 5-membered heterocyclyl includes, but are not limited to, tetrahydrofuranyl, tetrahydrothienyl, pyrrolidinyl, thiazolidinyl, isothiazolidinyl, oxazolidinyl, isoxazolidinyl, imidazolidinyl, pyrazolidinyl, dioxypentanyl, oxothiofuranyl, dithiofuranyl, or isomers and stereoisomers thereof. Examplary 6-membered heterocyclyl includes, but are not limited to, piperidinyl, tetrahydropyranyl, cyclopentyl sulfide, morpholinyl, thiomorpholinyl, dithianyl, dioxanyl, piperazinyl, triazinyl, or isomers and stereoisomers thereof; examplary 7-membered heterocyclyl includes, but are not limited to, azacycloheptyl, oxyheterocyclic heptyl, thioheterocyclic heptyl, and diazocycloheptyl, isomers and stereoisomers thereof. In some embodiments, "heterocycloalkyl" is $C_2$-$C_5$ heterocycloalkyl, wherein the heteroatom is selected from one or more of N, O and S, and the number of heteroatoms is 1, 2 or 3.

In some embodiments, the heterocycloalkyl group is a saturated, bridge-linked heterocyclic hydrocarbon group; preferably 5-8-membered heterocyclyl; the 5-8-membered heterocyclyl is bridged-linked, the heteroatoms in the 5-8-membered heterocyclyl are selected from N, O, and S, and the number of heteroatoms is one, two, or three;

In this application, as a group or as part of another group, the term "aryl" refers to a group ("$C_6$-$C_{14}$ aryl") having 6-14 ring atoms and a monocyclic or polycyclic(such as a bicyclic or tricyclic) 4n+2 aromatic ring system without heteroatoms in the aromatic ring system(for example, having 6, 10, or 14 shared p-electrons in cyclic array ). Examples of aryl units mentioned above include phenyl, naphthyl, phenanthryl, or anthranyl.

In this application, as a group or as part of another group, the term "heteroaryl" refers to a group ("4-16 heteroaryl") of a 4-16 membered monocyclic ring or bicyclic 4n+2 aromatic ring system (e.g., having 6 or 10 shared p-electrons in

cyclic array) with carbon atoms and 1-3 heteroatoms (wherein, each heteroatom is independently selected from nitrogen, oxygen and sulfur) provided in the aromatic ring system. In heteroaryl groups comprising one or more nitrogen atoms, the point of attachment may be either a carbon or a nitrogen atom, as far as the valence state allows.

In some embodiments, the heteroaryl is a 4-6-membered heteroaryl with 1-3 heteroatoms selected from one or more of N, O, and S, preferably a 5-6-membered heteroaryl group.

Examples of 5-membered heteroaryl groups include, but are not limited to, pyrrolyl, furanyl, thiophenyl, imidazolyl, pyrazolyl, oxazolyl, isoxazolyl, thiazolyl, isothiazole, triazole, oxadiazolyl, thiadiazole, furzanyl, oxadiazolyl or tetrazolyl. Exemples of 6-membered heteroaryl groups include but are not limited to pyridinyl, pyrazinyl, pyridazinyl, pyrimidinyl, triazinyl or tetrazinyl.

As used herein, the terms "moiety," "structure moiety," "chemical moiety," "group," "chemical group" refer to a specific fragment or functional group in a molecule. A chemical moiety is generally considered to be a chemical entity embedded in or attached to a molecule.

When no indication is given in the enumerated substituents as to the atom via which they are attached to a compound included, but not specifically mentioned, in the general formula of the chemical structure, such substituents may be bonded via any of their atoms. Combinations of substituents and/or variants thereof are only allowed if such combinations would yield stable compounds.

In various parts of the present invention, connecting substituents are described. When the structure clearly requires a linking group, the Markush variables listed for that group should be understood as linkting groups. For example, if the structure requires linking groups and the definition of the Markush group for this variable lists "alkyl" or "aryl", it should be understood that the "alkyl" or "aryl" respectively represent a linking alkylidene group or arylidene group.

In some specific structures, when the alkyl group is clearly represented as a linking group, the alkyl group represents a linking alkylidene group. For example, the $C_1$-$C_6$ alkyl in the group "halogenated $C_1$~$C_6$ alkyl" should be understood as $C_1$~$C_6$ alkylene.

The term "alkylene" refers to a saturated divalent hydrocarbon group obtained by removing two hydrogen atoms from a saturated straight or branched chain hydrocarbon group. Examples of alkylene groups include methylene ($-CH_2-$), ethylene {including - $CH_2CH_2$- or -CH ($CH_3$)-}, isopropylidene {including $-CH(CH_3)CH_2-$ or - $C(CH_3)_2$-}, and so on.

Unless otherwise specified, all technical and scientific terms used in this article have the standard meanings of the field that require the protection of the subject matter. If there are multiple definitions for a certain term, the definition herein shall prevail.

It should be understood that the singular form used in the present invention, such as "a", includes plural references, unless otherwise specified. In addition, the term "include" refers to an open-ended expression, i.e., means including what is specified in the present invention, but does not exclude other aspects.

Unless otherwise specified, the present invention adopts traditional methods of mass spectrometry and elemental analysis, and each step and condition can refer to conventional operating steps and conditions in the art.

Unless otherwise specified, the invention adopts standard nomenclature and standard laboratory steps and techniques in analytical chemistry, organic synthetic chemistry and optics. In some cases, standard techniques are used for chemical synthesis, chemical analysis, and performance testing by light-emitting devices.

In addition, it should be noted that unless otherwise explicitly stated, the description mode used in the present invention should be broadly understood as "... independently", which means that the described individuals are independent of each other and can independently represent the same or different specific groups. More specifically, the description "... independently" can refer to either that the specific options expressed between the same symbols in different groups do not affect each other, or that the specific options expressed between the same symbols in the same groups do not affect each other.

It will be understood for those skilled in the art that, according to the conventions used in the art, "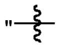" used in the structural formulae describing the moieties in the present application means that the corresponding moiety is connected to other fragments, moieties in the compound through this site.

"Pharmaceutically acceptable" means conditions that can be used to prepare pharmaceutical compositions, which are generally safe and non-toxic, and are not biologically or otherwise undesirable, and include conditions that are acceptable for veterinary use and for pharmaceutical use in humans.

The term "excipient" refers to a pharmaceutically acceptable chemical substance, such as a reagent known to ordinary technicians in the pharmaceutical field to aid in the administration of medicinal substances. It is a compound that can be used to prepare pharmaceutical components, usually safe, non-toxic, and unexpected biologically or in other aspects, and includes excipients that are acceptable for veterinary and human pharmaceuticals. The common excipients include adhesives, surfactants, diluents, disintegrants, and lubricants.

The term "therapeutically effective amount" refers to the amount of the compound used to achieve the treatment of a disease state when given to a subject. The "therapeutically effective amount" will vary according to the compound, the state of the disease being treated, the severity of the disease being treated, the age and relative health of the subject,

the route and mode of administration, and the judgment of the attending medical or veterinary practitioner.

The term 'treatment' or 'in treatment' used in the present patent refers to obtaining beneficial or expected results, including clinical outcomes. Beneficial or expected clinical outcomes include, but are not limited to, the reduction or improvement of one or more disorders or symptoms, reduction in the severity of the disease, the stabilization of disease status (if not worsening), the prevention of disease transmission, the delay or slowing of disease progression, the improvement or remission of a disease status, and partial or total improvement, whether detectable or undetectable. Compared to the expected survival without treatment, the term can also refer to extending survival.

The term mammal refers to humans or any mammal animal, such as primates, farm animals, pet animals, or experimental animals. Examples of these animals include monkeys, cows, sheep, horses, pigs, dogs, cats, rabbits, mice, and rats. Mammals are preferred to humans.

On the basis of not violating common knowledge in the field, the above preferred conditions can be arbitrarily combined to obtain each preferred example of the present invention.

The reagents and raw materials used in the present invention are commercially available.

The positive progressive effect of the present invention is that the present invention provides a dihydropyrimidine compound, which has better anti-HBV (Hepatitis B virus) activity, as well as lower cytotoxicity, and can be used in the preparation of drugs for the treatment and prevention of HBV infection.

## Embodiment of the present invention

The present invention is further explained through embodiments, but it is not limited to the scope of the embodiments. The experimental methods without specific conditions specified in the following embodiments shall be selected according to conventional methods and conditions, or according to the products' instructions.

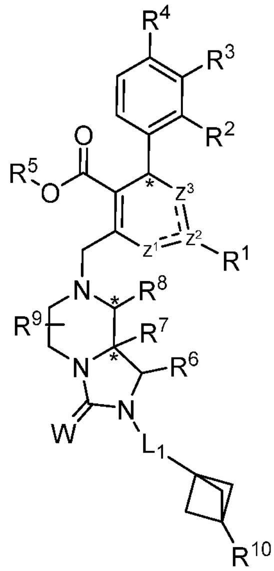

### General synthesis route 1:

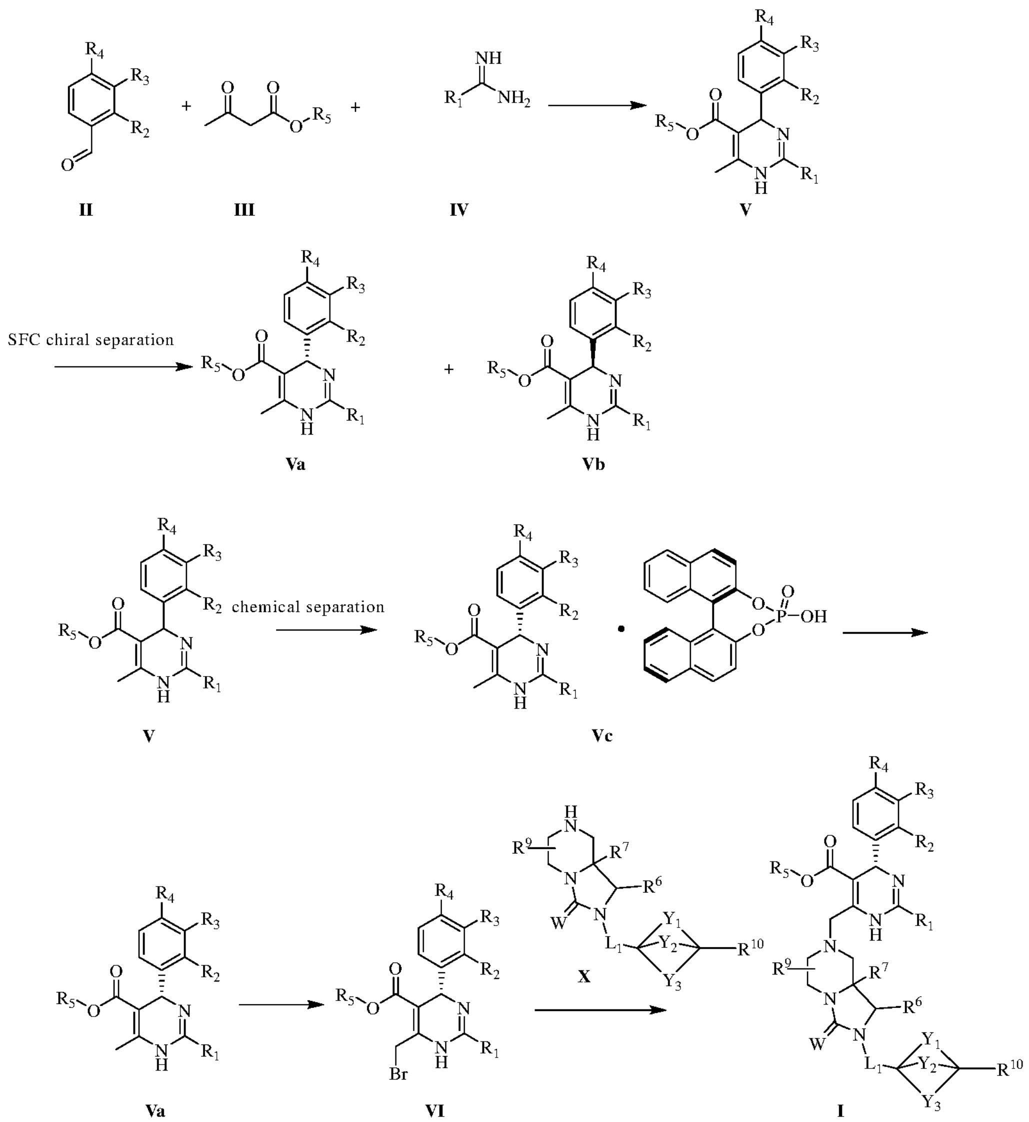


II III IV V

Va Vb

V Vc

Va VI X I

The target **compound** I can be prepared by general synthesis route. **Compound II, Compound III and Compound IV** are prepared by a one-pot reaction under base conditions to obtain **Compound V. Compound V** can be separated by chromatography to obtain **Compound Va** or separated by chemical resolution to obtain **Compound Va,** which undergoes bromination to obtain **Compound VI,** and **Compound VI** reacts with **Compound X** or a salt thereof to obtain the target **Compound I.**

**General synthesis route 2:**

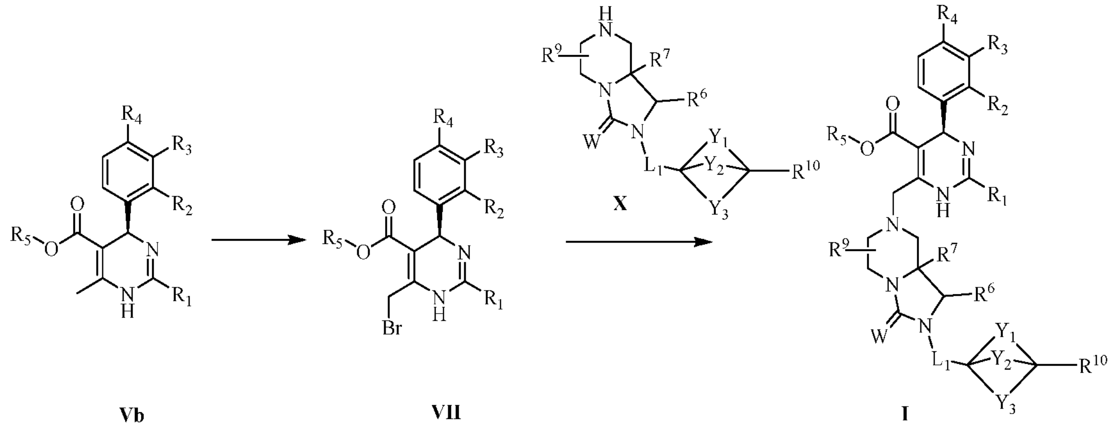

Vb VII I

The target **compound I** can be prepared through general synthesis route 2. **Compound Vb** undergoes bromination reaction to obtain **compound VII,** which reacts with **compound X** or salt thereof to obtain target **compound I.**

**General synthesis route 3:**

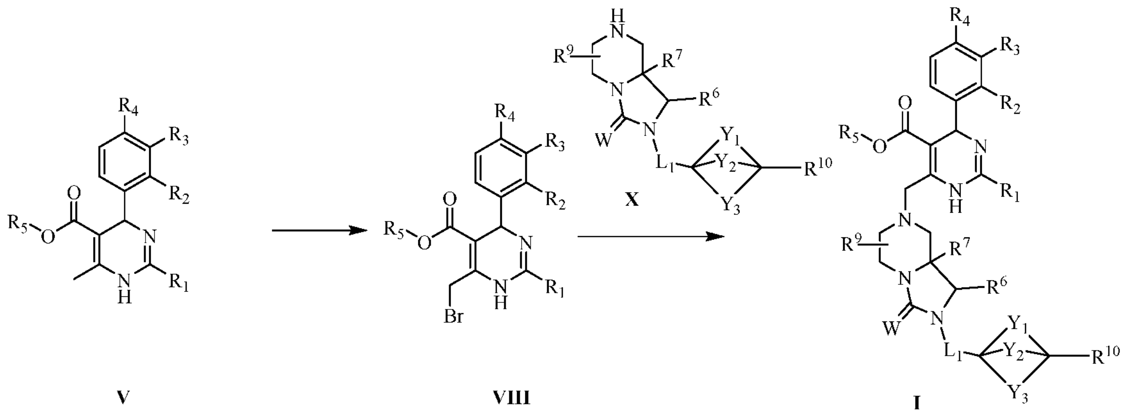

V VIII I

The target **compound I** can be prepared through general synthesis route 3. **Compound V** undergoes bromination reaction to obtain **compound VIII,** which reacts with compound X or salt thereof to obtain target **compound I.** or,

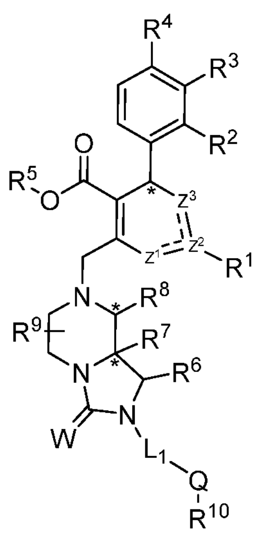

I’

**General synthesis route 1’:**

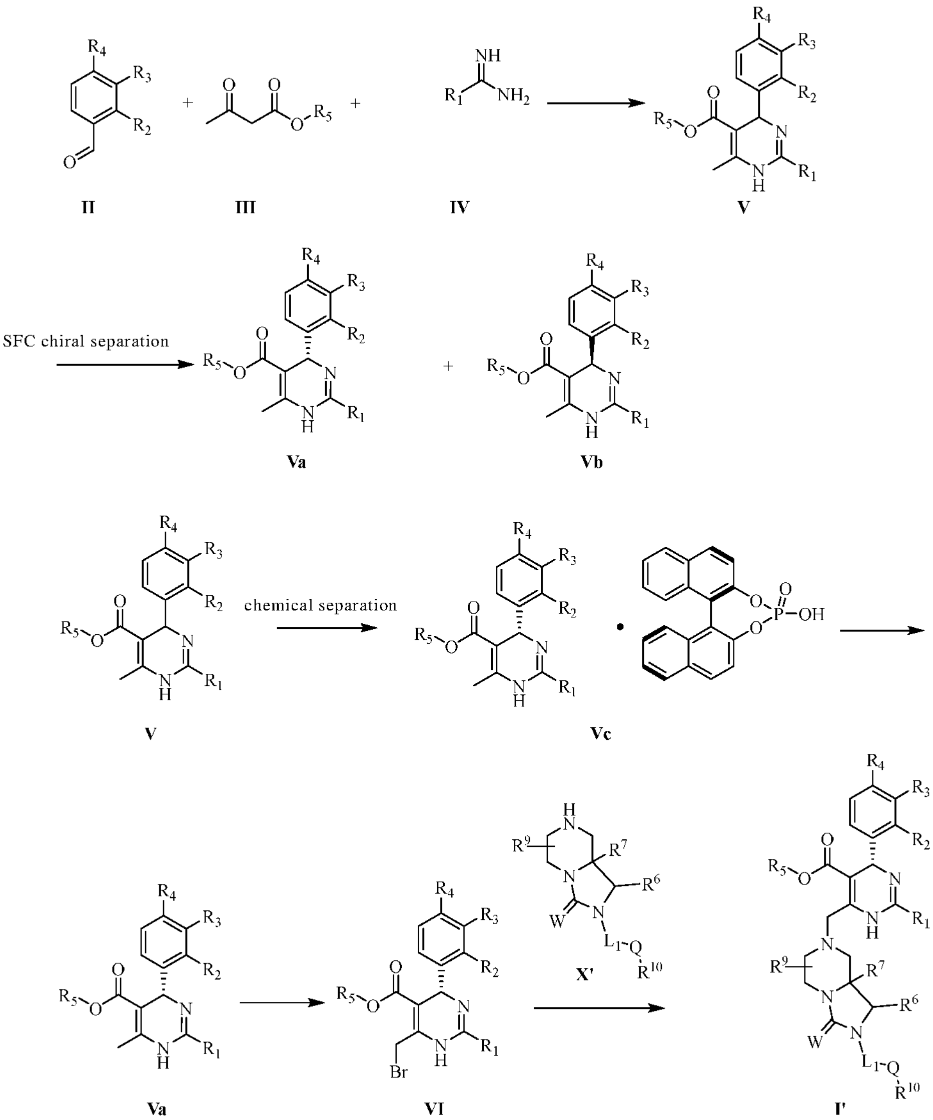


II III IV V

Va Vb

V Vc

Va VI X' I'

The target **compound I** can be prepared through general synthesis route. **Compound II, compound III,** and **compound IV** are prepared by a one pot reaction under base conditions to obtain **compound V. Compound V** can be separated by chromatography to obtain **compound Va** or separated by chemical resolution to obtain **compound Va. Compound Va** undergoes bromination reaction to obtain **compound VI. Compound VI** reacts with **compound X**’or salt thereof to obtain target **compound I’.**

**General synthesis route 2’:**

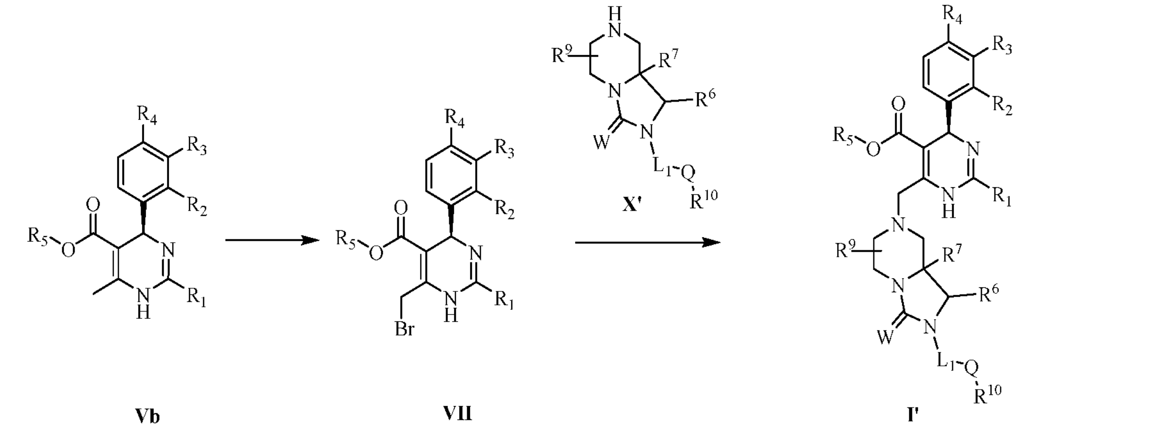

Vb VII I'

The target **compound I** can be prepared through general synthesis route 2. **Compound Vb** undergoes bromination reaction to obtain **compound VII**, which reacts with **compound X**' or salt thereof to obtain target **compound I'**.

**General synthesis route 3':**

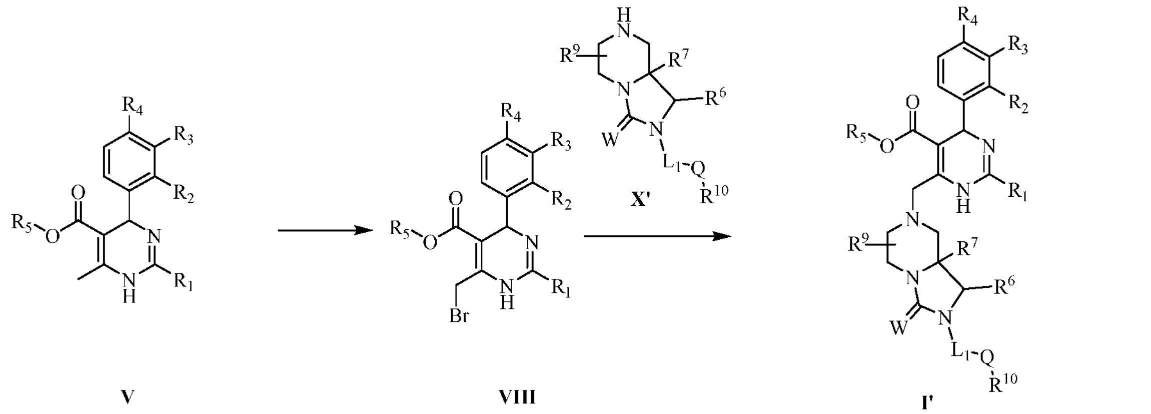

V VIII I'

The target **compound I** can be prepared through general synthesis route 3. **Compound V** undergoes a bromination reaction to obtain **compound VIII,** which reacts with **compound X'** or salt thereof to obtain the target **compound I'.**

In the following embodiments, the structure of the compound is determined by nuclear magnetic resonance (NMR) or/and mass spectrometry (MS). NMR shift(8) are given in units of 10-6 (ppm). The determination of NMR is carried out with Bruker AVANCE-400 nuclear magnetic spectrometer. The solvents for determination are Deuterated DMSO (DMSO-$d_6$), Deuterated chloroform ($CDCl_3$), Deuterated methanol ($CD_3OD$), and the internal standard is Tetramethylsilane (TMS).

SHIMADZU LC system (column: Xselect® CSHTM Prep-C18, 19 * 150 mm, liquid handler LH-40, pump LC-20AP, detector SPD-20A, system controller CBM-20A, solvent system: acetonitrile and 0.05% aqueous solution of trifluoroacetic acid).

LC/MS spectra of the compounds are obtained using LC/MS (Agilent Technologies 1200 Series).The LC/MS conditions are as follows (run time 10 min):

Acidic condition: A: 0.05% aqueous solution of trifluoroacetic acid; B: 0.05% acetonitrile solution of trifluoroacetic acid;
Basic condition: A: 0.05% aqueous solution of $NH_3$-$H_2O$; B: acetonitrile
Neutral condition: A: 10 mM aqueous solution of $NH_4OAC$; B: acetonitrile
Unless otherwise specified, in the following Examples, the intermediates and final compounds were purified using silica gel column chromatography, or purified using an Xselecto,R CSH™ Prep-C18 (5μm, OBD™ 19 * 150mm) column or using XBridgeTM Prep Phenyl (5μm, OBD™ 30 * 100mm) on a reversed-phase chromatography column by preparative HPLC.

The silica gel column chromatography generally uses Yantai Huanghai silica gel 200-300 mesh silica gel as the carrier.

The CombiFlash rapid preparator uses Combiflash Rf200 (TELEDYNE ISCO).

The silica gel plate for Thin-layer chromatography (TLC) uses Yantai Huanghai HSGF254 or Qingdao GF254 silica gel plate. The specification of silica gel plate used for thin layer chromatography detection products is 0.15mm-0.2mm, and the specification of thin layer chromatography for separation and purification of products is 0.4mm~0.5mm.

The known starting materials of the present invention can be synthesized using or according to methods known in the art or can be purchased from companies such as ABCR GmbH & Co.KG, Acros Organics, Aldrich Chemical Company, Accela ChemBio Inc, Darui Chemicals, etc.

**Abbreviations:**

AcOH: Acetic acid; ACN: Acetonitrile; AcCl: Acetyl chloride; $BH_3$Borane; BoczO: tert-butyldicarbonate; Boc: t-Butyloxy carbonyl; $BF_3Et_2O$: Boron (tri) fluoride etherate;Cbz: benzyloxycarbonyl; CbzCl: benzyl chloroformate;$CBr_4$: Carbon tetrabromide; $CH_3I$ (MeI): Iodomethane; con. HCl: con. hydrochloric acid; $Cs_2CO_3$: Caesium carbonate; CuI: Copper(I) iodate;DCM: dichloromethane; DIEA: N,N-diisopropylethylamine; Dioxane: 1,4-Dioxane; DMAP: 4-Dimethylaminopyridine; DMA: N,N-dimethylacetamide; DMF: N,N-dimethylformamide; DMSO: dimethyl sulfoxide; EtOH: Ethanol; $H_2$: hydrogen; HATU: 2-(7-Azabenzotriazol-1-yl)-N,N,N',N'-tetramethyluronium hexafluorophosphate; $K_2CO_3$: Potassium carbonate; $LiAlH_4$: Lithium Aluminum Hydride; LiHMDS: Lithium bis(trimethylsilyl)amide ; LiOH : Lithium hydroxide ; LDA: Lithium diisopropylamide ;MeOH: methanol; MsCl: Methanesulfonyl chloride; MW: Microwave; $NaCNBH_3$: Sodium cyanoborohydride; NaH : Sodium hydride ; NaHCOs : sodium bicarbonate; NaOH: Sodium hydroxide; $NaN_3$: Sodium azide ; NaOMe: Sodium methoxide;$Pd(OAc)_2$: Palladium( II )acetate; Pd/C: Palladium 10% on Carbon; $Pd_2(dba)_3$: Tris(dibenzylideneacetone)dipalladium; $PPh_3$: triphenylphosphine; t-BuOK: Potassium t-butoxide; TEA($Et_3N$): triethylamine; TFA: trifluoroacetic acid; TFAA: Trifluoroacetic anhydride; TMSCN: Trimethylsilyl Cyanide;THF: Tetrahydrofuran; TLC: thin layer chromatography.

**Intermediate A1**

**ethyl (S)-6-(bromomethyl)-4-(3-fluoro-2-methylphenyl)-2-(thiazol-2-yl)-1,4-dihydropyrimidine-5-carboxylate**

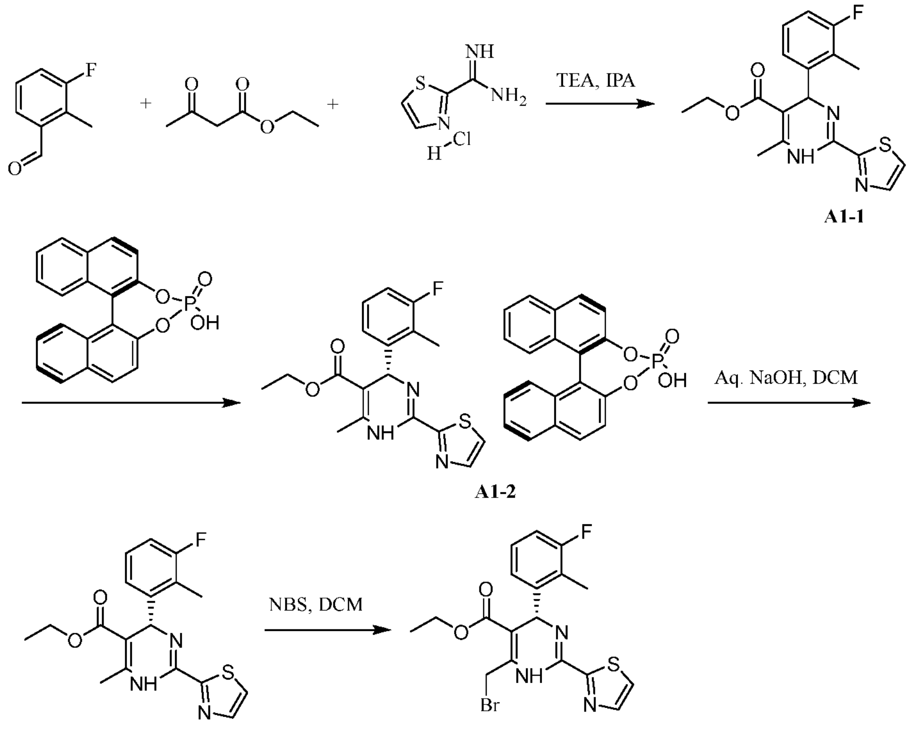


A1-1

A1-2

A1-1a

A1

**Step 1: Preparation of Intermediate A1-1**

To a stirred mixture solution of 3-fluoro-2-methyl benzaldehyde (77.0 g, 0.56 mol) and ethyl 3-oxobutanoate (113.2 g, 0.87 mol) in IPA (770 mL) was added piperidine (4.75 g, 0.056 mol) and acetic acid (3.7 g, 0.06 mol) at 20-30°C, then the resulting mixture was stirred at 20 °C for 4 hours. Then thiazole-2-carboxamidine hydrochloride (104.9 g, 78 wt%, 0.49 mol) and triethylamine (72.8 g, 0.72 mol) was added to the above mixture solution over 50 mins. The resulting reaction mixture was stirred at 25-30°C for another 12 hours and then the mixture was stirred at 70-75°C for 8 hours. After the reaction was completed, the reaction mixture was cooled to 30°C over 4 hours and water (770 mL) was added to the reaction mixture over 50 mins. The suspension was stirred at 20-30°C for another 10 hours and the solid was collected by filtration, washed with IPA/water (v/v=1:1, 80 mL) and water (80 mL). The wet cake was dried in a vacuum to afford ethyl 4-(3-fluro-2-methyl-phenyl)-6-methyl-2-thiazol-2-yl-1,4-dihydropyrimidine-5-carboxylate (**Intermediate A1-1**, 160 g, 80%) as a yellow solid.

**Step 2: Preparation of Intermediate A1-2**

To a stirred mixture solution of 4-(3-fluro-2-methyl-phenyl)-6-methyl-2-thiazol-2-yl-1,4-dihydropyrimidine-5-carboxylate (**Intermediate A1-1**, 180 g, 0.5 mol) in ethanol (1.8 L) was added (R)-(-)-1,1'-binaphthyl-2,2'-diyl hydrogen phosphate (174.5 g, 0.5 mol) at 75°C. Then the resulting mixture was stirred at 75 °C for 4 hours. After the reaction was completed, the reaction mixture was cooled to 30°C over 4 hours, and then filtered. The collected solid was washed with ethanol (180 mL) and then charged back to a 5 L reactor. DCM (1.8 L) was added and the suspension was heated to reflux. To the suspension was added ethanol (0.9 L) over 0.5 hour and the agitation was maintained for 0.5 hour untill all solid was dissolved. Additional ethanol (0.9 L) was added over 0.5 hour at 40°C. DCM was distilled off at 38-60°C, the resulting mixture was stirred at 60°C for 1 hour and then cooled to 30°C over 8 hours. The chiral salt was collected by filtration and washed with ethanol (180 mL). The wet cake was dried in a vacuum oven for 24 hours to afford ethyl (S)-4-(3-fluoro-2-methylphenyl)-6-methyl-2-(thiazol-2-yl)-1,4-dihydropyrimidine-5-carboxylate mono (R)-(-)-1,1'-Binaphthyl-2,2'-diyl hydrogenphosphate salt (**Intermediate A1-2**,160 g, 45%) as a light yellow solid.$^1$H NMR (DMSO-$d_6$, 400 MHz) δ 8.02-8.11 (m, 6H), 7.42-7.52 (m, 4H), 7.32-7.36 (m, 2H), 7.16-7.22 (m, 3H), 7.02-7.10 (m, 2H), 5.83 (s, 1H), 3.95 (q, *J* = 7.2 Hz, 2H), 2.45 (s, 3H), 2.41 (s, 2H), 1.02 (t, *J* = 7.2 Hz, 3 H).

**Step 3: Preparation of Intermediate A1-1a**

To a stirred suspension of ethyl (S)-4-(3-fluoro-2-methylphenyl)-6-methyl-2-(thiazol-2-yl)-1,4-dihydropyrimidine-5-carboxylate mono (R)-(-)-1,1'-Binaphthyl-2,2'-diyl hydrogenphosphate salt (**Intermediate A1-2**, 22.5g, 31.8mmol)in DCM (225 mL) was added 20% NaOH solution (6.36g, 31.8mmol) at 25 °C, then the resulting mixture was stirred at 25 °C for 1 hour. After the reaction was completed, the reaction mixture was filtered and washed with DCM. The combined organic layers were washed with brine, dried over anhydrous $Na_2SO_4$, filtered and concentrated in vacuo to afford **Intermediate A1-1a** as a yellow solid.

**Step 4: Preparation of Intermediate A1**

To a stirred suspension of (S)-4-(3-fluoro-2-methylphenyl)-6-methyl-2-(thiazol-2-yl)-1,4-dihydropyrimidine-5-carboxylate (**Intermediate A1-1a**, 11.39 g, 31.7 mmol) in DCM (115 mL) was added NBS (5.92g, 33.3mmol) at 42 °C, then the resulting mixture was stirred at 42 °C for 0.5 hour. After the reaction was completed, the reaction mixture was concentrated in vacuo to afford **Intermediate A1.**

**Intermediate A2 and Intermediate A2-b**

**ethyl (R)-6-(bromomethyl)-4-(2-chloro-3-fluorophenyl)-2-(thiazol-2-yl)-1,4-dihydropyrimidine-5-carboxylate (Intermediate A2)**

**ethyl (S)-6-(bromomethyl)-4-(2-chloro-3-fluorophenyl)-2-(thiazol-2-yl)-1,4-dihydropyrimidine-5-carboxylate (Intermediate A2-b)**

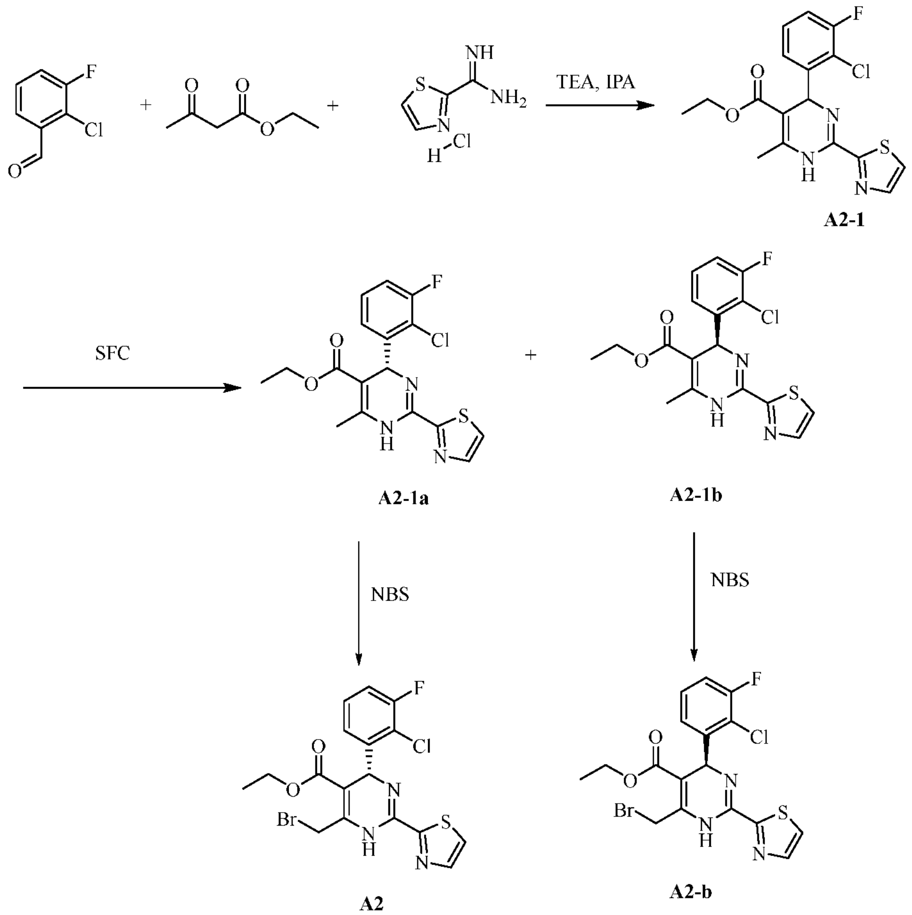


A2-1

A2-1a A2-1b

A2 A2-b

The title compound **Intermediate A2-1** as a yellow solid was prepared in analogy to the preparation of **Intermediate A1-1** by using2-chloro-3-fluorobenzaldehyde instead of 3-fluoro-2-methylbenzaldehyde.

**Intermediate A2-1** was separated by SFC chiral separation (CHIRALPAK-AD-H, 5 cm x 25 cm, 5μm column, 20% EtOH in $CO_2$) to afford the faster peak **Intermediate A2** - **1a** ($t_R$ = 1.08 mins) and the solwer peak **Intermediate**

**A2 -1b** ($t_R$ = 1.28 mins) both as yellow solids. MS: 380.2 $(M+H)^+$.

The title compound **Intermediate A2** was prepared in analogy to the preparation of **Intermediate A1** by using **Intermediate A2-1a** instead of **Intermediate A1-1a.**

The title compound **Intermediate A2-b** was prepared in analogy to the preparation of **Intermediate A1** by using **Intermediate A2-1b** instead of **Intermediate A1-1a.**

**Intermediate A3**

**ethyl (R)-6-(bromomethyl)-4-(2-chloro-4-fluorophenyl)-2-(thiazol-2-yl)-1,4-dihydropyrimidine-5-carboxylate**

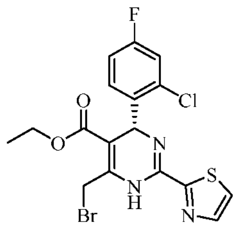

**A3**

The title compound **Intermediate A3** as a yellow solid was prepared in analogy to the preparation of **Intermediate A1** by using2-chloro-4-fluorobenzaldehyde instead of 3-fluoro-2-methylbenzaldehyde.

**Intermediate A4**

**ethyl (S)-6-(bromomethyl)-4-(3,4-difluorophenyl)-2-(thiazol-2-yl)-1,4-dihydropyrimidine-5-carboxylate**

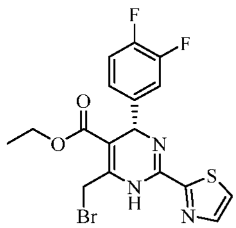

**A4**

The title compound **Intermediate A4** as a yellow solid was prepared in analogy to the preparation of **Intermediate A1** by using3,4-difluorobenzaldehyde instead of 3-fluoro-2-methylbenzaldehyde.

**Intermediate A5**

**ethyl (S)-6-(bromomethyl)-4-(4-fluoro-2-methylphenyl)-2-(thiazol-2-yl)-1,4-dihydropyrimidine-5-carboxylate**

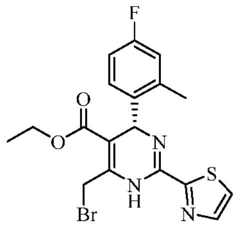

A5

The title compound **Intermediate A5** as a yellow solid was prepared in analogy to the preparation of **Intermediate A1** by using4-fluoro-2-methylbenzaldehyde instead of 3-fluoro-2-methylbenzaldehyde.

**Intermediate A6**

**methyl (R)-6-(bromomethyl)-4-(2-chloro-4-fluorophenyl)-2-(thiazol-2-yl)-1,4-dihydropyrimidine-5-carboxylate**

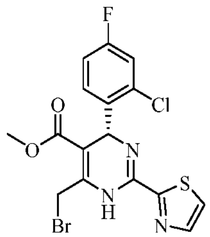

A6

The title compound **Intermediate A6** as a yellow solid was prepared in analogy to the preparation of **Intermediate A1** by usingmethyl 3-oxobutanoate instead of ethyl 3-oxobutanoate and 2-chloro-4-fluorobenzaldehyde instead of 3-fluoro-2-methylbenzaldehyde.

**Intermediate A7**

**methyl (R)-4-(2-bromo-4-fluorophenyl)-6-(bromomethyl)-2-(thiazol-2-yl)-1,4-dihydropyrimidine-5-carboxylate**

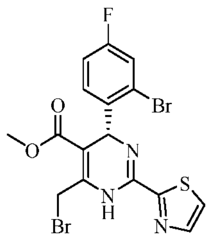

A7

The title compound **Intermediate A7** as a yellow solid was prepared in analogy to the preparation of **Intermediate A1** by usingmethyl 3-oxobutanoate instead of ethyl 3-oxobutanoate and 2-bromo-4-fluorobenzaldehyde instead of 3-fluoro-2-methylbenzaldehyde.

**Intermediate A8**

**methyl (S)-6-(bromomethyl)-4-(3,4-difluoro-2-methylphenyl)-2-(thiazol-2-yl)-1,4-dihydropyrimidine-5-carboxylate**

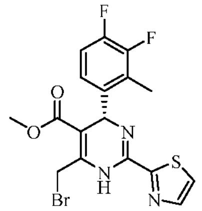

**A8**

The title compound **Intermediate A8** as a yellow solid was prepared in analogy to the preparation of **Intermediate A1** by usingmethyl 3-oxobutanoate instead of ethyl 3-oxobutanoate and 3,4-difluoro-2-methylbenzaldehyde instead of 3-fluoro-2-methylbenzaldehyde.

**Intermediate 1-5**

**(S)-3-(3-oxohexahydroimidazo[1,5-a]pyrazin-2(3H)-yl)bicyclo[1.1.1]pentane-1-carboxylic acid**

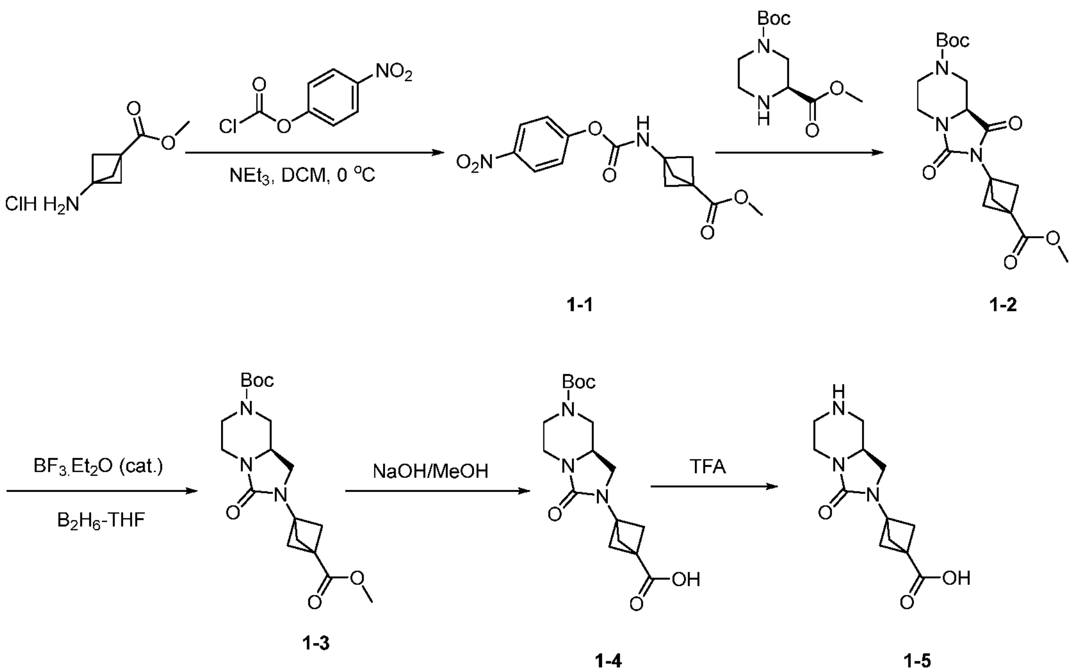


**1-1** **1-2** **1-3** **1-4** **1-5**

**Step 1: Preparation of Intermediate 1-2**

To a stirred mixture solution of methyl 3-aminobicyclo[1.1.1]pentane-1-carboxylate hydrochloride (178mg, 1.0 mmol) in DCM (30 mL) was added TEA(304 mg, 3.0 mmol) and 4-nitrophenyl carbonochloridate (222 mg, 1.1 mmol) at 0 °C, then the resulting mixture was stirred at 0 °C for 1 hour. After the reaction was completed, 1-(tert-butyl) 3-methyl (S)-piperazine-1,3-dicarboxylatethe (244 mg, 1.0 mmol) was added to the above mixture solution at 20 °C and the resulting mixture was stirred at 20 °C for 12 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography

to affordtert-butyl (S)-2-(3-(methoxycarbonyl)bicyclo[1.1.1]pentan-1-yl)-1,3-dioxohexahydroimidazo[1,5-a]pyrazine-7(1H)-carboxylate (**Intermediate 1-2**,136.9 mg, 36.1%) as a white solid. MS: 781.4 $(2M+Na)^+$. $^1$H-NMR ($CDCl_3$, 400 MHz) $\delta$ 4.48 (brs, 1H), 4.12 (brs, 1H), 4.04-4.00 (m, 1H), 3.89-3.84 (m, 1H), 3.69 (s, 3H), 2.96 brs, 1H), 2.76-2.66 (m, 2H), 2.59 (s, 6H), 1.47 (s, 9H).

**Step 2: Preparation of Intermediate 1-3**

To a stirred mixture solution of tert-butyl (S)-2-(3-(methoxycarbonyl)bicyclo[1.1.1]pentan-1-yl)-1,3-dioxohexahydroimidazo[1,5-a]pyrazine-7(1H)-carboxylate (**Intermediate 1-2**,142 mg, 0.374 mmol) in THF (5 mL) was added 3 drops of $BF_3{\cdot}Et_2O$ and 1.0 M $BH_3$-THF solution (0.75 mL, 0.75 mmol) at 0 °C, then the resulting mixture was stirred at 0 °C for 1 hour. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to affordtert-butyl (R)-2-(3-(methoxycarbonyl)bicyclo[1.1.1]pentan-1-yl)-3-oxohexahydroimidazo[1,5-a]pyrazine-7(1H)-carboxylate (**Intermediate 1-3**, 90 mg, 65.7%) as a white solid. MS: 366.2 $(M+H)^+$.

**Step 3: Preparation of Intermediate 1-4**

To a stirred mixture solution of tert-butyl (R)-2-(3-(methoxycarbonyl)bicyclo[1.1.1]pentan-1-yl)-3-oxohexahydroimidazo[1,5-a]pyrazine-7(1H)-carboxylate (**Intermediate 1-3**, 135 mg, 0.37 mmol) in MeOH (5 mL) was added 1N NaOH solution (0.74 mL, 0.74 mmol) at 0 °C, then the resulting mixture was stirred at 20 °C for 12 hours. After the reaction was completed, the reaction mixture was poured into ice-water and extracted with EA. The combined organic layers were washed with 1N HCl solution, brine, dried over anhydrous $Na_2SO_4$, filtered and concentrated in vacuo to give a crude product, which was purified by silica gel flash chromatography to afford(R)-3-(7-(tert-butoxycarbonyl)-3-oxohexahydroimidazo[1,5-a]pyrazin-2(3H)-yl)bicyclo[1.1.1]pentane-1-carboxylic acid (**Intermediate 1-4**, 123 mg, 95%) as a white solid. MS: 725.4 $(2M+Na)^+$.

**Step 4: Preparation of Intermediate 1-5**

A mixture solution of (R)-3-(7-(tert-butoxycarbonyl)-3-oxohexahydroimidazo[1,5-a]pyrazin-2(3H)-yl)bicyclo[1.1.1]pentane-1-carboxylic acid (**Intermediate 1-4**, 119 mg, 0.339 mmol) in DCM (7 mL) and TFA (1 mL) was stirred at 20 °C for 5 hours. After the reaction was completed, the reaction mixture was concentrated in vacuo to afford (S)-3-(3-oxohexahydroimidazo[1,5-a]pyrazin-2(3H)-yl)bicyclo[1.1.1]pentane-1-carboxylic acid (**Intermediate 1-5**, 85 mg) as a yellow oil. MS: 252.1 $(M+H)^+$.

**Intermediate 2-2**

**(S)-2-(3-(hydroxymethyl)bicyclo[1.1.1]pentan-1-yl)hexahydroimidazo[1,5-a]pyrazin-3(2H)-one**

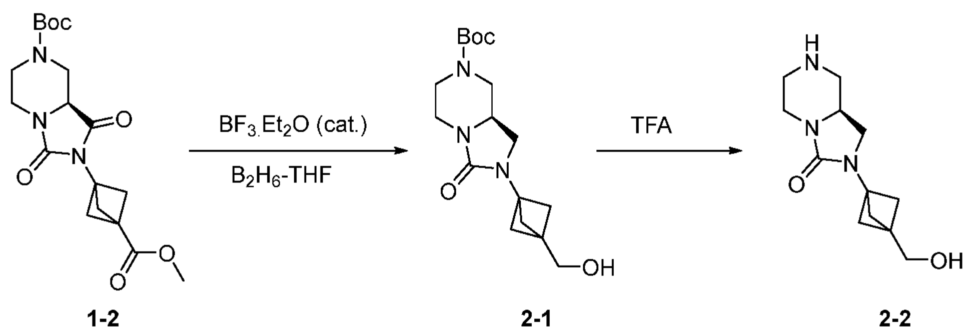


**1-2** **2-1** **2-2**

**Step 1: Preparation of Intermediate 2-1**

To a stirred mixture solution of tert-butyl (S)-2-(3-(methoxycarbonyl)bicyclo[1.1.1]pentan-1-yl)-1,3-dioxohexahydroimidazo[1,5-a]pyrazine-7(1H)-carboxylate (**Intermediate 1-2**,284 mg, 0.748 mmol) in THF (15 mL) was added 3 drops of $BF_3{\cdot}Et_2O$ and 1.0 M $BH_3$-THF solution (3 mL, 3 mmol) at 0 °C, then the resulting mixture was stirred at 0 °C

for 12 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to affordtert-butyl (R)-2-(3-(hydroxymethyl)bicyclo[1.1.1]pentan-1-yl)-3-oxohexahydroimidazo[1,5-a]pyrazine-7(1H)-carboxylate (**Intermediate 2-1**, 180 mg, 71.4%) as a white solid. MS: 338.2 $(M+H)^+$.

**Step 2: Preparation of Intermediate 2-2**

The title compound **Intermediate 2-2** was prepared in analogy to the preparation of **Intermediate 1-5** by usingtert-butyl (R)-2-(3-(hydroxymethyl)bicyclo[1.1.1]pentan-1-yl)-3-oxohexahydroimidazo[1,5-a]pyrazine-7(1H)-carboxylate (**Intermediate 2-1**) instead of (R)-3-(7-(tert-butoxycarbonyl)-3-oxohexahydroimidazo[1,5-a]pyrazin-2(3H)-yl)bicyclo[1.1.1]pentane-1-carboxylic acid (**Intermediate 1-4**). MS: 238.1 $(M+H)^+$.

**Intermediate 3-2**

**(S)-2-(3-(methoxymethyl)bicyclo[1.1.1]pentan-1-yl)hexahydroimidazo[1,5-a]pyrazin-3(2H)-one**

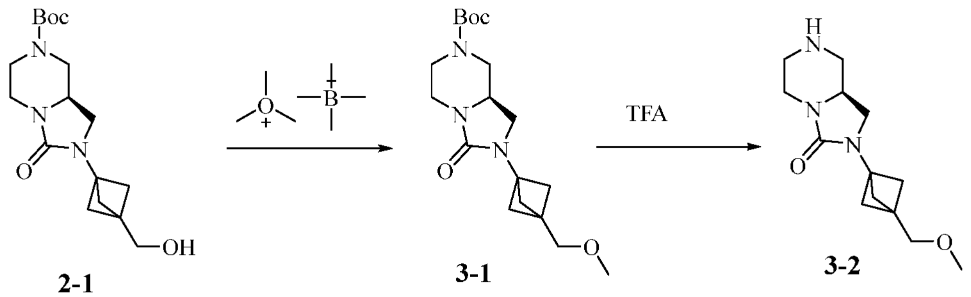


2-1 3-1 3-2

**Step 1: Preparation of Intermediate 3-1**

A mixture solution of tert-butyl (R)-2-(3-(hydroxymethyl)bicyclo[1.1.1]pentan-1-yl)-3-oxohexahydroimidazo[1,5-a]pyrazine-7(1H)-carboxylate (**Intermediate 2-1**, 50 mg, 0.148 mmol), trimethyloxonium tetramethylborate (23.48 mg, 0.178 mmol) and $K_2CO_3$ (61.4 mg, 0.445 mmol) in ACN (2 mL) was stirred at 40 °C for 3 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to affordtert-butyl (R)-2-(3-(methoxymethyl)bicyclo[1.1.1]pentan-1-yl)-3-oxohexahydroimidazo[1,5-a]pyrazine-7(1H)-carboxylate (**Intermediate 3-1**, 20 mg, 38%) as a white solid. MS: 352.2 $(M+H)^+$.

**Step 2: Preparation of Intermediate 3-2**

The title compound **Intermediate 3-2** was prepared in analogy to the preparation of **Intermediate 1-5** by usingtert-butyl (R)-2-(3-(methoxymethyl)bicyclo[1.1.1]pentan-1-yl)-3-oxohexahydroimidazo[1,5-a]pyrazine-7(1H)-carboxylate (**Intermediate 3-1**) instead of (R)-3-(7-(tert-butoxycarbonyl)-3-oxohexahydroimidazo[1,5-a]pyrazin-2(3H)-yl)bicyclo[1.1.1]pentane-1-carboxylic acid (**Intermediate 1-4**). MS: 252.1 $(M+H)^+$.

**Intermediate 4-4**

**(S)-2,2-dimethyl-3-(3-(3-oxohexahydroimidazo[1,5-a]pyrazin-2(3H)-yl)bicyclo[1.1.1]pentan-1-yl)propanoic acid**

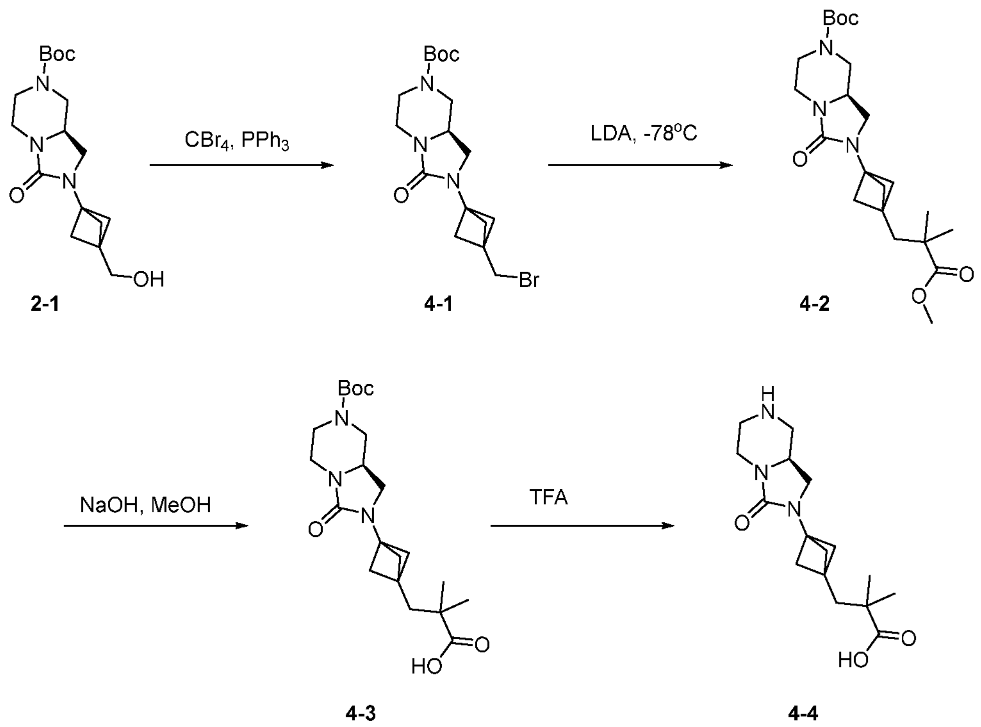


2-1 4-1 4-2 4-3 4-4

**Step 1: Preparation of Intermediate 4-1**

To a stirred mixture solution of tert-butyl (R)-2-(3-(hydroxymethyl)bicyclo[1.1.1]pentan-1-yl)-3-oxohexahydroimidazo[1,5-a]pyrazine-7(1H)-carboxylate (**Intermediate 2-1**, 337 mg, 1.0 mmol) in DCM (5 mL) was added $PPh_3$ (445 mg, 1.698 mmol) and $CBr_4$ (563 mg, 1.698 mmol) at 0 °C, then the resulting mixture was stirred at 20 °C for 1 hour. After the reaction was completed, the reaction mixture was quenched by adding ice-water and extracted with EA. The combined organic layers were washed with brine, dried over anhydrous $Na_2SO_4$, filtered and concentrated in vacuo to give a crude product, which was purified by silica gel flash chromatography to afford tert-butyl (R)-2-(3-(bromomethyl)bicyclo[1.1.1]pentan-1-yl)-3-oxohexahydroimidazo[1,5-a]pyrazine-7(1H)-carboxylate(**Intermediate 4-1**, 310 mg, 78%) as a yellow oil. MS: 400.1 $(M+H)^+$.

**Step 2: Preparation of Intermediate 4-2**

To a stirred mixture solution of ethyl isobutyrate (116 mg, 1.0 mmol) in THF (5 mL) was added 2.0 M LDA THF solution (1 mmol) at -78 °C and stirred at -78 °C for 0.5 hour, a solution of tert-butyl (R)-2-(3-(bromomethyl)bicyclo[1.1.1]pentan-1-yl)-3-oxohexahydroimidazo[1,5-a]pyrazine-7(1H)-carboxylate(**Intermediate 4-1**, 310 mg, 0.775 mmol) in THF (5 mL) was added to the above mixture at -78 °C, then the resulting mixture was stirred at -78 °C for 2 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to affordtert-butyl (R)-2-(3-(3-methoxy-2,2-dimethyl-3-oxopropyl)bicyclo[1.1.1]pentan-1-yl)-3-oxohexahydroimidazo[1,5-a]pyrazine-7(1H)-carboxylate (**Intermediate 4-2**,50 mg, 15%) as a white solid. MS: 422.5 $(M+H)^+$.

**Step 3: Preparation of Intermediate 4-4**

The title compound **Intermediate 4-4** was prepared in analogy to the preparation of **Intermediate 1-5** by usingtert-butyl (R)-2-(3-(3-methoxy-2,2-dimethyl-3-oxopropyl)bicyclo[1.1.1]pentan-1-yl)-3-oxohexahydroimidazo[1,5-a]pyrazine-7(1H)-carboxylate (**Intermediate 4-2**) instead of tert-butyl (R)-2-(3-(methoxycarbonyl)bicyclo[1.1.1]pentan-1-yl)-3-oxohexahydroimidazo[1,5-a]pyrazine-7(1H)-carboxylate (**Intermediate 1-3**). MS: 308.1 $(M+H)^+$.

**Intermediate 5-3**

**(S)-3-((3-oxohexahydroimidazo[1,5-a]pyrazin-2(3H)-yl)methyl)bicyclo[1.1.1]pentane-1-carboxylic acid**

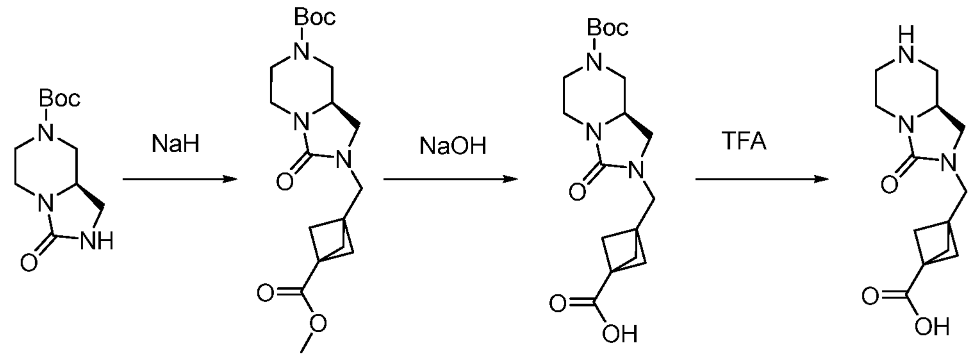


**5-1** **5-2** **5-3**

**Step 1: Preparation of Intermediate 5-1**

To a stirred mixture solution of tert-butyl (R)-3-oxohexahydroimidazo[1,5-a]pyrazine-7(1H)-carboxylate (200 mg, 0.833 mmol) in THF (5 mL) was added NaH (64 mg, 1.66 mmol) at 0 °C and the resulting mixture was stirred at 0 °C for 1 hour. Then methyl 3-(bromomethyl)bicyclo[1.1.1]pentane-1-carboxylate (273 mg, 1.249 mmol) was added to the above mixture solution at 0 °C and the resulting mixture was stirred at 20 °C for 15 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford tert-butyl (R)-2-((3-(methoxycarbonyl)bicyclo[1.1.1]pentan-1-yl)methyl)-3-oxohexahydroimidazo[1,5-a]pyrazine-7(1H)-carboxylate (**Intermediate 5-1**,100 mg, 32%) as a colorless oil. MS: 380.2 $(M+H)^+$.

**Step 2: Preparation of Intermediate 5-3**

The title compound **Intermediate 5-3** was prepared in analogy to the preparation of **Intermediate 1-5** by usingtert-butyl (R)-2-((3-(methoxycarbonyl)bicyclo[1.1.1]pentan-1-yl)methyl)-3-oxohexahydroimidazo[1,5-a]pyrazine-7(1H)-carboxylate (**Intermediate 5-1**) instead of tert-butyl (R)-2-(3-(methoxycarbonyl)bicyclo[1.1.1]pentan-1-yl)-3-oxohexahydroimidazo[1,5-a]pyrazine-7(1H)-carboxylate (**Intermediate 1-3**). MS: 266.1 $(M+H)^+$.

**Intermediate 26-2**

**(S)-N-methyl-3-(3-oxohexahydroimidazo[1,5-a]pyrazin-2(3H)-yl)bicyclo[1.1.1]pentane-1-carboxamide**

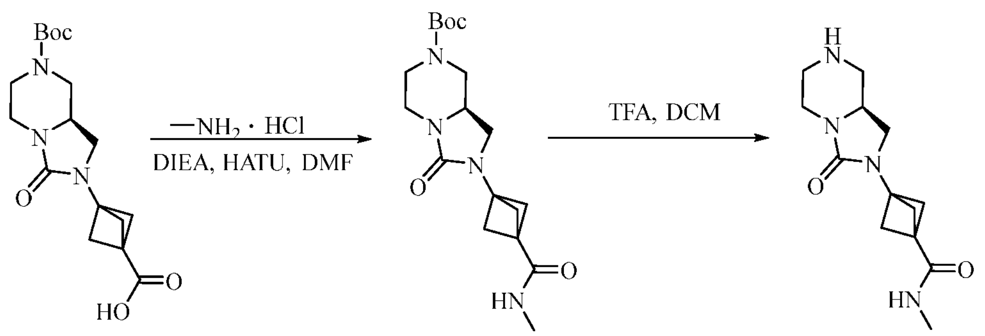


**1-4** **26-1** **26-2**

**Step 1: Preparation of Intermediate 26-1**

To a stirred mixture solution of (R)-3-(7-(tert-butoxycarbonyl)-3-oxohexahydroimidazo[1,5-a]pyrazin-2(3H)-yl)bicyclo[1.1.1]pentane-1-carboxylic acid **(Intermediate 1-4,** 50 mg, 0.142 mmol) in DMF (2 mL) was added methylamine hydrochloride (28.8 mg, 0.427 mmol), DIEA (55.1 mg, 0.427 mmol) and HATU (81 mg, 0.213 mmol) at 20 °C and the resulting mixture was stirred at 20 °C for 5 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford tert-butyl (R)-2-(3-(methylcarbamoyl)bicyclo[1.1.1]pentan-1-yl)-3-oxohexahydroimidazo[1,5-a]pyrazine-7(1H)-carboxylate (**Intermediate 26-1**,32 mg, 61.7%%) as a colorless oil. MS: 365.2 $(M+H)^+$.

**Step 2: Preparation of Intermediate 26-2**

The title compound **Intermediate 26-2** was prepared in analogy to the preparation of **Intermediate 1-5** by usingtert-butyl (R)-2-(3-(methylcarbamoyl)bicyclo[1.1.1]pentan-1-yl)-3-oxohexahydroimidazo[1,5-a]pyrazine-7(1H)-carboxylate (**Intermediate 26-1**) instead of(R)-3-(7-(tert-butoxycarbonyl)-3-oxohexahydroimidazo[1,5-a]pyrazin-2(3H)-yl)bicyclo[1.1.1]pentane-1-carboxylic acid (**Intermediate 1-4**). MS: 265.1 $(M+H)^+$.

**Intermediate 6-2**

**(S)-2-(3-(pyrrolidine-1-carbonyl)bicyclo[1.1.1]pentan-1-yl)hexahydroimidazo[1,5-a]pyrazin-3(2H)-one**

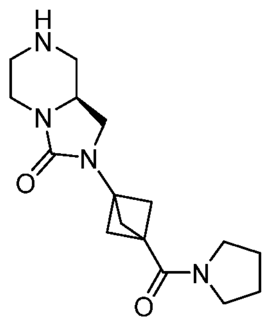

**6-2**

The title compound **Intermediate 6-2** was prepared in analogy to the preparation of **Intermediate 26-2** by usingpyrrolidine instead ofmethylamine hydrochloride. MS: 305.2 $(M+H)^+$.

**Intermediate 7-2**

**(S)-3-(3-oxohexahydroimidazo[1,5-a]pyrazin-2(3H)-yl)bicyclo[1.1.1]pentane-1-carboxamide**

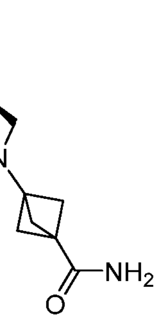

**7-2**

The title compound **Intermediate 7-2** was prepared in analogy to the preparation of **Intermediate 26-2** by using ammonium chlorideinstead ofmethylamine hydrochloride. MS: 251.1 $(M+H)^+$.

**Intermediate 8-2**

**(S)-2-(3-((methylsulfonyl)methyl)bicyclo[1.1.1]pentan-1-yl)hexahydroimidazo[1,5-a]pyrazin-3(2H)-one**

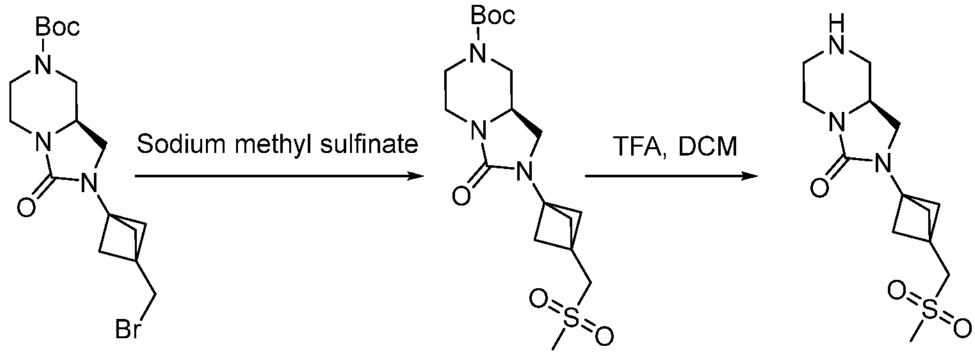


**4-1** **8-1** **8-2**

**Step 1: Preparation of Intermediate 8-1**

A mixture solution of tert-butyl (R)-2-(3-(bromomethyl)bicyclo[1.1.1]pentan-1-yl)-3-oxohexahydroimidazo[1,5-a]pyrazine-7(1H)-carboxylate(**Intermediate 4-1**, 200 mg, 0.5 mmol) and sodium methylsulfite in DMF (2 mL) was stirred at 80 °C for 3 hours. After the reaction was completed, the reaction mixture was quenched by adding ice-water and extracted with EA. The combined organic layers were washed with brine, dried over anhydrous $Na_2SO_4$, filtered and concentrated in vacuo to give a crude product, which was purified by silica gel flash chromatography to afford tert-butyl (R)-2-(3-((methylsulfonyl)methyl)bicyclo[1.1.1]pentan-1-yl)-3-oxohexahydroimidazo[1,5-a]pyrazine-7(1H)-carboxylate(**Intermediate 8-1**, 150 mg, 75%) as a white solid. MS: 400.2 $(M+H)^+$.

**Step 2: Preparation of Intermediate 8-2**

A mixture solution of tert-butyl (R)-2-(3-((methylsulfonyl)methyl)bicyclo[1.1.1]pentan-1-yl)-3-oxohexahydroimidazo[1,5-a]pyrazine-7(1H)-carboxylate(**Intermediate 8-1**, 150 mg, 0.38 mmol) in DCM (7 mL) and TFA (1 mL) was stirred at 20 °C for 5 hours. After the reaction was completed, the reaction mixture was concentrated in vacuo to afford (S)-2-(3-((methylsulfonyl)methyl)bicyclo[1.1.1]pentan-1-yl)hexahydroimidazo[1,5-a]pyrazin-3(2H)-one (**Intermediate 8-2**, 101 mg) as a yellow oil. MS: 300.1 $(M+H)^+$.

**Intermediate 9-5**

**(S)-N-(3-(3-oxohexahydroimidazo[1,5-a]pyrazin-2(3H)-yl)bicyclo[1.1.1]pentan-1-yl)methanesulfonamide**

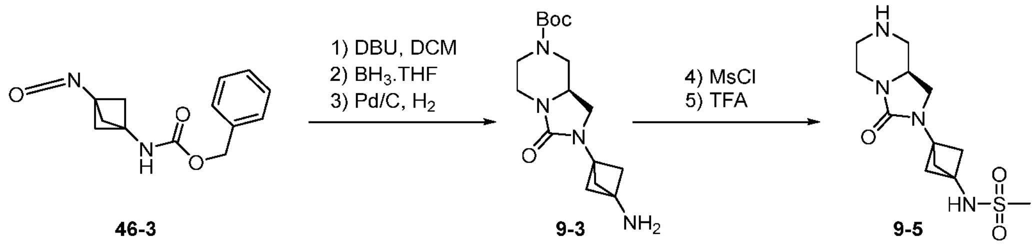


**46-3** **9-3** **9-5**

The title compound **Intermediate 9-5** was prepared in analogy to the preparation of **Intermediate 46-9** by usingl-(tert-butyl)3-methyl (s)-piperazine-1,3-dicarboxylate instead of 1-(tert-butyl)2,3-dimethyl(2S, 3S)- piperazine-1,2,3-tricarboxylate. MS: 301.2 $(M+H)^+$.

**Intermediate 10-2**

**(S)-N-(3-(3-oxohexahydroimidazo[1,5-a]pyrazin-2(3H)-yl)bicyclo[1.1.1]pentan-1-yl)acetamide**

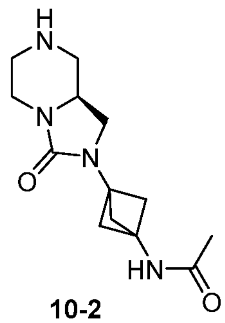

**10-2**

The title compound **Intermediate 10-2** was prepared in analogy to the preparation of **Intermediate 9-5** by usingacetyl chloride instead of methyl sulfonyl chloride. MS: 265.2 $(M+H)^+$.

**Intermediate 11-2**

**(S)-N-methyl-N-(3-(3-oxohexahydroimidazo[1,5-a]pyrazin-2(3H)-yl)bicyclo[1.1.1]pentan-1-yl)methanesulfonamide**

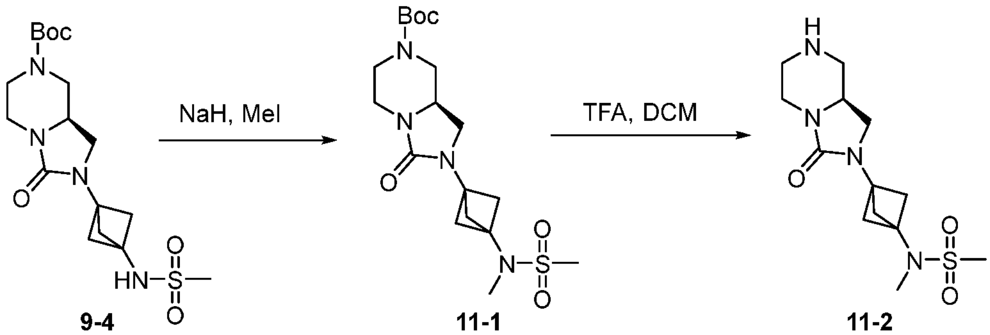


**Step 1: Preparation of Intermediate 11-1**

To a stirred mixture solution of tert-butyl (R)-2-(3-(methylsulfonamido)bicyclo[1.1.1]pentan-1-yl)-3-oxohexahydroimidazo[1,5-a]pyrazine-7(1H)-carboxylate (**Intermediate 9-4**, 100 mg, 0.25 mmol) in THF (5 mL) was added NaH (15 mg, 0.38 mmol) at 0 °C and the resulting mixture was stirred at 20 °C for 1 hour. Then $CH_3I$ (54 mg, 0.38 mmol) was added to the above mixture solution at 0 °C and the resulting mixture was stirred at 20 °C for 15 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford tert-butyl (R)-2-(3-(N-methylmethylsulfonamido)bicyclo[1.1.1]pentan-1-yl)-3-oxohexahydroimidazo[1,5-a]pyrazine-7(1H)-carboxylate **(Intermediate 11-1**, 88 mg, 85%) as a yellow oil. MS: 415.2 $(M+H)^+$.

**Step 2: Preparation of Intermediate 11-2**

A mixture solution of tert-butyl (R)-2-(3-(N-methylmethylsulfonamido)bicyclo[1.1.1]pentan-1-yl)-3-oxohexahydroimidazo[1,5-a]pyrazine-7(1H)-carboxylate (**Intermediate 11-1**, 88 mg, 0.21 mmol) in DCM (7 mL) and TFA (1 mL)

was stirred at 20 °C for 5 hours. After the reaction was completed, the reaction mixture was concentrated in vacuo to afford (S)-N-methyl-N-(3-(3-oxohexahydroimidazo[1,5-a]pyrazin-2(3H)-yl)bicyclo[1.1.1]pentan-1-yl)methanesulfonamide (**Intermediate 11-2**, 60 mg) as a yellow oil. MS: 315.1 $(M+H)^+$.

**Intermediate 12-2**

**(S)-N-methyl-N-(3-(3-oxohexahydroimidazo[1,5-a]pyrazin-2(3H)-yl)bicyclo[1.1.1]pentan-1-yl)acetamide**

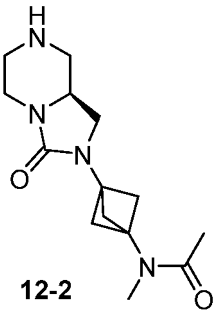

**12-2**

The title compound **Intermediate 12-2** was prepared in analogy to the preparation of **Intermediate 11-2** by usingacetyl chloride instead of methyl sulfonyl chloride. MS: 279.2 $(M+H)^+$.

**Intermediate 13-3**

**(S)-2-(3-(2-oxopyrrolidin-1-yl)bicyclo[1.1.1]pentan-1-yl)hexahydroimidazo[1,5-a]pyrazin-3(2H)-one**

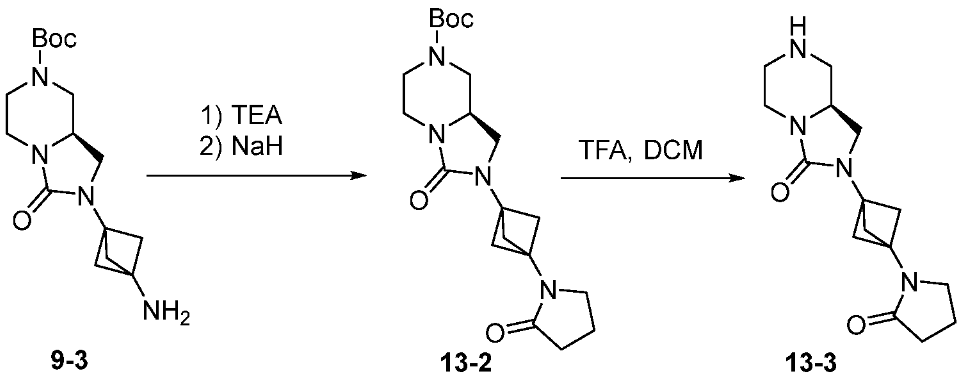


**9-3** **13-2** **13-3**

**Step 1: Preparation of Intermediate 13-2**

To a stirred mixture solution of tert-butyl (R)-2-(3-aminobicyclo[1.1.1]pentan-1-yl)-3-oxohexahydroimidazo[1,5-a]pyrazine-7(1H)-carboxylate **(Intermediate 9-3,** 100 mg, 0.31 mmol) in THF (5 mL) was added TEA(63 mg, 0.62 mmol) and 4-chlorobutyryl chloride (52 mg, 0.37 mmol) at 0 °C and the resulting mixture was stirred at 20 °C for 12 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford (**Intermediate 13-1,** 114 mg, 86%) as a colorless oil. MS: 472.2 $(M+H)^+$.

A mixture solution of (**Intermediate 13-1,** 114 mg, 0.27 mmol) and NaH (16 mg, 0.4 mmol) in THF (5 mL) was stirred at 10 °C for 12 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford tert-butyl (R)-3-oxo-2-(3-(2-oxopyrrolidin-1-yl)bicyclo[1.1.1]pentan-1-yl)hexahydroimidazo[1,5-a]pyrazine-7(1H)-carboxylate (**Intermediate 13-2**, 85 mg, 81%) as a colorless oil. MS: 391.2 $(M+H)^+$.

**Step 2: Preparation of Intermediate 13-3**

The title compound **Intermediate 13-3** was prepared in analogy to the preparation of **Intermediate 11-2** by using **Intermediate 13-2** instead of **Intermediate 11-1**. MS: 291.2 $(M+H)^+$.

**Intermediate 14-3**

**(S)-N-((3-(3-oxohexahydroimidazo[1,5-a]pyrazin-2(3H)-yl)bicyclo[1.1.1]pentan-1-yl)methyl)methanesulfonamide**

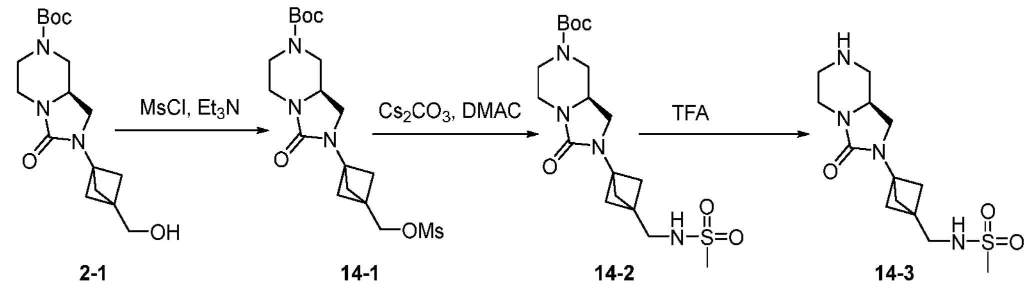


**2-1** **14-1** **14-2** **14-3**

**Step 1: Preparation of Intermediate 14-1**

To a stirred mixture solution of tert-butyl (R)-2-(3-(hydroxymethyl)bicyclo[1.1.1]pentan-1-yl)-3-oxohexahydroimidazo[1,5-a]pyrazine-7(1H)-carboxylate (**Intermediate 2-1**, 337 mg, 0.999 mmol) in DCM (5 mL) was added TEA (121 mg, 1.199 mmol) and MsCl (137 mg, 1.199 mmol) at 0 °C and the resulting mixture was stirred at 0 °C for 2 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford tert-butyl (R)-2-(3-(((methylsulfonyl)oxy)methyl)bicyclo[1.1.1]pentan-1-yl)-3-oxohexahydroimidazo[1,5-a]pyrazine-7(1H)-carboxylate (**Intermediate 14-1**, 380 mg, 92%) as a yellow oil. MS: 416.5 $(M+H)^+$.

**Step 2: Preparation of Intermediate 14-2**

To a stirred mixture solution of tert-butyl (R)-2-(3-(((methylsulfonyl)oxy)methyl)bicyclo[1.1.1]pentan-1-yl)-3-oxohexahydroimidazo[1,5-a]pyrazine-7(1H)-carboxylate (**Intermediate 14-1**, 330 mg, 0.794 mmol) in DMAC (6 mL) was added methanesulfonamide (83 mg, 0.874 mmol) and $Cs_2CO_3$ (388 mg, 1.191 mmol) at 20 °C and the resulting mixture was stirred at 80 °C for 2 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford tert-butyl (R)-2-(3-(methylsulfonamidomethyl)bicyclo[1.1.1]pentan-1-yl)-3-oxohexahydroimidazo[1,5-a]pyrazine-7(1H)-carboxylate (**Intermediate 14-2**, 170 mg, 52%) as a yellow oil. MS: 415.5 $(M+H)^+$.

**Step 3: Preparation of Intermediate 14-3**

The title compound **Intermediate 14-3** was prepared in analogy to the preparation of **Intermediate 1-5** by usingtert-butyl (R)-2-(3-(methylsulfonamidomethyl)bicyclo[1.1.1]pentan-1-yl)-3-oxohexahydroimidazo[1,5-a]pyrazine-7(1H)-carboxylate (**Intermediate 14-2**) instead of (R)-3-(7-(tert-butoxycarbonyl)-3-oxohexahydroimidazo[1,5-a]pyrazin-2(3H)-yl)bicyclo[1.1.1]pentane-1-carboxylic acid (**Intermediate 1-4**). MS: 315.5 $(M+H)^+$.

**Intermediate 15**

**(S)-N-((3-(3-oxohexahydroimidazo[1,5-a]pyrazin-2(3H)-yl)bicyclo[1.1.1]pentan-1-yl)methyl)acetamide**

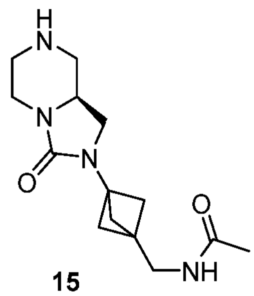

15

The title compound **Intermediate 15** was prepared in analogy to the preparation of **Intermediate 14-3** by usingtert-butyl (R)-2-(3-(bromomethyl)bicyclo[1.1.1]pentan-1-yl)-3-oxohexahydroimidazo[1,5-a]pyrazine-7(1H)-carboxylate(**Intermediate 4-1**) instead of tert-butyl (R)-2-(3-(((methylsulfonyl)oxy)methyl)bicyclo[1.1.1]pentan-1-yl)-3-oxohexahydroimidazo[1,5-a]pyrazine-7(1H)-carboxylate (**Intermediate 14-1**) and acetamide instead of methanesulfonamide. MS: 279.1 $(M+H)^+$.

**Intermediate 16**

**diethyl (S)-((3-(3-oxohexahydroimidazo[1,5-a]pyrazin-2(3H)-yl)bicyclo[1.1.1]pentan-1-yl)methyl)phosphonate**

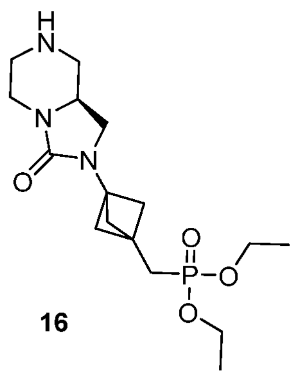

16

The title compound **Intermediate 16** was prepared in analogy to the preparation of **Intermediate 14-3** by usingtert-butyl (R)-2-(3-(bromomethyl)bicyclo[1.1.1]pentan-1-yl)-3-oxohexahydroimidazo[1,5-a]pyrazine-7(1H)-carboxylate(**Intermediate 4-1**) instead of tert-butyl (R)-2-(3-(((methylsulfonyl)oxy)methyl)bicyclo[1.1.1]pentan-1-yl)-3-oxohexahydroimidazo[1,5-a]pyrazine-7(1H)-carboxylate (**Intermediate 14-1**), NaH instead of $Cs_2CO_3$, THF instead of DMAC and diethyl phosphate instead of methanesulfonamide.

**Intermediate 17-3**

**(S)-3-(3-oxohexahydroimidazo[1,5-a]pyrazin-2(3H)-yl)bicyclo[1.1.1]pentane-1-carbonitrile**

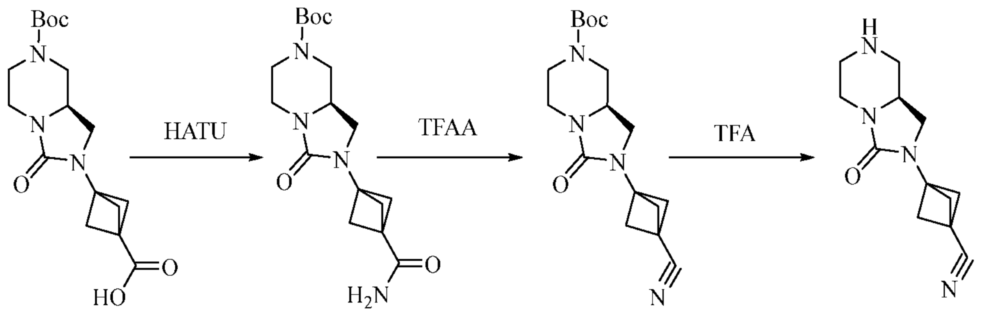


1-4 17-1 17-2 17-3

**Step 1: Preparation of Intermediate 17-1**

The title compound **Intermediate 17-1** was prepared in analogy to the preparation of **Intermediate 26-1** by usingammonium chloride instead of methylamine hydrochloride.

**Step 2: Preparation of Intermediate 17-2**

To a stirred mixture solution of (**17-1**,100 mg, 0.285 mmol) and TEA (43.3 mg, 0.428 mmol) in DCM (6 mL) was added TFAA (90 mg, 0.428 mmol) at 0 °C and the resulting mixture was stirred at 0 °C for 1 hour. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford tert-butyl (R)-2-(3-cyanobicyclo[1.1.1]pentan-1-yl)-3-oxohexahydroimidazo[1,5-a]pyrazine-7(1H)-carboxylate (**Intermediate 17-2**, 65 mg, 68.5 %) as a yellow oil. MS: 277.1 $(M-56+H)^+$.

**Step 3: Preparation of Intermediate 17-3**

The title compound **Intermediate 17-3** was prepared in analogy to the preparation of **Intermediate 3-2** by usingtert-butyl (R)-2-(3-cyanobicyclo[1.1.1]pentan-1-yl)-3-oxohexahydroimidazo[1,5-a]pyrazine-7(1H)-carboxylate (**Intermediate 17-2**)instead of tert-butyl (R)-2-(3-(methoxymethyl)bicyclo[1.1.1]pentan-1-yl)-3-oxohexahydroimidazo[1,5-a]pyrazine-7(1H)-carboxylate (**Intermediate 3-1**). MS: 233.1 $(M+H)^+$.

**Intermediate 18-2**

**(S)-2-(3-(fluoromethyl)bicyclo[1.1.1]pentan-1-yl)hexahydroimidazo[1,5-a]pyrazin-3(2H)-one**

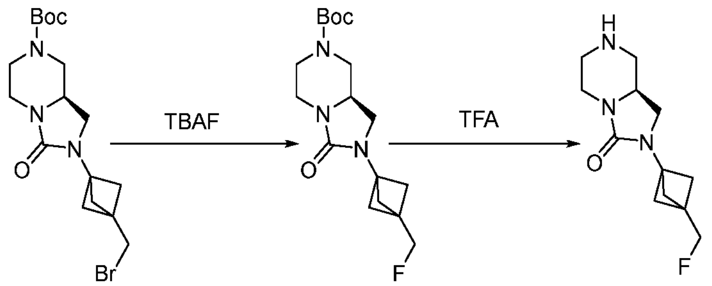


4-1 18-1 18-2

**Step 1: Preparation of Intermediate 18-1**

A mixture solution of tert-butyl (R)-2-(3-(bromomethyl)bicyclo[1.1.1]pentan-1-yl)-3-oxohexahydroimidazo[1,5-a]pyrazine-7(1H)-carboxylate(**Intermediate 4-1,** 100 mg, 0.25 mmol), 1.0 M TBAF solution in THF (0.3 mL) in DMF (2 mL) was stirred at 80 °C for 1 hour. After the reaction was completed, the reaction mixture was extracted with EA. The combined organic layers were washed with brine, dried over anhydrous $Na_2SO_4$, filtered and concentrated in vacuo to

give a crude product, which was purified by silica gel flash chromatography to afford tert-butyl (R)-2-(3-(fluoromethyl)bicyclo[1.1.1]pentan-1-yl)-3-oxohexahydroimidazo[1,5-a]pyrazine-7(1H)-carboxylate (**Intermdiate 18-1**, 66 mg, 78%) as a yellow oil. MS: 340.4 $(M+H)^+$.

**Step 2: Preparation of Intermediate 18-2**

The title compound **Intermediate 18-2** was prepared in analogy to the preparation of **Intermediate 11-2** by using **Intermediate 18-1**instead of **Intermediate 11-1**. MS: 240.2 $(M+H)^+$.

**Intermediate 19-3**

**(S)-2-(3-(1,1-dioxidoisothiazolidin-2-yl)bicyclo[1.1.1]pentan-1-yl)hexahydroimidazo[1,5-a]pyrazin-3(2H)-one**

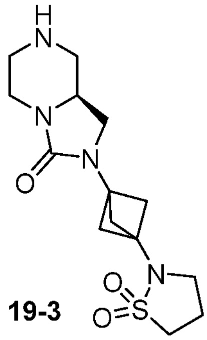

**19-3**

The title compound **Intermediate 19-3** was prepared in analogy to the preparation of **Intermediate 13-3** by using 3-chloropropane-1-sulfonyl chlorideinstead of 4-chlorobutyryl chloride. MS: 372.2 $(M+H)^+$.

**Intermediate 20-2**

**(S)-2-(3-(3-oxohexahydroimidazo[1,5-a]pyrazin-2(3H)-yl)bicyclo[1.1.1]pentan-1-yl)acetonitrile**

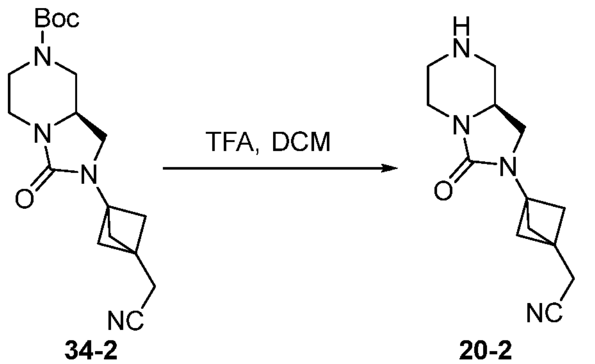


**34-2** **20-2**

The title compound **Intermediate 20-2** was prepared in analogy to the preparation of **Intermediate 11-2** by using **Intermediate 34-2**instead of **Intermediate 11-1.** MS: 247.2 $(M+H)^+$.

**Intermediate 21-2**

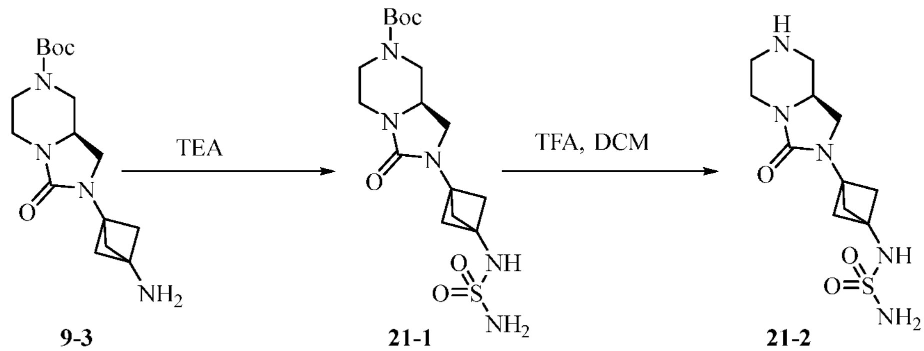


9-3 21-1 21-2

The title compound **Intermediate 21-2** was prepared in analogy to the preparation of **Intermediate 9-5** by usingsulfamoyl chlorideinstead of methyl sulfonyl chloride. MS: 302.2 $(M+H)^+$.

**Intermediate 49-4**

**(S)-N-methyl-N-(3-(3-oxohexahydroimidazo[1,5-a]pyrazin-2(3H)-yl)bicyclo[1.1.1]pentan-1-yl)glycine**

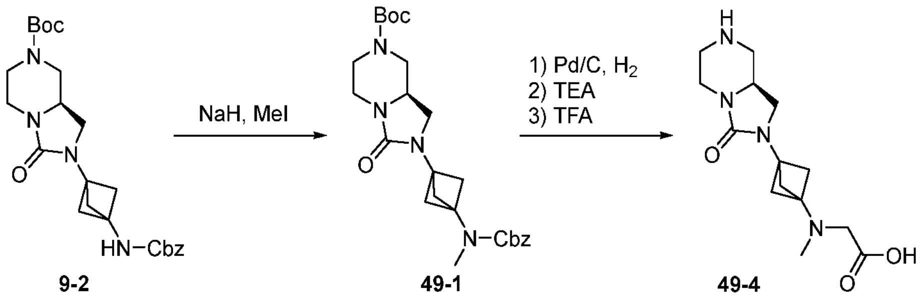


9-2 49-1 49-4

**Step 1: Preparation of Intermediate 49-1**

The title compound **Intermediate 49-1** was prepared in analogy to the preparation of **Intermediate 11-1** by using **Intermediate 9-2** instead of **Intermediate 9-4.** MS: 471.2 $(M+H)^+$.

**Step 1: Preparation of Intermediate 49-2**

The title compound **Intermediate 49-4** was prepared in analogy to the preparation of **Intermediate 9-5** by using **Intermediate 49-1** instead of **Intermediate 9-2** andtert butyl 2-bromoacetate instead of methyl sulfonyl chloride. MS: 295.2 $(M+H)^+$.

**Intermediate 23-3**

**(S)-N-((3-(3-oxohexahydroimidazo[1,5-a]pyrazin-2(3H)-yl)bicyclo[1.1.1]pentan-1-yl)methyl)cyclopropanesulfonamide**

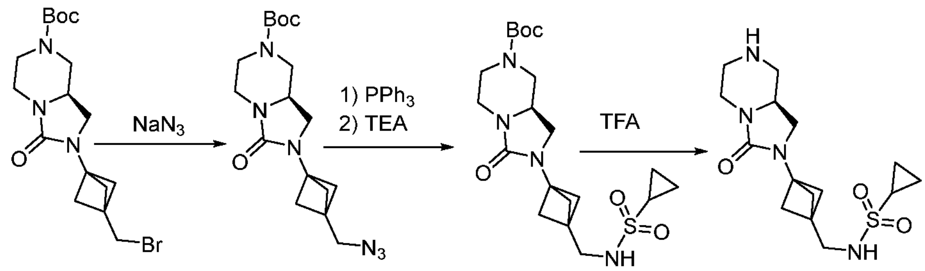


4-1 23-1 23-2 23-3

**Step 1: Preparation of Intermediate 23-1**

A mixture solution of tert-butyl (R)-2-(3-(bromomethyl)bicyclo[1.1.1]pentan-1-yl)-3-oxohexahydroimidazo[1,5-a]pyrazine-7(1H)-carboxylate(**Intermediate 4-1**, 511 mg, 1.276 mmol) and $NaN_3$ (91 mg, 1.404 mmol) in DMF (6 mL) and water (1 mL) was stirred at 80 °C for 1 hour. After the reaction was completed, the reaction mixture was extracted with EA. The combined organic layers were washed with brine, dried over anhydrous $Na_2SO_4$, filtered and concentrated in vacuo to give a crude product, which was purified by silica gel flash chromatography to afford tert-butyl (R)-2-(3-(azidomethyl)bicyclo[1.1.1]pentan-1-yl)-3-oxohexahydroimidazo[1,5-a]pyrazine-7(1H)-carboxylate (**Intermdiate 23-1,** 463 mg, 100%) as a yellow oil. MS: 363.4 $(M+H)^+$.

**Step 2: Preparation of Intermediate 23-2**

To a stirred mixture solution of tert-butyl (R)-2-(3-(azidomethyl)bicyclo[1.1.1]pentan-1-yl)-3-oxohexahydroimidazo[1,5-a]pyrazine-7(1H)-carboxylate **(Intermdiate 23-1,** 460 mg, 1.269 mmol) in THF (6 mL) and water (2 mL) was added $PPh_3$ (366 mg, 1.396 mmol) at 20 °C and the resulting mixture was stirred at 30 °C for 5 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to afford **(Intermediate 23-2-1,** 400 mg, 94%) as a yellow oil. MS: 337.4 $(M+ H)^+$.

To a stirred mixture solution of (**Intermdiate 23-2-1,** 80 mg, 0.238 mmol) in THF (6 mL) was added TEA (372.2 mg, 0.713 mmol) and cyclopropyl sulfonyl chloride (40.1 mg, 0.285 mmol) at 0 °C and the resulting mixture was stirred at 0 °C for 2 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to afford tert-butyl (R)-2-(3-(cyclopropanesulfonamidomethyl)bicyclo[1.1.1]pentan-1-yl)-3-oxohexahydroimidazo[1,5-a]pyrazine-7(1H)-carboxylate **(Intermediate 23-2,** 105 mg, 100%) as a yellow oil. MS: 441.5 $(M+ H)^+$.

**Step 3: Preparation of Intermediate 23-3**

The title compound **Intermediate 23-3** was prepared in analogy to the preparation of **Intermediate 3-2** by using tert-butyl (R)-2-(3-(cyclopropanesulfonamidomethyl)bicyclo[1.1.1]pentan-1-yl)-3-oxohexahydroimidazo[1,5-a]pyrazine-7(1H)-carboxylate **(Intermediate 23-2**) instead of tert-butyl (R)-2-(3-(methoxymethyl)bicyclo[1.1.1]pentan-1-yl)-3-oxohexahydroimidazo[1,5-a]pyrazine-7(1H)-carboxylate (**Intermediate 3-1**). MS: 341.5 $(M+H)^+$.

**Intermediate 24-1**

**(S) - 3-oxo-2 - (3 - ((aminosulfonylamino) methyl) bicyclo [1.1.1] pentane-1-yl) hexahydroimidazo [1,5-a] pyrazine**

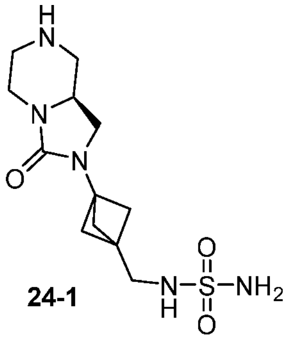

24-1

The title compound **Intermediate 24-1** was prepared in analogy to the preparation of **Intermediate 23-3** by using sulfamyl chloride instead of cyclopropyl sulfonyl chloride. MS: 316.1 $(M+H)^+$.

**Intermediate 25-3**

**(S)-2-(3-((2-oxopyrrolidin-1-yl)methyl)bicyclo[1.1.1]pentan-1-yl)hexahydroimidazo[1,5-a]pyrazin-3(2H)-one**

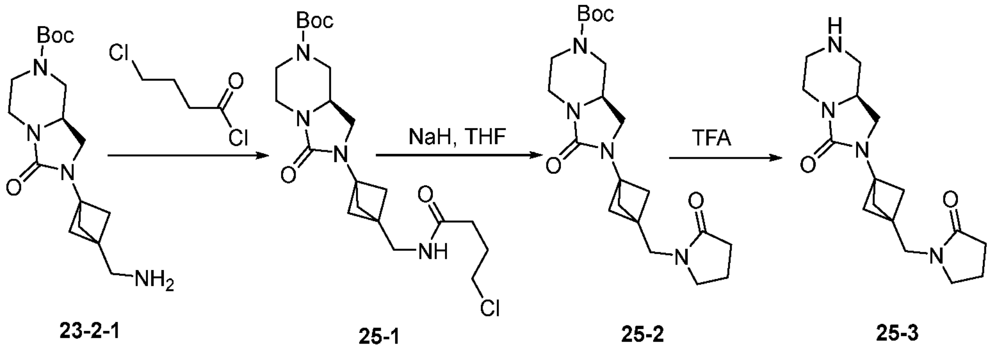


23-2-1 25-1 25-2 25-3

**Step 1: Preparation of Intermediate 25-1**

To a stirred mixture solution of tert-butyl (R)-2-(3-(aminomethyl)bicyclo[1.1.1]pentan-1-yl)-3-oxohexahydroimidazo[1,5-a]pyrazine-7(1H)-carboxylate **(Intermediate 23-2-1,** 80 mg, 0.238 mmol) in DCM (3 mL) was added TEA (72.2 mg, 0.713 mmol) and 4-chlorobutyryl chloride (36.9 mg, 0.262 mmol) at 0 °C and the resulting mixture was stirred at 0 °C for 2 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to afford tert-butyl (R)-2-(3-((4-chlorobutanamido)methyl)bicyclo[1.1.1]pentan-1-yl)-3-oxohexahydroimidazo[1,5-a]pyrazine-7(1H)-carboxylate (**Intermediate 25-1**, 105 mg, 100%) as a yellow oil. MS: 441.2 $(M+H)^+$.

**Step 2: Preparation of Intermediate 25-2**

To a stirred mixture solution of tert-butyl (R)-2-(3-((4-chlorobutanamido)methyl)bicyclo[1.1.1]pentan-1-yl)-3-oxohexahydroimidazo[1,5-a]pyrazine-7(1H)-carboxylate (**Intermediate 25-1**, 105 mg, 0.238 mmol) in THF (4 mL) was added NaH (13.7 mg, 0.357 mmol) at 0 °C and the resulting mixture was stirred at 20 °C for 2 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to afford tert-butyl (R)-3-oxo-2-(3-((2-oxopyrrolidin-1-yl)methyl)bicyclo[1.1.1]pentan-1-yl)hexahydroimidazo[1,5-a]pyrazine-7(1H)-carboxylate (**Intermediate 25-2**,96 mg, 100%) as a colorless oil. MS: 405.2 $(M+H)^+$.

**Step 3: Preparation of Intermediate 25-3**

The title compound **Intermediate 25-3** was prepared in analogy to the preparation of **Intermediate 14-3** by

usingtert-butyl (R)-3-oxo-2-(3-((2-oxopyrrolidin-1-yl)methyl)bicyclo[1.1.1]pentan-1-yl)hexahydroimidazo[1,5-a]pyrazine-7(1H)-carboxylate (**Intermediate 25-2**) instead of tert-butyl (R)-2-(3-(methylsulfonamidomethyl)bicyclo[1.1.1]pentan-1-yl)-3-oxohexahydroimidazo[1,5-a]pyrazine-7(1H)-carboxylate (**Intermediate 14-2**). MS: 305.2 $(M+H)^+$.

**Intermediate 27-2**

**(S)-2-(3-(morpholine-4-carbonyl)bicyclo[1.1.1]pentan-1-yl)hexahydroimidazo[1,5-a]pyrazin-3(2H)-one**

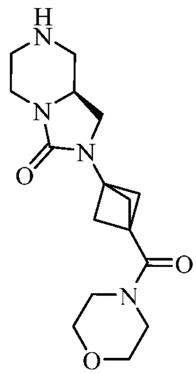

**27-2**

The title compound **Intermediate 27-2** was prepared in analogy to the preparation of **Intermediate 26-2** by usingmorpholine instead of methylamine hydrochloride. MS: 302.1 $(M+H)^+$.

**Intermediate 28-2**

**(S)-N-(methylsulfonyl)-3-(3-oxohexahydroimidazo[1,5-a]pyrazin-2(3H)-yl)bicyclo[1.1.1]pentane-1-carboxamide**

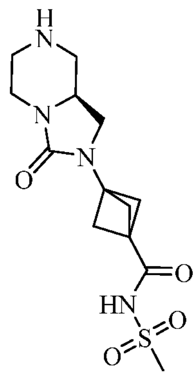

**28-2**

The title compound **Intermediate 28-2** was prepared in analogy to the preparation of **Intermediate 26-2** by usingmethanesulfonamide instead of methylamine hydrochloride. MS: 329.2 $(M+H)^+$.

**Intermediate 29-2**

**(S)-N-(3-(3-oxohexahydroimidazo[1,5-a]pyrazin-2(3H)-yl)bicyclo[1.1.1]pentan-1-yl)cyclopropanesulfonamide**

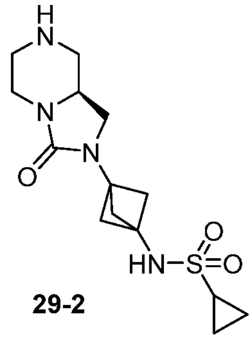

29-2

The title compound **Intermediate 29-2** was prepared in analogy to the preparation of **Intermediate 9-5** by usingcyclopropyl sulfonyl chloride instead of methyl sulfonyl chloride. MS: 327.2 $(M+H)^+$.

**Intermediate 30-2**

**2-(3-((S)-3-oxohexahydroimidazo[1,5-a]pyrazin-2(3H)-yl)bicyclo[1.1.1]pentan-1-yl)propanoic acid**

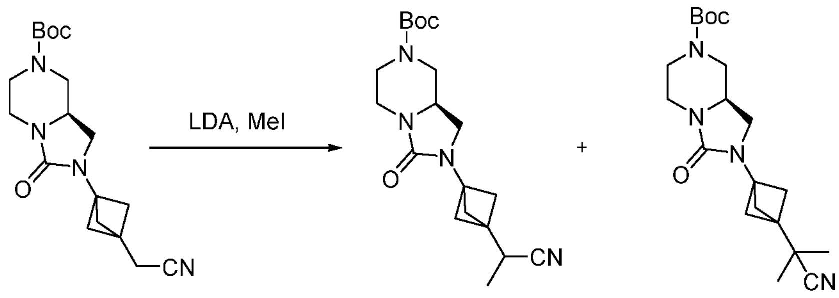


34-2 30-1a 30-1b

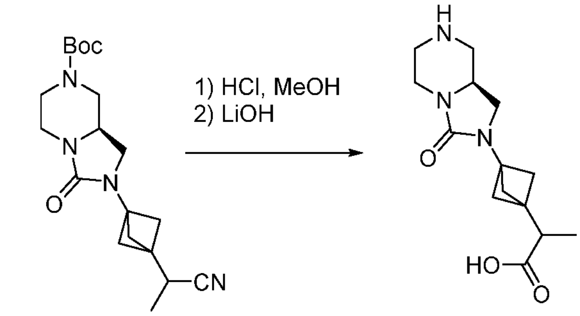


30-1a 30-2

**Step 1: Preparation of Intermediate 30-1a and 30-1b**

To a stirred mixture solution of tert-butyl (R)-2-(3-(cyanomethyl)bicyclo[1.1.1]pentan-1-yl)-3-oxohexahydroimidazo[1,5-a]pyrazine-7(1H)-carboxylate (**Intermediate 34-2**, 500 mg, 1.45 mmol) in THF (14 mL) was added 2.0 M LDA THF solution (2.2 mmol) at -78 °C and stirred at -78 °C for 1 hour, then $CH_3I$(246 mg, 1.72 mmol) was added to the above mixture at -78 °C, then the resulting mixture was stirred at 20°C for 21 hour. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to affordtert-butyl (8aR)-2-(3-(1-cyanoethyl)bicyclo[1.1.1]pentan-1-yl)-3-oxohexahydroimidazo[1,5-a]pyrazine-7(1H)-carboxylate (**Intermediate 30-1a**,208 mg, 40%) as a colorless oil. MS: 361.2 $(M+ H)^+$ and tert-butyl (R)-2-(3-(2-cyanopropan-2-yl)bicyclo[1.1.1]pentan-1-yl)-3-oxohexahydroimidazo[1,5-a]pyrazine-7(1H)-carboxylate (**Intermediate 30-1b**,110 mg, 20%) as a colorless oil. MS: 375.2 $(M+ H)^+$.

**Step 2: Preparation of Intermediate 30-2**

The title compound **Intermediate 30-2** was prepared in analogy to the preparation of **Intermediate 34-4** by using **Intermediate 30-1a** instead of **Intermediate 34-2.** MS: 280.2 $(M+H)^+$.

**Intermediate 42-1**

**(S)-2-methyl-2-(3-(3-oxohexahydroimidazo[1,5-a]pyrazin-2(3H)-yl)bicyclo[1.1.1]pentan-1-yl)propanoic acid**

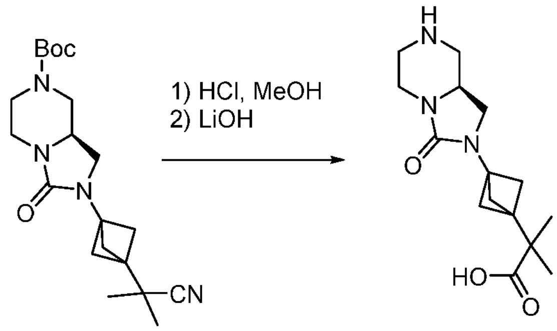


**30-1b** **42-1**

The title compound **Intermediate 42-1** was prepared in analogy to the preparation of **Intermediate 34-4** by using **Intermediate 30-1b** instead of **Intermediate 34-2.** MS: 294.2 $(M+H)^+$.

**Intermediate 32-5**

**(S)-3-(3-thioxohexahydroimidazo[1,5-a]pyrazin-2(3H)-yl)bicyclo[1.1.1]pentane-1-carboxylic acid**

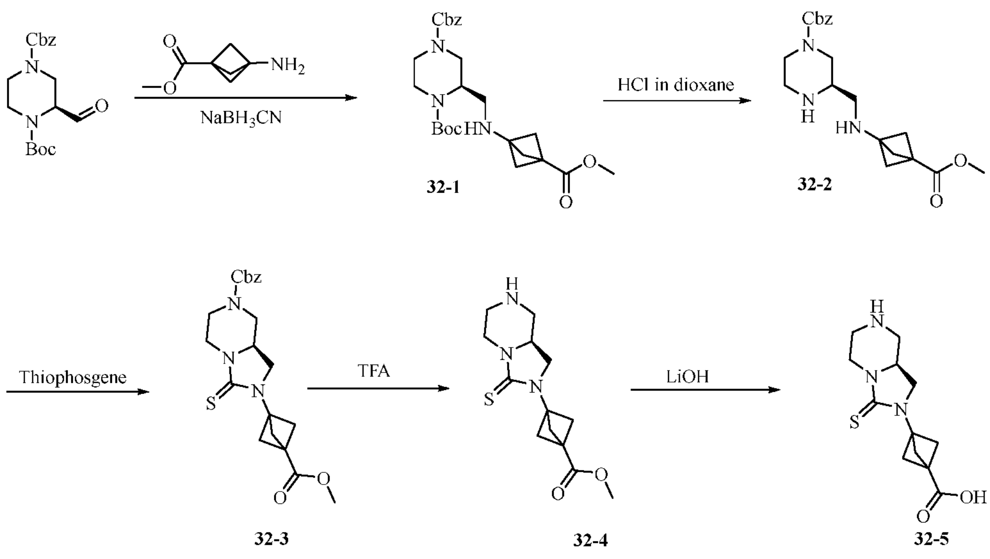


32-1 32-2 32-3 32-4 32-5

**Step 1: Preparation of Intermediate 32-1**

To a stirred mixture solution of methyl 3-aminobicyclo[1.1.1]pentane-1-carboxylate hydrochloride (0.765 g, 4.31 mmol) in Ethanol (10 mL) was added TEA (0.435 g, 4.31 mmol) and a solution of 4-benzyl 1-(tert-butyl) (S)-2-formylpiperazine-1,4-dicarboxylate (1 g, 2.87 mmol) in Ethanol (10 mL) at 0 °C, then the resulting mixture was stirred at 20 °C for 1 hour. Then $NaBH_3CN$ (0.433 g, 6.89 mmol) was added to the above mixture solution at 0 °C, then the resulting

mixture was stirred at 0 °C for 2 hours. After the reaction was completed, the reaction mixture was poured into ice-water and extracted with EA. The combined organic layers were washed with brine, dried over anhydrous $Na_2SO_4$, filtered and concentrated in vacuo to afford4-benzyl 1-(tert-butyl) (R)-2-(((3-(methoxycarbonyl)bicyclo[1.1.1]pentan-1-yl)amino)methyl)piperazine-1,4-dicarboxylate **(Intermediate 32-1,700** mg, 51.5%) as a yellow solid. MS: 474.3 $(M+H)^+$.

**Step 2: Preparation of Intermediate 32-2**

To a stirred mixture solution of 4-benzyl 1-(tert-butyl) (R)-2-(((3-(methoxycarbonyl)bicyclo[1.1.1]pentan-1-yl)amino)methyl)piperazine-1,4-dicarboxylate (**Intermediate 32-1**,700 mg, 1.478 mmol) in DCM (5 mL) was added 4N HCl dioxane solution (1 mL) at 0 °C, then the resulting mixture was stirred at 20 °C for 2 hours. After the reaction was completed, the reaction mixture was poured into ice-water and extracted with EA. The combined organic layers were washed with brine, dried over anhydrous $Na_2SO_4$, filtered and concentrated in vacuo to affordbenzyl (R)-3-(((3-(methoxycarbonyl)bicyclo[1.1.1]pentan-1-yl)amino)methyl)piperazine-1-carboxylate (**Intermediate 32-2**,520 mg, 86%) as a yellow solid. MS: 374.3 $(M+H)^+$.

**Step 3: Preparation of Intermediate 32-3**

To a stirred mixture solution of benzyl (R)-3-(((3-(methoxycarbonyl)bicyclo[1.1.1]pentan-1-yl)amino)methyl)piperazine-1-carboxylate (**Intermediate 32-2**,250 mg, 0.610 mmol) in DCM (15 mL) was added TEA (61.6 mg, 0.610 mmol) and sulfur phosgene (70.1 mg, 0.610 mmol) at 0 °C, then the resulting mixture was stirred at 20 °C for 12 hours. After the reaction was completed, the reaction mixture was poured into ice-water and extracted with EA. The combined organic layers were washed with brine, dried over anhydrous $Na_2SO_4$, filtered and concentrated in vacuo to affordbenzyl (R)-3-(((3-(methoxycarbonyl)bicyclo[1.1.1]pentan-1-yl)amino)methyl)piperazine-1-carboxylate (**Intermediate 32-3**,160 mg, 63.1%) as a yellow solid. MS: 416.2 $(M+H)^+$.

**Step 4: Preparation of Intermediate 32-4**

A mixture solution of benzyl (R)-3-(((3-(methoxycarbonyl)bicyclo[1.1.1]pentan-1-yl)amino)methyl)piperazine-1-carboxylate (**Intermediate 32-3**,75 mg, 0.181 mmol) in TFA (5 mL) was stirred at 70 °C for 1 hour. After the reaction was completed, the reaction mixture was concentrated in vacuo to afford methyl (S)-3-(3-thioxohexahydroimidazo[1,5-a]pyrazin-2(3H)-yl)bicyclo[1.1.1]pentane-1-carboxylate (**Intermediate 32-4**,30 mg, 59%) as a yellow oil. MS: 282.1 $(M+H)^+$.

**Step 5: Preparation of Intermediate 32-5**

To a stirred mixture solution of methyl (S)-3-(3-thioxohexahydroimidazo[1,5-a]pyrazin-2(3H)-yl)bicyclo[1.1.1]pentane-1-carboxylate (**Intermediate 32-4**,30 mg, 0.107 mmol) in THF (5 mL) and water (1 mL) was added LiOH (12.77 mg, 0.533 mmol) at 20 °C, then the resulting mixture was stirred at 20 °C for 12 hours. After the reaction was completed, the reaction mixture was poured into ice-water and extracted with EA. The combined organic layers were washed with brine, dried over anhydrous $Na_2SO_4$, filtered and concentrated in vacuo to afford (S)-3-(3-thioxohexahydroimidazo[1,5-a]pyrazin-2(3H)-yl)bicyclo[1.1.1]pentane-1-carboxylic acid (**Intermediate 32-5**,60 mg) as a yellow solid. MS: 268.1 $(M+H)^+$.

**Intermediate 34-4**

**(S)-2-(3-(3-oxohexahydroimidazo[1,5-a]pyrazin-2(3H)-yl)bicyclo[1.1.1]pentan-1-yl)acetic acid**

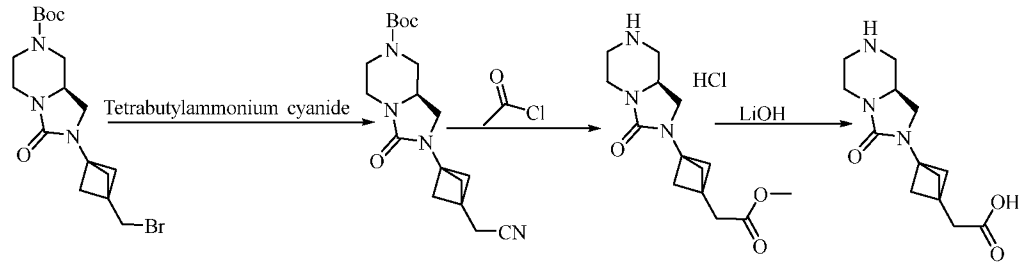


4-1 34-2 34-3 34-4

**Step 1: Preparation of Intermediate 34-2**

To a stirred mixture solution of tert-butyl (R)-2-(3-(bromomethyl)bicyclo[1.1.1]pentan-1-yl)-3-oxohexahydroimidazo[1,5-a]pyrazine-7(1H)-carboxylate (**Intermediate 4-1,1.2** g, 3.00 mmol) in DMF (5 mL) was added tetrabutylammonium cyanide (0.845 g, 3.15 mmol) at 20 °C, then the resulting mixture was stirred at 80 °C for 1 hour. After the reaction was completed, the reaction mixture was poured into ice-water and extracted with EA. The combined organic layers were washed with brine, dried over anhydrous $Na_2SO_4$, filtered and concentrated in vacuo to afford tert-butyl (R)-2-(3-(cyanomethyl)bicyclo[1.1.1]pentan-1-yl)-3-oxohexahydroimidazo[1,5-a]pyrazine-7(1H)-carboxylate (**Intermediate 34-2**,1.4 g) as a yellow solid. MS: 347.2 $(M+H)^+$.

**Step 2: Preparation of Intermediate 34-3**

To a stirred mixture solution of tert-butyl (R)-2-(3-(cyanomethyl)bicyclo[1.1.1]pentan-1-yl)-3-oxohexahydroimidazo[1,5-a]pyrazine-7(1H)-carboxylate (**Intermediate 34-2**,1.4 g, 4.04 mmol) in MeOH (20 mL) was added AcCl (10 mL) at 0 °C, then the resulting mixture was stirred at 80 °C for 12 hours. After the reaction was completed, the reaction mixture was poured into ice-water and extracted with EA. The combined organic layers were washed with brine, dried over anhydrous $Na_2SO_4$, filtered and concentrated in vacuo to afford methyl (S)-2-(3-(3-oxohexahydroimidazo[1,5-a]pyrazin-2(3H)-yl)bicyclo[1.1.1]pentan-1-yl)acetate (**Intermediate 34-3**,1 g, 89%) as a yellow solid. MS: 280.2 $(M+H)^+$.

**Step 3: Preparation of Intermediate 34-4**

The title compound **Intermediate 34-4** was prepared in analogy to the preparation of **Intermediate 32-5** by using **Intermediate 34-3** instead of **Intermediate 32-4.** MS: 266.1 $(M+H)^+$.

**Intermediate 46-9**

**(8S,8aR)-2-(3-(methylsulfonamido)bicyclo[1.1.1]pentan-1-yl)-3-oxooctahydroimidazo[1,5-a]pyrazine-8-carboxylic acid**

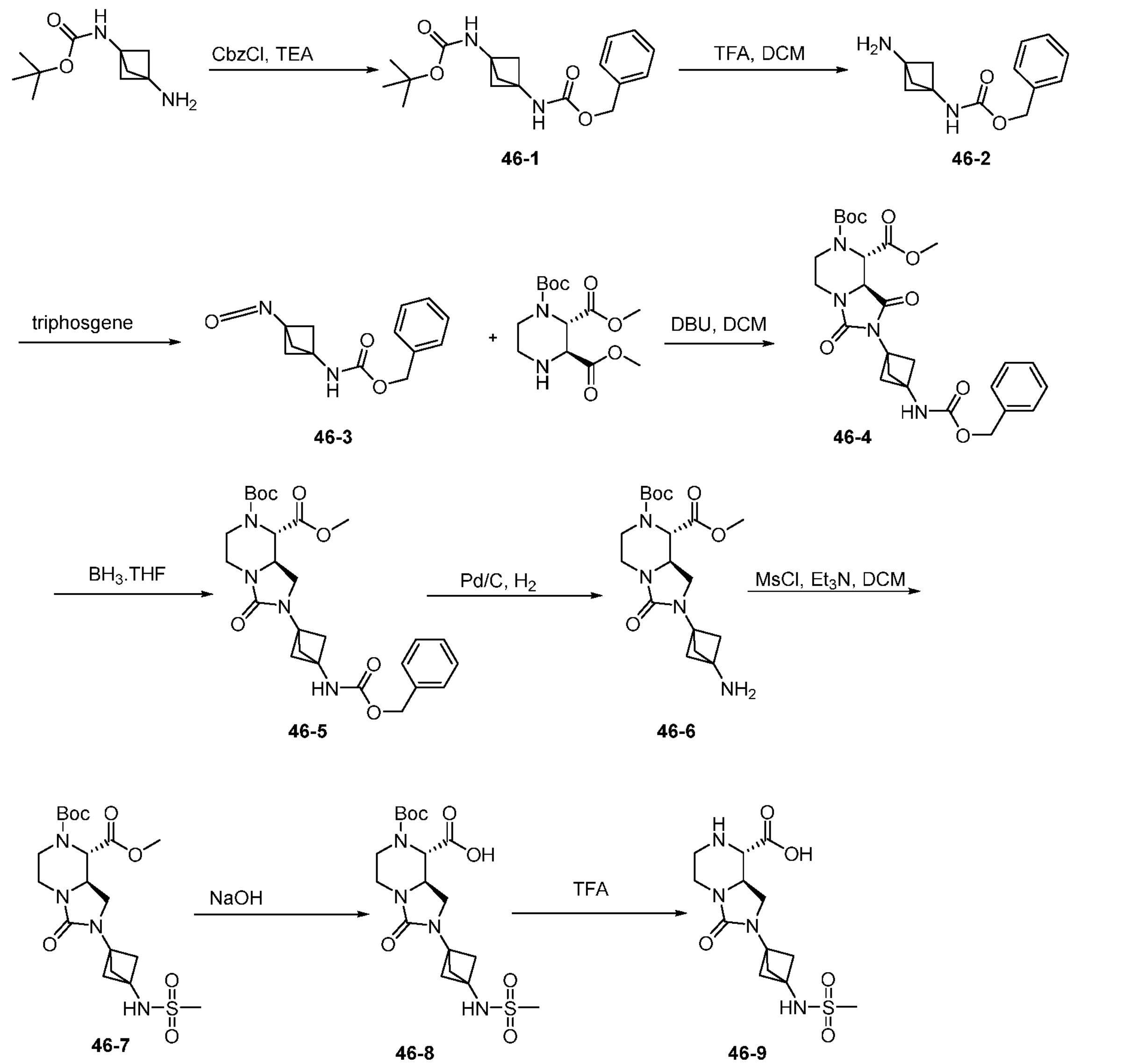


46-1

46-2

46-3

46-4

46-5

46-6

46-7

46-8

46-9

**Step 1: Preparation of Intermediate 46-1**

To a stirred mixture solution of tert-butyl (3-aminobicyclo[1.1.1]pentan-1-yl)carbamate (5.0 g, 25.2 mmol) in THF (80 mL) was added TEA (2.81 g, 27.7 mmol) and benzyl carbonochloridate (4.52 g, 26.5 mmol) at 0 °C, then the resulting mixture was stirred at 20 °C for 12 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to affordbenzyl tert-butyl bicyclo[1.1.1]pentane-1,3-diyldicarbamate **(Intermediate 46-1**,6.34 g, 76%) as a yellow oil. MS:333.4 $(M+ H)^+$.

**Step 2: Preparation of Intermediate 46-2**

The title compound **Intermediate 46-2** was prepared in analogy to the preparation of **Intermediate 14-3** by using **Intermediate 46-1** instead of **Intermediate 14-2.** MS: 233.1 $(M+H)^+$.

**Step 3: Preparation of Intermediate 46-3**

To a stirred mixture solution of benzyl (3-aminobicyclo[1.1.1]pentan-1-yl)carbamate (**Intermediate 46-2**,4.43 g, 19.1 mmol) and triphosgene (1.98 g, 6.685 mmol in DCM (40 mL) was added 10% $NaHCO_3$ solution (40 mL) at 0 °C, then the resulting mixture was stirred at 0 °C for 1 hour. After the reaction was completed, the reaction mixture was

poured into ice-water and extracted with EA. The combined organic layers were washed with brine, dried over anhydrous $Na_2SO_4$, filtered and concentrated in vacuo to affordbenzyl (3-isocyanatobicyclo[1.1.1]pentan-1-yl)carbamate (**Intermediate 46-3**, 4.93 g) as a yellow oil.

**Step 4: Preparation of Intermediate 46-4**

To a stirred mixture solution of benzyl (3-isocyanatobicyclo[1.1.1]pentan-1-yl)carbamate (**Intermediate 46-3**, 4.93 g, 19.1 mmol) in DCM (40 mL) was added a solution of 1-(tert-butyl) 2,3-dimethyl (2S,3S)-piperazine-1,2,3-tricarboxylate ((5.77 g, 19.1 mmol) in DCM (10 mL) and DBU (872 mg, 5.73 mmol) at 0 °C, then the resulting mixture was stirred at 20 °C for 12 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to afford7-(tert-butyl) 8-methyl (8S,8aS)-2-(3-(((benzyloxy)carbonyl)amino)bicyclo[1.1.1]pentan-1-yl)-1,3-dioxohexahydroimidazo[1,5-a]pyrazine-7,8(1H)-dicarboxylate (**Intermediate 46-4**,3.32 g, 33%) as a yellow oil. MS: 529.2 $(M+ H)^+$.

**Step 5: Preparation of Intermediate 46-5**

To a stirred mixture solution of 7-(tert-butyl) 8-methyl (8S,8aS)-2-(3-(((benzyloxy)carbonyl)amino)bicyclo[1.1.1]pentan-1-yl)-1,3-dioxohexahydroimidazo[1,5-a]pyrazine-7,8(1H)-dicarboxylate (**Intermediate 46-4**,3.32 g, 6.30 mmol) in THF (30 mL) was added $BF_3 \cdot Et_2O$ (0.5 mL) and 1.0 M $BH_3$-THF solution (24 mL, 24 mmol) at 0 °C, then the resulting mixture was stirred at 20 °C for 12 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to afford7-(tert-butyl) 8-methyl (8S,8aR)-2-(3-(((benzyloxy)carbonyl)amino)bicyclo[1.1.1]pentan-1-yl)-3-oxohexahydroimidazo[1,5-a]pyrazine-7,8(1H)-dicarboxylate (**Intermediate 46-5**, 1.32 g, 40%) as a white solid. MS: 515.2 $(M+H)^+$.

**Step 6: Preparation of Intermediate 46-6**

A mixture solution of 7-(tert-butyl) 8-methyl (8S,8aR)-2-(3-(((benzyloxy)carbonyl)amino)bicyclo[1.1.1]pentan-1-yl)-3-oxohexahydroimidazo[1,5-a]pyrazine-7,8(1H)-dicarboxylate (**Intermediate 46-5**, 650 mg, 1.264 mmol)and Pd/C (30 mg) in MeOH (10 mL) under $H_2$ atmosphere was stirred at 45 °C for 2 hours. After the reaction was completed, filtered and concentrated in vacuo to give a crude product, which was purified by silica gel flash chromatography to afford7-(tert-butyl) 8-methyl (8S,8aR)-2-(3-aminobicyclo[1.1.1]pentan-1-yl)-3-oxohexahydroimidazo[1,5-a]pyrazine-7,8(1H)-dicarboxylate (**Intermediate 46-6**,450 mg, 93%) as a yellow oil. MS: 381.2 $(M+H)^+$.

**Step 7: Preparation of Intermediate 46-7**

To a stirred mixture solution of 7-(tert-butyl) 8-methyl (8S,8aR)-2-(3-aminobicyclo[1.1.1]pentan-1-yl)-3-oxohexahydroimidazo[1,5-a]pyrazine-7,8(1H)-dicarboxylate (**Intermediate 46-6**,200 mg, 0.526 mmol) in DCM (5 mL) was added TEA (159 mg, 1.579 mmol) and MsCl (90.0 mg, 0.789 mmol) at 0 °C and the resulting mixture was stirred at 0 °C for 2 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford 7-(tert-butyl) 8-methyl (8S,8aR)-2-(3-(methylsulfonamido)bicyclo[1.1.1]pentan-1-yl)-3-oxohexahydroimidazo[1,5-a]pyrazine-7,8(1H)-dicarboxylate (**Intermediate 46-7**,210 mg, 87%) as a yellow oil. MS: 459.2 $(M+H)^+$.

**Step 8: Preparation of Intermediate 46-8**

To a stirred mixture solution of 7-(tert-butyl) 8-methyl (8S,8aR)-2-(3-(methylsulfonamido)bicyclo[1.1.1]pentan-1-yl)-3-oxohexahydroimidazo[1,5-a]pyrazine-7,8(1H)-dicarboxylate (**Intermediate 46-7**,210 mg, 0.457 mmol) in MeOH (5 mL) was added NaOH (73.2 mg, 1.830 mmol) at 20 °C, then the resulting mixture was stirred at 50 °C for 10 hours. After the reaction was completed, the reaction mixture was poured into ice-water and extracted with EA. The combined organic layers were washed with 1N HCl solution, brine, dried over anhydrous $Na_2SO_4$, filtered and concentrated in vacuo to give a crude product, which was purified by silica gel flash chromatography to afford(8S,8aR)-7-(tert-butoxycarbonyl)-2-(3-(methylsulfonamido)bicyclo[1.1.1]pentan-1-yl)-3-oxooctahydroimidazo[1,5-a]pyrazine-8-carboxylic acid (**Intermediate 46-8**, 40 mg, 19%) as a yellow oil. MS: 445.1 $(M+H)^+$.

**Step 9: Preparation of Intermediate 46-9**

The title compound **Intermediate 46-9** was prepared in analogy to the preparation of **Intermediate 1-5** by using **Intermediate 46-8** instead of **Intermediate 1-4.** MS: 345.2 $(M+H)^+$.

**Intermediate 47-9**

**(8S,8aR)-2-(3-(methoxymethyl)bicyclo[1.1.1]pentan-1-yl)-3-oxooctahydroimidazo[1,5-a]pyrazine-8-carboxylic acid**

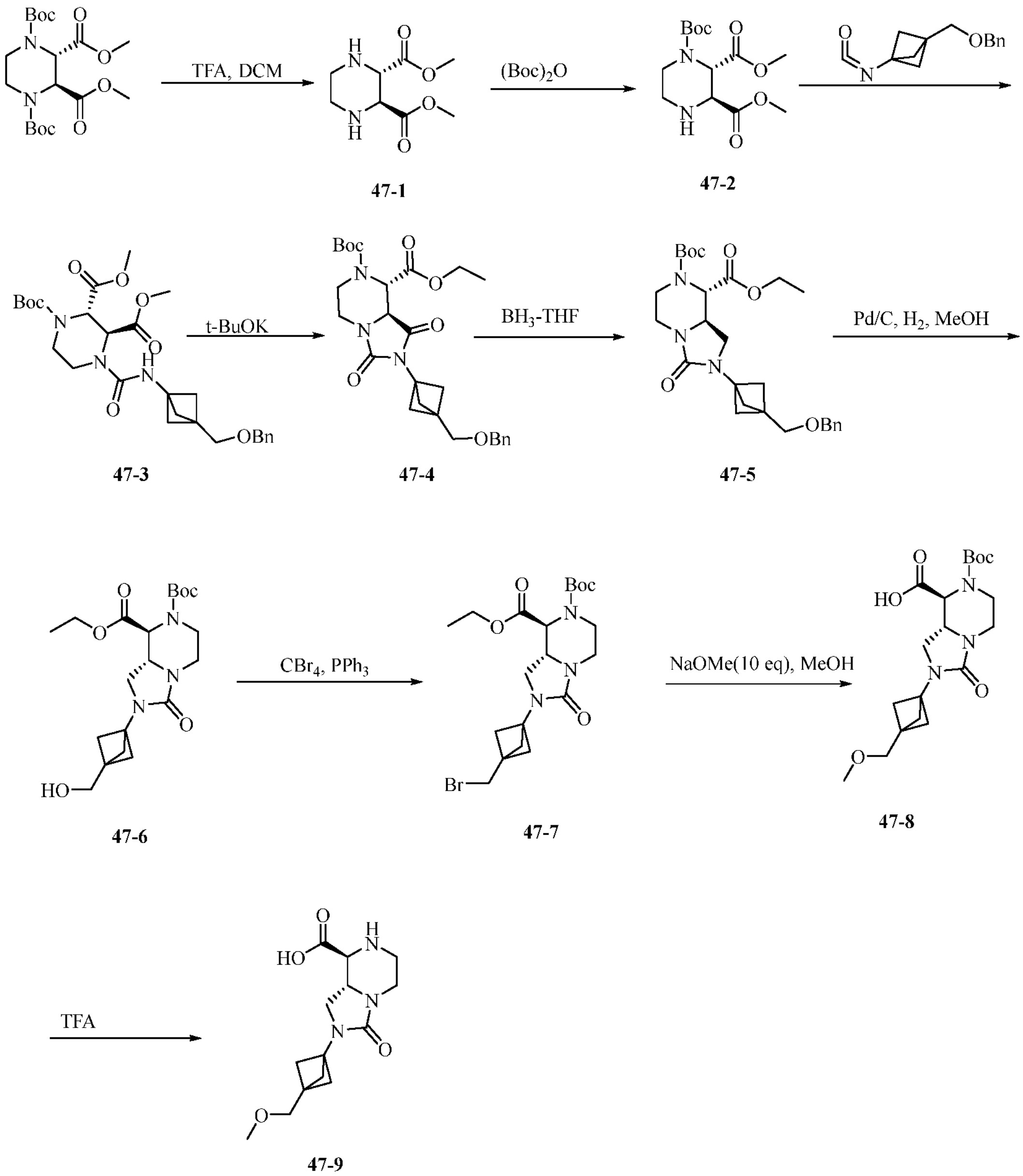


47-1 47-2 47-3 47-4 47-5 47-6 47-7 47-8 47-9

**Step 1: Preparation of Intermediate 47-1**

A mixture solution of 1,4-di-tert-butyl 2,3-dimethyl (2S,3S)-piperazine-1,2,3,4-tetracarboxylate (8 g, 19.88 mmol) in DCM (100 mL) and TFA (20 mL) was stirred at 40 °C for 12 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel

flash chromatography to afforddimethyl (2S,3S)-piperazine-2,3-dicarboxylate (**Intermediate 47-1**,3.5 g, 87%) as a yellow oil. MS: 203.1 (M+ H)$^+$.

**Step 2: Preparation of Intermediate 47-2**

To a stirred mixture solution of dimethyl (2S,3S)-piperazine-2,3-dicarboxylate (**Intermediate 47-1**,4 g, 19.78 mmol) in DCM (30 mL) was added TEA (5.99 g, 59.3 mmol) and BoczO (4.32 g, 19.78 mmol) at 0 °C and the resulting mixture was stirred at 20 °C for 2 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to afford1-(tert-butyl) 2,3-dimethyl (2S,3S)-piperazine-1,2,3-tricarboxylate (**Intermediate 47-2**,4.2 g, 70.2%) as a yellow oil. MS: 303.1 (M+ H)$^+$.

**Step 3: Preparation of Intermediate 47-3**

To a stirred mixture solution of 1-(tert-butyl) 2,3-dimethyl (2S,3S)-piperazine-1,2,3-tricarboxylate (**Intermediate 47-2**,2.95 g, 9.77 mmol) in DCM (30 mL) was added 1-((benzyloxy)methyl)-3-isocyanatobicyclo[1.1.1]pentane (2.8 g, 12.21 mmol) at 0 °C and the resulting mixture was stirred at 0 °C for 2 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to afford1-(tert-butyl) 2,3-dimethyl (2S,3S)-4-((3-((benzyloxy)methyl)bicyclo[1.1.1]pentan-1-yl)carbamoyl)piperazine-1,2,3-tricarboxylate (**Intermediate 47-3**,5.2 g, 80%) as a yellow oil. MS: 523.3 (M+ H)$^+$.

**Step 4: Preparation of Intermediate 47-4**

To a stirred mixture solution of 1-(tert-butyl) 2,3-dimethyl (2S,3S)-4-((3-((benzyloxy)methyl)bicyclo[1.1.1]pentan-1-yl)carbamoyl)piperazine-1,2,3-tricarboxylate (**Intermediate 47-3**,5.2 g, 9.78 mmol) in Ethanol (30 mL) was added t-BuOK (0.220 g, 1.956 mmol) at 0 °C and the resulting mixture was stirred at 0 °C for 1 hour. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to afford7-(tert-butyl) 8-ethyl (8S,8aS)-2-(3-((benzyloxy)methyl)bicyclo[1.1.1]pentan-1-yl)-1,3-dioxohexahydroimidazo[1,5-a]pyrazine-7,8(1H)-dicarboxylate (**Intermediate 47-4**,3.4 g, 67.7%) as a yellow oil. MS: 514.2 (M+ H)$^+$.

**Step 5: Preparation of Intermediate 47-6**

The title compound **Intermediate 47-6** was prepared in analogy to the preparation of **Intermediate 46-6** by using **Intermediate 47-4** instead of **Intermediate 46-4.** MS: 410.2 (M+H)$^+$.

**Step 6: Preparation of Intermediate 47-7**

The title compound **Intermediate 47-7** was prepared in analogy to the preparation of **Intermediate 4-1** by using **Intermediate 47-6** instead of **Intermediate 2-1.** MS: 472.1 (M+H)$^+$.

**Step 7: Preparation of Intermediate 47-8**

To a stirred mixture solution of 7-(tert-butyl) 8-ethyl (8S,8aR)-2-(3-(bromomethyl)bicyclo[1.1.1]pentan-1-yl)-3-oxohexahydroimidazo[1,5-a]pyrazine-7,8(1H)-dicarboxylate (**Intermediate 47-7**,110 mg, 0.233 mmol) in MeOH (5 mL) was added MeONa (25.2 mg, 0.466 mmol) at 0 °C and the resulting mixture was stirred at 70 °C for 48 hours. After the reaction was completed, the reaction mixture was poured into ice-water and extracted with EA. The combined organic layers were washed with 1N HCl solution, brine, dried over anhydrous $Na_2SO_4$, filtered and concentrated in vacuo to give a crude product, which was purified by silica gel flash chromatography to afford(8S,8aR)-7-(tert-butoxycarbonyl)-2-(3-(methoxymethyl)bicyclo[1.1.1]pentan-1-yl)-3-oxooctahydroimidazo[1,5-a]pyrazine-8-carboxylic acid (**Intermediate 47-8**, 92 mg) as a yellow oil. MS: 396.2 (M+ H)$^+$.

**Step 8: Preparation of Intermediate 47-9**

The title compound **Intermediate 47-9** was prepared in analogy to the preparation of **Intermediate 1-5** by using

**Intermediate 47-8** instead of **Intermediate 1-4.** MS: 296.2 $(M+H)^+$.

**Intermediate 31-1**

**ethyl (8S,8aR)-2-(3-(hydroxymethyl)bicyclo[1.1.1]pentan-1-yl)-3-oxooctahydroimidazo[1,5-a]pyrazine-8-carboxylate**

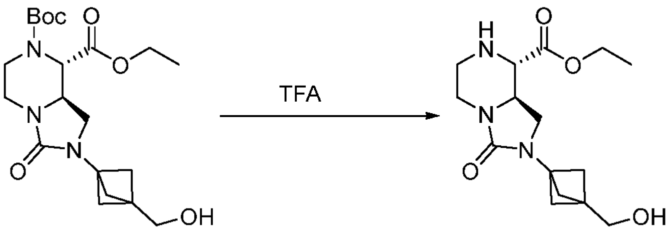


**47-6** **31-1**

The title compound **Intermediate 31-1** was prepared in analogy to the preparation of **Intermediate 1-5** by using **Intermediate 47-6** instead of **Intermediate 1-4.** MS: 310.2 $(M+H)^+$.

**Intermediate 33-2**

**(8S,8aR)-2-(3-(hydroxymethyl)bicyclo[1.1.1]pentan-1-yl)-3-oxooctahydroimidazo[1,5-a]pyrazine-8-carboxylic acid**

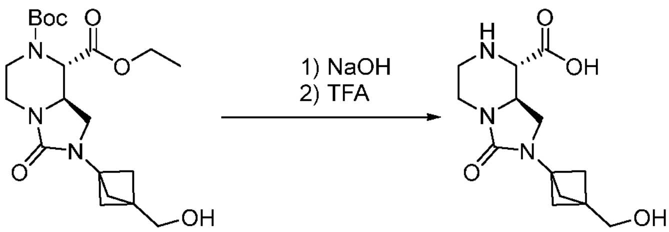


**47-6** **33-2**

The title compound **Intermediate 33-2** was prepared in analogy to the preparation of **Intermediate 1-5** by using **Intermediate 47-6** instead of **Intermediate 1-3.** MS: 282.2 $(M+H)^+$.

**Intermediate 50-5**

**2-hydroxy-2-(3-((S)-3-oxohexahydroimidazo[1,5-a]pyrazin-2(3H)-yl)bicyclo[1.1.1]pentan-1-yl)acetic acid**

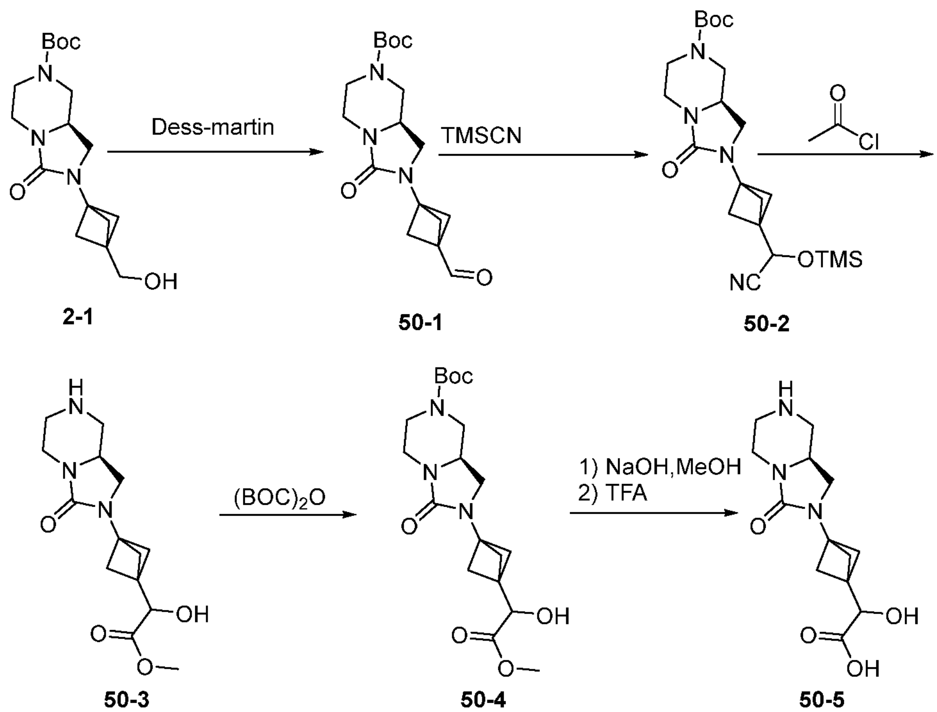


**Step 1: Preparation of Intermediate 50-1**

To a stirred mixture solution of tert-butyl (R)-2-(3-(hydroxymethyl)bicyclo[1.1.1]pentan-1-yl)-3-oxohexahydroimidazo[1,5-a]pyrazine-7(1H)-carboxylate (**Intermediate 2-1**,100 mg, 0.296 mmol) in DCM (5 mL) was added dess-martinperiodinane (151 mg, 0.356 mmol) at 0 °C and the resulting mixture was stirred at 0 °C for 3 hours. After the reaction was completed, the reaction mixture was poured into ice-water and extracted with EA. The combined organic layers were washed with 1N HCl solution, brine, dried over anhydrous $Na_2SO_4$, filtered and concentrated in vacuo to give a crude product, which was purified by silica gel flash chromatography to affordtert-butyl (R)-2-(3-formylbicyclo[1.1.1]pentan-1-yl)-3-oxohexahydroimidazo[1,5-a]pyrazine-7(1H)-carboxylate (**Intermediate 50-1,** 76 mg, 76%) as a yellow oil. MS: 336.4 $(M+H)^+$.

**Step 2: Preparation of Intermediate 50-2**

To a stirred mixture solution of tert-butyl (R)-2-(3-formylbicyclo[1.1.1]pentan-1-yl)-3-oxohexahydroimidazo[1,5-a]pyrazine-7(1H)-carboxylate (**Intermediate 50-1**,1.4 g, 4.17 mmol) in DCM (25 mL) was added TEA (0.042 g, 0.417 mmol) and TMSCN (0.621 g, 6.26 mmol) at 0 °C and the resulting mixture was stirred at 20 °C for 3 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to affordtert-butyl (8aR)-2-(3-(cyano((trimethylsilyl)oxy)methyl)bicyclo[1.1.1]pentan-1-yl)-3-oxohexahydroimidazo[1,5-a]pyrazine-7(1H)-carboxylate (**Intermediate 50-2**, 1.08 g, 60%) as a yellow oil. MS: 435.6 $(M+H)^+$.

**Step 3: Preparation of Intermediate 50-3**

To a stirred mixture solution of tert-butyl (8aR)-2-(3-(cyano((trimethylsilyl)oxy)methyl)bicyclo[1.1.1]pentan-1-yl)-3-oxohexahydroimidazo[1,5-a]pyrazine-7(1H)-carboxylate (**Intermediate 50-2**, 1.08 g, 2.485 mmol) in MeOH (25 mL) was added AcCl (5 mL) at 0 °C and the resulting mixture was stirred at 70 °C for 13 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford methyl 2-hydroxy-2-(3-((S)-3-oxohexahydroimidazo[1,5-a]pyrazin-2(3H)-yl)bicyclo[1.1.1]pentan-1-yl)acetate (**Intermediate 50-3**, 1.08 g) as a yellow oil. MS: 296.6 $(M+H)^+$.

**Step 4: Preparation of Intermediate 50-4**

To a stirred mixture solution of methyl 2-hydroxy-2-(3-((S)-3-oxohexahydroimidazo[1,5-a]pyrazin-2(3H)-yl)bicyclo[1.1.1]pentan-1-yl)acetate (**Intermediate 50-3**, 733 mg, 2.485 mmol) in DCM (10 mL) was added a solution of $NaHCO_3$ (5 mL) and BoczO (1.085 g, 4.97 mmol) at 0 °C and the resulting mixture was stirred at 20 °C for 2 hours. After the reaction was completed, the reaction mixture was poured into ice-water and extracted with EA. The combined organic layers were washed with brine, dried over anhydrous $Na_2SO_4$, filtered and concentrated in vacuo to give a crude product, which was purified by silica gel flash chromatography to affordtert-butyl (8aR)-2-(3-(1-hydroxy-2-methoxy-2-oxoethyl)bicyclo[1.1.1]pentan-1-yl)-3-oxohexahydroimidazo[1,5-a]pyrazine-7(1H)-carboxylate (**Intermediate 50-4**, 400 mg, 41%) as a yellow oil. MS: 396.2 $(M+H)^+$.

**Step 5: Preparation of Intermediate 50-5**

The title compound **Intermediate 50-5** was prepared in analogy to the preparation of **Intermediate 1-5** by using **Intermediate 50-4** instead of **Intermediate 1-3.** MS: 282.2 $(M+H)^+$.

**Intermediate 51-2**

**2-methoxy-2-(3-((S)-3-oxohexahydroimidazo[1,5-a]pyrazin-2(3H)-yl)bicyclo[1.1.1]pentan-1-yl)acetic acid**

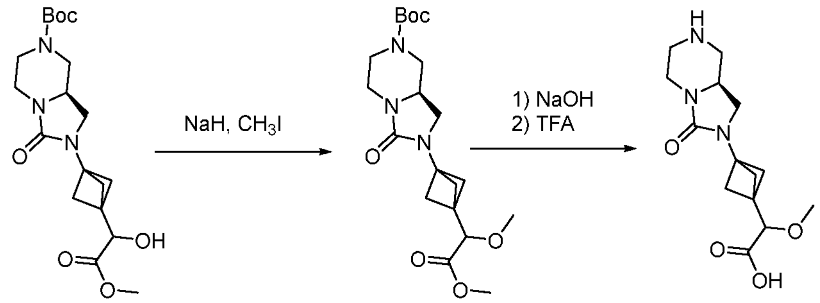


50-4 51-1 51-2

**Step 1: Preparation of Intermediate 51-1**

To a stirred mixture solution of tert-butyl (8aR)-2-(3-(1-hydroxy-2-methoxy-2-oxoethyl)bicyclo[1.1.1]pentan-1-yl)-3-oxohexahydroimidazo[1,5-a]pyrazine-7(1H)-carboxylate (**Intermediate 50-4,** 150 mg, 0.379 mmol) in THF (5 mL) was added NaH (21.8 mg, 0.569 mmo) at 0 °C and the resulting mixture was stirred at 20 °C for 1 hour. Then $CH_3I$ (80.7 mg, 0568 mmol) was added to the above mixture solution at 0 °C and the resulting mixture was stirred at 20 °C for 15 hours. After the reaction was completed, the reaction mixture was quenched by adding ice-water and extracted with EA. The combined organic layers were washed with a solution of $NaHCO_3$, brine, dried over anhydrous $Na_2SO_4$, filtered and concentrated in vacuo to give a crude product, which was purified by silica gel flash chromatography to afford tert-butyl (8aR)-2-(3-(1,2-dimethoxy-2-oxoethyl)bicyclo[1.1.1]pentan-1-yl)-3-oxohexahydroimidazo[1,5-a]pyrazine-7(1H)-carboxylate (**Intermediate 51-1,** 50 mg, 34%) as a colorless oil. MS: 410.2 $(M+H)^+$.

**Step 2: Preparation of Intermediate 51-2**

The title compound **Intermediate 51-2** was prepared in analogy to the preparation of **Intermediate 1-5** by using **Intermediate 51-1** instead of **Intermediate 1-3.** MS: 296.2 $(M+H)^+$.

**Intermediate 43-7**

**2-(3-((8S,8aR)-8-(methoxymethyl)-3-oxohexahydroimidazo[1,5-a]pyrazin-2(3H)-yl)bicyclo[1.1.1]pentan-1-yl)acetic acid**

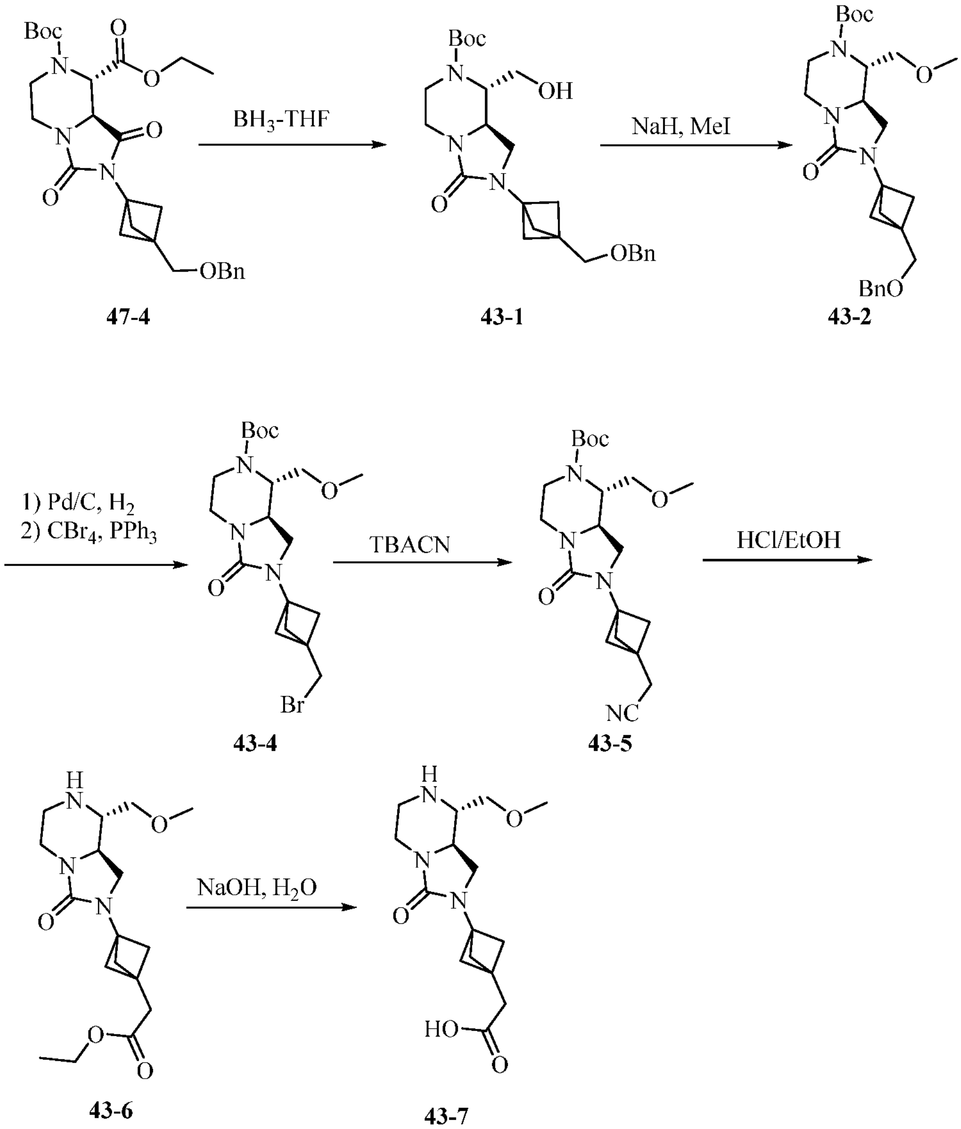


47-4 43-1 43-2 43-4 43-5 43-6 43-7

**Step 1: Preparation of Intermediate 43-1**

The title compound **Intermediate 43-1** was prepared in analogy to the preparation of **Intermediate 46-5** by using **Intermediate 47-4** instead of **Intermediate 46-4.** MS: 458.1 $(M+H)^+$.

**Step 2: Preparation of Intermediate 43-2**

The title compound **Intermediate 43-2** was prepared in analogy to the preparation of **Intermediate 51-1** by using **Intermediate 43-1** instead of **Intermediate 50-4.** MS: 472.3 $(M+H)^+$.

**Step 3: Preparation of Intermediate 43-4**

The title compound **Intermediate 43-4** was prepared in analogy to the preparation of **Intermediate 47-7** by using **Intermediate 43-2** instead of **Intermediate 47-5.** MS: 444.3 $(M+H)^+$.

**Step 4: Preparation of Intermediate 43-5**

The title compound **Intermediate 43-5** was prepared in analogy to the preparation of **Intermediate 34-2** by using **Intermediate 43-4** instead of **Intermediate 4-1.** MS: 391.2 $(M+H)^+$.

**Step 5: Preparation of Intermediate 43-6**

The title compound **Intermediate 43-6** was prepared in analogy to the preparation of **Intermediate 50-3** by using **Intermediate 43-5** instead of **Intermediate 50-2.** MS: 338.2 $(M+H)^+$.

**Step 6: Preparation of Intermediate 43-7**

The title compound **Intermediate 43-7** was prepared in analogy to the preparation of **Intermediate 46-8** by using **Intermediate 43-6** instead of **Intermediate 46-7.** MS: 310.2 $(M+H)^+$.

**Intermediate A9**

**ethyl (S)-6-(bromomethyl)-4-(3,4-difluoro-2-methylphenyl)-2-(thiazol-2-yl)-1,4-dihydropyrimidine-5-carboxylate**

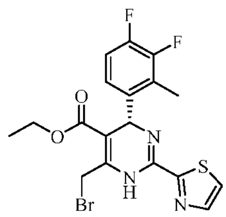

**A9**

The title compound **Intermediate A9** as a yellow solid was prepared in analogy to the preparation of **Intermediate A1** by using3,4-difluoro-2-methylbenzaldehyde instead of 3-fluoro-2-methylbenzaldehyde.

**Intermediate 52-5**

**(S)-4-(3-oxohexahydroimidazo[1,5-a]pyrazin-2(3H)-yl)bicyclo[2.2.2]octane-1-carboxylic acid**

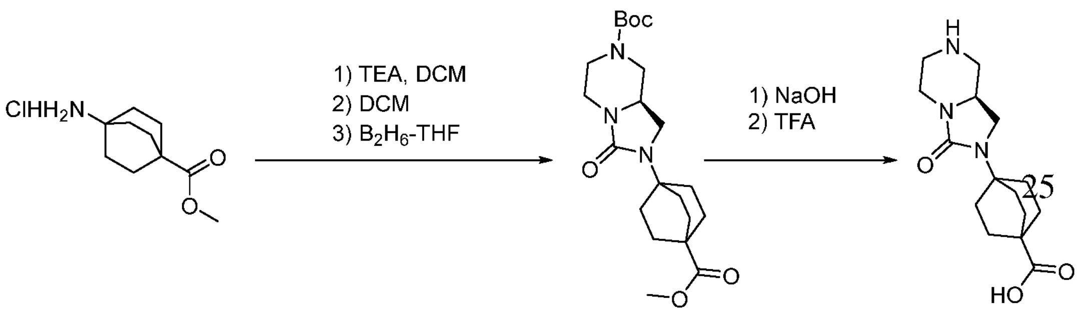


**52-3** **52-5**

The title compound **Intermediate 52-5** was prepared in analogy to the preparation of **Intermediate 1-5** by usingmethyl 4-aminobicyclo[2.2.2]octane-1-carboxylate hydrochloride instead of methyl 3-aminobicyclo[1.1.1]pentane-1-carboxylate hydrochloride. MS: 294.2 $(M+H)^+$.

**Intermediate 53-5**

**(S)-4-(3-oxohexahydroimidazo[1,5-a]pyrazin-2(3H)-yl)bicyclo[2.1.1]hexane-1-carboxylic acid**

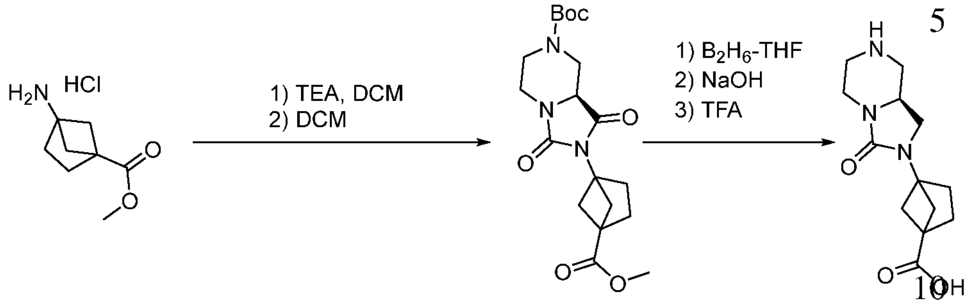


53-2 53-5

The title compound **Intermediate 53-5** was prepared in analogy to the preparation of **Intermediate 1-5** by usingmethyl 4-aminobicyclo[2.1.1]hexane-1-carboxylate hydrochloride instead of methyl 3-aminobicyclo[1.1.1]pentane-1-carboxylate hydrochloride. MS: 266.2 $(M+H)^+$.

**Intermediate 54-5**

**(S)-2-(4-(3-oxohexahydroimidazo[1,5-a]pyrazin-2(3H)-yl)bicyclo[2.1.1]hexan-1-yl)acetic acid**

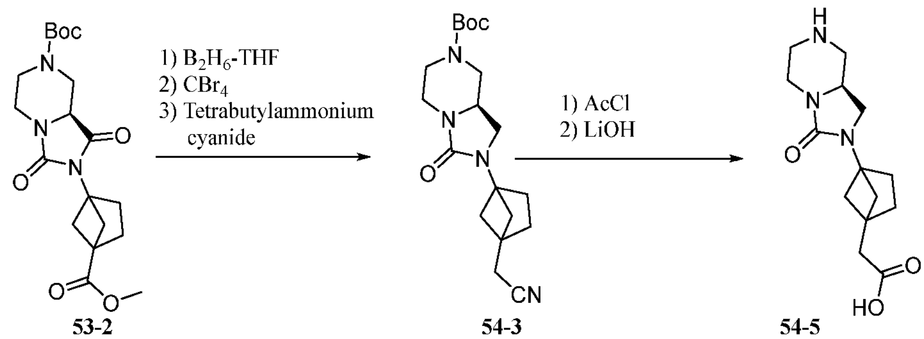


53-2 54-3 54-5

The title compound **Intermediate 54-5** was prepared in analogy to the preparation of **Intermediate 34-4** by using **Intermediate 53-2** instead of **Intermediate 1-2.** MS: 280.2 $(M+H)^+$.

**Intermediate 55-5**

**(S)-4-(3-oxohexahydroimidazo[1,5-a]pyrazin-2(3H)-yl)bicyclo[2.2.1]heptane-1-carboxylic acid**

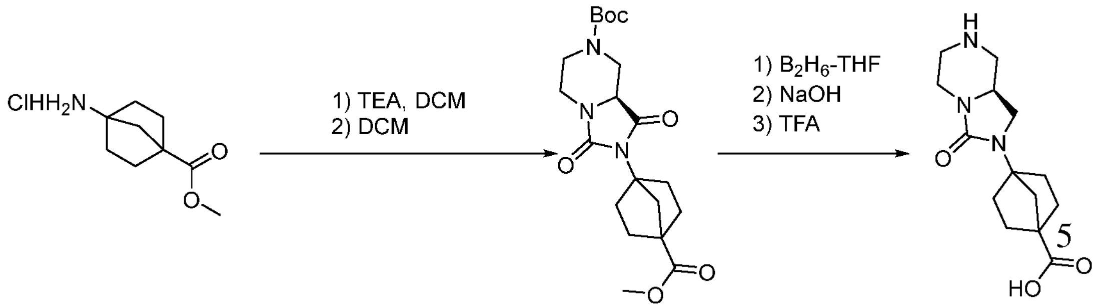


55-2 55-5

The title compound **Intermediate 55-5** was prepared in analogy to the preparation of **Intermediate 1-5** by usingmethyl 4-aminobicyclo[2.2.1]heptane-1-carboxylate hydrochloride instead of methyl 3-aminobicyclo[1.1.1]pentane-1-carboxylate hydrochloride. MS: 280.2 $(M+H)^+$.

**Intermediate 56-5**

**(S)-4-(3-oxohexahydroimidazo[1,5-a]pyrazin-2(3H)-yl)cubane-1-carboxylic acid**

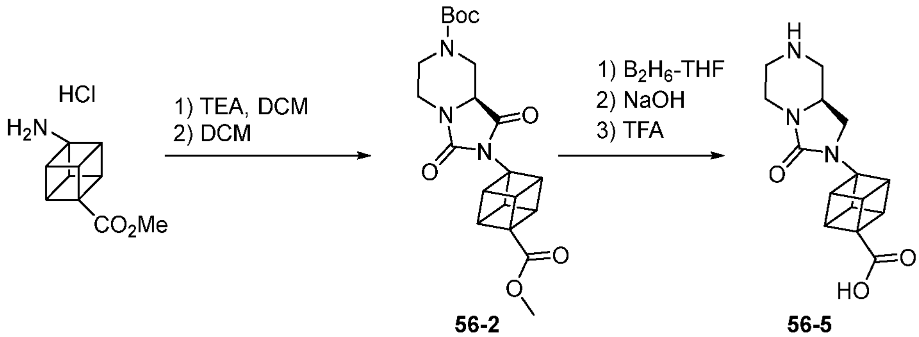


56-2 56-5

The title compound **Intermediate 56-5** was prepared in analogy to the preparation of **Intermediate 1-5** by usingmethyl (1r,2R,3R,4s,5s,6S,7S,8r)-4-aminocubane-1-carboxylate hydrochloride instead of methyl 3-aminobicyclo[1.1.1]pentane-1-carboxylate hydrochloride. MS: 288.2 $(M+H)^+$.

**Intermediate 57-5**

**(S)-2-(4-(3-oxohexahydroimidazo[1,5-a]pyrazin-2(3H)-yl)bicyclo[2.2.1]heptan-1-yl)acetic acid**

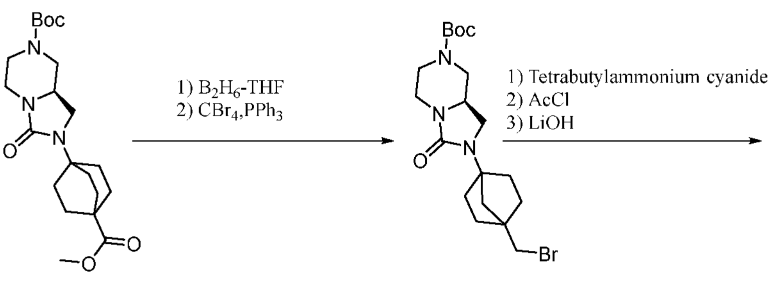


52-3 57-2 57-5

The title compound **Intermediate 57-5** was prepared in analogy to the preparation of **Intermediate 34-4** by using **Intermediate 52-3** instead of **Intermediate 1-2.** MS: 294.1 $(M+H)^+$.

**Example 1**

**Compound 1: 3-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentane-1-carboxylic acid**

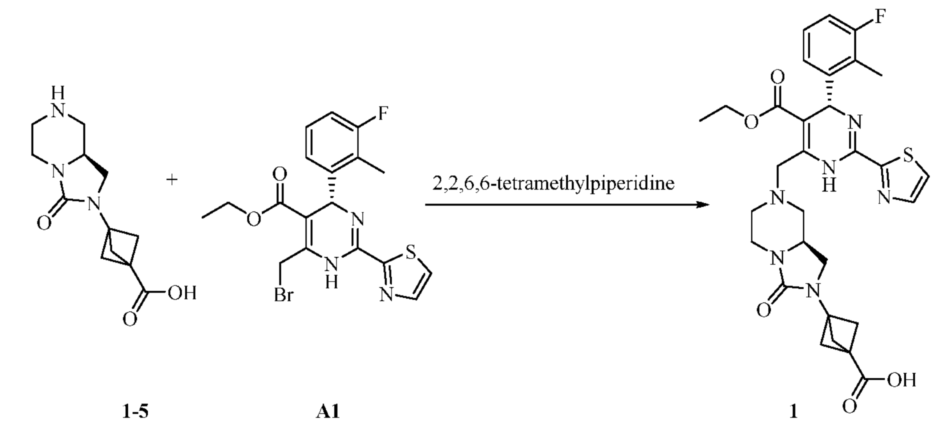


1-5 A1 1

To a stirred mixture solution of (S)-3-(3-oxohexahydroimidazo[1,5-a]pyrazin-2(3H)-yl)bicyclo[1.1.1]pentane-1-carboxylic acid (**Intermediate 1-5,** 85 mg, 0.339 mmol) in DCM (6 mL) was added 2,2,6,6-tetramethylpiperidine (85 mg, 0.6 mmol) and a solution of ethyl (S)-6-(bromomethyl)-4-(3-fluoro-2-methylphenyl)-2-(thiazol-2-yl)-1,4-dihydropyrimidine-5-carboxylate (**Intermediate A1**,149 mg, 0.339 mmol) in DCM (6 mL) at 0 °C, then the resulting mixture was stirred at 20 °C for 12 hours. After the reaction was completed, the reaction mixture was adjusted pH with 1N HCl solution and extracted with EA. The combined organic layers were washed with brine, dried over anhydrous $Na_2SO_4$, filtered and concentrated in vacuo to give a crude product, which was purified by pre-HPLC to afford3-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentane-1-carboxylic acid(**Compound 1,** 30 mg, 14.5%) as a yellow solid. MS: 609.3 $(M+H)^+$. $^1H$ NMR ($CD_3OD$, 400 MHz) $\delta$ 7.92 (d, J = 3.2 Hz, 1H), 7.72 (d, J = 3.2 Hz, 1H), 7.17-7.09 (m, 2H), 6.96-6.89 (m, 1H), 5.99 (s, 1H), 4.15 (d, J = 16.0 Hz, 1H), 4.43 (q, J = 7.2 Hz, 2H), 3.96 (d, J = 16.0 Hz, 1H), 3.88-3.78 (m, 2H), 3.42 (t, J = 8.8 Hz, 1H), 3.19-3.09 (m, 1H), 3.05-3.01 (m, 1H), 2.95-2.88 (m, 2H), 2.51 (d, J = 2.0 Hz, 3H), 2.37-2.26 (m, 1H), 2.20 (s, 6H), 2.19 (t, J = 10.8 Hz, 1H), 1.18 (t, J = 7.2 Hz, 3H).

## Example 2

**Compound 2: ethyl (S)-4-(3-fluoro-2-methylphenyl)-6-(((S)-2-(3-(hydroxymethyl)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

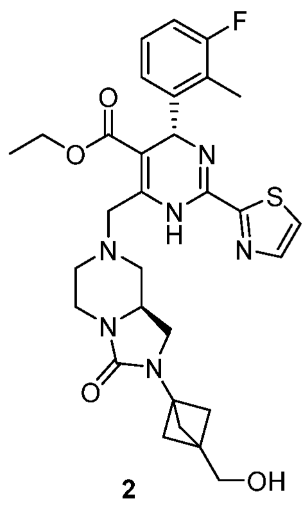

2

The title **Compound 2** as a yellow foam was prepared in analogy to the preparation of **Compound 1** by using (S)-2-(3-(hydroxymethyl)bicyclo[1.1.1]pentan-1-yl)hexahydroimidazo[1,5-a]pyrazin-3(2H)-one(**Intermediate 2-2**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 2** (15 mg). MS: 595.3 $(M+H)^+$. $^1H$ NMR ($CD_3OD$, 400 MHz) $\delta$ 7.92 (d, J = 3.2 Hz, 1H), 7.72 (d, J

= 3.2 Hz, 1H), 7.14-7.07 (m, 2H), 6.96-6.88 (m, 1H), 5.98 (s, 1H), 4.09 (d, J = 16.0 Hz, 1H), 4.42 (q, J = 7.2 Hz, 2H), 3.87 (d, J = 16.0 Hz, 1H), 3.86-3.72 (m, 2H), 3.61 (s, 2H), 3.47 (t, J = 8.8 Hz, 1H), 3.14-3.09 (m, 1H), 3.07-3.02 (m, 1H), 2.99-2.81 (m, 2H), 2.52 (d, J = 2.0 Hz, 3H), 2.35-2.32 (m, 1H), 2.16 (t, J = 10.8 Hz, 1H), 1.96 (s, 6H), 1.18 (t, J = 7.2 Hz, 3H).

## Example 3

**Compound 3:ethyl (S)-4-(3-fluoro-2-methylphenyl)-6-(((S)-2-(3-(methoxymethyl)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

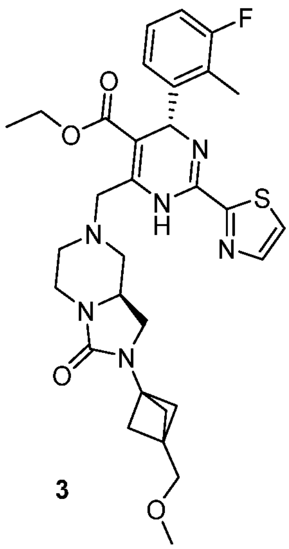

**3**

The title **Compound 3** as a yellow foam was prepared in analogy to the preparation of **Compound 1** by using (S)-2-(3-(methoxymethyl)bicyclo[1.1.1]pentan-1-yl)hexahydroimidazo[1,5-a]pyrazin-3(2H)-one(**Intermediate 3-2)** instead of (S)-3-(3-oxohexahydroimidazo[1,5-a]pyrazin-2(3H)-yl)bicyclo[1.1.1]pentane-1-carboxylic acid **(Intermediate 1-5).**

**Compound 3** (18.6 mg). MS: 609.1 $(M+H)^+$. $^1$H NMR (400 MHz, Methanol-$d_4$) $\delta$ 7.98 (d, $J$ = 3.1 Hz, 1H), 7.88 (d, $J$ = 3.1 Hz, 1H), 7.29 - 7.24 (m, 1H), 7.23 - 7.16 (m, 1H), 7.03 - 6.96 (m, 1H), 5.98 (s, 1H), 4.64 (s, 1H), 4.49 (d, $J$ = 16.1 Hz, 1H), 4.15 - 3.98 (m, 4H), 3.69 - 3.56 (m, 3H), 3.49 (s, 2H), 3.41 (d, $J$ = 12.5 Hz, 1H), 3.35 (s, 3H), 3.17 (dd, $J$ = 9.7, 3.8 Hz, 3H), 2.45 (d, $J$ = 2.1 Hz, 3H), 2.01 (s, 6H), 1.11 (t, $J$ = 7.1 Hz, 3H).

## Example 4

**Compound 4:3-(3-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)-2,2-dimethylpropanoic acid**

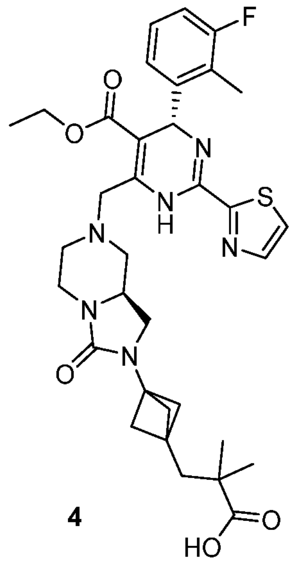

4

The title **Compound 4** as a yellow foam was prepared in analogy to the preparation of **Compound 1** by using (S)-2,2-dimethyl-3-(3-(3-oxohexahydroimidazo[1,5-a]pyrazin-2(3H)-yl)bicyclo[1.1.1]pentan-1-yl)propanoic acid(**Intermediate 4-4**) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 4** (16.7 mg). MS: 665.1 $(M+H)^+$. $^1H$ NMR (400 MHz, Methanol-$d_4$) δ 7.98 (d, *J* = 3.0 Hz, 1H), 7.88 (d, *J* = 3.0 Hz, 1H), 7.27 (d, *J* = 7.8 Hz, 1H), 7.19 (q, *J* = 7.3 Hz, 1H), 7.00 (t, *J* = 8.9 Hz, 1H), 5.98 (s, 1H), 4.70 (d, *J* = 16.1 Hz, 1H), 4.52 (d, *J* = 16.1 Hz, 1H), 4.06 (m, 4H), 3.69 (d, *J* = 12.0 Hz, 2H), 3.60 - 3.53 (m, 1H), 3.40 (t, *J* = 13.4 Hz, 1H), 3.25 - 3.08 (m, 3H), 2.45 (s, 3H), 2.00 (s, 6H), 1.86 (s, 2H), 1.18 (s, 6H), 1.10 (t, *J* = 7.1 Hz, 3H).

### Example 5

**Compound 5:3-(((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)methyl)bicyclo[1.1.1]pentane-1-carboxylic acid**

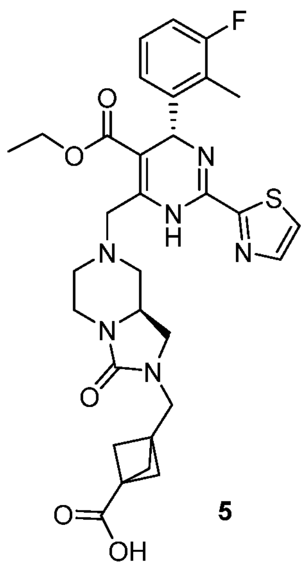

5

The title **Compound 5** as a yellow foam was prepared in analogy to the preparation of **Compound 1** by using (S)-3-((3-oxohexahydroimidazo[1,5-a]pyrazin-2(3H)-yl)methyl)bicyclo[1.1.1]pentane-1-carboxylic acid(**Intermediate 5-3**) instead of (S)-3-(3-oxohexahydroimidazo[1,5-a]pyrazin-2(3H)-yl)bicyclo[1.1.1]pentane-1-carboxylic acid **(Intermediate 1-5)**.

**Compound 5** (25.1 mg). MS: 623.1 (M+H)$^+$. $^1$H NMR (400 MHz, Methanol-$d_4$) δ 7.98 (d, *J* = 2.9 Hz, 1H), 7.88 (d, *J* = 3.1 Hz, 1H), 7.28 (d, *J* = 7.9 Hz, 1H), 7.24 - 7.17 (m, 1H), 7.00 (t, *J* = 8.9 Hz, 1H), 5.99 (s, 1H), 4.71 (dd, *J* = 16.1, 4.6 Hz, 1H), 4.53 (d, *J* = 16.0 Hz, 1H), 4.08 (q, *J* = 7.2 Hz, 4H), 3.70 (q, *J* = 8.7, 6.6 Hz, 3H), 3.51 - 3.32 (m, 2H), 3.29 - 3.07 (m, 4H), 2.45 (d, *J* = 2.0 Hz, 3H), 2.00 (d, *J* = 2.8 Hz, 6H), 1.11 (t, *J* = 7.1 Hz, 3H).

**Example 6**

**Compound 6:ethyl (S)-4-(3-fluoro-2-methylphenyl)-6-(((S)-3-oxo-2-(3-(pyrrolidine-1-carbonyl)bicyclo[1.1.1]pentan-1-yl)hexahydroimidazo[1,5-a]pyrazin-7(1H)-yl)methyl)-2-(thiazol-2-yl)-1,4-dihydropyrimidine-5-carboxylate**

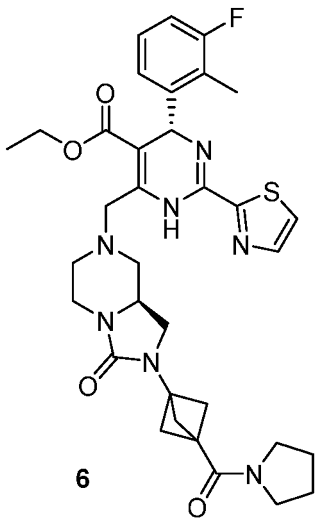

**6**

The title **Compound 6** as a yellow foam was prepared in analogy to the preparation of **Compound 1** by using (S)-2-(3-(pyrrolidine-1-carbonyl)bicyclo[1.1.1]pentan-1-yl)hexahydroimidazo[1,5-a]pyrazin-3(2H)-one **(Intermediate 6-2)** instead of (S)-3-(3-oxohexahydroimidazo[1,5-a]pyrazin-2(3H)-yl)bicyclo[1.1.1]pentane-1-carboxylic acid **(Intermediate 1-5).**

**Compound 6** (55.1 mg). MS: 662.3 (M+H)$^+$. $^1$H NMR (400 MHz, Methanol-$d_4$) δ7.99 (d, J = 3.0 Hz, 1H), 7.89 (d, J = 3.1 Hz, 1H), 7.27 (d, J = 7.8 Hz, 1H), 7.20 (m, 1H), 7.00 (t, J =9.0 Hz, 1H), 5.99 (s, 1H), 4.71 (d, J = 16.1 Hz, 1H), 4.54 (d, J = 16.1 Hz, 1H), 4.08 (m, 4H), 3.71 (d, J = 12.3 Hz, 3H), 3.65 - 3.54 (m, 3H), 3.43 (t, J = 6.9 Hz, 3H), 3.26 - 3.08 (m, 4H), 2.43 (d, J = 17.1 Hz, 9H), 1.99 (m, 2H), 1.85 (m, 2H), 1.11 (t, J = 7.1 Hz, 3H).

**Example 7**

**Compound 7: ethyl (S)-6-(((S)-2-(3-carbamoylbicyclo[1.1.1]pentan-1-yl)-3-oxohexahydroimidazo[1,5-a]pyrazin-7(1H)-yl)methyl)-4-(3-fluoro-2-methylphenyl)-2-(thiazol-2-yl)-1,4-dihydropyrimidine-5-carboxylate**

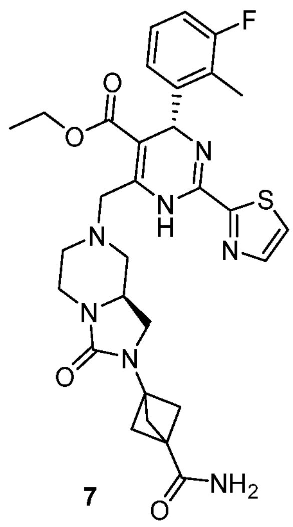

**7**

The title **Compound 7** as a yellow foam was prepared in analogy to the preparation of **Compound 1** by using (S)-3-(3-oxohexahydroimidazo[1,5-a]pyrazin-2(3H)-yl)bicyclo[1.1.1]pentane-1-carboxamide **(Intermediate 7-2)** instead of (S)-3-(3-oxohexahydroimidazo[1,5-a]pyrazin-2(3H)-yl)bicyclo[1.1.1]pentane-1-carboxylic acid **(Intermediate 1-5).**

**Compound 7** (25.1 mg). MS: 608.2 $(M+H)^+$. $^1H$ NMR (400 MHz, Methanol-$d_4$) $\delta$ 7.99 (d, J = 3.1 Hz, 1H), 7.89 (d, J = 3.1 Hz, 1H), 7.27 (d, J = 7.6 Hz, 1H), 7.20 (m, 1H), 7.00 (t, J = 8.9 Hz, 1H), 5.99 (s, 1H), 4.70 (d, J = 16.1 Hz, 1H), 4.53 (d, J = 16.0 Hz, 1H), 4.17 - 4.01 (m, 4H), 3.70 (d, J = 12.2 Hz, 2H), 3.61 (t, J = 9.0 Hz, 1H), 3.26 - 3.11 (m, 3H), 2.45 (d, J = 2.0 Hz, 3H), 2.31 (s, 6H), 1.11 (t, J = 7.1 Hz, 3H).

**Example 8**

**Compound 8:ethyl (S)-4-(3-fluoro-2-methylphenyl)-6-(((S)-2-(3-((methylsulfonyl)methyl)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

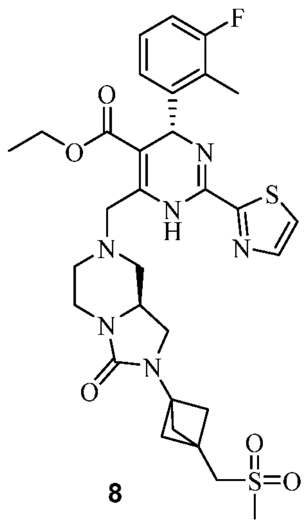

**8**

The title **Compound 8** as a yellow foam was prepared in analogy to the preparation of **Compound 1** by using (S)-2-(3-((methylsulfonyl)methyl)bicyclo[1.1.1]pentan-1-yl)hexahydroimidazo[1,5-a]pyrazin-3(2H)-one **(Intermediate 8-2)** instead of (S)-3-(3-oxohexahydroimidazo[1,5-a]pyrazin-2(3H)-yl)bicyclo[1.1.1]pentane-1-carboxylic acid **(Intermediate 1-5).**

**Compound 8** (17.3 mg). MS: 657.2 $(M+H)^+$. $^1H$ NMR (400 MHz, Methanol-$d_4$) $\delta$ 7.98 (d, J = 3.1 Hz, 1H), 7.88 (d, J = 3.1 Hz, 1H), 7.28 (d, J = 7.9 Hz, 1H), 7.20 (q, J = 7.4 Hz, 1H), 7.00 (t, J = 9.0 Hz, 1H), 5.99 (s, 1H), 4.72 (d, J = 16.0 Hz, 1H), 4.53 (d, J = 16.0 Hz, 1H), 4.22 - 3.97 (m, 4H), 3.72 (d, J = 12.1 Hz, 2H), 3.63 - 3.56 (m, 1H), 3.44 (d, J =

11.8 Hz, 3H), 3.26 - 3.08 (m, 3H), 2.95 (s, 3H), 2.45 (s, 3H), 2.24 (s, 6H), 1.10 (t, J = 7.1 Hz, 3H).

**Example 9**

**Compound 9:ethyl (S)-4-(3-fluoro-2-methylphenyl)-6-(((S)-2-(3-(methylsulfonamido)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

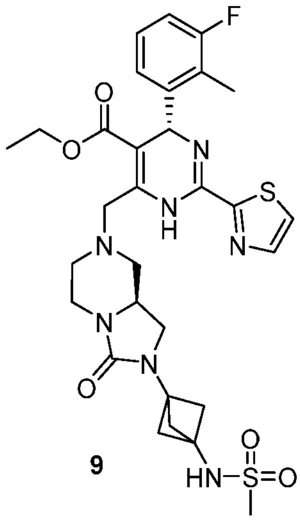

**9**

The title **Compound 9** as a yellow foam was prepared in analogy to the preparation of **Compound 1** by using (S)-N-(3-(3-oxohexahydroimidazo[1,5-a]pyrazin-2(3H)-yl)bicyclo[1.1.1]pentan-1-yl)methanesulfonamide **(Intermediate 9-5)** instead of (S)-3-(3-oxohexahydroimidazo[1,5-a]pyrazin-2(3H)-yl)bicyclo[1.1.1]pentane-1-carboxylic acid **(Intermediate 1-5).**

**Compound 9** (15.8 mg). MS: 658.1 $(M+H)^+$. $^1H$ NMR (400 MHz, Methanol-$d_4$) δ 7.98 (d, J = 3.0 Hz, 1H), 7.88 (d, J = 3.1 Hz, 1H), 7.27 (d, J = 7.9 Hz, 1H), 7.20 (q, J = 7.3 Hz, 1H), 7.00 (t, J = 8.9 Hz, 1H), 5.98 (s, 1H), 4.70 (d, J = 16.1 Hz, 1H), 4.52 (d, J = 16.1 Hz, 1H), 4.21 - 3.99 (m, 4H), 3.70 (d, J = 12.1 Hz, 2H), 3.61 (t, J = 8.9 Hz, 1H), 3.42 (t, J = 13.3 Hz, 1H), 3.27 - 3.07 (m, 3H), 2.95 (s, 3H), 2.45 (d, J = 2.1 Hz, 3H), 2.32 (s, 6H), 1.11 (t, J = 7.1 Hz, 3H).

**Example 10**

**Compound 10: ethyl (S)-6-(((S)-2-(3-acetamidobicyclo[1.1.1]pentan-1-yl)-3-oxohexahydroimidazo[1,5-a]pyrazin-7(1H)-yl)methyl)-4-(3-fluoro-2-methylphenyl)-2-(thiazol-2-yl)-1,4-dihydropyrimidine-5-carboxylate**

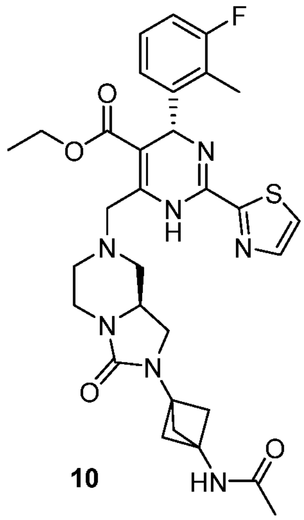

**10**

The title **Compound 10** as a yellow foam was prepared in analogy to the preparation of **Compound 1** by using (S)-N-(3-(3-oxohexahydroimidazo[1,5-a]pyrazin-2(3H)-yl)bicyclo[1.1.1]pentan-1-yl)acetamide(Intermediate 10-2) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 10** (15.3 mg). MS: 622.1 $(M+H)^+$. $^1H$ NMR (400 MHz, Methanol-$d_4$) δ 7.98 (d, J = 3.0 Hz, 1H), 7.88 (d, J = 3.0 Hz, 1H), 7.27 (d, J = 7.9 Hz, 1H), 7.20 (q, J = 7.4 Hz, 1H), 7.00 (t, J = 9.0 Hz, 1H), 5.99 (s, 1H), 4.71 (d, J = 15.9 Hz, 1H), 4.53 (d, J = 16.0 Hz, 1H), 4.22 - 3.98 (m, 4H), 3.71 (d, J = 12.0 Hz, 2H), 3.60 (d, J = 5.3 Hz, 1H), 3.42 (t, J = 13.3 Hz, 1H), 3.27 - 3.12 (m, 3H), 2.45 (s, 3H), 2.33 (s, 6H), 1.89 (s, 3H), 1.11 (t, J = 7.1 Hz, 3H).

**Example 11**

**Compound 11: ethyl (S)-4-(3-fluoro-2-methylphenyl)-6-(((S)-2-(3-(N-methylmethylsulfonamido)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

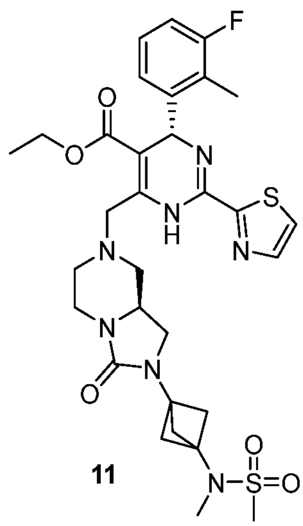

**11**

The title **Compound 11** as a yellow foam was prepared in analogy to the preparation of **Compound 1** by using (S)-N-methyl-N-(3-(3-oxohexahydroimidazo[1,5-a]pyrazin-2(3H)-yl)bicyclo[1.1.1]pentan-1-yl)methanesulfonamide(**Intermediate 11-2**) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 11** (11.3 mg). MS: 672.1 $(M+H)^+$. $^1H$ NMR (400 MHz, Methanol-$d_4$) δ 7.98 (d, J = 3.1 Hz, 1H), 7.88

(d, J = 3.1 Hz, 1H), 7.28 (d, J = 7.8 Hz, 1H), 7.20 (td, J = 7.9, 5.4 Hz, 1H), 7.00 (t, J = 8.9 Hz, 1H), 5.99 (s, 1H), 4.72 (d, J = 15.9 Hz, 1H), 4.53 (d, J = 16.1 Hz, 1H), 4.16 (td, J = 8.3, 4.1 Hz, 1H), 4.12 - 4.00 (m, 3H), 3.78 - 3.67 (m, 2H), 3.63 (d, J = 9.0 Hz, 1H), 3.44 (ddd, J = 15.0, 12.6, 3.1 Hz, 1H), 3.28 - 3.11 (m, 3H), 2.87 (d, J = 9.9 Hz, 6H), 2.45 (d, J = 2.1 Hz, 3H), 2.37 (s, 6H), 1.10 (t, J = 7.1 Hz, 3H).

Example 12

Compound 12:ethyl (S)-4-(3-fluoro-2-methylphenyl)-6-(((S)-2-(3-(N-methylacetamido)bicyclo[1.1.1]pentan-1-yl)-3-oxohexahydroimidazo[1,5-a]pyrazin-**7(1H)-yl)methyl)-2-(thiazol-2-yl)-1,4-dihydropyrimidine-5-carboxylate**

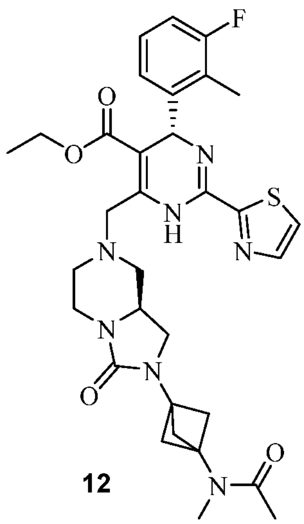

**12**

The title Compound 12 as a yellow foam was prepared in analogy to the preparation of Compound 1 by using (S)-N-methyl-N-(3-(3-oxohexahydroimidazo[1,5-a]pyrazin-2(3H)-yl)bicyclo[1.1.1]pentan-1-yl)acetamide(Intermediate 12-2) instead of (S)-3-(3-oxohexahydroimidazo[1,5-a]pyrazin-2(3H)-yl)bicyclo[1.1.1]pentane-1-carboxylic acid (Intermediate 1-5).

Compound 12 (15.9 mg). MS: 636.1 $(M+H)^+$. $^1H$ NMR (400 MHz, Methanol-$d_4$) δ 7.98 (d, J = 3.1 Hz, 1H), 7.89 (d, J = 3.1 Hz, 1H), 7.28 (d, J = 7.8 Hz, 1H), 7.20 (td, J = 7.9, 5.5 Hz, 1H), 7.00 (t, J = 8.9 Hz, 1H), 5.99 (s, 1H), 4.72 (d, J = 16.0 Hz, 1H), 4.53 (d, J = 16.0 Hz, 1H), 4.16 (dt, J = 7.6, 3.9 Hz, 1H), 4.08 (q, J = 7.0 Hz, 3H), 3.72 (d, J = 12.2 Hz, 2H), 3.67 - 3.55 (m, 1H), 3.44 (t, J = 13.6 Hz, 1H), 3.28 - 3.11 (m, 3H), 2.95 (d, J = 36.3 Hz, 3H), 2.56 - 2.30 (m, 9H), 2.10 (d, J = 26.7 Hz, 3H), 1.11 (t, J = 7.1 Hz, 3H).

**Example 13**

**Compound 13: ethyl (S)-4-(3-fluoro-2-methylphenyl)-6-(((S)-3-oxo-2-(3-(2-oxopyrrolidin-1-yl)bicyclo[1.1.1]pentan-1-yl)hexahydroimidazo[1,5-a]pyrazin-7(1H)-yl)methyl)-2-(thiazol-2-yl)-1,4-dihydropyrimidine-5-carboxylate**

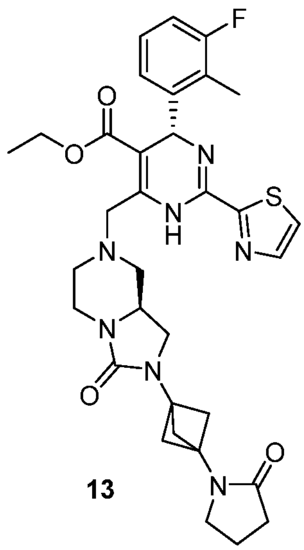

13

The title **Compound 13** as a yellow foam was prepared in analogy to the preparation of **Compound 1** by using (S)-2-(3-(2-oxopyrrolidin-1-yl)bicyclo[1.1.1]pentan-1-yl)hexahydroimidazo[1,5-a]pyrazin-3(2H)-one(**Intermediate 13-3**) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 13** (11.5 mg). MS: 648.1 $(M+H)^+$. $^1H$ NMR (400 MHz, Methanol-$d_4$) δ 7.98 (d, J = 3.1 Hz, 1H), 7.89 (d, J = 3.1 Hz, 1H), 7.27 (dd, J = 8.0, 1.3 Hz, 1H), 7.20 (td, J = 7.9, 5.5 Hz, 1H), 7.00 (ddd, J = 9.5, 8.1, 1.3 Hz, 1H), 5.99 (s, 1H), 4.71 (d, J = 16.0 Hz, 1H), 4.53 (d, J = 16.1 Hz, 1H), 4.20 - 4.00 (m, 4H), 3.72 (dd, J = 11.7, 4.0 Hz, 2H), 3.62 (dd, J = 9.6, 8.4 Hz, 1H), 3.49 - 3.38 (m, 3H), 3.26 - 3.12 (m, 3H), 2.43 (d, J = 11.2 Hz, 9H), 2.35 (t, J = 8.1 Hz, 2H), 2.02 (tt, J = 7.9, 6.8 Hz, 2H), 1.11 (t, J = 7.1 Hz, 3H).

**Example 14**

**Compound 14: ethyl (S)-4-(3-fluoro-2-methylphenyl)-6-(((S)-2-(3-(methylsulfonamidomethyl)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

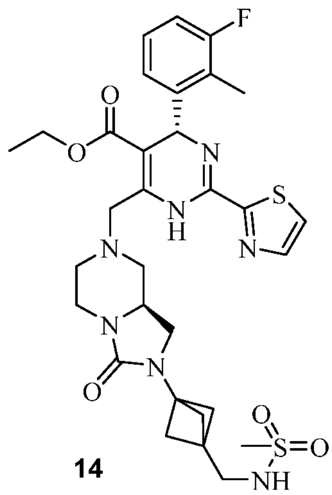

14

The title **Compound 14** as a yellow foam was prepared in analogy to the preparation of **Compound 1** by using (S)-N-((3-(3-oxohexahydroimidazo[1,5-a]pyrazin-2(3H)-yl)bicyclo[1.1.1]pentan-1-yl)methyl)methanesulfonamide(**Intermediate 14-3)** instead of (S)-3-(3-oxohexahydroimidazo[1,5-a]pyrazin-2(3H)-yl)bicyclo[1.1.1]pentane-1-carboxylic acid **(Intermediate 1-5).**

**Compound 14** (12.4 mg). MS: 672.1 $(M+H)^+$. $^1H$ NMR (400 MHz, Methanol-$d_4$) δ 8.03 - 7.94 (m, 1H), 7.93 - 7.84 (m, 1H), 7.27 (d, J = 7.9 Hz, 1H), 7.21 (t, J = 7.2 Hz, 1H), 7.00 (t, J = 9.0 Hz, 1H), 5.98 (s, 1H), 4.71 (d, J = 16.0 Hz, 1H), 4.53 (d, J = 16.1 Hz, 1H), 4.07 (tt, J = 14.2, 5.9 Hz, 4H), 3.70 (d, J = 12.0 Hz, 2H), 3.60 (t, J = 9.1 Hz, 1H), 3.41 (t, J = 13.5 Hz, 1H), 3.26 - 3.10 (m, 5H), 2.91 (d, J = 1.8 Hz, 3H), 2.45 (s, 3H), 2.01 (s, 6H), 1.11 (t, J = 7.1 Hz, 3H).

**Example 15**

**Compound 15: ethyl (S)-6-(((S)-2-(3-(acetamidomethyl)bicyclo[1.1.1]pentan-1-yl)-3-oxohexahydroimidazo[1,5-a]pyrazin-7(1H)-yl)methyl)-4-(3-fluoro-2-methylphenyl)-2-(thiazol-2-yl)-1,4-dihydropyrimidine-5-carboxylate**

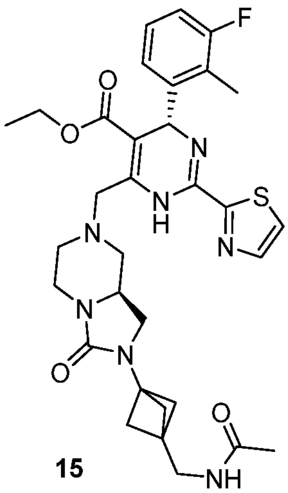

**15**

The title **Compound 15** as a yellow foam was prepared in analogy to the preparation of **Compound 1** by using (S)-N-((3-(3-oxohexahydroimidazo[1,5-a]pyrazin-2(3H)-yl)bicyclo[1.1.1]pentan-1-yl)methyl)acetamide(**Intermediate 15)** instead of (S)-3-(3-oxohexahydroimidazo[1,5-a]pyrazin-2(3H)-yl)bicyclo[1.1.1]pentane-1-carboxylic acid **(Intermediate 1-5).**

**Compound 15** (15.3 mg). MS: 636.1 $(M+H)^+$. $^1H$ NMR (400 MHz, Methanol-$d_4$) δ 7.98 (d, J = 3.1 Hz, 1H), 7.88 (d, J = 3.1 Hz, 1H), 7.27 (d, J = 7.8 Hz, 1H), 7.19 (td, J = 7.9, 5.5 Hz, 1H), 7.00 (t, J = 8.9 Hz, 1H), 5.98 (s, 1H), 4.70 (d, J = 16.0 Hz, 1H), 4.52 (d, J = 16.0 Hz, 1H), 4.17 - 4.00 (m, 4H), 3.70 (d, J = 12.0 Hz, 2H), 3.58 (t, J = 9.0 Hz, 1H), 3.47 - 3.38 (m, 1H), 3.35 (s, 2H), 3.25 - 3.10 (m, 3H), 2.45 (d, J = 2.0 Hz, 3H), 1.96 (d, J = 11.9 Hz, 9H), 1.11 (t, J = 7.1 Hz, 3H).

**Example 16**

**Compound 16: ethyl (S)-6-(((S)-2-(3-((diethoxyphosphoryl)methyl)bicyclo[1.1.1]pentan-1-yl)-3-oxohexahydroimidazo[1,5-a]pyrazin-7(1H)-yl)methyl)-4-(3-fluoro-2-methylphenyl)-2-(thiazol-2-yl)-1,4-dihydropyrimidine-5-carboxylate**

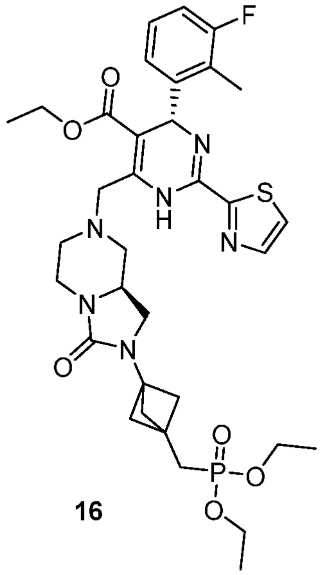

16

The title **Compound 16** as a yellow foam was prepared in analogy to the preparation of **Compound 1** by using diethyl (S)-((3-(3-oxohexahydroimidazo[1,5-a]pyrazin-2(3H)-yl)bicyclo[1.1.1]pentan-1-yl)methyl)phosphonate(**Intermediate 16**) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 16** (11.3 mg). MS: 715.1 $(M+H)^+$. $^1H$ NMR (400 MHz, Methanol-*$d_4$*) δ 7.98 (d, J = 3.1 Hz, 1H), 7.88 (d, J = 3.1 Hz, 1H), 7.27 (d, J = 7.8 Hz, 1H), 7.20 (td, J = 7.9, 5.4 Hz, 1H), 7.00 (t, J = 8.9 Hz, 1H), 5.98 (s, 1H), 4.70 (d, J = 16.1 Hz, 1H), 4.52 (d, J = 16.1 Hz, 1H), 4.16 - 4.00 (m, 8H), 3.69 (d, J = 11.8 Hz, 2H), 3.59 (t, J = 9.0 Hz, 1H), 3.37-3.44 (m, 1H), 3.25 - 3.09 (m, 3H), 2.45 (d, J = 2.0 Hz, 3H), 2.18 (d, J = 18.8 Hz, 2H), 2.11 (s, 6H), 1.32 (t, J = 7.0 Hz, 6H), 1.11 (t, J = 7.1 Hz, 3H).

**Example 17**

**Compound 17: ethyl (S)-6-(((S)-2-(3-cyanobicyclo[1.1.1]pentan-1-yl)-3-oxohexahydroimidazo[1,5-a]pyrazin-7(1H)-yl)methyl)-4-(3-fluoro-2-methylphenyl)-2-(thiazol-2-yl)-1,4-dihydropyrimidine-5-carboxylate**

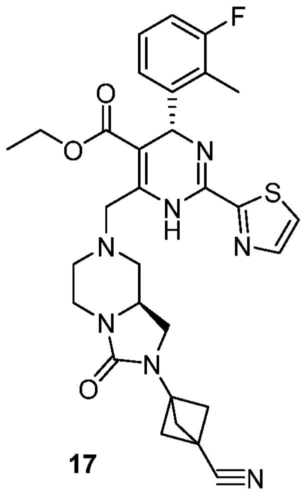

17

The title **Compound 17** as a yellow foam was prepared in analogy to the preparation of **Compound 1** by using (S)-3-(3-oxohexahydroimidazo[1,5-a]pyrazin-2(3H)-yl)bicyclo[1.1.1]pentane-1-carbonitrile(**Intermediate 17-3**) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 17** (17.3 mg). MS: 590.2 $(M+H)^+$. $^1H$ NMR (400 MHz, Methanol-*$d_4$*) δ7.98 (d, J = 3.1 Hz, 1H), 7.88

(d, J = 3.1 Hz, 1H), 7.26 (d, J = 7.9 Hz, 1H), 7.19 (m, 1H), 7.00 (t, J = 8.9 Hz, 1H), 5.98 (s, 1H), 4.67 (d, J = 16.1 Hz, 1H), 4.50 (d, J = 16.1 Hz, 1H), 4.06 (m, 4H), 3.70 - 3.57 (m, 3H), 3.40 (m, 1H), 3.21 - 3.05 (m, 3H), 2.55 (s, 7H), 2.45 (d, J = 2.0 Hz, 3H).

**Example 18**

**Compound 18: ethyl (S)-4-(3-fluoro-2-methylphenyl)-6-(((S)-2-(3-(fluoromethyl)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

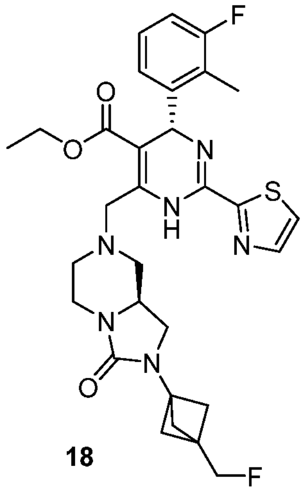

**18**

The title **Compound 18** as a yellow foam was prepared in analogy to the preparation of **Compound 1** by using (S)-2-(3-(fluoromethyl)bicyclo[1.1. 1]pentan-1-yl)hexahydroimidazo[1,5-a]pyrazin-3(2H)-one(**Intermediate 18-2)** instead of (S)-3-(3-oxohexahydroimidazo[1,5-a]pyrazin-2(3H)-yl)bicyclo[1.1.1]pentane-1-carboxylic acid **(Intermediate 1-5).**

**Compound 18** (8.9 mg). MS: 597.1 $(M+H)^+$. $^1$H NMR (400 MHz, DMSO-$d_6$) δ 9.58 (s, 1H), 7.99 (d, J = 3.2 Hz, 1H), 7.90 (d, J = 3.2 Hz, 1H), 7.22 - 7.09 (m, 1H), 7.02 (d, J = 8.3 Hz, 2H), 5.87 (s, 1H), 4.47 (d, J = 47.7 Hz, 2H), 4.07 - 3.82 (m, 4H), 3.64 (dt, J = 13.4, 6.4 Hz, 2H), 3.35 (s, 1H), 2.98 - 2.71 (m, 4H), 2.43 (d, J = 2.0 Hz, 3H), 2.18 (d, J = 3.4 Hz, 1H), 1.93 (s, 7H), 1.03 (t, J = 7.1 Hz, 3H).

**Example 19**

**Compound 19: ethyl (S)-6-(((S)-2-(3-(1,1-dioxidoisothiazolidin-2-yl)bicyclo[1.1.1]pentan-1-yl)-3-oxohexahydroimidazo[1,5-a]pyrazin-7(1H)-yl)methyl)-4-(3-fluoro-2-methylphenyl)-2-(thiazol-2-yl)-1,4-dihydropyrimidine-5-carboxylate**

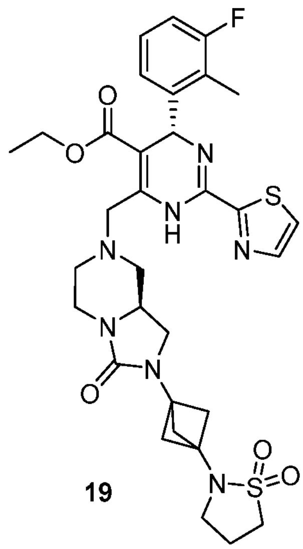

19

The title **Compound 19** as a yellow foam was prepared in analogy to the preparation of **Compound 1** by using (S)-2-(3-(1,1-dioxidoisothiazolidin-2-yl)bicyclo[1.1.1]pentan-1-yl)hexahydroimidazo[1,5-a]pyrazin-3(2H)-one(**Intermediate 19-3**) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 19** (5.9 mg). MS: 684.1 $(M+H)^+$. $^1H$ NMR (400 MHz, Methanol-$d_4$) δ 7.98 (d, J = 3.1 Hz, 1H), 7.88 (d, J = 3.1 Hz, 1H), 7.27 (d, J = 7.8 Hz, 1H), 7.20 (td, J = 8.0, 5.5 Hz, 1H), 7.00 (dd, J = 9.7, 8.0 Hz, 1H), 5.98 (s, 1H), 4.70 (d, J = 16.1 Hz, 1H), 4.52 (d, J = 16.1 Hz, 1H), 4.19 - 3.99 (m, 4H), 3.70 (d, J = 12.2 Hz, 2H), 3.61 (t, J = 9.0 Hz, 1H), 3.42 (ddd, J = 15.0, 12.7, 3.1 Hz, 1H), 3.30 - 3.26 (m, 2H), 3.25 - 3.10 (m, 5H), 2.45 (d, J = 2.0 Hz, 3H), 2.40 - 2.26 (m, 8H), 1.11 (t, J = 7.1 Hz, 3H).

## Example 20

**Compound 20: ethyl (S)-6-(((S)-2-(3-(cyanomethyl)bicyclo[1.1.1]pentan-1-yl)-3-oxohexahydroimidazo[1,5-a] pyrazin-7(1H)-yl)methyl)-4-(3-fluoro-2-methylphenyl)-2-(thiazol-2-yl)-1,4-dihydropyrimidine-5-carboxylate**

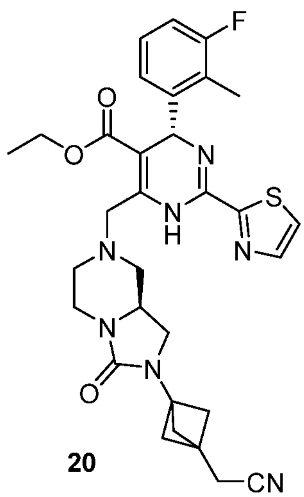

20

The title **Compound 20** as a yellow foam was prepared in analogy to the preparation of **Compound 1** by using (S)-2-(3-(3-oxohexahydroimidazo[1,5-a]pyrazin-2(3H)-yl)bicyclo[1.1.1]pentan-1-yl)acetonitrile **(Intermediate 20-2)** instead of (S)-3-(3-oxohexahydroimidazo[1,5-a]pyrazin-2(3H)-yl)bicyclo[1.1.1]pentane-1-carboxylic acid **(Intermediate 1-5).**

**Compound 20** (25.9 mg). MS: 604.1 $(M+H)^+$. $^1H$ NMR (400 MHz, Methanol-$d_4$) δ 7.98 (d, J = 3.1 Hz, 1H), 7.88

(d, J = 3.1 Hz, 1H), 7.27 (d, J = 7.7 Hz, 1H), 7.20 (td, J = 8.0, 5.5 Hz, 1H), 7.00 (dd, J = 9.7, 7.9 Hz, 1H), 5.98 (s, 1H), 4.70 (d, J = 16.1 Hz, 1H), 4.53 (d, J = 16.0 Hz, 1H), 4.21 - 3.97 (m, 4H), 3.70 (dd, J = 12.2, 3.1 Hz, 2H), 3.61 (dd, J = 9.6, 8.4 Hz, 1H), 3.42 (ddd, J = 15.0, 12.5, 3.1 Hz, 1H), 3.26 - 3.09 (m, 3H), 2.81 (s, 2H), 2.45 (d, J = 2.0 Hz, 3H), 2.11 (s, 6H), 1.11 (t, J = 7.1 Hz, 3H).

**Example 21**

**Compound 21: ethyl (S)-4-(3-fluoro-2-methylphenyl)-6-(((S)-3-oxo-2-(3-(sulfamoylamino)bicyclo[1.1.1]pentan-1-yl)hexahydroimidazo[1,5-a]pyrazin-7(1H)-yl)methyl)-2-(thiazol-2-yl)-1,4-dihydropyrimidine-5-carboxylate**

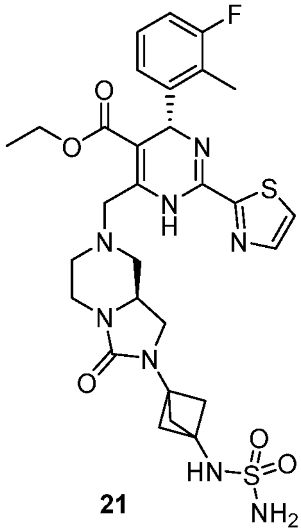

**21**

The title **Compound 21** as a yellow foam was prepared in analogy to the preparation of **Compound 1** by using **Intermediate 21-2** instead of (S)-3-(3-oxohexahydroimidazo[1,5-a]pyrazin-2(3H)-yl)bicyclo[1.1.1]pentane-1-carboxylic acid **(Intermediate 1-5). Compound 21** (4.3 mg). MS: 659.1 $(M+H)^+$. $^1H$ NMR (400 MHz, Methanol-$d_4$) δ 7.98 (d, J = 3.1 Hz, 1H), 7.88 (d, J = 3.2 Hz, 1H), 7.26 (d, J = 7.8 Hz, 1H), 7.23 - 7.16 (m, 1H), 7.00 (dd, J = 9.7, 8.1 Hz, 1H), 5.98 (s, 1H), 4.68 (d, J = 16.1 Hz, 1H), 4.51 (d, J = 16.1 Hz, 1H), 4.17 - 4.00 (m, 4H), 3.72 - 3.55 (m, 3H), 3.40 (dd, J = 13.8, 10.7 Hz, 1H), 3.25 - 3.06 (m, 3H), 2.45 (d, J = 2.0 Hz, 3H), 2.32 (s, 6H), 1.11 (t, J = 7.1 Hz, 3H).

**Example 22**

**Compound 22: 2-(3-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)acetic acid**

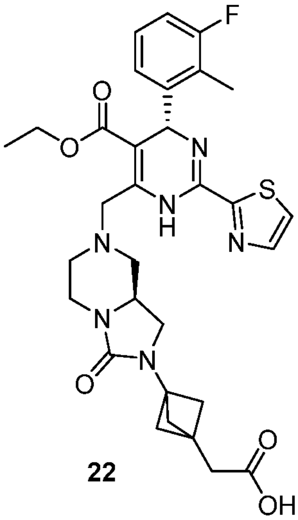

22

The title **Compound 22** as a yellow foam was prepared in analogy to the preparation of **Compound 1** by using (S)-2-(3-(3-oxohexahydroimidazo[1,5-a]pyrazin-2(3H)-yl)bicyclo[1.1.1]pentan-1-yl)acetic acid(**Intermediate 34-4**) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 22** (6.3 mg). MS: 623.1 $(M+H)^+$. $^1H$ NMR (400 MHz, Methanol-$d_4$) δ 7.98 (d, J = 3.1 Hz, 1H), 7.88 (d, J = 3.1 Hz, 1H), 7.27 (d, J = 7.8 Hz, 1H), 7.20 (td, J = 7.9, 5.5 Hz, 1H), 7.00 (t, J = 8.9 Hz, 1H), 5.98 (s, 1H), 4.71 (d, J = 16.0 Hz, 1H), 4.53 (d, J = 16.1 Hz, 1H), 4.22 - 3.95 (m, 4H), 3.70 (dd, J = 12.1, 3.0 Hz, 2H), 3.59 (t, J = 9.0 Hz, 1H), 3.42 (td, J = 14.7, 13.7, 3.1 Hz, 1H), 3.27 - 3.08 (m, 3H), 2.57 (s, 2H), 2.45 (d, J = 2.0 Hz, 3H), 2.07 (s, 6H), 1.11 (t, J = 7.1 Hz, 3H).

**Example** 23

**Compound 23: ethyl (S)-6-(((S)-2-(3-(cyclopropanesulfonamidomethyl)bicyclo[1.1.1]pentan-1-yl)-3-oxohexahydroimidazo[1,5-a]pyrazin-7(1H)-yl)methyl)-4-(3-fluoro-2-methylphenyl)-2-(thiazol-2-yl)-1,4-dihydropyrimidine-5-carboxylate**

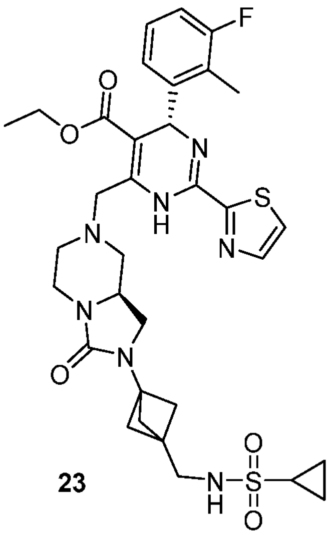

23

The title **Compound 23** as a yellow foam was prepared in analogy to the preparation of **Compound 1** by using (S)-N-((3-(3-oxohexahydroimidazo[1,5-a]pyrazin-2(3H)-yl)bicyclo[1.1.1]pentan-1-yl)methyl)cyclopropanesulfonamide(**Intermediate 23-3**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 23** (9 mg). MS: 698.1 $(M+H)^+$. $^1H$ NMR (400 MHz, Methanol-$d_4$) δ 7.98 (d, J = 3.1 Hz, 1H), 7.88 (d, J = 3.1 Hz, 1H), 7.27 (d, J = 7.8 Hz, 1H), 7.20 (td, J = 7.9, 5.4 Hz, 1H), 7.00 (t, J = 8.9 Hz, 1H), 5.98 (s, 1H), 4.70 (d, J = 16.1 Hz, 1H), 4.53 (d, J = 16.1 Hz, 1H), 4.18 - 4.00 (m, 4H), 3.76 - 3.65 (m, 2H), 3.63 - 3.55 (m, 1H), 3.41 (t, J = 12.1 Hz, 1H), 3.29 - 3.11 (m, 5H), 2.54 - 2.41 (m, 4H), 2.01 (s, 6H), 1.11 (t, J = 7.1 Hz, 3H), 1.05 - 0.93 (m, 4H).

**Example 24**

**Compound 24: ethyl (S)-4-(3-fluoro-2-methylphenyl)-6-(((S)-3-oxo-2-(3-((sulfamoylamino)methyl)bicyclo[1.1.1] pentan-1-yl)hexahydroimidazo[1,5-a]pyrazin-7(1H)-yl)methyl)-2-(thiazol-2-yl)-1,4-dihydropyrimidine-5-carboxylate**

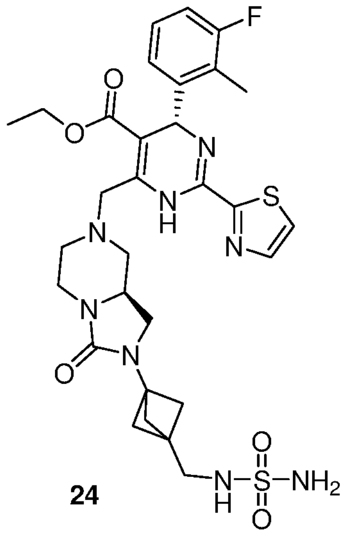

**24**

The title **Compound 24** as a yellow foam was prepared in analogy to the preparation of **Compound 1** by using (S)-3-oxo-2-(3-((aminosulfonylamino) methyl)bicyclo [1.1.1]pentane-1-yl) hexahydroimidazo[1,5-a] pyrazine(**Intermediate 24-1**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 24** (5 mg). MS: 673.1 $(M+H)^+$.

**Example 25**

**Compound 25: ethyl (S)-4-(3-fluoro-2-methylphenyl)-6-(((S)-3-oxo-2-(3-((2-oxopyrrolidin-1-yl)methyl)bicyclo[1.1.1]pentan-1-yl)hexahydroimidazo[1,5-a]pyrazin-7(1H)-yl)methyl)-2-(thiazol-2-yl)-1,4-dihydropyrimidine-5-carboxylate**

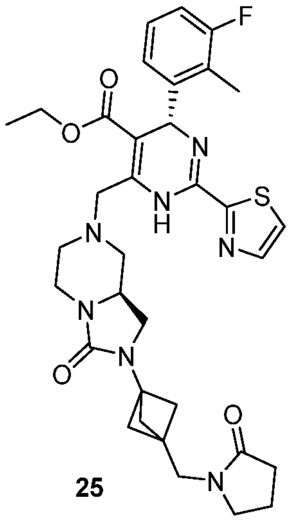

**25**

The title **Compound 25** as a yellow foam was prepared in analogy to the preparation of **Compound 1** by using (S)-2-(3-((2-oxopyrrolidin-1-yl)methyl)bicyclo[1.1.1]pentan-1-yl)hexahydroimidazo[1,5-a]pyrazin-3(2H)-one(**Intermediate 25-3**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 25** (15 mg). MS: 662.1 $(M+H)^+$. $^1H$ NMR (400 MHz, Methanol-$d_4$) δ 7.98 (d, J = 3.1 Hz, 1H), 7.88 (d, J = 3.1 Hz, 1H), 7.27 (d, J = 7.7 Hz, 1H), 7.20 (td, J = 8.0, 5.5 Hz, 1H), 7.00 (t, J = 8.8 Hz, 1H), 5.98 (s, 1H), 4.70 (d, J = 16.0 Hz, 1H), 4.53 (d, J = 16.1 Hz, 1H), 4.19 - 3.99 (m, 4H), 3.70 (d, J = 12.0 Hz, 2H), 3.62 - 3.36 (m, 7H), 3.25 - 3.09 (m, 3H), 2.45 (d, J = 2.1 Hz, 3H), 2.37 (t, J = 8.1 Hz, 2H), 2.10 - 1.96 (m, 8H), 1.11 (t, J = 7.1 Hz, 3H).

**Example 26**

**Compound 26: ethyl (S)-4-(3-fluoro-2-methylphenyl)-6-(((S)-2-(3-(methylcarbamoyl)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

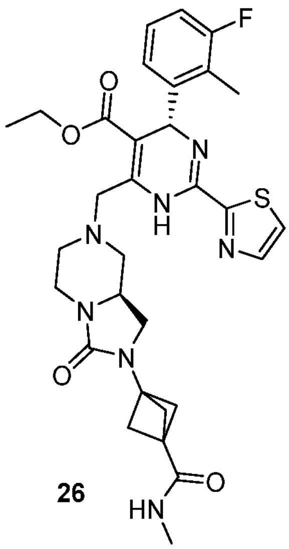

**26**

The title **Compound 26** as a yellow foam was prepared in analogy to the preparation of **Compound 1** by using (S)-N-methyl-3-(3-oxohexahydroimidazo[1,5-a]pyrazin-2(3H)-yl)bicyclo[1.1.1]pentane-1-carboxamide(**Intermediate 26-2**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 26** (35 mg). MS: 622.1 $(M+H)^+$. $^1H$ NMR (400 MHz, Methanol-$d_4$) δ 7.98 (d, *J* = 3.1 Hz, 1H), 7.89

(d, *J* = 3.1 Hz, 1H), 7.27 (d, *J* = 7.7 Hz, 1H), 7.20 (q, *J* = 7.5 Hz, 1H), 7.00 (t, *J* = 8.9 Hz, 1H), 5.98 (s, 1H), 4.70 (d, *J* = 16.0 Hz, 1H), 4.53 (d, *J* = 16.0 Hz, 1H), 4.08 (q, *J* = 6.9 Hz, 4H), 3.70 (d, *J* = 12.3 Hz, 2H), 3.61 (t, *J* = 9.0 Hz, 1H), 3.41 (t, *J* = 13.4 Hz, 1H), 3.25 - 3.09 (m, 3H), 2.71 (s, 3H), 2.45 (d, *J* = 2.2 Hz, 3H), 2.29 (s, 6H), 1.11 (t, *J* = 7.1 Hz, 3H).

**Example 27**

**Compound 27: ethyl (S)-4-(3-fluoro-2-methylphenyl)-6-(((S)-2-(3-(morpholine-4-carbonyl)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

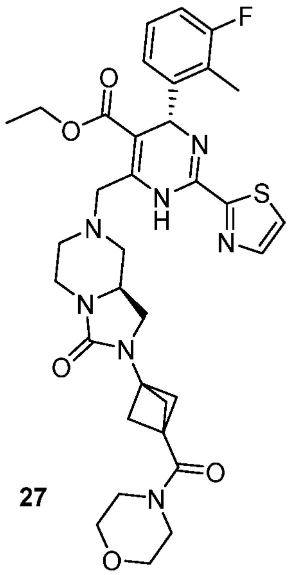

**27**

The title **Compound 27** as a yellow foam was prepared in analogy to the preparation of **Compound 1** by using (S)-2-(3-(morpholine-4-carbonyl)bicyclo[1.1.1]pentan-1-yl)hexahydroimidazo[1,5-a]pyrazin-3(2H)-one(**Intermediate 27-2**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 27** (25.1 mg). MS: 678.1 $(M+H)^+$. $^1H$ NMR (400 MHz, Methanol-$d_4$) δ 8.06 - 7.83 (m, 2H), 7.32 - 7.14 (m, 2H), 7.00 (t, *J* = 8.9 Hz, 1H), 5.98 (s, 1H), 4.70 (d, *J* = 16.1 Hz, 1H), 4.52 (d, *J* = 16.1 Hz, 1H), 4.17 - 4.01 (m, 4H), 3.71 - 3.62 (m, 9H), 3.59 - 3.53 (m, 2H), 3.42 (t, *J* = 13.3 Hz, 1H), 3.19 (ddd, *J* = 22.8, 15.1, 9.9 Hz, 3H), 2.44 (d, *J* = 10.0 Hz, 9H), 1.11 (t, *J* = 7.1 Hz, 3H).

**Example 28**

**Compound 28: ethyl (S)-4-(3-fluoro-2-methylphenyl)-6-(((S)-2-(3-((methylsulfonyl)carbamoyl)bicyclo[1.1.1]pentan-1-yl)-3-oxohexahydroimidazo[1,5-a]pyrazin-7(1H)-yl)methyl)-2-(thiazol-2-yl)-1,4-dihydropyrimidine-5-carboxylate**

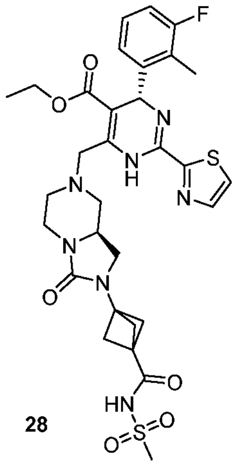

28

The title **Compound 28** as a yellow foam was prepared in analogy to the preparation of **Compound 1** by using (S)-N-(methylsulfonyl)-3-(3-oxohexahydroimidazo[1,5-a]pyrazin-2(3H)-yl)bicyclo[1.1.1]pentane-1-carboxamide(**Intermediate 28-2**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 28** (15.1 mg). MS: 686.1 $(M+H)^+$. $^1H$ NMR (400 MHz, Methanol-$d_4$) δ 7.98 (d, *J* = 3.1 Hz, 1H), 7.88 (d, *J* = 3.1 Hz, 1H), 7.27 (d, *J* = 7.8 Hz, 1H), 7.20 (td, *J* = 7.9, 5.5 Hz, 1H), 7.00 (t, *J* = 8.9 Hz, 1H), 5.99 (s, 1H), 4.70 (d, *J* = 16.0 Hz, 1H), 4.56 - 4.50 (m, 1H), 4.19 - 4.01 (m, 4H), 3.77 - 3.67 (m, 2H), 3.61 (t, *J* = 9.0 Hz, 1H), 3.47 - 3.37 (m, 1H), 3.25 - 3.11 (m, 5H), 3.05 (s, 1H), 2.51 - 2.26 (m, 9H), 1.11 (t, *J* = 7.1 Hz, 3H).

## Example 29

**Compound 29: ethyl (S)-6-(((S)-2-(3-(cyclopropanesulfonamido)bicyclo[1.1.1]pentan-1-yl)-3-oxohexahydroimidazo[1,5-a]pyrazin-7(1H)-yl)methyl)-4-(3-fluoro-2-methylphenyl)-2-(thiazol-2-yl)-1,4-dihydropyrimidine-5-carboxylate**

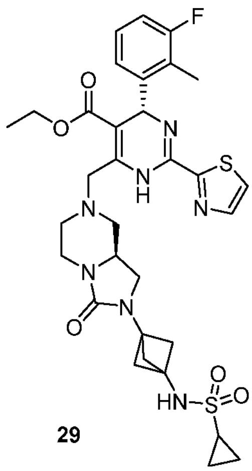

29

The title **Compound 29** as a yellow foam was prepared in analogy to the preparation of **Compound 1** by using (S)-N-(3-(3-oxohexahydroimidazo[1,5-a]pyrazin-2(3H)-yl)bicyclo[1.1.1]pentan-1-yl)cyclopropanesulfonamide(**Intermediate 29-2**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 29** (35.1 mg). MS: 684.1 $(M+H)^+$. $^1H$ NMR (400 MHz, Methanol-$d_4$) δ 7.98 (d, J = 3.1 Hz, 1H), 7.88 (d, J = 3.1 Hz, 1H), 7.27 (d, J = 7.7 Hz, 1H), 7.20 (q, J = 7.3 Hz, 1H), 7.00 (t, J = 8.9 Hz, 1H), 5.98 (s, 1H), 4.69 (d, J = 16.1 Hz, 1H), 4.51 (d, J = 16.1 Hz, 1H), 4.19 - 3.98 (m, 4H), 3.68 (d, J = 12.1 Hz, 2H), 3.61 (t, J = 9.0 Hz, 1H), 3.41 (t, J = 12.4 Hz, 1H), 3.23 - 3.07 (m, 3H), 2.51 (td, J = 8.0, 4.0 Hz, 1H), 2.45 (d, J = 2.1 Hz, 3H), 2.32 (s, 6H), 1.11 (t, J = 7.1 Hz, 3H), 1.08 - 0.97 (m, 4H).

**Example 30**

**Compound 30: 2-(3-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)propanoic acid**

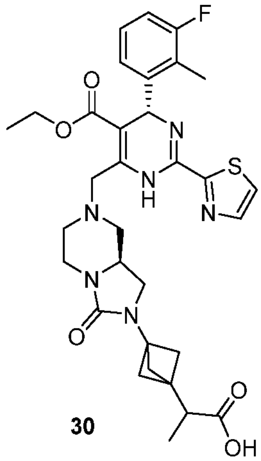

**30**

The title **Compound 30** as a yellow foam was prepared in analogy to the preparation of **Compound 1** by using 2-(3-((S)-3-oxohexahydroimidazo[1,5-a]pyrazin-2(3H)-yl)bicyclo[1.1.1]pentan-1-yl)propanoic acid(**Intermediate 30-2**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 30** (25.1 mg). MS: 637.1 $(M+H)^+$. $^1H$ NMR (400 MHz, Methanol-*$d_4$*) $\delta$ 7.98 (d, J = 3.1 Hz, 1H), 7.88 (d, J = 3.1 Hz, 1H), 7.27 (d, J = 7.8 Hz, 1H), 7.20 (dd, J = 8.1, 5.7 Hz, 1H), 7.00 (t, J = 8.9 Hz, 1H), 5.98 (s, 1H), 4.69 (d, J = 16.1 Hz, 1H), 4.52 (d, J = 16.1 Hz, 1H), 4.16 - 3.99 (m, 4H), 3.68 (dd, J = 12.1, 3.1 Hz, 2H), 3.64 - 3.54 (m, 1H), 3.47 - 3.34 (m, 1H), 3.23 - 3.08 (m, 3H), 2.66 (q, J = 7.0 Hz, 1H), 2.45 (d, J = 2.0 Hz, 3H), 2.00 (s, 6H), 1.11 (dt, J = 7.1, 3.6 Hz, 6H).

**Example 31**

**Compound 31: ethyl (8aR)-7-(((S)-5-(ethoxycarbonyl)-6-(3-fluoro-2-methylphenyl)-2-(thiazol-2-yl)-3,6-dihydropyrimidin-4-yl)methyl)-2-(3-(hydroxymethyl)bicyclo[1.1.1]pentan-1-yl)-3-oxooctahydroimidazo[1,5-a]pyrazine-8-carboxylate**

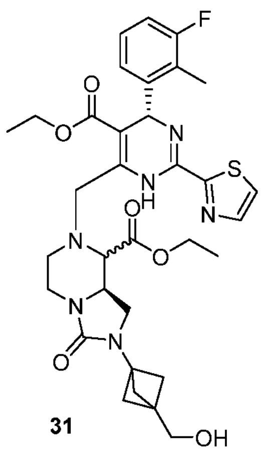

**31**

The title **Compound 31** as a yellow foam was prepared in analogy to the preparation of **Compound 1** by using

ethyl (8S,8aR)-2-(3-(hydroxymethyl)bicyclo[1.1.1]pentan-1-yl)-3-oxooctahydroimidazo[1,5-a]pyrazine-8-carboxylate(**Intermediate 31-1**) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 31** (19.1 mg). MS: 667.2 (M+H)$^+$.

**Example 32**

**Compound 32: 3-((S)-7-(((S)-5-(ethoxycarbonyl)-6-(3-fluoro-2-methylphenyl)-2-(thiazol-2-yl)-3,6-dihydropyrimidin-4-yl)methyl)-3-thioxohexahydroimidazo[1,5-a]pyrazin-2(3H)-yl)bicyclo[1.1.1]pentane-1-carboxylic acid**

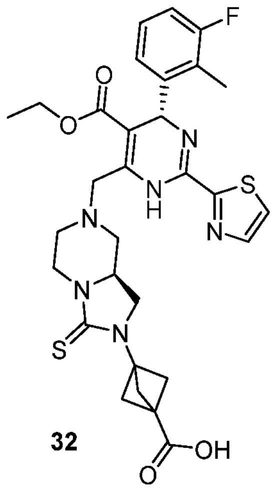

**32**

The title **Compound 32** as a yellow foam was prepared in analogy to the preparation of **Compound 1** by using (S)-3-(3-thioxohexahydroimidazo[1,5-a]pyrazin-2(3H)-yl)bicyclo[1.1.1]pentane-1-carboxylic acid(**Intermediate 32-5**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 32** (35 mg). MS: 625.2 (M+H)$^+$. $^1$H NMR (400 MHz, Methanol-*d$_4$*) δ 7.98 (d, J = 2.9 Hz, 1H), 7.87 (s, 1H), 7.31 - 7.13 (m, 2H), 6.99 (t, J = 8.8 Hz, 1H), 5.99 (s, 1H), 4.73 - 4.56 (m, 2H), 4.45 (d, J = 16.2 Hz, 1H), 4.30 (s, 1H), 4.07 (q, J = 7.4 Hz, 2H), 3.84 (q, J = 10.2 Hz, 1H), 3.64 (d, J = 14.5 Hz, 2H), 3.41 (t, J = 12.9 Hz, 2H), 3.34 (s, 1H), 3.11 (s, 1H), 2.51 (d, J = 4.4 Hz, 6H), 2.46 (s, 3H), 1.11 (t, J = 7.3 Hz, 3H).

**Example 33**

**Compound 33: (8S,8aR)-7-(((S)-5-(ethoxycarbonyl)-6-(3-fluoro-2-methylphenyl)-2-(thiazol-2-yl)-3,6-dihydropyrimidin-4-yl)methyl)-2-(3-(hydroxymethyl)bicyclo[1.1.1]pentan-1-yl)-3-oxooctahydroimidazo[1,5-a]pyrazine-8-carboxylic acid**

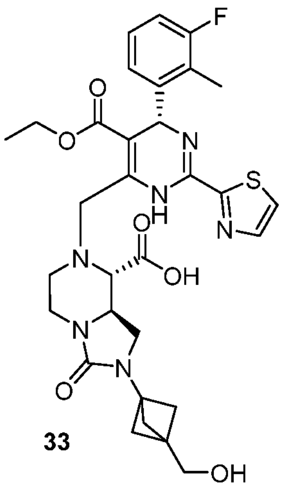

33

The title **Compound 33** as a yellow foam was prepared in analogy to the preparation of **Compound 1** by using (8S,8aR)-2-(3-(hydroxymethyl)bicyclo[1.1.1]pentan-1-yl)-3-oxooctahydroimidazo[1,5-a]pyrazine-8-carboxylic acid(**Intermediate 33-2**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 33** (18.1 mg). MS: 639.2 $(M+H)^+$. $^1H$ NMR (400 MHz, Methanol-d4) δ 8.17 - 8.06 (m, 2H), 7.26 (m, 2H), 7.04 (t, J=8.4 Hz, 1H), 6.09 (d, J=6.1 Hz, 1H), 4.32 (dd, J=16.9, 4.5 Hz, 1H), 4.09 (m, 2H), 3.97 (dd, J = 16.6, 3.8 Hz, 1H), 3.88 - 3.75 (m, 2H), 3.65 - 3.56 (m, 3H), 3.50 (d, J = 10.0 Hz, 2H), 3.12 (m, 2H), 2.78 - 2.59 (m, 1H), 2.49 (s, 3H), 1.99 (s, 6H), 1.12 (t, J = 7.2 Hz, 3H).

**Example 34**

**Compound 34: 2-(3-((S)-7-(((R)-6-(2-chloro-3-fluorophenyl)-5-(ethoxycarbonyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)acetic acid**

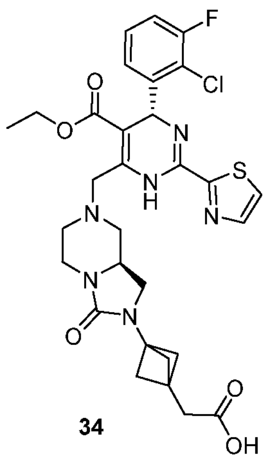

34

The title **Compound 34** as a yellow foam was prepared in analogy to the preparation of **Compound 1** by using (S)-2-(3-(3-oxohexahydroimidazo[1,5-a]pyrazin-2(3H)-yl)bicyclo[1.1.1]pentan-1-yl)acetic acid(**Intermediate 34-4**) instead of (S)-3-(3-oxohexahydroimidazo[1,5-a]pyrazin-2(3H)-yl)bicyclo[1.1.1]pentane-1-carboxylic acid (**Intermediate 1-5**) and ethyl (R)-6-(bromomethyl)-4-(2-chloro-3-fluorophenyl)-2-(thiazol-2-yl)-1,4-dihydropyrimidine-5-carboxylate (**Intermediate A2**) instead of ethyl (S)-6-(bromomethyl)-4-(3-fluoro-2-methylphenyl)-2-(thiazol-2-yl)-1,4-dihydropyrimidine-5-carboxylate (**Intermediate A1).**

**Compound 34** (25.1 mg). MS: 643.2 $(M+H)^+$. $^1H$ NMR (400 MHz, Methanol-$d_4$) δ 7.99 (d, *J* = 3.1 Hz, 1H), 7.89 (d, *J* = 3.0 Hz, 1H), 7.38 - 7.32 (m, 2H), 7.28 - 7.18 (m, 1H), 6.24 (s, 1H), 4.77 - 4.68 (m, 1H), 4.58 - 4.49 (m, 1H), 4.17 - 3.99 (m, 4H), 3.75 - 3.66 (m, 2H), 3.59 (t, *J* = 9.0 Hz, 1H), 3.40 (t, *J* = 13.2 Hz, 1H), 3.26 - 3.11 (m, 3H), 2.57 (s, 2H),

2.07 (s, 6H), 1.11 (t, *J* = 7.1 Hz, 3H).

**Example 35**

**Compound 35: 2-(3-((S)-7-(((R)-6-(2-chloro-4-fluorophenyl)-5-(ethoxycarbonyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)acetic acid**

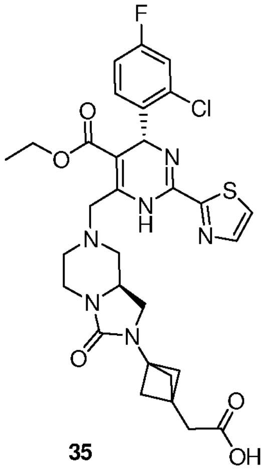

**35**

The title **Compound 35** as a yellow foam was prepared in analogy to the preparation of **Compound 1** by using (S)-2-(3-(3-oxohexahydroimidazo[1,5-a]pyrazin-2(3H)-yl)bicyclo[1.1.1]pentan-1-yl)acetic acid(**Intermediate 34-4**) instead of (S)-3-(3-oxohexahydroimidazo[1,5-a]pyrazin-2(3H)-yl)bicyclo[1.1.1]pentane-1-carboxylic acid (**Intermediate 1-5**) and ethyl (R)-6-(bromomethyl)-4-(2-chloro-4-fluorophenyl)-2-(thiazol-2-yl)-1,4-dihydropyrimidine-5-carboxylate (**Intermediate A3**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 35** (65 mg). MS: 643.2 $(M+H)^+$. $^1H$ NMR (400 MHz, Methanol-$d_4$) δ 7.99 (d, *J* = 3.0 Hz, 1H), 7.89 (d, *J* = 3.0 Hz, 1H), 7.53 (dd, *J* = 8.7, 6.0 Hz, 1H), 7.28 (dd, *J* = 8.6, 2.4 Hz, 1H), 7.11 (td, *J* = 8.4, 2.4 Hz, 1H), 6.19 (s, 1H), 4.72 (d, *J* = 16.0 Hz, 1H), 4.53 (d, *J* = 16.0 Hz, 1H), 4.18 - 3.99 (m, 4H), 3.70 (d, *J* = 12.1 Hz, 2H), 3.59 (t, *J* = 9.0 Hz, 1H), 3.40 (t, *J* = 13.0 Hz, 1H), 3.27 - 3.08 (m, 3H), 2.57 (s, 2H), 2.07 (s, 6H), 1.12 (t, *J* = 7.1 Hz, 3H).

**Example 36**

**Compound 36: 2-(3-((S)-7-(((S)-6-(3,4-difluorophenyl)-5-(ethoxycarbonyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)acetic acid**

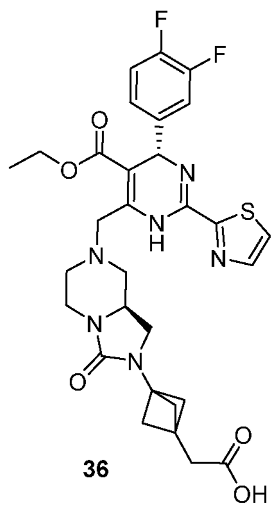

**36**

The title **Compound 36** as a yellow foam was prepared in analogy to the preparation of **Compound 1** by using (S)-2-(3-(3-oxohexahydroimidazo[1,5-a]pyrazin-2(3H)-yl)bicyclo[1.1.1]pentan-1-yl)acetic acid(**Intermediate 34-4**) instead of (S)-3-(3-oxohexahydroimidazo[1,5-a]pyrazin-2(3H)-yl)bicyclo[1.1.1]pentane-1-carboxylic acid (**Intermediate 1-5**) and ethyl (S)-6-(bromomethyl)-4-(3,4-difluorophenyl)-2-(thiazol-2-yl)-1,4-dihydropyrimidine-5-carboxylate (**Intermediate A4**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 36** (25 mg). MS: 627.1 $(M+H)^+$. $^1H$ NMR (400 MHz, Methanol-$d_4$ $\delta$ 7.99 (d, J = 3.0 Hz, 1H), 7.83 (d, J = 2.7 Hz, 1H), 7.19 (s, 3H), 5.70 (s, 1H), 4.30 (d, J = 16.0 Hz, 1H), 4.20 - 4.02 (m, 3H), 3.97 - 3.81 (m, 2H), 3.49 (t, J = 8.9 Hz, 1H), 3.29 - 3.01 (m, 5H), 2.55 (s, 3H), 2.04 (s, 6H), 1.20 (t, J = 7.2 Hz, 3H).

**Example 37**

**Compound 37: 2-(3-((S)-7-(((S)-5-(ethoxycarbonyl)-6-(4-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)acetic acid**

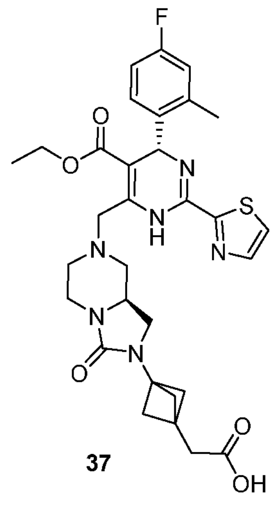

**37**

The title **Compound 37** as a yellow foam was prepared in analogy to the preparation of **Compound 1** by using (S)-2-(3-(3-oxohexahydroimidazo[1,5-a]pyrazin-2(3H)-yl)bicyclo[1.1.1]pentan-1-yl)acetic acid(**Intermediate 34-4**) instead of (S)-3-(3-oxohexahydroimidazo[1,5-a]pyrazin-2(3H)-yl)bicyclo[1.1.1]pentane-1-carboxylic acid (**Intermediate 1-5**) and ethyl (S)-6-(bromomethyl)-4-(4-fluoro-2-methylphenyl)-2-(thiazol-2-yl)-1,4-dihydropyrimidine-5-carboxylate (**Intermediate A5**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 37** (5 mg). MS: 623.1 $(M+H)^+$. $^1H$ NMR (400 MHz, Methanol-$d_4$) δ 7.37 (t, J = 7.2 Hz, 1H), 6.99 - 6.83 (m, 2H), 5.93 (s, 1H), 4.52 (d, J = 16.3 Hz, 1H), 4.34 (d, J = 16.3 Hz, 1H), 4.04 (dd, J = 21.8, 13.5 Hz, 4H), 3.55 (t, J = 9.0 Hz, 1H), 3.46 (s, 2H), 3.13 (dd, J = 10.1, 3.9 Hz, 1H), 2.91 (d, J = 43.7 Hz, 2H), 2.57 (d, J = 4.5 Hz, 5H), 2.06 (s, 6H), 1.12 (t, J = 7.2 Hz, 3H).

## Example 38

**Compound 38: 2-(3-((S)-7-(((R)-6-(2-chloro-4-fluorophenyl)-5-(methoxycarbonyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)acetic acid**

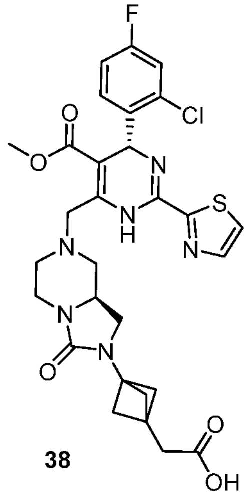

**38**

The title **Compound 38** as a yellow foam was prepared in analogy to the preparation of **Compound 1** by using (S)-2-(3-(3-oxohexahydroimidazo[1,5-a]pyrazin-2(3H)-yl)bicyclo[1.1.1]pentan-1-yl)acetic acid(**Intermediate 34-4**) instead of (S)-3-(3-oxohexahydroimidazo[1,5-a]pyrazin-2(3H)-yl)bicyclo[1.1.1]pentane-1-carboxylic acid (**Intermediate 1-5**) and methyl (R)-6-(bromomethyl)-4-(2-chloro-4-fluorophenyl)-2-(thiazol-2-yl)-1,4-dihydropyrimidine-5-carboxylate (**Intermediate A6**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 38** (9 mg). MS: 630.1 $(M+H)^+$. $^1H$ NMR (400 MHz, Methanol-$d_4$) δ 7.99 (s, 1H), 7.89 (s, 1H), 7.51 (t, J = 7.5 Hz, 1H), 7.27 (d, J = 8.9 Hz, 1H), 6.17 (s, 1H), 4.72 (d, J = 16.0 Hz, 1H), 4.54 (d, J = 16.0 Hz, 1H), 4.16 - 3.99 (m, 2H), 3.70 (d, J = 12.1 Hz, 2H), 3.63 (s, 3H), 3.58 (d, J = 8.9 Hz, 1H), 3.40 (t, J = 13.7 Hz, 1H), 3.17 (q, J = 12.4, 11.3 Hz, 3H), 2.57 (s, 2H), 2.07 (s, 6H).

## Example 39

**Compound 39: 2-(3-((S)-7-(((R)-6-(2-bromo-4-fluorophenyl)-5-(methoxycarbonyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)acetic acid**

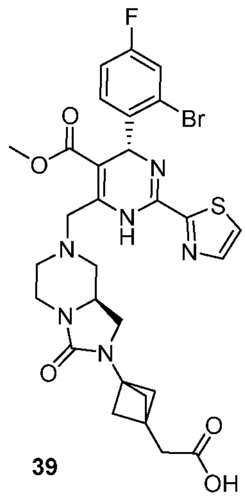

39

The title **Compound 39** as a yellow foam was prepared in analogy to the preparation of **Compound 1** by using (S)-2-(3-(3-oxohexahydroimidazo[1,5-a]pyrazin-2(3H)-yl)bicyclo[1.1.1]pentan-1-yl)acetic acid(**Intermediate 34-4**) instead of (S)-3-(3-oxohexahydroimidazo[1,5-a]pyrazin-2(3H)-yl)bicyclo[1.1.1]pentane-1-carboxylic acid (**Intermediate 1-5**) and methyl (R)-4-(2-bromo-4-fluorophenyl)-6-(bromomethyl)-2-(thiazol-2-yl)-1,4-dihydropyrimidine-5-carboxylate (**Intermediate A7**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 39** (5.9 mg). MS: 674.1 $(M+H)^+$. $^1H$ NMR (400 MHz, Methanol-$d_4$) δ 7.99 (s, 1H), 7.89 (s, 1H), 7.52 (t, J = 7.5 Hz, 1H), 7.45 (d, J = 8.5 Hz, 1H), 7.14 (t, J = 8.5 Hz, 1H), 6.17 (s, 1H), 4.73 (d, J = 16.0 Hz, 1H), 4.54 (d, J = 16.0 Hz, 1H), 4.17 - 3.99 (m, 2H), 3.70 (d, J = 12.5 Hz, 2H), 3.63 (s, 3H), 3.58 (d, J = 9.2 Hz, 1H), 3.40 (t, J = 13.6 Hz, 1H), 3.19 (dd, J = 21.2, 11.2 Hz, 3H), 2.57 (s, 2H), 2.07 (s, 6H).

**Example 40**

**Compound 40: 3-(3-((S)-7-(((R)-6-(2-chloro-4-fluorophenyl)-5-(ethoxycarbonyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)-2,2-dimethylpropanoic acid**

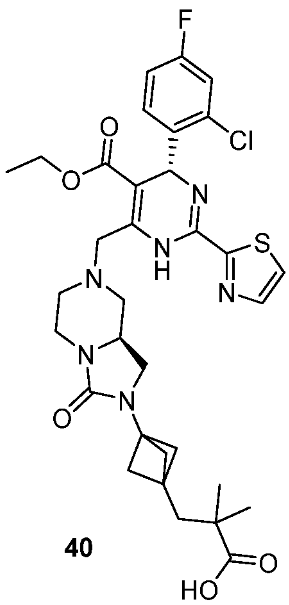

40

The title **Compound 40** as a yellow foam was prepared in analogy to the preparation of **Compound 1** by using

(S)-2,2-dimethyl-3-(3-(3-oxohexahydroimidazo[1,5-a]pyrazin-2(3H)-yl)bicyclo[1.1.1]pentan-1-yl)propanoic acid(**Intermediate 4-4**) instead of (S)-3-(3-oxohexahydroimidazo[1,5-a]pyrazin-2(3H)-yl)bicyclo[1.1.1]pentane-1-carboxylic acid (**Intermediate 1-5**) and methyl (R)-6-(bromomethyl)-4-(2-chloro-4-fluorophenyl)-2-(thiazol-2-yl)-1,4-dihydropyrimidine-5-carboxylate (**Intermediate A6**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 40** (25 mg). MS: 686.1 $(M+H)^+$. $^1H$ NMR (400 MHz, Methanol-d4) δ 7.99 (d, J = 3.1 Hz, 1H), 7.89 (d, J = 3.1 Hz, 1H), 7.52 (dd, J = 8.7, 6.0 Hz, 1H), 7.28 (dd, J = 8.6, 2.6 Hz, 1H), 7.14 - 7.07 (m, 1H), 6.18 (s, 1H), 4.71 (d, J = 16.0 Hz, 1H), 4.52 (d, J = 16.0 Hz, 1H), 4.06 (qd, J = 15.1, 14.3, 5.2 Hz, 4H), 3.68 (d, J = 12.2 Hz, 2H), 3.56 (t, J = 9.3 Hz, 1H), 3.43 - 3.34 (m, 1H), 3.23 - 3.09 (m, 3H), 2.00 (s, 6H), 1.86 (s, 2H), 1.18 (s, 7H), 1.12 (t, J = 7.1 Hz, 3H).

### Example 41

### Compound 41: 2-(3-((S)-7-(((S)-6-(3,4-difluoro-2-methylphenyl)-5-(methoxycarbonyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)acetic acid

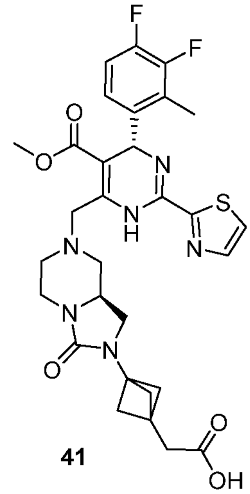

**41**

The title **Compound 41** as a yellow foam was prepared in analogy to the preparation of **Compound 1** by using (S)-2-(3-(3-oxohexahydroimidazo[1,5-a]pyrazin-2(3H)-yl)bicyclo[1.1.1]pentan-1-yl)acetic acid(**Intermediate 34-4**) instead of (S)-3-(3-oxohexahydroimidazo[1,5-a]pyrazin-2(3H)-yl)bicyclo[1.1.1]pentane-1-carboxylic acid (**Intermediate 1-5**) and methyl (S)-6-(bromomethyl)-4-(3,4-difluoro-2-methylphenyl)-2-(thiazol-2-yl)-1,4-dihydropyrimidine-5-carboxylate (**Intermediate A8**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 41** (16 mg). MS: 627.1 $(M+H)^+$. $^1H$ NMR (400 MHz, Methanol-$d_4$) δ 7.98 (d, J = 3.1 Hz, 1H), 7.88 (d, J = 3.1 Hz, 1H), 7.29 - 7.21 (m, 1H), 7.09 (q, J = 8.9 Hz, 1H), 5.93 (s, 1H), 4.70 (d, J = 16.1 Hz, 1H), 4.52 (d, J = 16.1 Hz, 1H), 4.13 - 4.01 (m, 4H), 3.71 (s, 1H), 3.59 (t, J = 9.1 Hz, 1H), 3.41 (t, J = 13.1 Hz, 1H), 3.17 (ddd, J = 13.0, 7.8, 4.3 Hz, 2H), 2.57 (s, 2H), 2.49 (d, J = 2.2 Hz, 3H), 2.07 (s, 6H), 1.12 (t, J = 7.1 Hz, 3H).

### Example 42

### Compound 42: 2-(3-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)-2-methylpropanoic acid

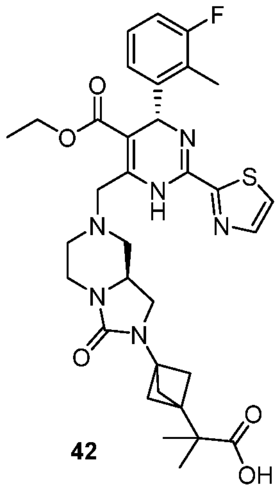

42

The title **Compound 42** as a yellow foam was prepared in analogy to the preparation of **Compound 1** by using (S)-2-methyl-2-(3-(3-oxohexahydroimidazo[1,5-a]pyrazin-2(3H)-yl)bicyclo[1.1.1]pentan-1-yl)propanoic acid(**Intermediate 42-1**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 42** (15 mg). MS: 651.1 $(M+H)^+$. $^1H$ NMR (400 MHz, Methanol-$d_4$) δ 7.97 (d, J = 3.1 Hz, 1H), 7.85 (d, J = 3.1 Hz, 1H), 7.30 - 7.13 (m, 2H), 6.98 (t, J = 8.9 Hz, 1H), 5.98 (s, 1H), 4.59 (d, J = 16.1 Hz, 1H), 4.41 (d, J = 16.3 Hz, 1H), 4.10 - 3.96 (m, 4H), 3.58 - 3.49 (m, 3H), 3.36 (s, 1H), 3.14 (dd, J = 9.8, 3.9 Hz, 1H), 3.04 (s, 1H), 2.95 (s, 1H), 2.46 (d, J = 2.0 Hz, 3H), 1.97 (s, 6H), 1.13 (d, J = 15.7 Hz, 9H).

**Example 43**

**Compound 43: 2-(3-((8S,8aR)-7-(((S)-5-(ethoxycarbonyl)-6-(3-fluoro-2-methylphenyl)-2-(thiazol-2-yl)-3,6-dihydropyrimidin-4-yl)methyl)-8-(methoxymethyl)-3-oxohexahydroimidazo[1,5-a]pyrazin-2(3H)-yl)bicyclo[1.1.1]pentan-1-yl)acetic acid**

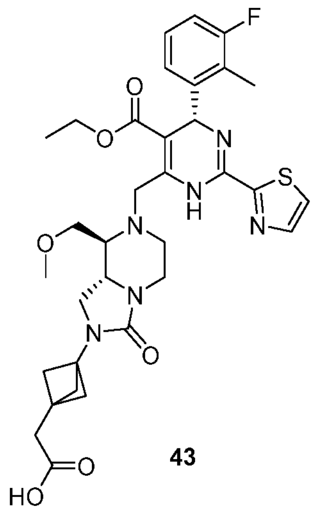

43

The title **Compound 43** as a yellow foam was prepared in analogy to the preparation of **Compound 1** by using 2-(3-((8S,8aR)-8-(methoxymethyl)-3-oxohexahydroimidazo[1,5-a]pyrazin-2(3H)-yl)bicyclo[1.1.1]pentan-1-yl)acetic acid(**Intermediate 43-7**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 43** (25 mg). MS: 667.1 $(M+H)^+$. $^1H$ NMR (400 MHz, Methanol-$d_4$) δ 8.01 (d, J = 3.1 Hz, 1H), 7.93 (d, J = 3.0 Hz, 1H), 7.32 - 7.16 (m, 2H), 7.01 (t, J = 8.9 Hz, 1H), 6.01 (s, 1H), 4.96 (d, J = 16.3 Hz, 1H), 4.60 (d, J = 16.3

Hz, 1H), 4.30 (d, J = 7.6 Hz, 1H), 4.10 (q, J = 7.1 Hz, 2H), 3.91 (dt, J = 13.3, 8.6 Hz, 4H), 3.54 (d, J = 20.0 Hz, 5H), 3.46 - 3.34 (m, 2H), 3.25 (d, J = 12.0 Hz, 1H), 2.57 (s, 2H), 2.46 (s, 3H), 2.08 (s, 6H), 1.12 (t, J = 7.1 Hz, 3H).

**Example 44**

**Compound 44: ethyl (S)-6-(((8S,8aR)-2-(3-(2-ethoxy-2-oxoethyl)bicyclo[1.1.1]pentan-1-yl)-8-(methoxymethyl)-3-oxohexahydroimidazo[1,5-a]pyrazin-7(1H)-yl)methyl)-4-(3-fluoro-2-methylphenyl)-2-(thiazol-2-yl)-1,4-dihydropyrimidine-5-carboxylate**

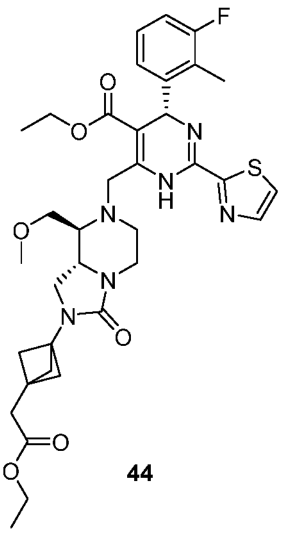

**44**

The title **Compound 44** as a yellow foam was prepared in analogy to the preparation of **Compound 1** by using **Intermediate 43-6** instead of (S)-3-(3-oxohexahydroimidazo[1,5-a]pyrazin-2(3H)-yl)bicyclo[1.1.1]pentane-1-carboxylic acid **(Intermediate 1-5)**.

**Compound 44** (25.3 mg). MS: 695.1 $(M+H)^+$. $^1H$ NMR (400 MHz, Methanol-*$d_4$*) δ 8.01 (d, J = 3.1 Hz, 1H), 7.93 (d, J = 3.1 Hz, 1H), 7.23 (dt, J = 21.5, 7.7 Hz, 2H), 7.01 (t, J = 8.9 Hz, 1H), 6.01 (s, 1H), 4.96 (d, J = 16.2 Hz, 1H), 4.60 (d, J = 16.3 Hz, 1H), 4.32 (q, J = 7.0, 5.7 Hz, 1H), 4.11 (dq, J = 11.5, 7.2 Hz, 4H), 3.91 (dt, J = 13.4, 8.7 Hz, 4H), 3.55 (d, J = 30.2 Hz, 5H), 3.40 (ddd, J = 21.7, 11.6, 4.3 Hz, 2H), 3.25 (dd, J = 12.8, 3.1 Hz, 1H), 2.60 (s, 2H), 2.46 (s, 3H), 2.07 (s, 6H), 1.25 (t, J = 7.1 Hz, 3H), 1.12 (t, J = 7.1 Hz, 3H).

**Example 45**

**Compound 46: (8S,8aR)-7-(((S)-5-(ethoxycarbonyl)-6-(3-fluoro-2-methylphenyl)-2-(thiazol-2-yl)-3,6-dihydropyrimidin-4-yl)methyl)-2-(3-(methylsulfonamido)bicyclo[1.1.1]pentan-1-yl)-3-oxooctahydroimidazo[1,5-a]pyrazine-8-carboxylic acid**

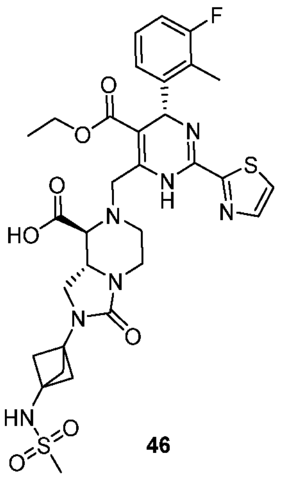

**46**

The title **Compound 46** as a yellow foam was prepared in analogy to the preparation of **Compound 1** by using (8S,8aR)-2-(3-(methylsulfonamido)bicyclo[1.1.1]pentan-1-yl)-3-oxooctahydroimidazo[1,5-a]pyrazine-8-carboxylic acid(**Intermediate 46-9**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 46** (9.8 mg). MS: 702.1 $(M+H)^+$.

**Example 46**

**Compound 47&Compound 48: (8S,8aR)-7-(((S)-5-(ethoxycarbonyl)-6-(3-fluoro-2-methylphenyl)-2-(thiazol-2-yl)-3,6-dihydropyrimidin-4-yl)methyl)-2-(3-(methoxymethyl)bicyclo[1.1.1]pentan-1-yl)-3-oxooctahydroimidazo[1,5-a]pyrazine-8-carboxylic acid & (8R,8aR)-7-(((S)-5-(ethoxycarbonyl)-6-(3-fluoro-2-methylphenyl)-2-(thiazol-2-yl)-3,6-dihydropyrimidin-4-yl)methyl)-2-(3-(methoxymethyl)bicyclo[1.1.1]pentan-1-yl)-3-oxooctahydroimidazo[1,5-a]pyrazine-8-carboxylic acid**

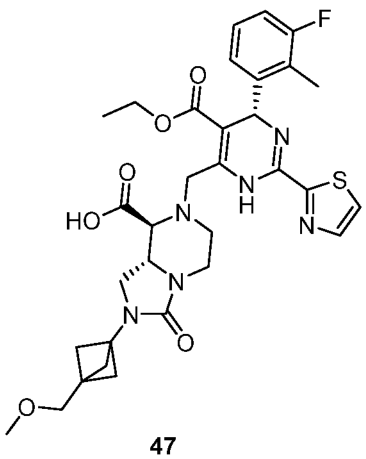

**47**

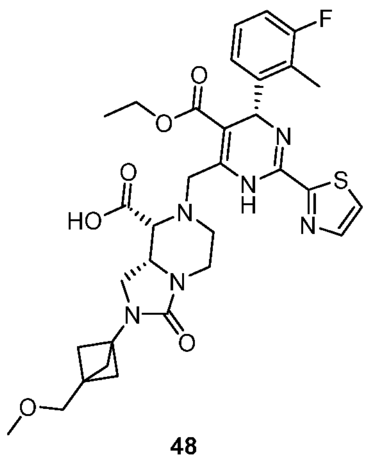

**48**

The title **Compound 47** & **Compound 48** as a yellow foam was prepared in analogy to the preparation of **Compound 1** by using (8S,8aR)-2-(3-(methoxymethyl)bicyclo[1.1.1]pentan-1-yl)-3-oxooctahydroimidazo[1,5-a]pyrazine-8-carboxylic acid(**Intermediate 47-9**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 47** (16.8 mg). MS: 653.2 $(M+H)^+$. $^1H$ NMR (400 MHz, Methanol-$d_4$) δ 8.12 (dd, J = 8.6, 3.0 Hz, 1H), 8.06 (t, J = 3.8 Hz, 1H), 7.24 (dd, J = 12.9, 6.3 Hz, 2H), 6.08 (d, J = 6.3 Hz, 1H), 4.09 (q, J = 7.1 Hz, 2H), 3.97 (dd, J = 17.0, 6.4 Hz, 1H), 3.86 - 3.74 (m, 2H), 3.58 (td, J = 9.0, 3.8 Hz, 1H), 3.49 (d, J = 6.9 Hz, 4H), 3.35 (s, 3H), 3.14 (t, J = 12.4 Hz, 2H), 2.67 (dt, J = 31.0, 10.3 Hz, 1H), 2.49 (s, 3H), 2.02 (s, 6H), 1.12 (t, J = 7.1 Hz, 3H).

**Compound 48** (19.9 mg). MS: 653.2 $(M+H)^+$. $^1H$ NMR (400 MHz, Methanol-d4) δ 8.12 (dd, J = 8.6, 3.0 Hz,

1H), 8.06 (t, J = 3.8 Hz, 1H), 7.24 (dd, J = 12.9, 6.3 Hz, 2H), 6.08 (d, J = 6.3 Hz, 1H), 4.09 (q, J = 7.1 Hz, 2H), 3.97 (dd, J = 17.0, 6.4 Hz, 1H), 3.86 - 3.74 (m, 2H), 3.58 (td, J = 9.0, 3.8 Hz, 1H), 3.49 (d, J = 6.9 Hz, 4H), 3.35 (s, 3H), 3.14 (t, J = 12.4 Hz, 2H), 2.67 (dt, J = 31.0, 10.3 Hz, 1H), 2.49 (s, 3H), 2.02 (s, 6H), 1.12 (t, J = 7.1 Hz, 3H).

**Example 47**

**Compound 49: N-(3-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)-N-methylglycine**

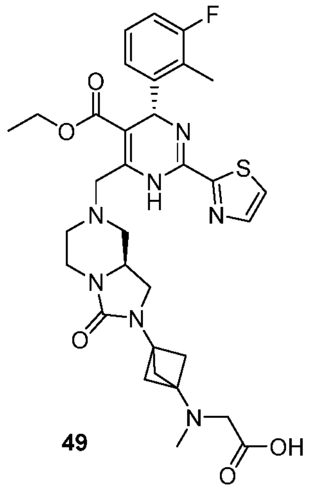

**49**

The title **Compound 49** as a yellow foam was prepared in analogy to the preparation of **Compound 1** by using (S)-N-methyl-N-(3-(3-oxohexahydroimidazo[1,5-a]pyrazin-2(3H)-yl)bicyclo[1.1.1]pentan-1-yl)glycine(**Intermediate 49-4**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 49** (45 mg). MS: 652.1 $(M+H)^+$. $^1H$ NMR (400 MHz, Methanol-$d_4$) δ 7.98 (d, J = 3.1 Hz, 1H), 7.88 (d, J = 3.1 Hz, 1H), 7.27 (d, J = 7.8 Hz, 1H), 7.24 - 7.15 (m, 1H), 6.99 (t, J = 8.9 Hz, 1H), 5.98 (s, 1H), 4.68 (d, J = 16.0 Hz, 1H), 4.51 (d, J = 16.0 Hz, 1H), 4.17 (s, 1H), 4.11 - 4.01 (m, 5H), 3.77 - 3.57 (m, 3H), 3.46 - 3.38 (m, 1H), 3.26 - 3.07 (m, 3H), 2.91 (s, 3H), 2.44 (d, J = 6.5 Hz, 9H), 1.10 (t, J = 7.1 Hz, 3H).

**Example 48**

**Compound 50: 2-(3-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)-2-hydroxyacetic acid**

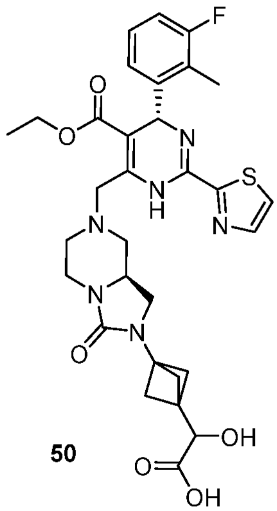

50

The title **Compound 50** as a yellow foam was prepared in analogy to the preparation of **Compound 1** by using 2-hydroxy-2-(3-((S)-3-oxohexahydroimidazo[1,5-a]pyrazin-2(3H)-yl)bicyclo[1.1.1]pentan-1-yl)acetic acid(**Intermediate 50-5**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 50** (5.8 mg). MS: 639.1 $(M+H)^+$. $^1$H NMR (400 MHz, Methanol-$d_4$) δ 7.97 (d, J = 3.1 Hz, 1H), 7.86 (d, J = 3.0 Hz, 1H), 7.29 - 7.14 (m, 2H), 6.99 (t, J = 8.9 Hz, 1H), 5.98 (s, 1H), 4.65 (d, J = 16.1 Hz, 1H), 4.48 (d, J = 16.2 Hz, 1H), 4.22 (s, 1H), 3.99-4.11 (m, 4H), 3.68 - 3.54 (m, 3H), 3.39 (t, J = 13.3 Hz, 1H), 3.20 - 3.02 (m, 3H), 2.45 (s, 3H), 2.05 (s, 6H), 1.10 (t, J = 7.1 Hz, 3H).

**Example 49**

**Compound 51: 2-(3-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[1.1.1]pentan-1-yl)-2-methoxyacetic acid**

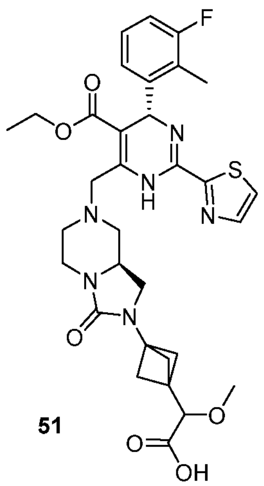

51

The title **Compound 51** as a yellow foam was prepared in analogy to the preparation of **Compound 1** by using 2-methoxy-2-(3-((S)-3-oxohexahydroimidazo[1,5-a]pyrazin-2(3H)-yl)bicyclo[1.1.1]pentan-1-yl)acetic acid(**Intermediate 51-2**) instead of (S)-3-(3-oxohexahydroimidazo[1,5-a]pyrazin-2(3H)-yl)bicyclo[1.1.1]pentane-1-carboxylic acid **(Intermediate 1-5).**

**Compound 51** (19.3 mg). MS: 653.1 $(M+H)^+$。

**Example 50**

**Compound 52: 4-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[2.2.2]octane-1-carboxylic acid**

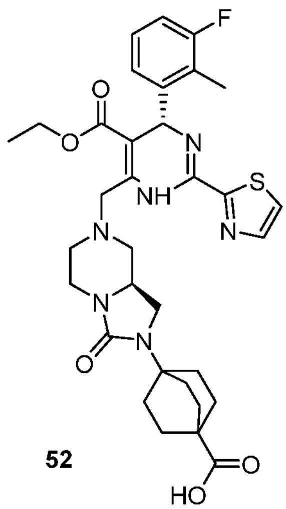

**52**

The title **Compound 52** as a yellow foam was prepared in analogy to the preparation of **Compound 1** by using (S)-4-(3-oxohexahydroimidazo[1,5-a]pyrazin-2(3H)-yl)bicyclo[2.2.2]octane-1-carboxylic acid (**Intermediate 52-5**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 52** (23 mg). MS: 651.7 $(M+H)^+$.

**Example 51**

**Compound 53: 4-((S)-7-(((S)-5-(ethoxycarbonyl)-6-(4-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[2.2.2]octane-1-carboxylic acid**

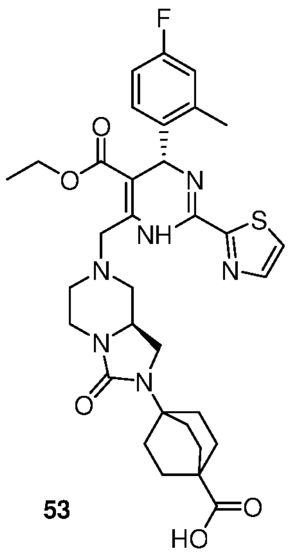

**53**

The title **Compound 53** as a yellow foam was prepared in analogy to the preparation of **Compound 1** by using

(S)-4-(3-oxohexahydroimidazo[1,5-a]pyrazin-2(3H)-yl)bicyclo[2.2.2]octane-1-carboxylic acid (**Intermediate 52-5**) instead of (S)-3-(3-oxohexahydroimidazo[1,5-a]pyrazin-2(3H)-yl)bicyclo[1.1.1]pentane-1-carboxylic acid (**Intermediate 1-5**) and ethyl (S)-6-(bromomethyl)-4-(4-fluoro-2-methylphenyl)-2-(thiazol-2-yl)-1,4-dihydropyrimidine-5-carboxylate (**Intermediate A5**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 53** (40 mg). MS: 651.7 $(M+H)^+$. $^1H$ NMR (400 MHz, Methanol-$d_4$) δ 7.99 (d, J = 3.3 Hz, 1H), 7.88 (d, J = 3.2 Hz, 1H), 7.42 (t, J = 7.1 Hz, 1H), 6.94 (t, J = 8.6 Hz, 2H), 5.94 (s, 1H), 4.68 (d, J = 16.3 Hz, 1H), 4.50 (d, J = 16.3 Hz, 1H), 4.06 (m, 4H), 3.63 (q, J = 9.8, 8.1 Hz, 3H), 3.35 (s, 1H), 3.12 (m, 3H), 2.57 (s, 3H), 1.97 (m, 12H), 1.18 - 1.08 (m, 3H).

**Example 52**

**Compound 54: 4-((S)-7-(((S)-6-(3,4-difluoro-2-methylphenyl)-5-(ethoxycarbonyl)-2-(thiazol-2-yl)-3,6-dihydropyrimidin-4-yl)methyl)-3-oxohexahydroimidazo[1,5-a]pyrazin-2(3H)-yl)bicyclo[2.2.2]octane-1-carboxylic acid**

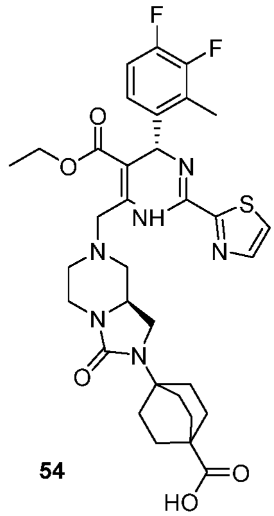

**54**

The title **Compound 54** as a yellow foam was prepared in analogy to the preparation of **Compound 1** by using (S)-4-(3-oxohexahydroimidazo[1,5-a]pyrazin-2(3H)-yl)bicyclo[2.2.2]octane-1-carboxylic acid (**Intermediate 52-5**) instead of (S)-3-(3-oxohexahydroimidazo[1,5-a]pyrazin-2(3H)-yl)bicyclo[1.1.1]pentane-1-carboxylic acid (**Intermediate 1-5**) and ethyl (S)-6-(bromomethyl)-4-(3,4-difluoro-2-methylphenyl)-2-(thiazol-2-yl)-1,4-dihydropyrimidine-5-carboxylate (**Intermediate A9**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 54** (45 mg). MS: 669.7 $(M+H)^+$. $^1H$ NMR (400 MHz, Methanol-$d_4$) δ 7.99 (t, J = 2.2 Hz, 1H), 7.89 (t, J = 2.3 Hz, 1H), 7.24 (t, J = 6.8 Hz, 1H), 7.14 - 7.05 (m, 1H), 5.94 (s, 1H), 4.70 (d, J = 16.1 Hz, 1H), 4.51 (d, J = 16.1 Hz, 1H), 4.16 - 3.95 (m, 4H), 3.72 - 3.56 (m, 3H), 3.31 - 3.04 (m, 4H), 2.50 (s, 3H), 2.12 - 1.85 (m, 13H), 1.13 (t, J = 7.1 Hz, 3H).

**Example 53**

**Compound 55: 4-((S)-7-(((R)-6-(2-chloro-4-fluorophenyl)-5-(ethoxycarbonyl)-2-(thiazol-2-yl)-3,6-dihydropyrimidin-4-yl)methyl)-3-oxohexahydroimidazo[1,5-a]pyrazin-2(3H)-yl)bicyclo[2.2.2]octane-1-carboxylic acid**

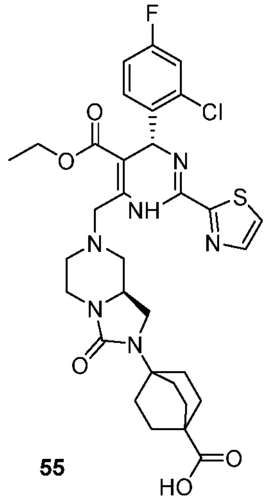

55

The title **Compound 55** as a yellow foam was prepared in analogy to the preparation of **Compound 1** by using (S)-4-(3-oxohexahydroimidazo[1,5-a]pyrazin-2(3H)-yl)bicyclo[2.2.2]octane-1-carboxylic acid (**Intermediate 52-5**) instead of (S)-3-(3-oxohexahydroimidazo[1,5-a]pyrazin-2(3H)-yl)bicyclo[1.1.1]pentane-1-carboxylic acid (**Intermediate 1-5**) and ethyl (R)-6-(bromomethyl)-4-(2-chloro-4-fluorophenyl)-2-(thiazol-2-yl)-1,4-dihydropyrimidine-5-carboxylate (**Intermediate A3**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 55** (65 mg). MS: 672.2 $(M+H)^+$. $^1H$ NMR (400 MHz, Methanol-$d_4$) δ 8.00 (t, J = 2.3 Hz, 1H), 7.89 (t, J = 2.4 Hz, 1H), 7.53 (dd, J = 8.6, 6.0 Hz, 1H), 7.29 (dd, J = 8.6, 2.5 Hz, 1H), 7.11 (t, J = 8.3 Hz, 1H), 6.20 (s, 1H), 4.71 (d, J = 16.0 Hz, 1H), 4.52 (d, J = 16.1 Hz, 1H), 4.06 (m,4H), 3.65 (m, 3H), 3.31 - 3.06 (m, 4H), 2.10 - 1.87 (m, 12H), 1.18 - 1.09 (m, 3H).

**Example 54**

**Compound 56: 4-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[2.1.1]hexane-1-carboxylic acid**

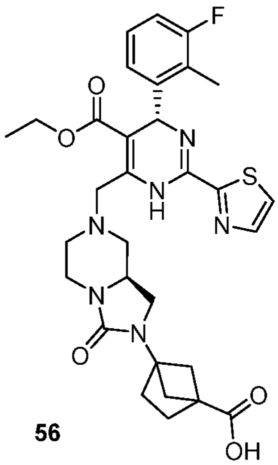

56

The title **Compound 56** as a yellow foam was prepared in analogy to the preparation of **Compound 1** by using (S)-4-(3-oxohexahydroimidazo[1,5-a]pyrazin-2(3H)-yl)bicyclo[2.1.1]hexane-1-carboxylic acid (**Intermediate 53-5**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 56** (110 mg). MS: 623.7 $(M+H)^+$. $^1H$ NMR (400 MHz, Methanol-$d_4$) δ 7.99 (d, J = 3.1 Hz, 1H), 7.89 (d, J = 3.1 Hz, 1H), 7.28 (d, J = 7.8 Hz, 1H), 7.25 - 7.16 (m, 1H), 7.00 (t, J = 8.9 Hz, 1H), 5.99 (s, 1H), 4.70 (d, J = 16.1 Hz, 1H), 4.52 (d, J = 16.1 Hz, 1H), 4.19 - 4.00 (m, 4H), 3.67 (m, 3H), 3.46 - 3.36 (m, 1H), 3.26 - 3.09 (m, 3H), 2.46 (d,

J = 2.0 Hz, 3H), 2.16 (dd, J = 15.4, 5.1 Hz, 2H), 2.02 - 1.89 (m, 4H), 1.82 (t, J = 4.8 Hz, 2H), 1.12 (t, J = 7.1 Hz, 3H).

**Example 55**

**Compound 57: 4-((S)-7-(((R)-6-(2-chloro-4-fluorophenyl)-5-(ethoxycarbonyl)-2-(thiazol-2-yl)-3,6-dihydropyrimidin-4-yl)methyl)-3-oxohexahydroimidazo[1,5-a]pyrazin-2(3H)-yl)bicyclo[2.1.1]hexane-1-carboxylic acid**

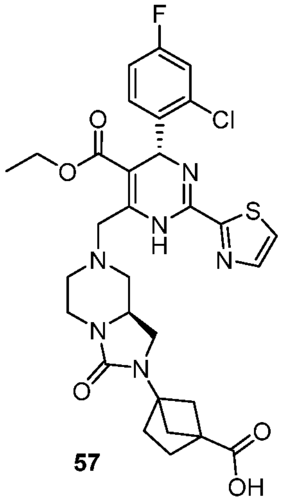

**57**

The title **Compound 57** as a yellow foam was prepared in analogy to the preparation of **Compound 1** by using (S)-4-(3-oxohexahydroimidazo[1,5-a]pyrazin-2(3H)-yl)bicyclo[2.1.1]hexane-1-carboxylic acid (**Intermediate 53-5**) instead of (S)-3-(3-oxohexahydroimidazo[1,5-a]pyrazin-2(3H)-yl)bicyclo[1.1.1]pentane-1-carboxylic acid (**Intermediate 1-5**) and ethyl (R)-6-(bromomethyl)-4-(2-chloro-4-fluorophenyl)-2-(thiazol-2-yl)-1,4-dihydropyrimidine-5-carboxylate (**Intermediate A3**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 57** (99 mg). MS: 644.1 $(M+H)^+$. $^1H$ NMR (400 MHz, Methanol-$d_4$) $\delta$ 7.99 (d, J = 3.1 Hz, 1H), 7.89 (d, J = 3.0 Hz, 1H), 7.54 (dd, J = 8.7, 6.0 Hz, 1H), 7.28 (dd, J = 8.7, 2.5 Hz, 1H), 7.11 (td, J = 8.3, 2.5 Hz, 1H), 6.20 (s, 1H), 4.72 (d, J = 16.0 Hz, 1H), 4.53 (d, J = 16.0 Hz, 1H), 4.19 - 4.00 (m, 4H), 3.67 (q, J = 10.2, 9.0 Hz, 3H), 3.49 - 3.37 (m, 1H), 3.27 - 3.11 (m, 3H), 2.16 (m, 2H), 2.02 - 1.89 (m, 4H), 1.82 (t, J = 4.9 Hz, 2H), 1.13 (t, J = 7.1 Hz, 3H).

**Example 56**

**Compound 58: 2-(4-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[2.1.1]hexan-1-yl)acetic acid**

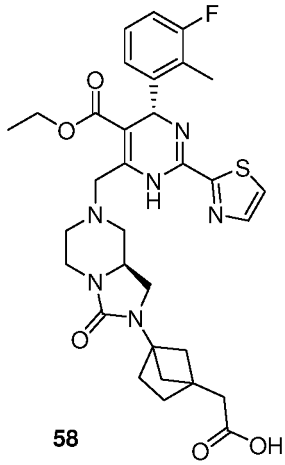

58

The title **Compound 58** as a yellow foam was prepared in analogy to the preparation of **Compound 1** by using (S)-2-(4-(3-oxohexahydroimidazo[1,5-a]pyrazin-2(3H)-yl)bicyclo[2.1.1]hexan-1-yl)acetic acid (**Intermediate 54-5**) instead of (S)-3-(3-oxohexahydroimidazo[1,5-a]pyrazin-2(3H)-yl)bicyclo[1.1.1]pentane-1-carboxylic acid **(Intermediate 1-5**).

**Compound 58** (18 mg). MS: 637.7 $(M+H)^+$. $^1$H NMR (400 MHz, Methanol-$d_4$) δ 7.98 (d, J = 3.1 Hz, 1H), 7.86 (d, J = 3.0 Hz, 1H), 7.32 - 7.16 (m, 2H), 6.99 (t, J = 8.8 Hz, 1H), 5.99 (s, 1H), 4.62 (d, J = 16.2 Hz, 1H), 4.44 (d, J = 16.2 Hz, 1H), 4.13 - 3.95 (m, 4H), 3.67 - 3.52 (m, 3H), 3.43 - 3.33 (m, 1H), 3.18 (m, 1H), 3.13 - 2.95 (m, 2H), 2.54 (s, 2H), 2.47 (d, J = 2.0 Hz, 3H), 1.96 - 1.88 (m, 2H), 1.80 (m, 2H), 1.67 (m,4H), 1.12 (t, J = 7.1 Hz, 3H).

**Example 57**

**Compound 59: 4-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[2.2.1]heptane-1-carboxylic acid**

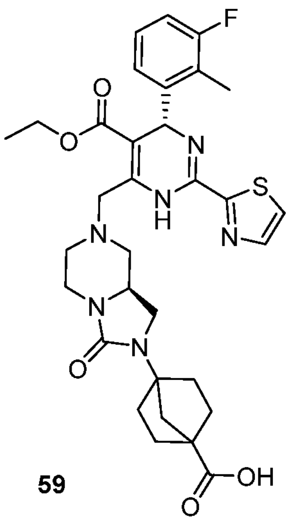

59

The title **Compound 59** as a yellow foam was prepared in analogy to the preparation of **Compound 1** by using (S)-4-(3-oxohexahydroimidazo[1,5-a]pyrazin-2(3H)-yl)bicyclo[2.2.1]heptane-1-carboxylic acid (**Intermediate 55-5**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 59** (85 mg). MS: 637.7 $(M+H)^+$. $^1$H NMR (400 MHz, Methanol-$d_4$) δ 7.99 (d, J = 3.1 Hz, 1H), 7.89 (d, J = 3.1 Hz, 1H), 7.28 (d, J = 7.9 Hz, 1H), 7.20 (q, J = 7.4 Hz, 1H), 7.01 (t, J = 8.9 Hz, 1H), 5.99 (s, 1H), 4.72 (d, J = 16.1 Hz, 1H), 4.54 (d, J = 16.1 Hz, 1H), 4.17 - 4.01 (m, 4H), 3.74 - 3.62 (m, 3H), 3.40 (t, J = 13.9 Hz, 1H), 3.28 - 3.11 (m, 3H), 2.46 (d, J = 2.0 Hz, 3H), 2.16 - 1.84 (m, 8H), 1.76 (d, J = 11.8 Hz, 2H), 1.12 (t, J = 7.1 Hz, 3H).

**Example 58**

**Compound 60: 4-((S)-7-(((R)-6-(2-chloro-4-fluorophenyl)-5-(ethoxycarbonyl)-2-(thiazol-2-yl)-3,6-dihydropyrimidin-4-yl)methyl)-3-oxohexahydroimidazo[1,5-a]pyrazin-2(3H)-yl)bicyclo[2.2.1]heptane-1-carboxylic acid**

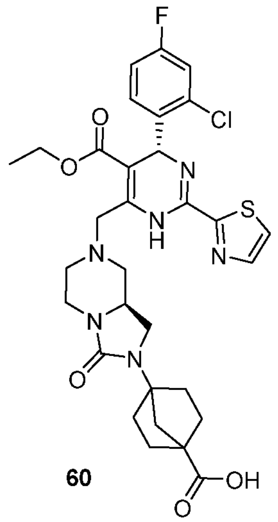

**60**

The title **Compound 60** as a yellow foam was prepared in analogy to the preparation of **Compound 1** by using (S)-4-(3-oxohexahydroimidazo[1,5-a]pyrazin-2(3H)-yl)bicyclo[2.2.1]heptane-1-carboxylic acid (**Intermediate 55-5**) instead of (S)-3-(3-oxohexahydroimidazo[1,5-a]pyrazin-2(3H)-yl)bicyclo[1.1.1]pentane-1-carboxylic acid **(Intermediate 1-5)** and ethyl (R)-6-(bromomethyl)-4-(2-chloro-4-fluorophenyl)-2-(thiazol-2-yl)-1,4-dihydropyrimidine-5-carboxylate (**Intermediate A3**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 60** (45 mg). MS: 658.1 $(M+H)^+$. $^1H$ NMR (400 MHz, Methanol-$d_4$) $\delta$ 8.00 (d, J = 3.0 Hz, 1H), 7.89 (d, J = 3.0 Hz, 1H), 7.54 (dd, J = 8.6, 6.1 Hz, 1H), 7.29 (dd, J = 8.7, 2.5 Hz, 1H), 7.11 (td, J = 8.3, 2.5 Hz, 1H), 6.20 (s, 1H), 4.73 (d, J = 16.0 Hz, 1H), 4.54 (d, J = 16.0 Hz, 1H), 4.17 - 3.98 (m, 4H), 3.68 (t, J = 8.9 Hz, 3H), 3.45 - 3.34 (m, 1H), 3.28 - 3.12 (m, 3H), 2.18 - 1.83 (m, 8H), 1.76 (d, J = 12.2 Hz, 2H), 1.13 (t, J = 7.1 Hz, 3H).

**Example 59**

**Compound 61:**

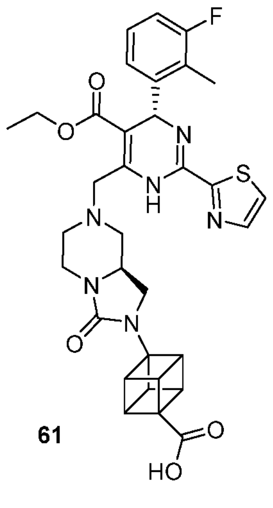

**61**

The title **Compound 61** as a yellow foam was prepared in analogy to the preparation of **Compound 1** by using (S)-4-(3-oxohexahydroimidazo[1,5-a]pyrazin-2(3H)-yl)cubane-1-carboxylic acid (**Intermediate 56-5**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 61** (10 mg). MS: 645.7 $(M+H)^+$. $^1H$ NMR (400 MHz, Methanol-$d_4$) δ 7.99 (d, J = 3.1 Hz, 1H), 7.88 (d, J = 3.1 Hz, 1H), 7.27 (d, J = 7.8 Hz, 1H), 7.25 - 7.17 (m, 1H), 7.00 (t, J = 8.9 Hz, 1H), 5.99 (s, 1H), 4.69 (d, J = 16.1 Hz, 1H), 4.52 (d, J = 16.1 Hz, 1H), 4.35 - 4.02 (m, 9H), 3.74 - 3.63 (m, 3H), 3.43 (t, J = 12.8 Hz, 1H), 3.28 - 3.11 (m, 3H), 2.46 (d, J = 2.0 Hz, 3H), 1.12 (t, J = 7.1 Hz, 3H).

**Example 60**

**Compound 62:**

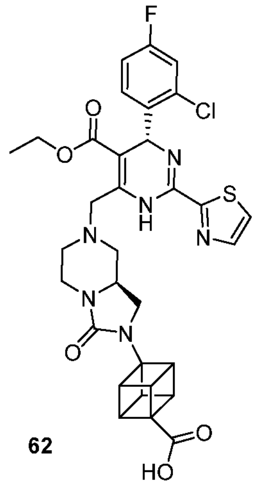

**62**

The title **Compound 62** as a yellow foam was prepared in analogy to the preparation of **Compound 1** by using (S)-4-(3-oxohexahydroimidazo[1,5-a]pyrazin-2(3H)-yl)cubane-1-carboxylic acid (**Intermediate 56-5**) instead of (S)-3-(3-oxohexahydroimidazo[1,5-a]pyrazin-2(3H)-yl)bicyclo[1.1.1]pentane-1-carboxylic acid (**Intermediate 1-5**) and ethyl (R)-6-(bromomethyl)-4-(2-chloro-4-fluorophenyl)-2-(thiazol-2-yl)-1,4-dihydropyrimidine-5-carboxylate (**Intermediate A3**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 62** (8 mg). MS: 665.1 $(M+H)^+$. $^1H$ NMR (400 MHz, Methanol-$d_4$) δ 7.99 (d, J = 3.1 Hz, 1H), 7.87 (d, J = 3.0 Hz, 1H), 7.52 (dd, J = 8.7, 6.1 Hz, 1H), 7.28 (dd, J = 9.0, 2.4 Hz, 1H), 7.11 (td, J = 8.2, 4.3 Hz, 1H), 6.19 (s, 1H), 4.63 (d, J = 16.2 Hz, 1H), 4.44 (d, J = 16.1 Hz, 1H), 4.31 (t, J = 5.1 Hz, 3H), 4.07 (m, , 6H), 3.69 - 3.55 (m, 3H), 3.39 (t, J = 13.3 Hz, 1H), 3.23 (m, 1H), 3.07 (m, 2H), 1.13 (t, J = 7.1 Hz, 3H).

**Example61**

**Compound 63: 2-(4-((S)-7-(((S)-5-(ethoxycarbonyl)-6-(3-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[2.2.1]heptan-1-yl)acetic acid**

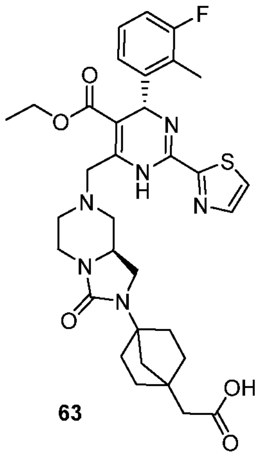

63

The title **Compound 63** as a yellow foam was prepared in analogy to the preparation of **Compound 1** by using (S)-2-(4-(3-oxohexahydroimidazo[1,5-a]pyrazin-2(3H)-yl)bicyclo[2.2.1]heptan-1-yl)acetic acid (**Intermediate 57-5**) instead of (S)-3-(3-oxohexahydroimidazo[1,5-a]pyrazin-2(3H)-yl)bicyclo[1.1.1]pentane-1-carboxylic acid (**Intermediate 1-5**).

**Compound 63** (25 mg). MS: 651.7 $(M+H)^+$. $^1H$ NMR (400 MHz, DMSO-$d_6$) δ 7.19 (d, J = 3.1 Hz, 1H), 7.09 (d, J = 3.1 Hz, 1H), 6.48 (d, J = 7.9 Hz, 1H), 6.40 (q, J = 7.7 Hz, 1H), 6.21 (t, J = 9.0 Hz, 1H), 5.19 (d, J = 1.5 Hz, 1H), 3.91 (dd, J = 16.1, 6.4 Hz, 1H), 3.74 (dd, J = 16.2, 3.0 Hz, 1H), 3.31-3.22 (m, 4H), 2.95 - 2.80 (m, 3H), 2.67 - 2.55 (m, 1H), 2.50 - 2.14 (m, 4H), 1.66 (t, J = 2.1 Hz, 5H), 1.28 - 0.74 (m, 10H), 0.32 (t, J = 7.1 Hz, 3H).

**Example62**

**Compound 64: 2-(4-((S)-7-(((S)-5-(ethoxycarbonyl)-6-(4-fluoro-2-methylphenyl)-2-(thiazol-2-yl)-3,6-dihydropyrimidin-4-yl)methyl)-3-oxohexahydroimidazo[1,5-a]pyrazin-2(3H)-yl)bicyclo[2.2.1]heptan-1-yl)acetic acid**

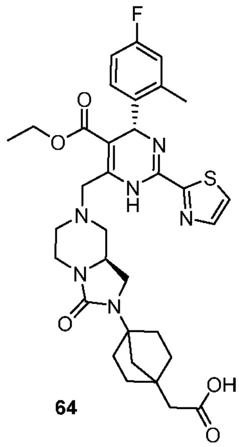

64

The title **Compound 64** as a yellow foam was prepared in analogy to the preparation of **Compound 63** by using ethyl (S)-6-(bromomethyl)-4-(4-fluoro-2-methylphenyl)-2-(thiazol-2-yl)-1,4-dihydropyrimidine-5-carboxylate (**Intermediate A5**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).

**Compound 64** (11 mg). MS: 651.7 $(M+H)^+$. $^1H$ NMR (400 MHz, Methanol-$d_4$) δ 7.19 (d, J = 3.1 Hz, 1H), 7.09 (d, J = 3.2 Hz, 1H), 6.65-6.61 (m, 1H), 6.18 - 6.10 (m, 2H), 5.14 (d, J = 1.4 Hz, 1H), 3.91 (dd, J = 16.1, 7.2 Hz, 1H), 3.73 (dd, J = 16.0, 3.8 Hz, 1H), 3.31-3.21 (m, 4H), 2.97 - 2.81 (m, 3H), 2.65 - 2.55 (m, 1H), 2.49 - 2.26 (m, 4H), 1.77 (s, 3H), 1.66 (d, J = 2.8 Hz, 2H), 1.26 - 0.73 (m, 10H), 0.32 (t, J = 7.1 Hz, 3H).

**Example63**

**Compound 65: 2-(4-((S)-7-(((R)-6-(2-chloro-4-fluorophenyl)-5-(ethoxycarbonyl)-2-(thiazol-2-yl)-3,6-dihydropyrimidin-4-yl)methyl)-3-oxohexahydroimidazo[1,5-a]pyrazin-2(3H)-yl)bicyclo[2.2.1]heptan-1-yl)acetic acid**

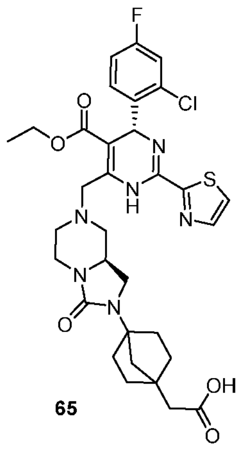

**65**

The title **Compound 65** as a yellow foam was prepared in analogy to the preparation of **Compound 1** by using ethyl (R)-6-(bromomethyl)-4-(2-chloro-4-fluorophenyl)-2-(thiazol-2-yl)-1,4-dihydropyrimidine-5-carboxylate **(Intermediate A3**) instead of ethyl (S)-6-(bromomethyl)-4-(3-fluoro-2-methylphenyl)-2-(thiazol-2-yl)-1,4-dihydropyrimidine-5-carboxylate (**Intermediate A1**).
**Compound 65** (20 mg). MS: 672.2 $(M+H)^+$.

**Cell-based anti-viral activity assay**

The Invention determined the anti-HBV (hepatitis B virus) activity of compounds in HepG2.2.15 cells (Table 1).
HepG2.2.15 is a cell line stably transfected with the HBV genome. HepG2.2.15 cells were cultured in DMEM (1X) with 10% fetal bovine serum (Corning), 100 U penicillin (Gibico), 100 μg/mL streptomycin (Gibico), 250 μg /mL geneticin (Gibco) and GlutaMAXTM-I medium (Invitrogen).
HepG2.2.15 cells were seeded into 96-well plates ($4\times10^4$ cells in 100μL of medium per well) and incubated overnight at 37 ° C. The test compounds were weighed and prepared as 10 mM stock solution with DMSO. The stock solutions of the compounds were diluted by a gradient of 3.16 times with DMSO, and then diluted by a gradient of 3.16 times with medium for 50 times. Finally, the diluted compound solutions were added to the cell plate with a total volume of 200μL and the final DMSO concentration was 1%. The 96-well culture plates loaded with drugs were transferred to an incubator at 37 ° C for 5 days. 2μL of nucleic acid releasing agent was added to 96-well PCR reaction plate, and 2μL of cell supernatant or HBV quantitative standards were added to the corresponding Wells. Centrifuged the plates to fully mix, and incubated at room temperature for 10min to fully release nucleic acid. Quantified HBV DNA content by fluorescent quantitative PCR (CFX Connect Real-Time System) using Sanxiang Bio hepatitis B virus fluorescent quantitative diagnostic kit.
The standard curve was established by taking the logarithm of the copy number (IU/ml) of the HBV standard sample (log10) as the abscordinate and the corresponding CT value as the ordinate. The copy number of the sample was calculated according to the corresponding CT of the positive sample and the standard curve, and the percentage of inhibition rate was calculated as follows:

% Inhibition = 100-100* (sample HBV copy number -high concentration reference compound column HBV copy number) /DMSO column HBV copy number -high concentration reference compound column HBV copy number).

**Cytotoxicity assay**

The Invention determined cytotoxicity of the compounds against HepG2.2.15 cells (Table 2)

HepG2.2.15 cells were seeded into 96-well plates (8000 cells per well) and cell viability was measured by the addition of 60 μL of CellTiter Glo reagent (Promega) five days post treatment with the compounds. Cell plates with CellTiter Glo added were shaken on a plate shaker for 20 min and then the chemiluminescence signal was stabilized at room temperature for 10 min. Chemiluminescence values were read using a microplate reader (Bio-TeK SYNERGY NEO2).

The concentration corresponding to 50% cytotoxicity ($CC_{50}$) was fitted by graphpad prism software. The cytotoxicity was calculated by the formula: % Cytotoxicity =100-(chemiluminescence value of sample Well -average chemiluminescence value of blank medium column)/(average chemiluminescence value of DMSO column -average chemiluminescence value of blank medium column) *100

Table 1: Anti-HBV activity of the test compounds in the Invention in HepG2.2.15 cells

| Compound No. | $EC_{50}$ (HepG2.2.15, nM) | Compound No. | $EC_{50}$ (HepG2.2.15, nM) |
|---|---|---|---|
| **1** | 33 | **2** | 3 |
| **3** | 4 | **4** | 8 |
| **5** | 73 | **6** | 8 |
| **7** | 7 | **8** | 4 |
| **9** | 4 | **10** | 8 |
| **11** | 4 | **12** | 3 |
| **13** | 8 | **14** | 4 |
| **15** | 4 | **16** | 9 |
| **17** | 5 | **18** | 3 |
| **19** | 3 | **20** | 3 |
| **21** | 15 | **22** | 7 |
| **23** | 4 | **24** | 9 |
| **25** | 2 | **26** | 5 |
| **27** | 6 | **28** | 21 |
| **29** | 6 | **30** | 11 |
| **31** | 25 | **32** | 30 |
| **33** | 202 | **34** | 22 |
| **35** | 28 | **36** | 48 |
| **37** | 18 | **38** | 43 |
| **39** | 32 | **40** | 12 |
| **41** | 8 | **42** | 11 |
| **43** | 197 | **44** | 183 |
| **46** | 61 | **47** | 18 |
| **48** | 15 | **49** | 17 |
| **50** | 3 | **51** | 139 |
| **52** | 3 | **53** | 9 |
| **54** | 3 | **55** | 2 |
| **56** | 16 | **57** | 12 |
| **58** | 6 | **59** | 12 |

(continued)

| Compound No. | $EC_{50}$ (HepG2.2.15, nM) | Compound No. | $EC_{50}$ (HepG2.2.15, nM) |
|---|---|---|---|
| **60** | 9 | **61** | 17 |
| **63** | 26 | **64** | 57 |
| **65** | 42 | | |

Table 2: Cytotoxicity of the test compounds in the Invention in HepG2.2.15 cells

| Compound No. | $CC_{50}$ (HepG2.2.15, $\mu$M) | Compound No. | $CC_{50}$ (HepG2.2.15, $\mu$M) |
|---|---|---|---|
| **1** | >50 | **2** | >50 |
| **5** | >50 | **17** | >50 |
| **18** | >50 | **22** | >50 |
| **28** | >50 | **29** | >50 |
| **33** | >50 | **35** | >50 |
| **37** | >50 | **38** | >50 |
| **44** | >50 | **46** | >50 |
| **47** | >50 | **48** | >50 |
| **49** | >50 | **52** | >50 |
| **53** | >50 | **55** | >50 |
| **56** | >50 | **57** | >50 |
| **58** | >50 | **59** | >50 |
| **60** | >50 | **61** | >50 |

Conclusion: The experimental data indicated the compounds in the Invention have good anti-HBV activity and low cytotoxicity.

**Pharmacokinetics (PK) evaluation in mice**

Test compounds were administered to ICR mice by gavage (po). The po dose was 10mg/kg, and the vehicle formulation was 10% DMSO + 40% PEG400 + 50% Saline (0.9% saline). Animals were fasted overnight (10-14 h) before administration and fed 4 h after administration. Blood was collected into heparin sodium anticoagulated tubes at multiple time points (0.5, 1, 3, 5 hours) after administration. Mice were euthanized with $CO_2$ and livers were collected at multiple time points (0.5, 1, 3, 5 h). The drug concentrations in plasma and liver were analyzed by LC-MS/MS method. Phenix WinNonlin was used to calculate the pharmacokinetic parameters. The results are presented in Tables 3 and 4.

Table 3: Drug concentrationof Compound 22 and Compound 58

| Compound No. | Time (hour) | Plasm drug conc. (ng/mL) | Liver drug conc. (ng/g) | Liver/Plasma ratio |
|---|---|---|---|---|
| Compound 22 | 0.5 | 849 | 102141 | 120 |
| | 1 | 826 | 94764 | 115 |
| | 3 | 180 | 24696 | 137 |
| | 5 | 41 | 12198 | 297 |

(continued)

| Compound No. | Time (hour) | Plasm drug conc. (ng/mL) | Liver drug conc. (ng/g) | Liver/Plasma ratio |
|---|---|---|---|---|
| Compound 58 | 0.5 | 2034 | 62519 | 31 |
| | 1 | 1544 | 61296 | 40 |
| | 3 | 470 | 26816 | 57 |
| | 5 | 324 | 14219 | 44 |

Table 4: Pharmacokinetic parameters of Compound 22 and Compound 58

| Compound No. | Plasma Cmax (ng/mL) | Plasma $AUC_{(0\rightarrow t)}$ (h*ng/mL) | Liver Cmax (ng/g) | $LiverAUC_{(0\rightarrow t)}$ (h*ng/g) |
|---|---|---|---|---|
| Compound 22 | 849 | 1859 | 102141 | 231116 |
| Compound 58 | 2034 | 4210 | 62519 | 175730 |

Conclusion: The experimental data indicated that the compounds of the Invention have special pharmacokinetic properties in mice, especially the higher liver-to-plasma drug concentration ratio (L/P). Considering that the liver is the reproduction organ of HBV virus, the compounds of the Invention are expected to achieve good efficacy and safety in anti-HBV therapy.

## Claims

**1.** A dihydropyrimidine compound of formula I' or a pharmaceutically acceptable salt thereof,

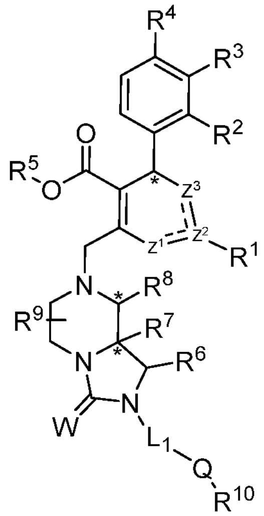

I'

wherein,

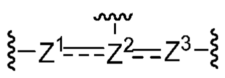 is 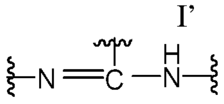 or 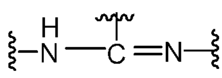;

W is O or S;
$R^1$ is 2-thiazolyl or 2-thiazolyl substituted by one or more $R^{11}$;
$R^{11}$ is halogen or methyl;
$R^2$, $R^3$ and $R^4$ are independently selected from H, halogen and $C_1$-$C_3$ alkyl;

$R^5$ is methyl or ethyl;
$R^6$ is H or $C_1$-$C_3$ alkyl;
$R^7$ is H, halogen, $C_1$-$C_3$alkl, $C_1$-$C_3$alkox or

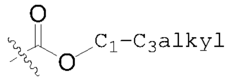
;

$R^8$ is H, COOH, $C_1$-$C_3$alkl substituted with Ci-Csalkoxy,

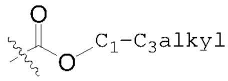
;

$R^9$ is H, halogen, $C_1$-$C_3$alkyl substituted with $C_1$-$C_3$alkoxy,

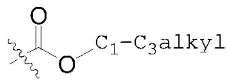
;

Li is single bond or $C_1$-$C_3$alkylene;
Q is a 5-8-membered bridged cycloalkyl or a 5-8-membered heterocyclyl; wherein the 5-8-membered heterocyclyl is bridged-linked, the heteroatoms in the 5-8-membered heterocyclyl are selected from N, O, and S, and the number of heteroatoms is one, two, or three;
$R^{10}$ is COOH, CN, $C_1$-$C_6$ alkyl substituted by one or more $R^A$, $C_1$-$C_6$ alkyl,

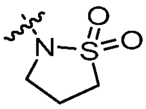
,

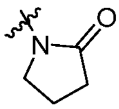
,

$NR^BR^C$, or

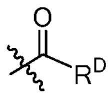
;

$R^A$ is a hydroxyl, halogen, $C_1$-$C_6$ alkoxy, COOH,

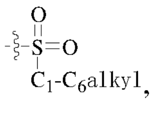
, 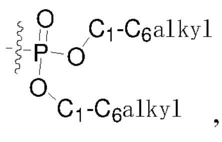
,

CN,

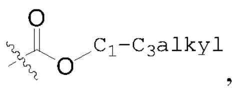
,

$C_4$-$C_6$ heterocyclyl substituted by a carbonyl,

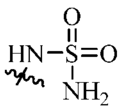, 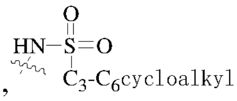,

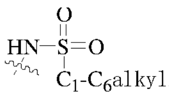 or 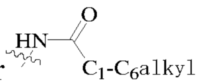;

wherein the number of heteroatoms in the $C_4$-$C_6$ heterocyclyl is one, two, or three, and the heteroatoms are selected from N, O, and S;
$R^B$ and $R^C$ are independently H,

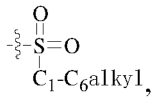, 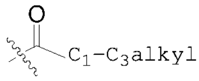,

$C_1$-$C_6$ alkyl,

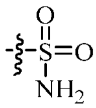,

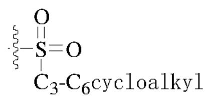

or -$C_1$-$C_6$ alkylene COOH;
$R^D$ is $C_4$-$C_6$ heterocyclyl, $NH_2$-, -$NHC_1$-$C_6$ alkyl,

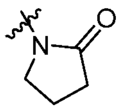 or 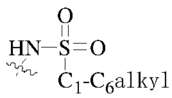,

wherein the number of heteroatoms in the $C_4$-$C_6$ heterocyclyl is one, two, or three, and the heteroatoms are selected from N, O, and S;
A carbon atom with "*" indicates that when the carbon atom is a chiral carbon atom, the carbon atom with "*" is in the S configuration, R configuration, or a mixture thereof; and

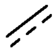 represents a single or double bond.

**2.** The dihydropyrimidine compound of formula I' according to claim 1, or a pharmaceutically acceptable salt thereof, wherein, Q is

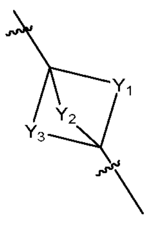 or 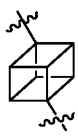;

wherein, $Y_1$ is - $CH_2$- or -$CH_2CH_2$-; $Y_2$ is -$CH_2$- or -$CH_2CH_2$-; $Y_3$ is -$CH_2$-, -$CH_2CH_2$-, - O-$CH_2$-, or -$CH_2$-O-.

**3.** The dihydropyrimidine compound of formula I' according to claim 1, or a pharmaceutically acceptable salt thereof, having a structure of formula I,

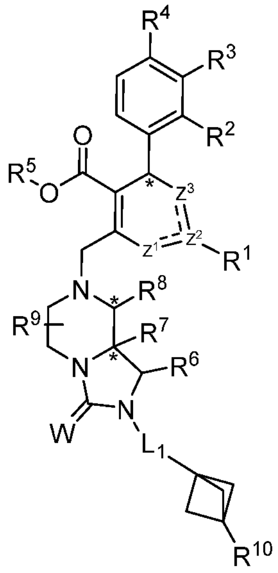

I

wherein,

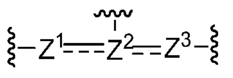 is 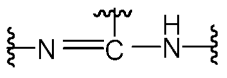 or 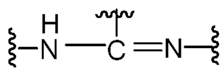;

W is O or S;
$R^1$ is 2-thiazolyl or 2-thiazolyl substituted by one or more $R^{11}$;
$R^{11}$ is halogen or methyl;
$R^2$, $R^3$ and $R^4$ are independently selected from H, halogen and $C_1$-$C_3$ alkyl;
$R^5$ is methyl or ethyl;
$R^6$ is H or $C_1$-$C_3$ alkyl;
$R^7$ is H, halogen, $C_1$-$C_3$alkyl, $C_1$-$C_3$alkoxy or

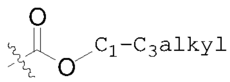;

$R^8$ is H, COOH, $C_1$-$C_3$alkyl substituted with $C_1$-$C_3$alkoxy,

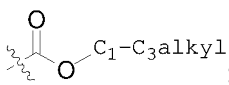;

$R^9$ is H, halogen, $C_1$-$C_3$alkyl substituted with $C_1$-$C_3$alkoxy,

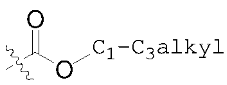;

Li is single bond or $C_1$-$C_3$alkylene;
$R^{10}$ is COOH, CN, $C_1$-$C_6$ alkyl substituted by one or more $R^A$, $C_1$-$C_6$ alkyl,

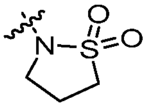,

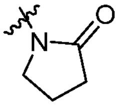,

$NR^BR^C$, or

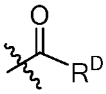;

$R^A$ is independently hydroxyl, halogen, $C_1$-$C_6$ alkoxy, COOH,

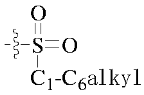,

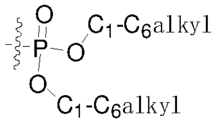,

CN,

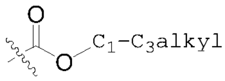,

$C_4$-$C_6$ heterocyclyl substituted by a carbonyl,

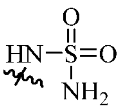, 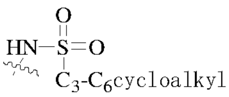, 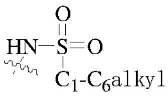 or 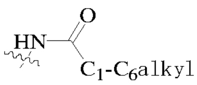;

wherein the number of heteroatoms in the $C_4$-$C_6$ heterocyclyl is one, two, or three, and the heteroatoms are selected from N, O, and S;
$R^B$ and $R^C$ are independently H,

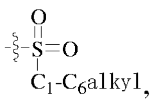, 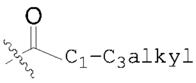,

$C_1$-$C_6$ alkyl,

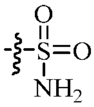,

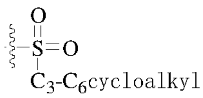

or -$C_1$-$C_6$ alkylene-COOH;
$R^D$ is $C_4$-$C_6$ heterocyclyl, $NH_2$-, -$NHC_1$-$C_6$ alkyl,

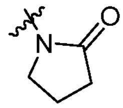 or 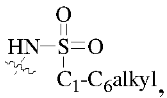,

wherein the number of heteroatoms in the $C_4$-$C_6$ heterocyclyl is one, two, or three, and the heteroatoms are selected from N, O, and S;
A carbon atom with "*" indicates that when the carbon atom is a chiral carbon atom, the carbon atom with "*" is in the S configuration, R configuration, or a mixture thereof;

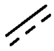 represents a single or double bond.

**4.** The dihydropyrimidine compound of formula I' according to claim 1, or a pharmaceutically acceptable salt thereof, wherein,

when $R^{11}$ is halogen, the halogen is fluorine, chlorine, bromine, or iodine;
and/or, when $R^2$, $R^3$, and $R^4$ are independently halogen, the halogen is fluorine, chlorine, bromine, or iodine;
and/or, when $R^2$, $R^3$, and $R^4$ are independently $C_1$-$C_3$ alkyl, the $C_1$-$C_3$ alkyl is methyl, ethyl, n-propyl, or isopropyl;
and/or, when $R^6$ is $C_1$-$C_3$ alkyl, the $C_1$-$C_3$ alkyl is methyl, ethyl, n-propyl or isopropyl;
and/or, when $R^7$ is halogen, the halogen is fluorine, chlorine, bromine, or iodine;
and/or, when $R^7$ is $C_1$-$C_3$ alkyl, the $C_1$-$C_3$ alkyl is methyl, ethyl, n-propyl or isopropyl;
and/or, when $R^7$ is $C_1$-$C_3$ alkoxy, the $C_1$-$C_3$ alkyl is methoxy, ethoxy, n-propoxy or isopropoxy;
and/or, when $R^7$ is

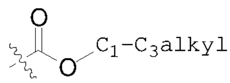,

the $C_1$-$C_3$ alkyl is methyl, ethyl, n-propyl or isopropyl;
and/or, when $R^8$ is $C_1$-$C_3$ alkyl substituted by $C_1$-$C_3$ alkoxy, the $C_1$-$C_3$ alkyl is methyl, ethyl, n-propyl or isopropyl;
and/or, when $R^8$ is $C_1$-$C_3$ alkyl substituted by $C_1$-$C_3$ alkoxy, the $C_1$-$C_3$ alkoxy is methoxy, ethoxy, n-propoxy or isopropoxy;
and/or, when $R^8$ is

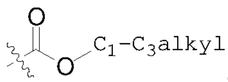,

the $C_1$-$C_3$ alkyl is methyl, ethyl, n-propyl or isopropyl;
and/or, when $R^9$ is halogen, the halogen is fluorine, chlorine, bromine, or iodine;
and/or, when $R^9$ is $C_1$-$C_3$ alkyl substituted by $C_1$-$C_3$ alkoxy, the $C_1$-$C_3$ alkyl is methyl, ethyl, n-propyl or isopropyl;
and/or, when $R^9$ is $C_1$-$C_3$ alkyl substituted by $C_1$-$C_3$ alkoxy, the $C_1$-$C_3$ alkoxy is methoxy, ethoxy, n-propoxy or isopropoxy;
and/or, when $R^9$ is

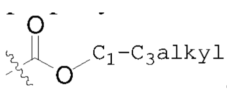,

the $C_1$-$C_3$ alkyl is methyl, ethyl, n-propyl or isopropyl;

and/or, when Liis $C_1$-$C_3$alkylene, the $C_1$-$C_3$alkylene is methylene,

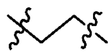, 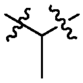,

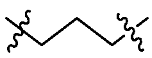, 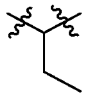, 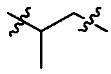 or 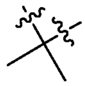;

and/or, when $R^{10}$ is $C_1$-$C_6$ alkyl substituted by one or more $R^A$, the $C_1$-$C_6$alkyl is methyl, ethyl, n-propyl, isopropyl, n-butyl, isobutyl, tert butyl, n-pentyl, isopentyl or neopentyl;
and/or, when $R^{10}$ is $C_1$-$C_6$ alkyl, the $C_1$-$C_6$ alkyl is methyl, ethyl, n-propyl, isopropyl, n-butyl, isobutyl, tert butyl, n-pentyl, isopentyl or neopentyl;
and/or, when $R^A$ is halogen, the halogen is fluorine, chlorine, bromine, or iodine;
and/or, when $R^A$ is $C_1$-$C_6$ alkoxy, the $C_1$-$C_6$ alkoxy is methoxy, ethoxy, n-propoxy, isopropoxy, n-butoxy, isobutyloxy, tert butoxy, n-pentoxy, isopentoxy or neopentoxy;
and/or, when $R^A$ is

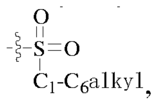,

the $C_1$-$C_6$ alkyl is methyl, ethyl, n-propyl, isopropyl, n-butyl, isobutyl, tert butyl, n-pentyl, isopentyl or neopentyl;
and/or, when $R^A$ is

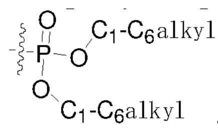,

the $C_1$-$C_6$ alkyl is independently methyl, ethyl, n-propyl, isopropyl, n-butyl, isobutyl, tert butyl, n-pentyl, isopentyl or neopentyl;
and/or, when $R^A$ is

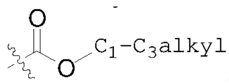,

the $C_1$-$C_3$ alkyl is methyl, ethyl, n-propyl or isopropyl;
and/or, when $R^A$ is $C_4$-$C_6$ heterocyclyl substituted by one carbonyl, the $C_4$-$C_6$ heterocyclyl is tetrahydropyrrolyl, tetrahydrofuryl, tetrahydrothienyl, piperidinyl, tetrahydropyranyl or morpholinyl;
and/or, when $R^A$ is

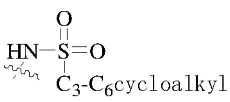,

the $C_3$-$C_6$ cycloalkyl is cyclopropyl, cyclobutyl, or cyclopentyl;
and/or, when $R^A$ is

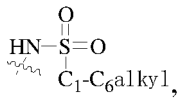

the $C_1$-$C_6$ alkyl is methyl, ethyl, n-propyl, isopropyl, n-butyl, isobutyl, tert butyl, n-pentyl, isopentyl or neopentyl; and/or, when $R^A$ is

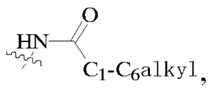

the $C_1$-$C_6$ alkyl is methyl, ethyl, n-propyl, isopropyl, n-butyl, isobutyl, tert butyl, n-pentyl, isopentyl or neopentyl; and/or, when $R^B$ and $R^C$ are independently

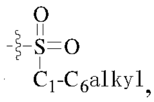

the $C_1$-$C_6$ alkyl is independently methyl, ethyl, n-propyl, isopropyl, n-butyl, isobutyl, tert butyl, n-pentyl, isopentyl or neopentyl;
and/or, when $R^B$ and $R^C$ are independently

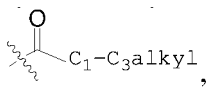

the $C_1$-$C_3$ alkyl is methyl, ethyl, n-propyl, or isopropyl;
and/or, when $R^B$ and $R^C$ are independently $C_1$-$C_6$ alkyl, the $C_1$-$C_6$ alkyl is methyl, ethyl, n-propyl, isopropyl, n-butyl, isobutyl, tert butyl, n-pentyl, isopentyl or neopentyl;
and/or, when $R^B$ and $R^C$ are independently

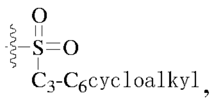

the $C_3$-$C_6$ cycloalkyl is cyclopropyl, cyclobutyl, cyclopentyl or cyclohexyl;
and/or, when $R^B$ and $R^C$ are independently -$C_1$-$C_6$ alkylene-COOH, the $C_1$-$C_6$ alkylene is methylene, ethylene, propylene, butylene, pentylene, or hexylene;
and/or, when $R^D$ is $C_4$-$C_6$ heterocyclyl, the $C_4$-$C_6$ heterocyclyl is tetrahydropyrrolyl, tetrahydrofuryl, tetrahydrothienyl, piperidinyl, tetrahydropyranyl or morpholinyl;
and/or, when $R^D$ is -NH$C_1$-$C_6$ alkyl, the $C_1$-$C_6$ alkyl is methyl, ethyl, n-propyl, isopropyl, n-butyl, isobutyl, tert butyl, n-pentyl, isopentyl or neopentyl;
and/or, when $R^D$ is

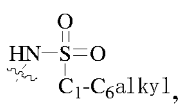

the $C_1$-$C_6$ alkyl is methyl, ethyl, n-propyl, isopropyl, n-butyl, isobutyl, tert butyl, n-pentyl, isopentyl or neopentyl;
and/or, when Q is a 5-8-membered heterocyclyl; the 5-8-membered heterocyclyl is bridged-linked, the heteroatom in the 5-8-membered heterocyclyl is O, and the number of heteroatoms is one;
and/or, when Q is 5-8-membered bridged cycloalkyl; the 5-8 membered bridged cycloalkyl is a 5-membered bridged cycloalkyl;
and/or, when Q is 5-8-membered bridged cycloalkyl; the 5-8 membered bridged cycloalkyl is a 6-8 membered

bridged cycloalkyl.

5. A dihydropyrimidine compound of formula I' according to claim 1, or a pharmaceutically acceptable salt thereof, wherein,

when $R^2$, $R^3$, and $R^4$ are independently halogen, the halogen is fluorine, chlorine, or bromine;
and/or, when $R^2$, $R^3$, and $R^4$ are independently $C_1$-$C_3$ alkyl, the $C_1$-$C_3$ alkyl is methyl;
and/or, when $R^8$ is $C_1$-$C_3$ alkyl substituted by $C_1$-$C_3$ alkoxy, the $C_1$-$C_3$ alkyl is methyl;
and/or, when $R^8$ is $C_1$-$C_3$ alkyl substituted by $C_1$-$C_3$ alkoxy, the $C_1$-$C_3$ alkoxy is methoxy;
and/or, when $R^8$ is

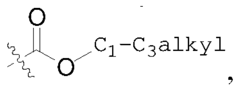,

the $C_1$-$C_3$ alkyl is ethyl;
and/or, when $L_1$ is $C_1$-$C_3$alkylene, the $C_1$-$C_3$alkylene is methylene;
and/or, when $R^{10}$ is $C_1$-$C_6$ alkyl substituted by one or more $R^A$, the $C_1$-$C_6$alkyl is methyl, ethyl, isopropyl or isobutyl;
and/or, when $R^A$ is halogen, the halogen is chlorine;
and/or, when $R^A$ is $C_1$-$C_6$ alkoxy, the $C_1$-$C_6$ alkoxy is methoxy;
and/or, when $R^A$ is

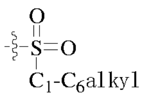,

the $C_1$-$C_6$ alkyl is methyl;
and/or, when $R^A$ is

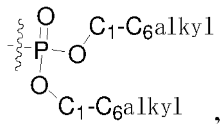,

the $C_1$-$C_6$ alkyl is independently ethyl;
and/or, when $R^A$ is

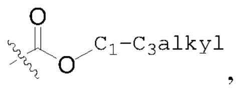,

the $C_1$-$C_3$ alkyl is methyl;
and/or, when $R^A$ is $C_4$-$C_6$ heterocyclyl substituted by one carbonyl, the $C_4$-$C_6$ heterocyclyl is tetrahydropyrrolyl;
and/or, when $R^A$ is

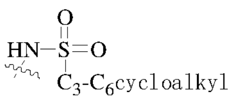,

the $C_3$-$C_6$ cycloalkyl is cyclopropyl;
and/or, when $R^A$ is

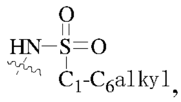

the $C_1$-$C_6$ alkyl is methyl;
and/or, when $R^A$ is

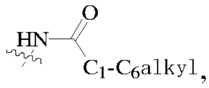

the $C_1$-$C_6$ alkyl is methyl;
and/or, when $R^B$ and $R^C$ are independently

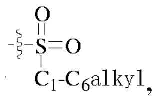

the $C_1$-$C_6$ alkyl is methyl;
and/or, when $R^B$ and $R^C$ are independently

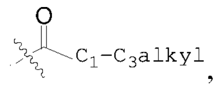

the $C_1$-$C_3$ alkyl is methyl;
and/or, when $R^B$ and $R^C$ are independently $C_1$-$C_6$ alkyl, the $C_1$-$C_6$ alkyl is methyl;
and/or, when $R^B$ and $R^C$ are independently

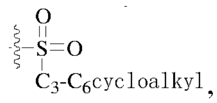

the $C_3$-$C_6$ cycloalkyl is cyclopropyl;
and/or, when $R^B$ and $R^C$ are independently -$C_1$-$C_6$ alkylene-COOH, the $C_1$-$C_6$ alkylene is methylene;
and/or, when $R^D$ is $C_4$-$C_6$ heterocyclyl, the $C_4$-$C_6$ heterocyclyl is

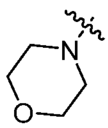 or 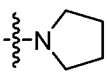;

and/or, when $R^D$ is -NH$C_1$-$C_6$ alkyl, the $C_1$-$C_6$ alkyl is methyl;
and/or, when $R^D$ is 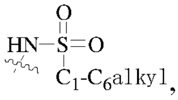 the $C_1$-$C_6$ alkyl is methyl;
and/or, when Q is 5-8-membered bridged cycloalkyl; the 5-8 membered bridged cycloalkyl is 5-membered bridged cycloalkyl;
and/or, when Q is 5-8-membered bridged cycloalkyl; the 5-8 membered bridged cycloalkyl is 6-8 membered bridged cycloalkyl, the 6-8 membered bridged cycloalkyl is

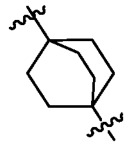,

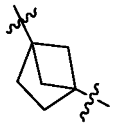, 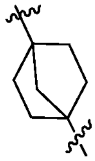 or 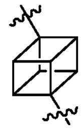.

**6.** A dihydropyrimidine compound of formula I' according to claim 1, or a pharmaceutically acceptable salt thereof, wherein,

when $R^8$ is $C_1$-$C_3$ alkyl substituted by $C_1$-$C_3$ alkoxy, the $C_1$-$C_3$ alkyl substituted by $C_1$-$C_3$ alkoxy is

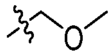;

and/or, when $R^A$ is

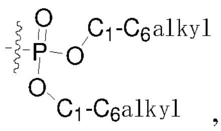, 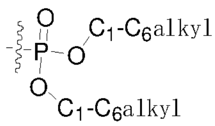 is 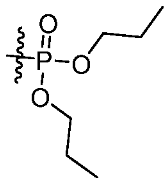;

and/or, when $R^A$ is $C_4$-$C_6$ heterocyclyl substituted by one carbonyl, the $C_4$-$C_6$ heterocyclyl substituted by one carbonyl is

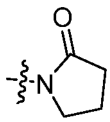;

and/or, when $R^{10}$ is $C_1$-$C_6$ alkyl substituted by one $R^A$, the $C_1$-$C_6$ alkyl substituted by a $R^A$ is

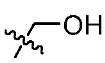, 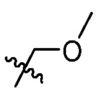, 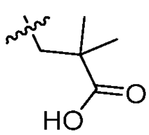, 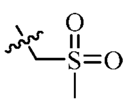, 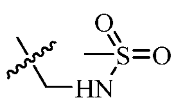, 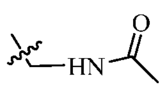, 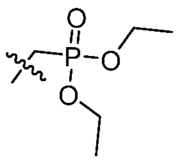,

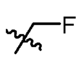, 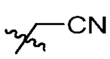, 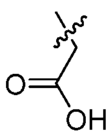, 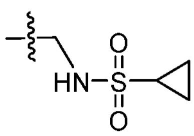, 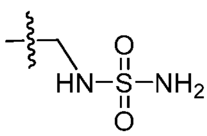, 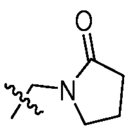, 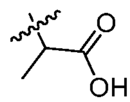,

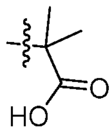 or 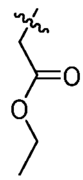 ;

and/or, when $R^{10}$ is $C_1$-$C_6$ alkyl substituted by more $R^A$, the $C_1$-$C_6$ alkyl substituted by more $R^A$ is

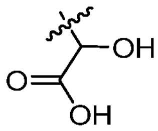 or 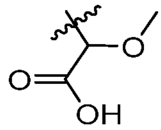 ;

and/or, when $R^{10}$ is $NR^BR^C$, $NR^BR^C$ is

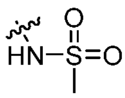 , 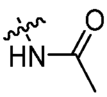 , 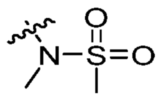 , 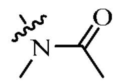 ,

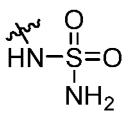 , 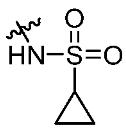 or 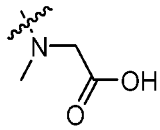 ;

and/or, when $R^{10}$ is

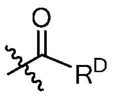 , 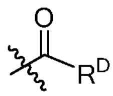 is 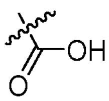 , 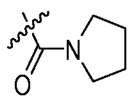 , 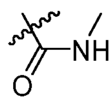 , 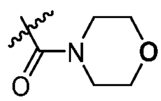

or

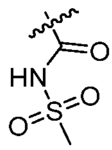 .

**7.** The dihydropyrimidine compound of formula I' according to claim 1, or a pharmaceutically acceptable salt thereof, wherein, the compound of formula I' is any of the following compounds:

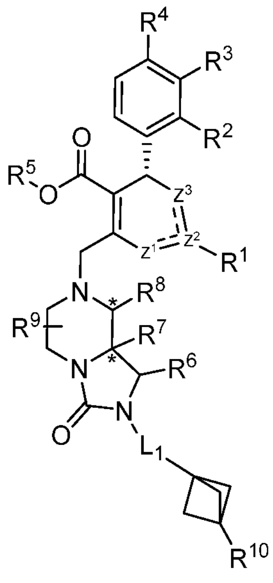,
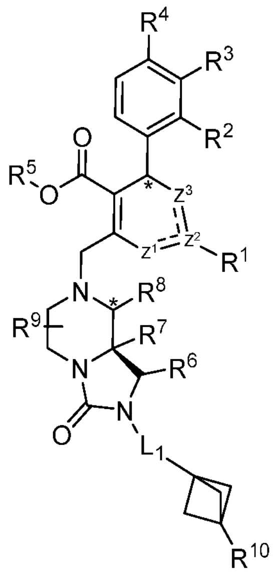,
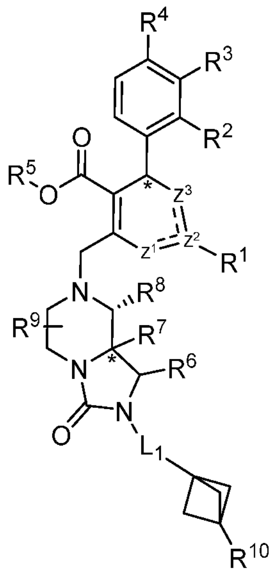,

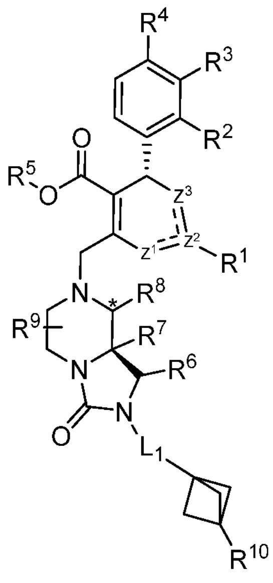,
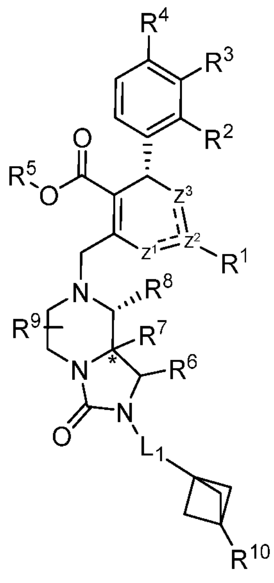,
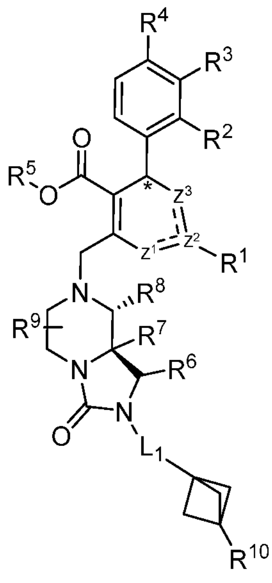,

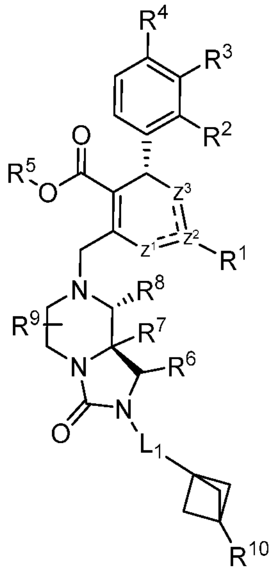 , 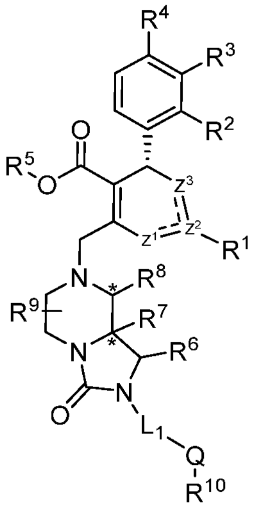 , 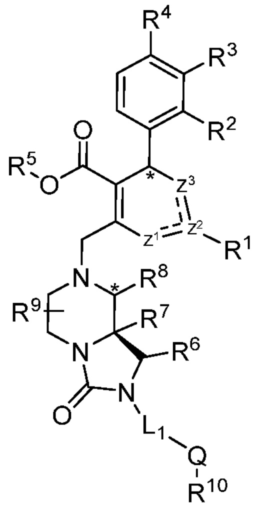 , 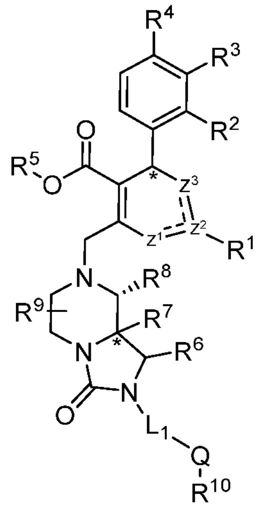 ,

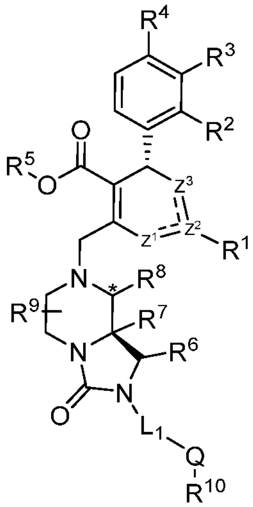 , 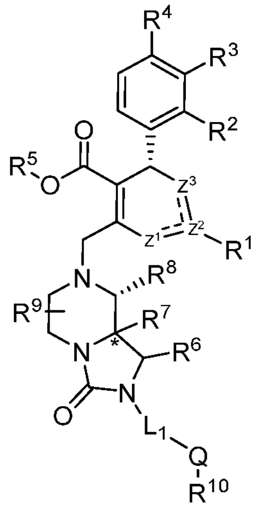 , 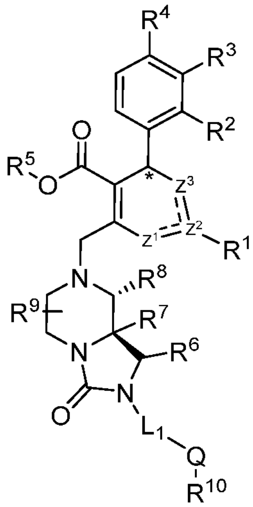 or 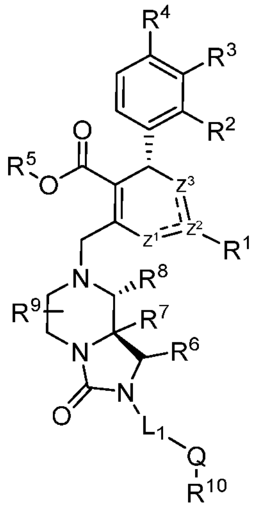 .

**8.** The dihydropyrimidine compound of formula I' according to claim 1, or a pharmaceutically acceptable salt thereof, wherein,

$R^1$ is 2-thiazolyl;
and/or, when $R^2$, $R^3$, and $R^4$ are independently H, fluorine, chlorine, bromine, or methyl;
and/or, $R^3$ and $R^4$ are not halogen at the same time;
and/or, $R^6$ is H;
and/or, $R^7$ is H;
and/or, $R^8$ is H or

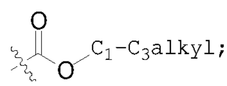 ;

and/or, $R^9$ is H;
and/or, Li is single bond or $C_1$-$C_3$alkylene;
and/or, $R^{10}$ is CN, $C_1$-$C_6$ alkyl substituted by one $R^A$, $C_1$-$C_6$ alkyl,

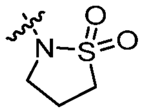, 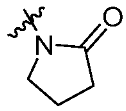,

$NR^BR^C$, or

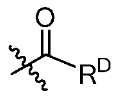;

and/or, $R^{10}$ is COOH, CN, $C_1$-$C_6$ alkyl substituted by one $R^A$, $C_1$-$C_6$ alkyl,

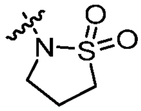,

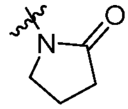,

$NR^BR^C$, or 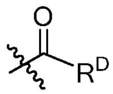;

and/or, $R^A$ is hydroxyl, halogen, $C_1$-$C_6$ alkoxy,

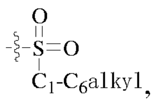, 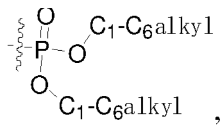,

CN,

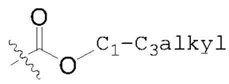,

$C_4$-$C_6$ heterocyclyl substituted by a carbonyl,

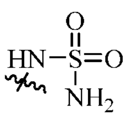, 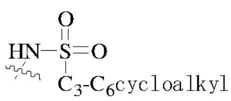,

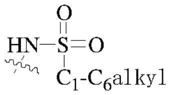 or 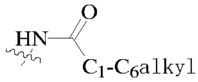;

and/or, $R^B$ and $R^C$ are independently H,

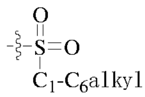, 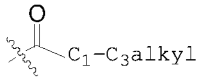,

$C_1$-$C_6$ alkyl or

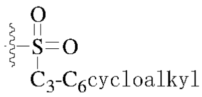;

and/or, $R^D$ is $C_4$-$C_6$ heterocyclyl, $NH_2$- or -$NHC_1$-$C_6$alkyl.

**9.** The dihydropyrimidine compound of formula I' according to claim 8, or a pharmaceutically acceptable salt thereof, wherein,

$R^2$, $R^3$ and $R^4$ are independently H, fluorine, or methyl;
and/or, $R^8$ is H;
and/or, Li is single bond.

**10.** The dihydropyrimidine compound of formula I' according to claim 1, or a pharmaceutically acceptable salt thereof, wherein, the compound of formula I' is any of the following scheme:

scheme 1:
wherein:

$R^1$ is 2-thiazolyl;
$R^6$ is H;
$R^7$ is H;
$R^9$ is H; and
Li is single bond or methylene;

scheme 2:
wherein:

$R^1$ is 2-thiazolyl;
$R^6$ is H;
$R^7$ is H;
$R^8$ is H,

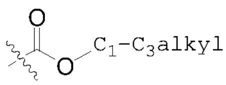;

$R^9$ is H;
Li is single bond; and
$R^{10}$ is COOH, CN, $C_1$-$C_6$ alkyl substituted by one $R^A$, $C_1$-$C_6$ alkyl,

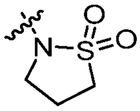,

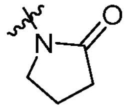,

$NR^BR^C$, or

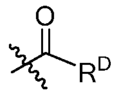;

scheme 3:
wherein:

W is O;
$R^1$ is 2-thiazolyl;
$R^2$, $R^3$, and $R^4$ are independently H, fluorine, or methyl; and $R^3$ and $R^4$ are preferably not fluorine at the same time;
$R^6$ is H;
$R^7$ is H;
$R^8$ is H;
$R^9$ is H;
Li is single bond;
$R^{10}$ is CN, $C_1$-$C_6$ alkyl substituted by one $R^A$, $C_1$-$C_6$ alkyl,

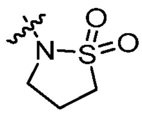, 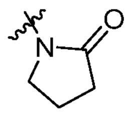,

$NR^BR^C$, or

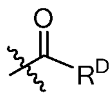;

and
$R^D$ is $C_4$-$C_6$ heterocyclyl, $NH_2$- or -$NHC_1$-$C_6$alkyl;

scheme 4:
wherein:

W is O;
$R^1$ is 2-thiazolyl;
$R^2$, $R^3$, and $R^4$ are independently H, fluorine, or methyl;
$R^6$ is H;
$R^7$ is H;
$R^8$ is H;
$R^9$ is H;
Li is single bond;
$R^{10}$ is CN, $C_1$-$C_6$ alkyl substituted by one $R^A$, $C_1$-$C_6$ alkyl,

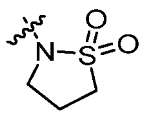, 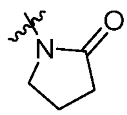,

$NR^BR^C$, or

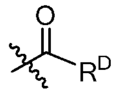;

$R^A$ is hydroxyl, halogen, $C_1$-$C_6$ alkoxy,

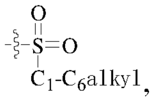, 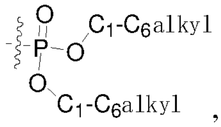, CN,

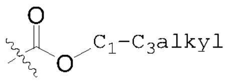,

$C_4$-$C_6$ heterocyclyl substituted by a carbonyl,

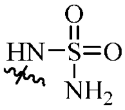, 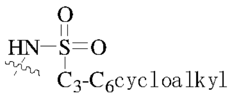,

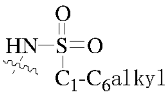 or 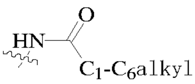;

$R^B$ and $R^C$ are independently H,

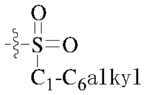, 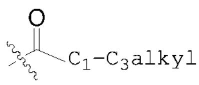,

$C_1$-$C_6$ alkyl or

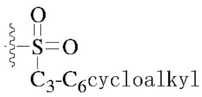;

$R^D$ is $C_4$-$C_6$ heterocyclyl, $NH_2$- or -$NHC_1$-$C_6$alkyl;

scheme 5:
wherein,

$R^1$ is 2-thiazolyl;
$R^6$ is H;
$R^7$ is H;
$R^8$ is H,

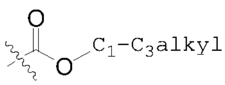;

$R^9$ is H;

Li is single bond;

$R^{10}$ is COOH, CN, $C_1$-$C_6$ alkyl substituted by one $R^A$, $C_1$-$C_6$ alkyl,

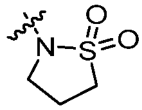

,

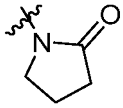

,

$NR^BR^C$, or

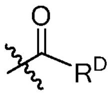

;

and

Q is

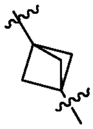

;

scheme 6:

wherein,

$R^1$ is 2-thiazolyl;

$R^6$ is H;

$R^7$ is H;

$R^8$ is H;

$R^9$ is H;

$L_1$ is single bond;

$R^{10}$ is COOH, CN, $C_1$-$C_6$ alkyl substituted by one $R^A$, $C_1$-$C_6$ alkyl,

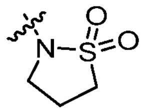

,

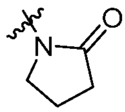

,

$NR^BR^C$, or

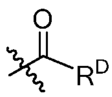

;

and

Q is

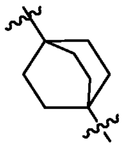, 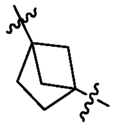, 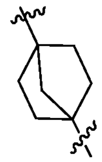 or 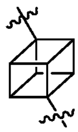;

scheme 7:
wherein,

W is O;
$R^1$ is 2-thiazolyl;
$R^2$, $R^3$, and $R^4$ are independently H, fluorine, or methyl; and $R^3$ and $R^4$ are preferably not fluorine at the same time;
$R^6$ is H;
$R^7$ is H;
$R^8$ is H;
$R^9$ is H;
Li is single bond;
$R^{10}$ is CN, $C_1$-$C_6$ alkyl substituted by one $R^A$, $C_1$-$C_6$ alkyl,

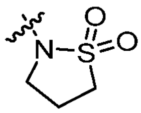, 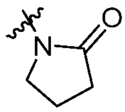,

$NR^BR^C$, or

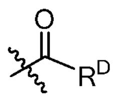;

$R^D$ is $C_4$-$C_6$ heterocyclyl, $NH_2$- or -$NHC_1$-$C_6$alkyl; and
Q is

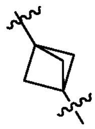;

scheme 8:
wherein,

W is O;
$R^1$ is 2-thiazolyl;
$R^2$, $R^3$, and $R^4$ are independently H, fluorine, or methyl; and $R^3$ and $R^4$ are preferably not fluorine at the same time;
$R^6$ is H;
$R^7$ is H;
$R^8$ is H;
$R^9$ is H;
Li is single bond;
$R^{10}$ is COOH or $C_1$-$C_6$ alkyl substituted by one $R^A$, wherein, $R^A$ is COOH; and
Q is

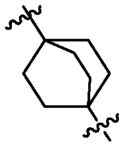, 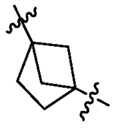, 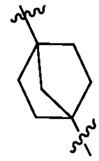 or 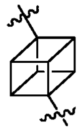;

scheme 9:
wherein,

W is O;
$R^1$ is 2-thiazolyl;
$R^2$, $R^3$, and $R^4$ are independently H, fluorine, or methyl;
$R^6$ is H;
$R^7$ is H;
$R^8$ is H;
$R^9$ is H;
Li is single bond;
$R^{10}$ is CN, $C_1$-$C_6$ alkyl substituted by one $R^A$, $C_1$-$C_6$ alkyl,

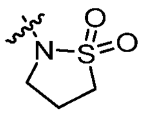, 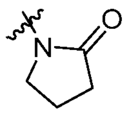,

$NR^BR^C$, or

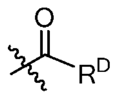;

$R^A$ is hydroxyl, halogen, $C_1$-$C_6$ alkoxy,

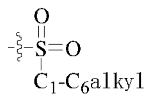, 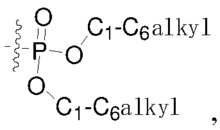,

CN,

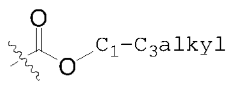,

$C_4$-$C_6$ heterocyclyl substituted by a carbonyl,

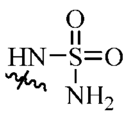, 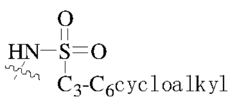,

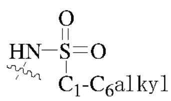 or 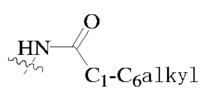;

$R^B$ and $R^C$ are independently H,

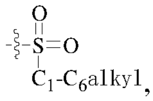, 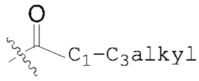,

$C_1$-$C_6$ alkyl or

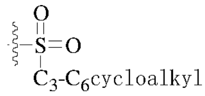;

$R^D$ is $C_4$-$C_6$ heterocyclyl, $NH_2$- or -$NHC_1$-$C_6$alkyl; and
Q is

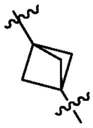;

scheme 10:
wherein,

W is O;
$R^1$ is 2-thiazolyl;
$R^2$, $R^3$, and $R^4$ are independently H, fluorine, or methyl;
$R^6$ is H;
$R^7$ is H;
$R^8$ is H;
$R^9$ is H;
Li is single bond;
$R^{10}$ is COOH; and
Q is

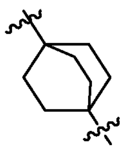.

**11.** The dihydropyrimidine compound of formula I' according to claim 1, or a pharmaceutically acceptable salt thereof, wherein,

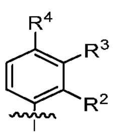 is 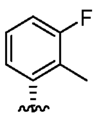, 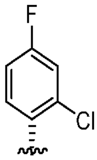, 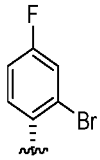, 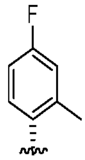, 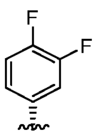, 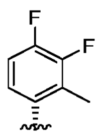 or 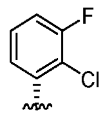;

and/or,

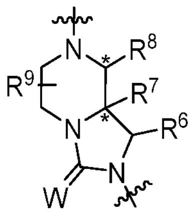 is 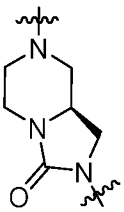, 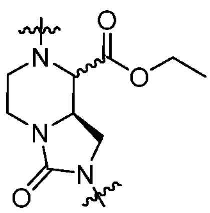, 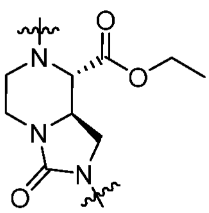, 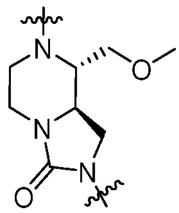

or

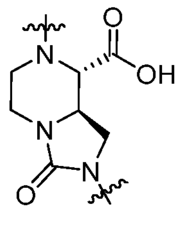;

and/or, Q is

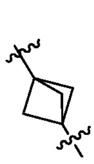, 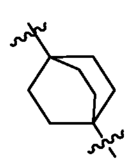, 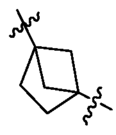, 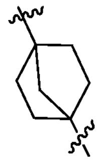 or 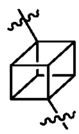;

and/or, $R^{10}$ is COOH,

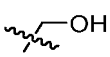, 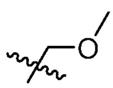, 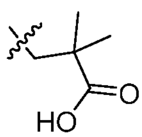, 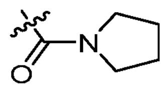, 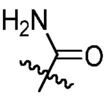,

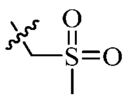, 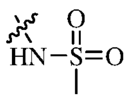, 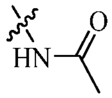, 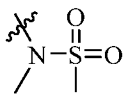, 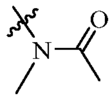, 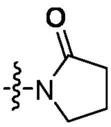, 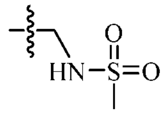, 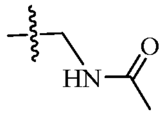,

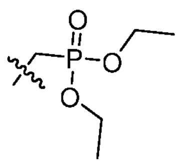,

CN,

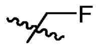, 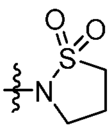, 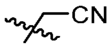, 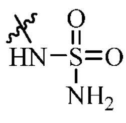, 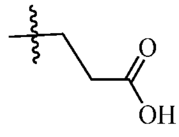, 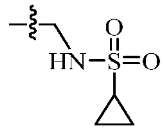,

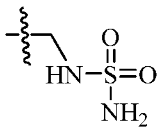, 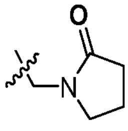, 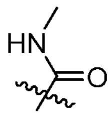, 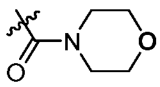, 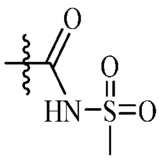, 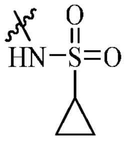, 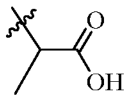,

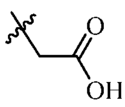, 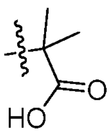, 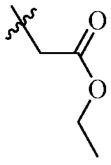, 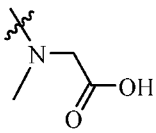, 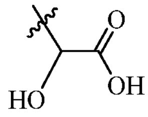 or 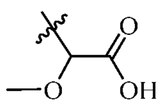.

**12.** The dihydropyrimidine compound as shown in formula I' according to claim 1, or a pharmaceutically acceptable salt thereof, wherein, the dihydropyrimidine compound as shown in formula I' is selected from the following compounds:

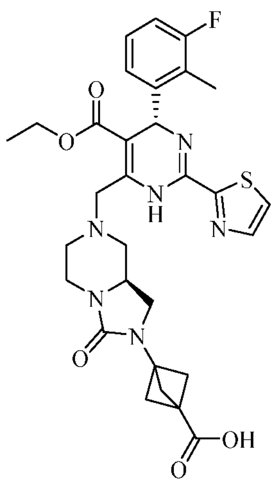, 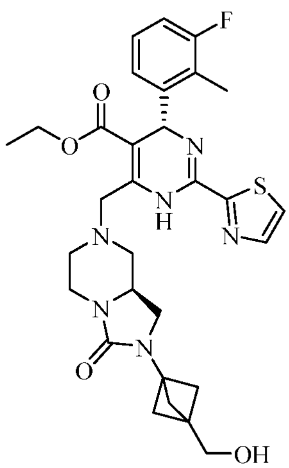, 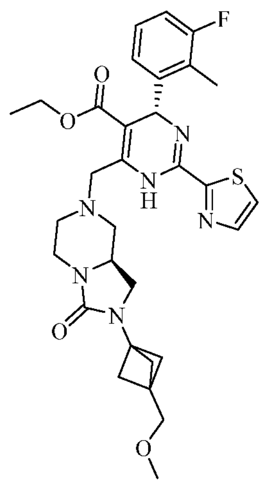, 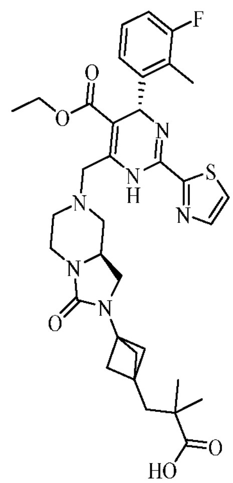,

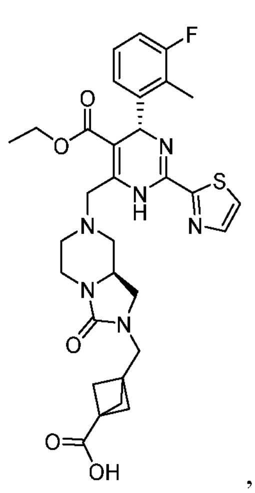, 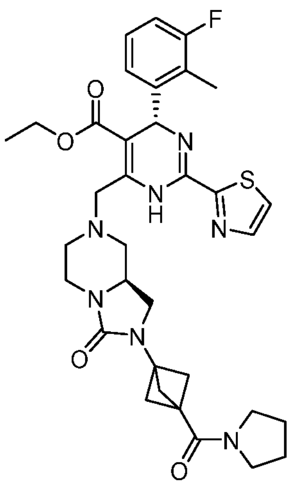, 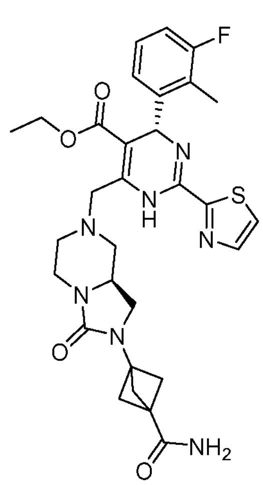, 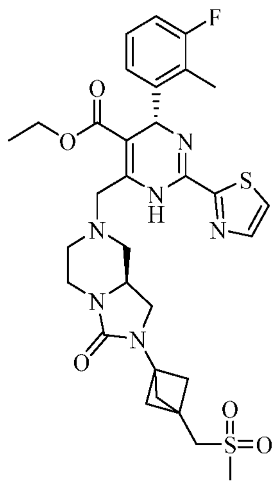,

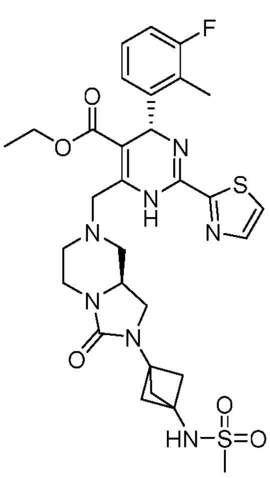, 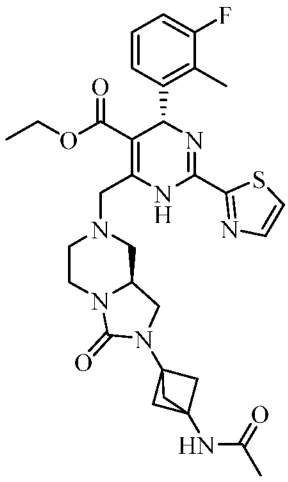, 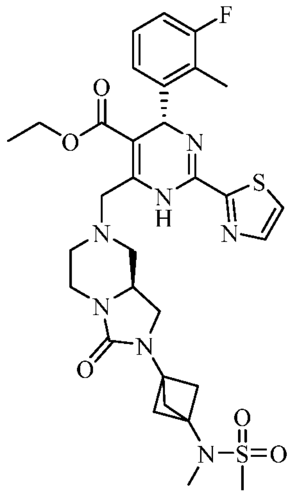, 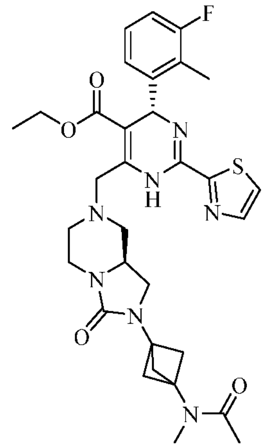,

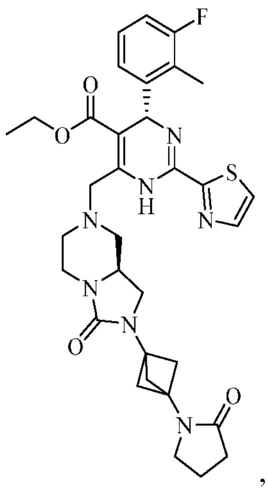, 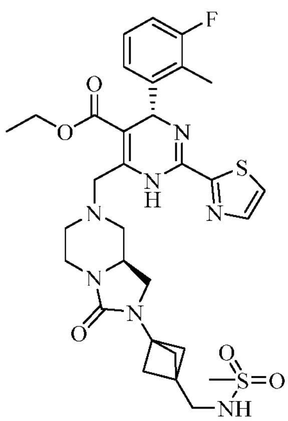, 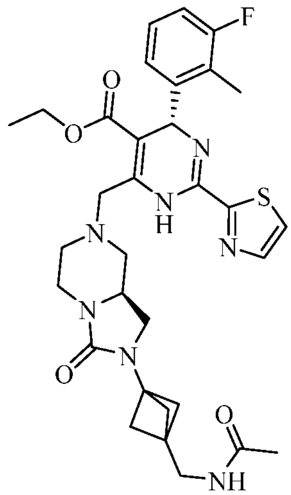, 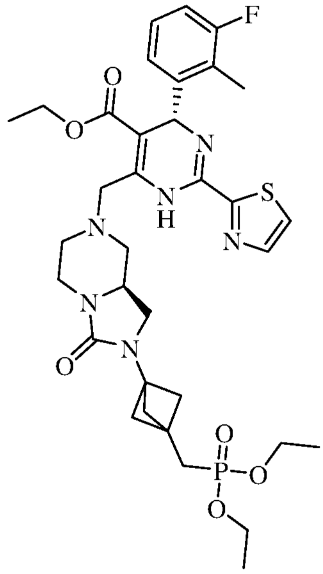,

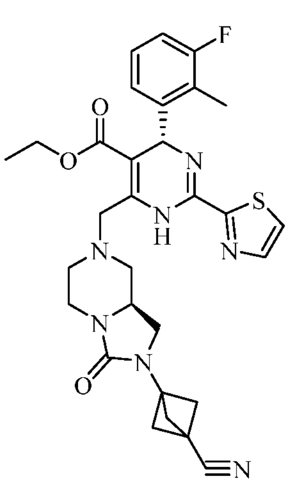, 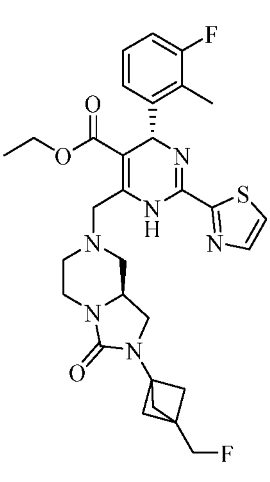, 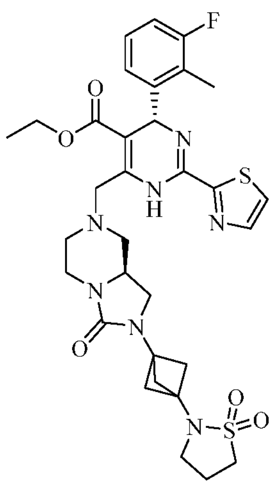, 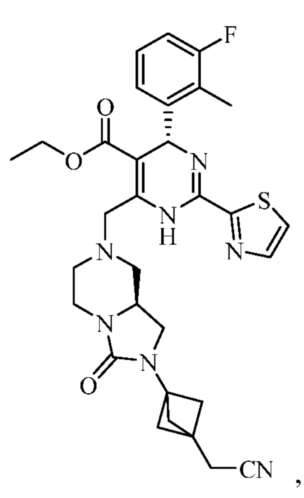,

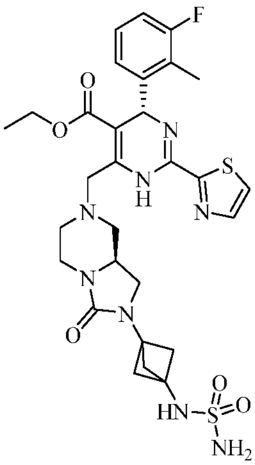,
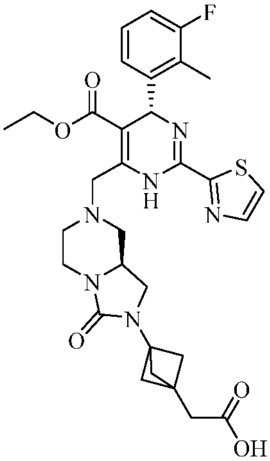,
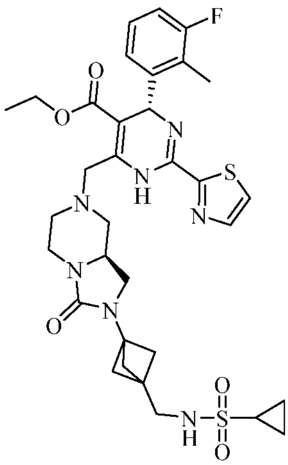,
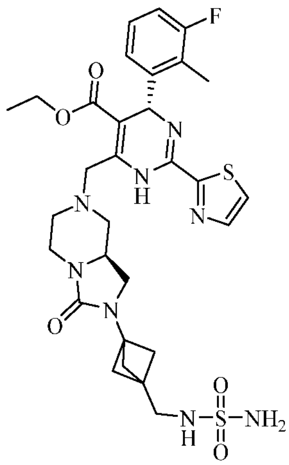,

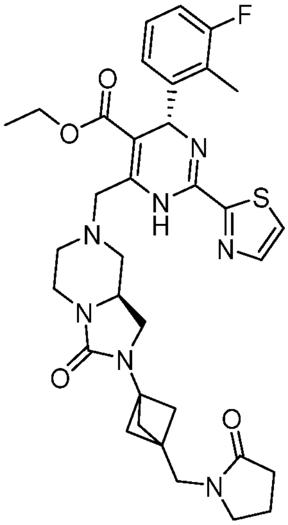,
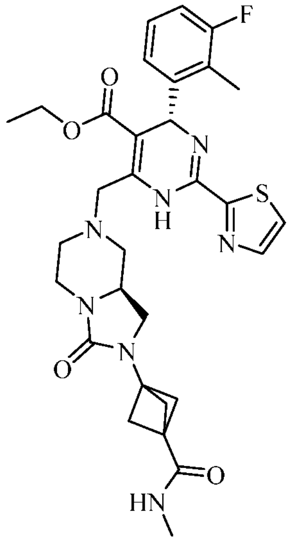,
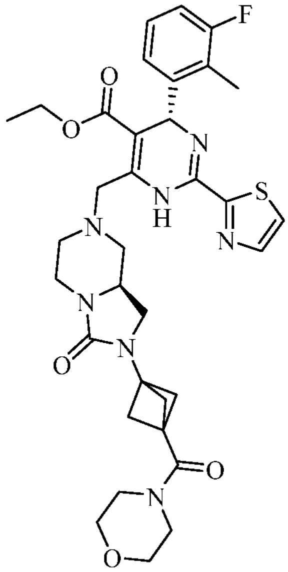,
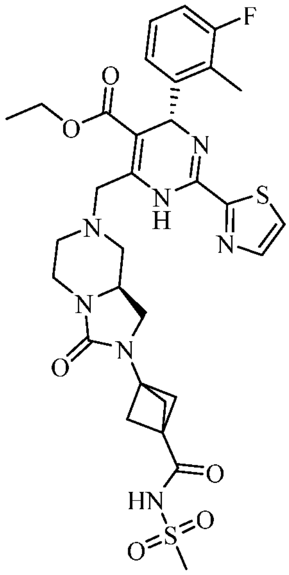,

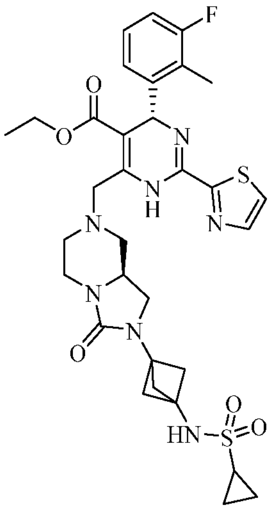,
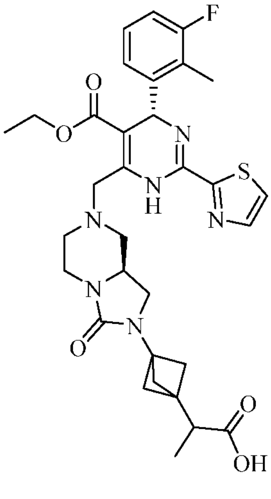,
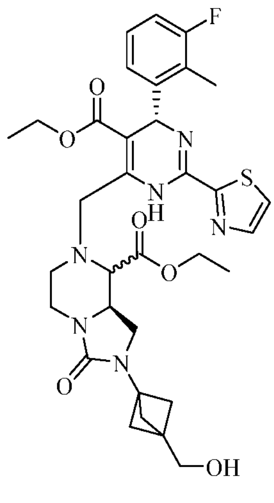,
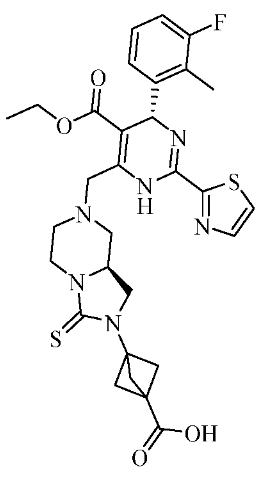,

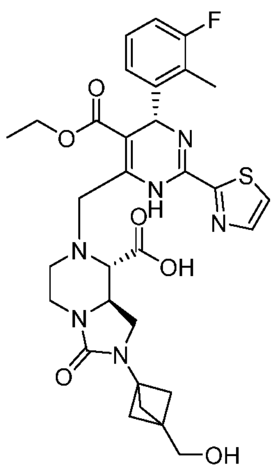 ,

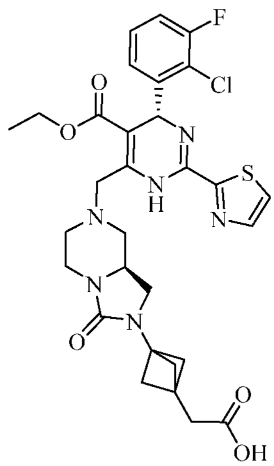 ,

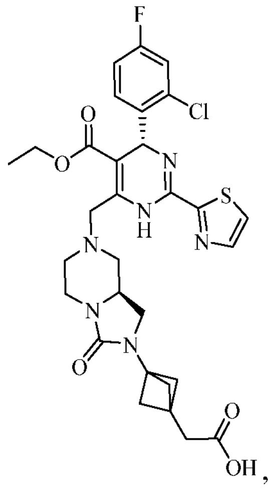 ,

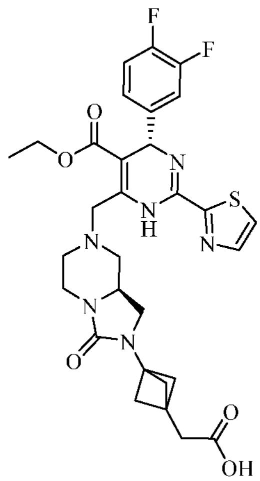 ,

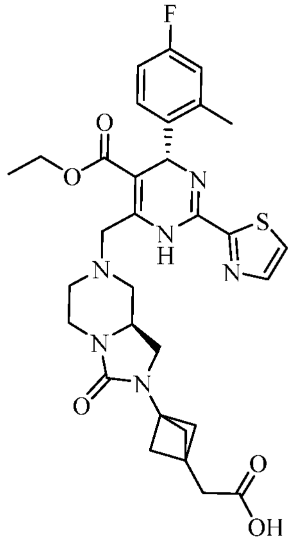 ,

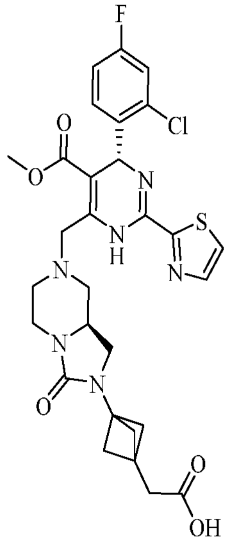 ,

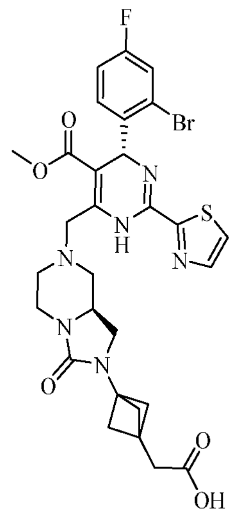 ,

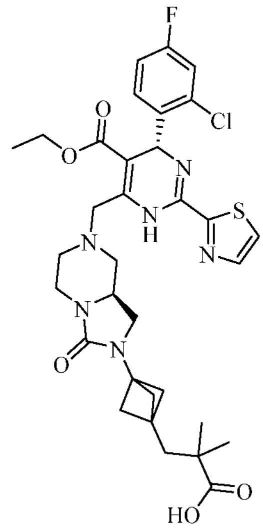 ,

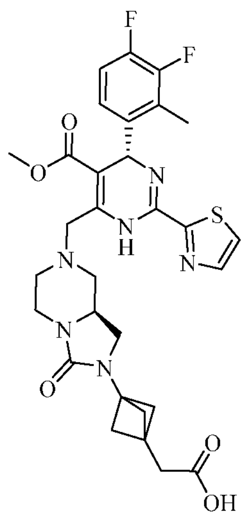 ,

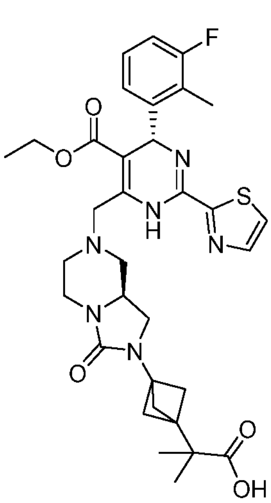 ,

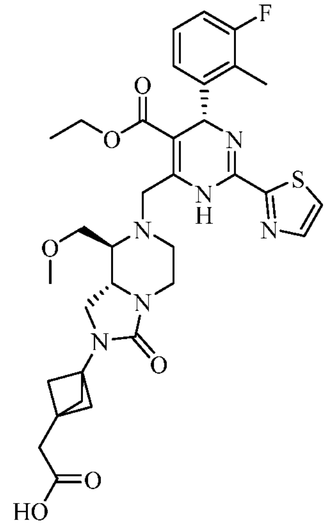 ,

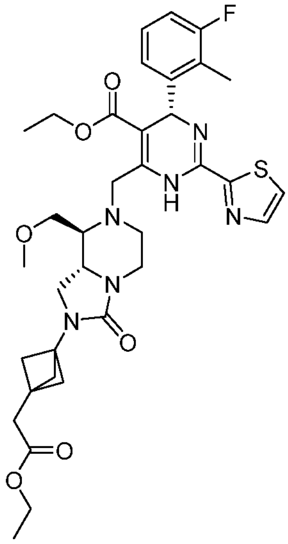 ,

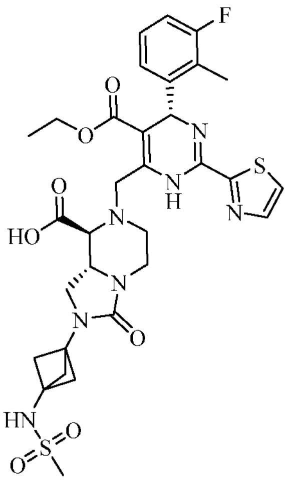

, 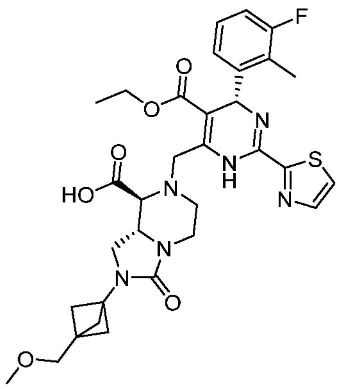

, 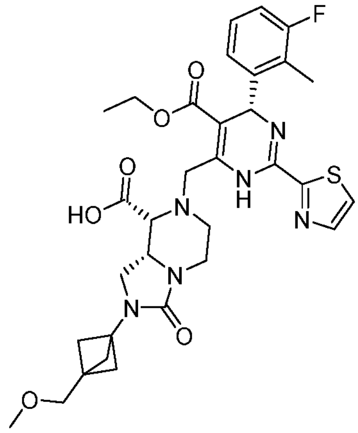

,

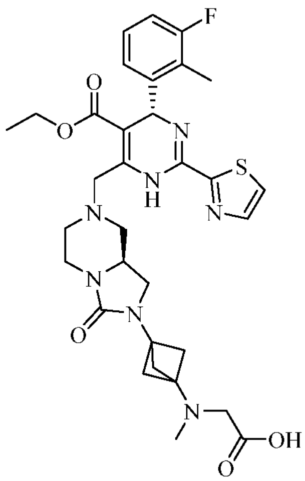

, 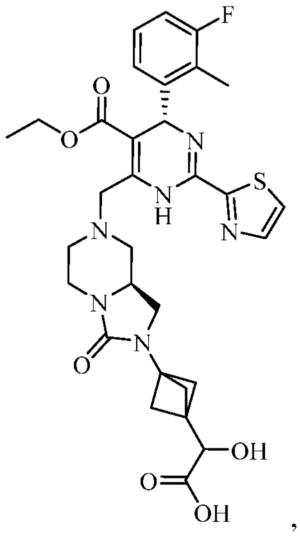

, 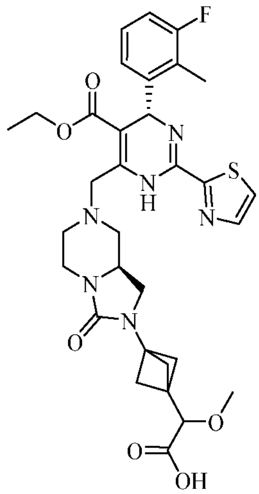

, 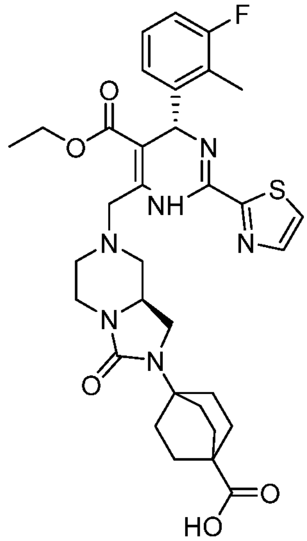

,

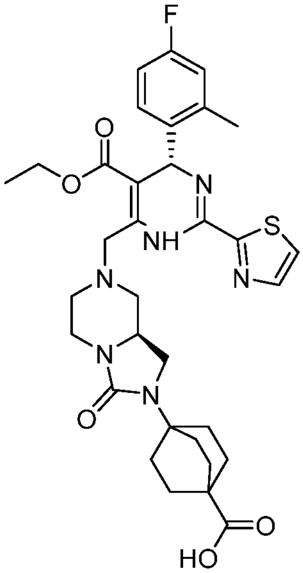

, 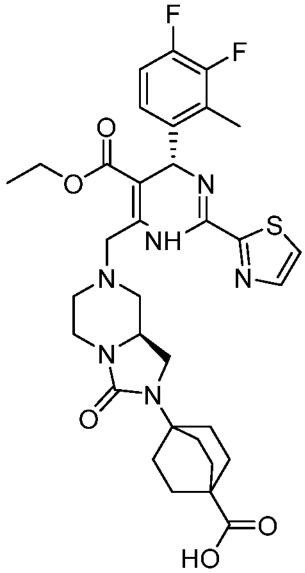

, 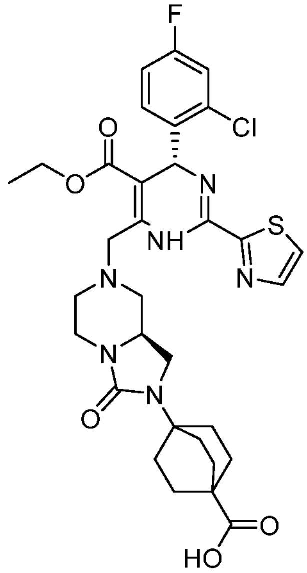

,

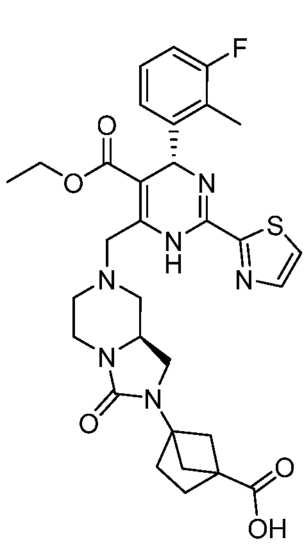 , 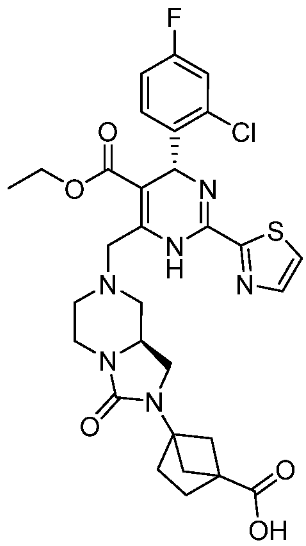 , 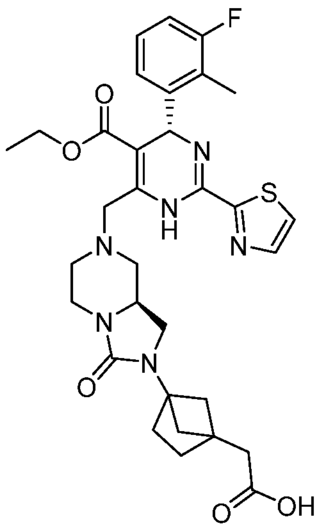 ,

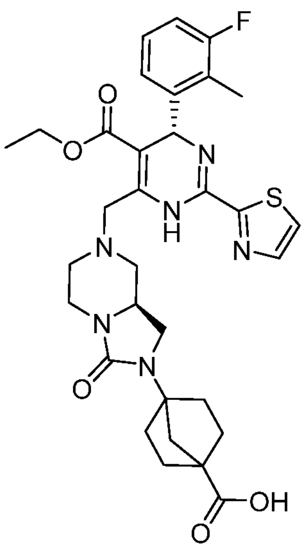 , 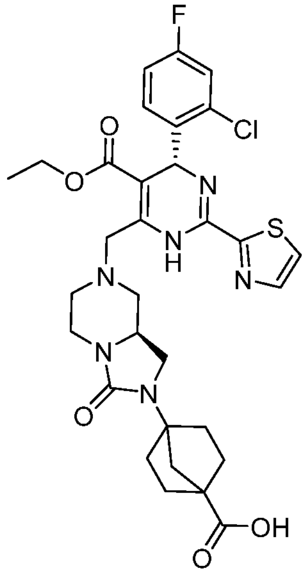 , 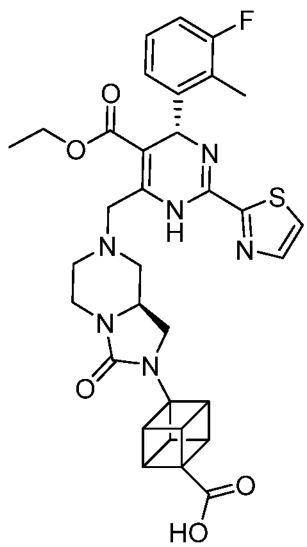 ,

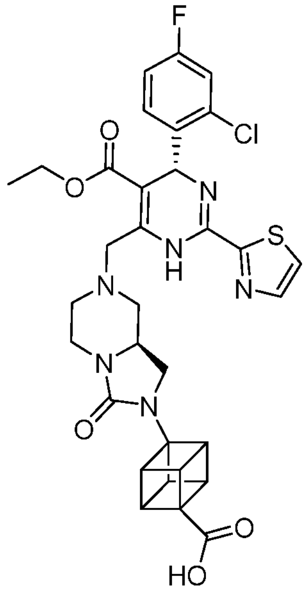
,
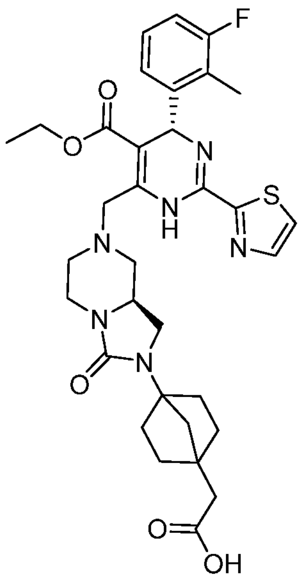
,
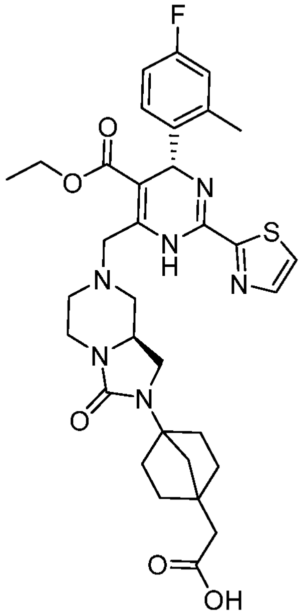

or

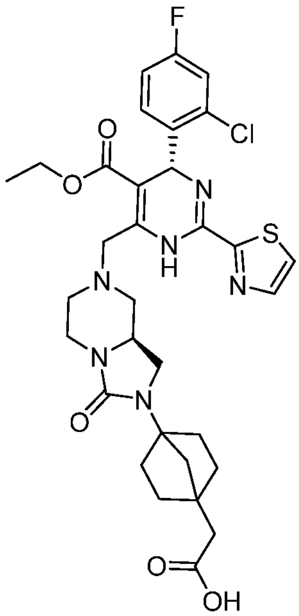
.

**13.** A preparation method for a compound as shown in formula I' according to at least one of claims 1-12, comprising the following steps:

in a solvent, in the presence of base, the compound of formula II and the compound of formula III' undergo the following substitution reaction to obtain the compound of formula I';

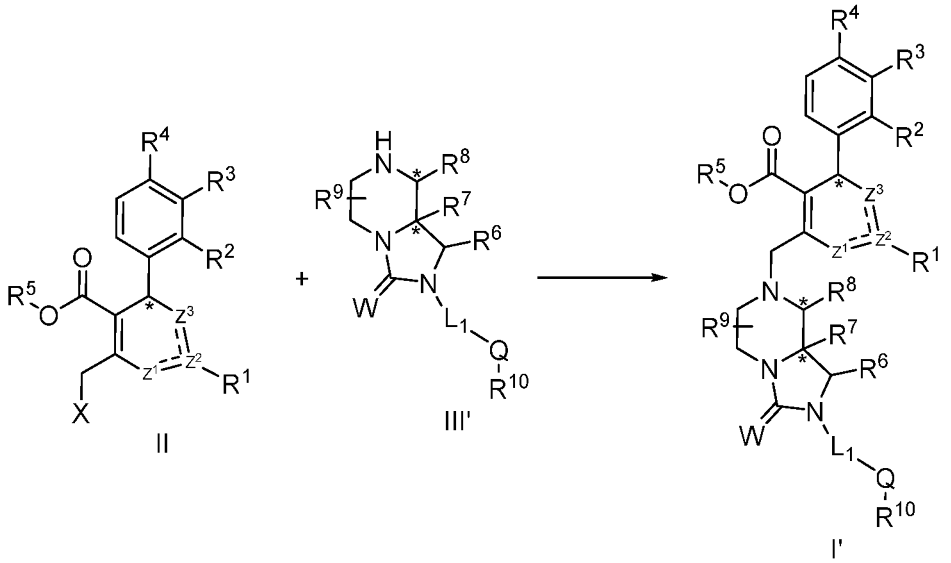

II III' I'

X is halogen,

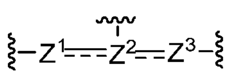,

$R^1$, $R^2$, $R^3$, $R^4$, $R^5$, $R^6$, $R^7$, $R^8$, $R^9$, $R^{10}$, $L_1$, Q, and W are as defined in any one of claims 1-12.

**14.** A pharmaceutical composition comprising a dihydropyrimidine compound as shown in formula I' according to at least one of claims 1-12, or a pharmaceutically acceptable salt thereof and pharmaceutical excipients, the dosage of the dihydropyrimidine compound as shown in formula I' or a pharmaceutically acceptable salt thereof can be therapeutically effective amount.

**15.** Use of the dihydropyrimidine compound according to at least one of claims 1-12, or pharmaceutically acceptable salts, or the pharmaceutical composition according to claim 14 in the preparation of an HBV inhibitor.

**16.** Use of the dihydropyrimidine compound according to at least one of claims 1-12, or pharmaceutically acceptable salts, or the pharmaceutical composition according to claim 14 in the preparation a medication for prevention and/or treatment of infections associated with HBV.

**INTERNATIONAL SEARCH REPORT**

International application No.
**PCT/CN2022/075494**

**A. CLASSIFICATION OF SUBJECT MATTER**

C07D 487/04(2006.01)i； A61K 31/4985(2006.01)i； A61P 31/12(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNPAT, CNKI, EPODOC, DWPI, STN(structure search), 二氢嘧啶, 乙型肝炎, dihydropyrimidine, HBV

**C. DOCUMENTS CONSIDERED TO BE RELEVANT**

| Category* | Citation of document, with indication, where appropriate, of the relevant passages | Relevant to claim No. |
|---|---|---|
| X | WO 2020125729 A1 (JANSSEN PHARMACEUTICA NV et al.) 25 June 2020 (2020-06-25)<br>claims 1, 14-19, description, page 35, general solution | 1-16 |
| X | WO 2020125730 A1 (JANSSEN PHARMACEUTICA NV et al.) 25 June 2020 (2020-06-25)<br>claims 1, 13-19 | 1-16 |
| X | CN 109678859 A (GUANGDONG HEC PHARMACEUTICAL CO., LTD.) 26 April 2019 (2019-04-26)<br>claims 1, 12-19, description paragraphs [0361]-[0362] | 1-16 |
| X | CN 106061978 A (F. HOFFMANN-LA ROCHE AG) 26 October 2016 (2016-10-26)<br>claims 1, 21-27, embodiments 35, 39 and 49 | 1-16 |
| A | WO 2020135439 A1 (GUANGDONG HEC PHARMACEUTICAL CO., LTD.) 02 July 2020 (2020-07-02)<br>claims 1-10 | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 March 2022** | **29 April 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/075494**

| Patent document cited in search report | Publication date (day/month/year) | Patent family member(s) | Publication date (day/month/year) |
|---|---|---|---|
| WO 2020125729 A1 | 25 June 2020 | KR 20210106421 A | 30 August 2021 |
| | | EP 3898628 A1 | 27 October 2021 |
| | | CA 3124317 A1 | 25 June 2020 |
| | | AU 2019409191 A1 | 27 May 2021 |
| | | JP 2022511819 A | 01 February 2022 |
| | | US 2022048919 A1 | 17 February 2022 |
| | | CN 113227090 A | 06 August 2021 |
| WO 2020125730 A1 | 25 June 2020 | IL 284116 D0 | 31 August 2021 |
| | | KR 20210106464 A | 30 August 2021 |
| | | SG 11202105338V A | 29 July 2021 |
| | | EP 3898632 A1 | 27 October 2021 |
| | | AU 2019410640 A1 | 27 May 2021 |
| | | TW 202035412 A | 01 October 2020 |
| | | CA 3118764 A1 | 25 June 2020 |
| | | CN 113195499 A | 30 July 2021 |
| | | JP 2022513297 A | 07 February 2022 |
| CN 109678859 A | 26 April 2019 | WO 2019076310 A1 | 25 April 2019 |
| | | EP 3697792 A1 | 26 August 2020 |
| | | SG 11202003320 S A | 28 May 2020 |
| | | AU 2018351400 A1 | 23 April 2020 |
| | | US 2020283445 A1 | 10 September 2020 |
| | | KR 20200072512 A | 22 June 2020 |
| | | JP 2020537677 A | 24 December 2020 |
| | | CA 3079557 A1 | 25 April 2019 |
| CN 106061978 A | 26 October 2016 | US 2016083383 A1 | 24 March 2016 |
| | | TW 201726682 A | 01 August 2017 |
| | | EP 3114128 A1 | 11 January 2017 |
| | | PL 3114128 T3 | 28 June 2019 |
| | | DK 3114128 T3 | 25 March 2019 |
| | | HU E041734 T2 | 28 May 2019 |
| | | KR 20160105978 A | 08 September 2016 |
| | | RS 58384 B1 | 30 April 2019 |
| | | MX 2016011515 A | 20 December 2016 |
| | | AU 2015226206 A1 | 07 July 2016 |
| | | KR 20180130016 A | 05 December 2018 |
| | | US 2020062753 A1 | 27 February 2020 |
| | | IL 246449 D0 | 31 August 2016 |
| | | CA 2935811 A1 | 11 September 2015 |
| | | PE 20161338 A1 | 12 December 2016 |
| | | PT 3114128 T | 27 February 2019 |
| | | EA 201691726 A1 | 30 December 2016 |
| | | CN 107513073 A | 26 December 2017 |
| | | LT 3114128 T | 25 March 2019 |
| | | HR P20190352 T1 | 05 April 2019 |
| | | UA 117518 C2 | 10 August 2018 |
| | | US 2015252057 A1 | 10 September 2015 |
| | | US 2018370969 A1 | 27 December 2018 |
| | | SG CN 11201607332 U A | 28 October 2016 |
| | | WO 2015132276 A1 | 11 September 2015 |

Form PCT/ISA/210 (patent family annex) (January 2015)

**INTERNATIONAL SEARCH REPORT**
**Information on patent family members**

International application No.
**PCT/CN2022/075494**

| Patent document cited in search report | Publication date (day/month/year) | Patent family member(s) | Publication date (day/month/year) |
|---|---|---|---|
| | | JP 2017507186 A | 16 March 2017 |
| | | TW 201546075 A | 16 December 2015 |
| | | MA 39721 A | 11 January 2017 |
| | | SI 3114128 T1 | 30 April 2019 |
| | | CL 2016002179 A1 | 17 February 2017 |
| | | HK 1245784 A1 | 31 August 2018 |
| | | CR 20160337 A | 20 September 2016 |
| | | ES 2714110 T3 | 27 May 2019 |
| | | PH 12016501631 A1 | 06 February 2017 |
| | | CN 109232613 A | 18 January 2019 |
| WO 2020135439 A1 | 02 July 2020 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2021101826790
- CN 202110773266X


### Non-patent literature cited in the description


- **MARCELLIN, PATRICK et al.** *The Lancet,* 2013, vol. 381 (9865), 468-475
- **TANG, LYDIA SY et al.** *Jama,* 2018, vol. 319 (17), 1802-1813
- **DIAB, AHMED et al.** *Antiviral research,* 2018, vol. 149, 211-220
- *Inactive Ingredient Guide,* 1996
- **ASH ; ASH.** Hand book of Pharmaceutical Additives. Synapse Information Resources, Inc, 2002
- Handbook of Chemistry and Physics. 1994
- **THOMAS SORRELL.** Organic Chemistry. University Science Books, 1999
- **MICHAEL B. SMITH ; JERRY MARCH.** March's Advanced Organic Chemistry. John Wiley&Sons, 2007